# FASCICULE DE BREVET EUROPEEN

(11) **EP 2 976 318 B1**
(45) Date de publication et mention de la délivrance du brevet: **09.12.2020**
(21) Numéro de dépôt: 14711823.6
(22) Date de dépôt: 24.02.2014
(51) Int. Cl.: C07C 21/18, C09K 5/04

(54) **COMPOSITION COMPRENANT HF ET 1,3,3,3-TETRAFLUOROPROPENE**
HF- UND 1,3,3,3-TETRAFLUORPROPEN-ENTHALTENDE ZUSAMMENSETZUNG
COMPOSITION COMPRISING HF AND 1,3,3,3-TETRAFLUOROPROPENE

(30) Priorité: 20.03.2013 FR 1352485
(43) Date de publication de la demande: 27.01.2016
(73) Titulaire: Arkema France, 92700 Colombes (FR)
(72) Inventeur: BONNET, Philippe, 69007 Lyon (FR); COLLIER, Bertrand, 69230 Saint-Genis-Laval (FR); DEUR-BERT, Dominique, 69390 Charly (FR); WENDLINGER, Laurent, 69510 Soucieu En Jarrest (FR)
(74) Mandataire: Arkema Patent
(86) Numéro de dépôt international: PCT/FR2014/050369
(87) Numéro de publication internationale: WO 2014/147313

(56) Documents cités:
- EP-A1- 2 527 313
- WO-A1-2008/054781
- WO-A1-2009/105521
- US-A1- 2012 056 122

## Description

La présente invention concerne des compositions azéotropiques ou quasi-azéotropiques comprenant du Z-1 ,3,3,3-tetrafluoropropene et du fluorure d'hydrogène. Ces compositions peuvent provenir des compositions intermédiaires de la production de 1,3,3,3-tetrafluoropropene et sont généralement utiles dans des procédés de recyclage de fluorure d'hydrogène.

La fabrication du 1,3,3,3-tetrafluoropropene s'accompagnant d'une multitude de sous-produits, ayant un point d'ébullition proche du HFO-1234ze, conduit à des étapes de purification assez complexes et coûteuses. La difficulté rencontrée au cours de la purification du HFO-1234ze implique généralement une perte conséquente en produit recherché. De plus, ces sous-produits peuvent former des compositions azéotropiques avec le 1,3,3,3-tetrafluoropropene, rendant la séparation par distillation simple, très difficile, voir impossible.

Les fluides à base de 1,3,3,3-tetrafluoropropene ont trouvé de nombreuses applications dans des domaines industriels variés, notamment en tant que fluide de transfert de chaleur, propulseurs, agents moussants, agents gonflants, diélectriques gazeux, milieu de polymérisation ou monomère, fluides supports, agents pour abrasifs, agents de séchage et fluides pour unité de production d'énergie.

Une importance particulière est donnée aux fluides ayant un faible impact sur l'environnement.

Le document US 2012/056122 décrit des compositions azéotropiques comprenant du fluorure d'hydrogène (HF), du trans-1,3,3,3-tetrafluoropropène (HFO-1234ze(E)) et du cis-1,3,3,3-tetrafluoropropene (HFO-1234ze(Z)); ou du trans-1,3,3,3-tetrafluoropropène (HFO-1234ze(E)), 1,1,1,3,3-pentafluoropropane (HFC-245fa) et du fluorure d'hydrogène (HF) ; ou du cis-1,3,3,3-tetrafluoropropene (HFO-1234ze(Z)), 1,1,1,3,3-pentafluoropropane (HFC-245fa) et du fluorure d'hydrogène (HF). Le document EP2527313 A1 décrit une composition binaire azéotropique contenant du fluorure d'hydrogène et du trans-1,3,3,3-tetrafluoropropène WO2009/105521 A1 décrit une composition azéotropique comprenant du fluorure d'hydrogène, du cis-1,3,3,3-tétrafluoropropène et du 2,3,3,3-tétrafluoropropène.

La présente invention a pour objet une composition azéotropique ou quasi-azéotropique comprenant du fluorure d'hydrogène, du Z-1,3,3,3-tetrafluoropropene, et un ou plusieurs composé(s) (hydro)halogénocarboné(s) choisi(s) parmi le 1,1,1,2,2-pentafluoropropane, le 2,3,3,3-tetrafluoropropene, le 3,3,3-trifluoropropene, le 3,3,3-trifluoro-2-chloropropene, le E-3,3,3-trifluoro-1-chloropropene, le trifluoropropyne, le 1,1,1,3,3-pentafluoropropène, le 1,1,1,2,3-pentafluoropropène et le 2-chloro,1,1,1,2-tetrafluoropropane ; caractérisé en ce que ladite composition comprend de 1 à 95 % en poids de fluorure d'hydrogène et de 99 à 5 % en poids de la somme des composés organiques, et le point d'ébullition de ladite composition est compris entre -20°C et 80°C, à une pression comprise entre 0,1 et 44 bars absolue.

Selon un mode de réalisation de l'invention, la composition est hétéro-azéotropique ou quasi-hétéro-azéotropique.

Un mélange hétéro-azéotropique ou quasi-hétéro-azéotropique est un mélange azéotropique ou quasi-azéotropique dont le liquide condensé forme deux solutions non miscibles qui peuvent être facilement séparées, par exemple par décantation. Cette propriété est un avantage considérable pour la récupération d'HF.

L'expression « quasi-azéotropique » ou « quasi-hétéro-azéotropique » a un sens large et est destinée à inclure les compositions qui sont strictement azéotropique ou strictement hétéro-azéotropique et celles qui se comportent comme un mélange azéotropique ou hétéro-azéotropique.

Un mélange est azéotropique lorsque que la pression au point de rosée est égale à celle au point de formation de bulle, ce qui signifie que la composition de vapeur est égale à celle du liquide condensée.

Un mélange est considéré comme quasi-azéotropique lorsque que la pression au point de rosée est substantiellement égale à celle au point de formation de bulle, ce qui signifie que la composition de vapeur est substantiellement égale à celle du liquide condensée.

Une autre manière de caractériser un mélange comme quasi-azéotropique lorsque la différence de pression entre la pression au point de rosée et la pression au point de formation de bulles est faible, préférentiellement inférieure ou égale à 5 %, sur la base de la pression au point de formation de bulles.

Les compositions selon l'invention concernent notamment les composés suivants dont les acronymes représentent :
- HF : fluorure d'hydrogène
- HCC-40: chlorométhane, ou CH₃Cl
- HCFC-115: chloropentafluoroéthane, ou C₂F₅Cl
- HCFC-124: chlorotétrafluoroéthane, ou C₂HF₄Cl
- HFC-125: pentafluoroéthane, ou C₂HF₅
- HCFC-133a: 1-chloro-2,2,2-trifluoroéthane, ou C₂H₂F₃Cl
- HFC-134a: 1,1,1,2-tétrafluoroéthane, ou C₂H₂F₄
- HCFC-142b: 1-chloro-1,1-difluoroéthane, ou C₂H₃F₂Cl
- HFC-143a : 1,1,1-trifluoroéthane, ou C₂H₃F₃
- HFC-152a : 1,1-difluoroéthane, ou C₂H₄F₂
- HFO-1132 : 1,2-difluoroéthylène, ou C₂H₂F₂
- HFO-1141 : fluoroéthylène, ou C₂H₃F
- HFO-1234yf : 2,3,3,3-tetrafluoropropene ou CH₂=CF-CF₃
- HFC-245cb : 1,1,1,2,2-pentafluoropropane ou CF₃-CF₂-CH₃
- HFO-1234zeE : E-1,3,3,3-tetrafluoropropene ou E-CF₃-CH=CHF
- HFO-1234zeZ : Z-1,3,3,3-tetrafluoropropene ou Z-CF₃-CH=CHF
- HFO-1243zf : 3,3,3-trifluoropropene ou CF₃-CH=CH₂
- HCFO-1233xf : 3,3,3-trifluoro-2-chloropropene ou CF₃-CCl=CH₂
- HCFO-1233zdE : E-3,3,3-trifluoro-1-chloropropene ou E-CF₃-CH=CHCl
- HCFO-1233zdZ : Z-3,3,3-trifluoro-1-chloropropene ou Z-CF₃-CH=CHCl
- HFO-1225yeZ : Z-1,1,1,2,3-pentafluoropropene ou Z-CHF=CF-CF₃
- HFO-1225yeE : E-1,1,1,2,3-pentafluoropropene ou E-CHF=CF-CF₃
- HFO-1225zc : 1,1,3,3,3-pentafluoropropene ou CF₂=CH-CF₃
- HFO-1225yc: 1,1,2,3,3-pentafluoropropene ou CF₂=CF-CF₂
- HCFC-1214 : dichlorotetrafluoropropène, ou C₃F₄Cl₂
- HCFO-1215 : chloropentafluoropropène, ou C₃F₅Cl
- HFO-1216 : hexafluoropropène, ou C₃F₆
- HCFO-1223 : dichlorotrifluoropropène, ou C₃HF₃Cl₂
- HCFO-1224 : chlorotetrafluoropropène, ou C₃HF₄Cl
- HCFO-1232 : dichlorodifluoropropène, ou C₃H₂F₂Cl₂
- HCFO-1233xc: 1,1,3-trifluoro-2-chloropropene ou CH₂F-CCl=CF₂
- HCFO-1233xe : 1,3,3-trifluoro-2-chloropropene ou CHF₂-CCl=CHF
- HCFO-1233yb : 1,2,3-trifluoro-1-chloropropene ou CH₂F-CF=CFCl
- HCFO-1233yc : 1,1,2-trifluoro-3-chloropropene ou CH₂Cl-CF=CF₂
- HCFO-1233yd : 2,3,3-trifluoro-1-chloropropene ou CHF₂-CF=CHCl
- HCFO-1233ye : 1,2,3-trifluoro-3-chloropropene ou CHClF-CF=CHF
- HCFO-1233yf : 2,3,3-trifluoro-3-chloropropene ou CClF₂-CF=CH₂
- HCFO-1233zb : 1,3,3-trifluoro-1-chloropropene ou CHF₂-CH=CFCl
- HCFO-1233zc: 1,1,3-trifluoro-3-chloropropene ou CHClF-CH=CF₂
- HCFO-1233ze : 1,3,3-trifluoro-3-chloropropene ou CClF₂-CH=CHF
- HFO-1234yc: 1,1,2,3-tetrafluoropropene ou CF₂=CF-CH₂F
- HFO-1234ye : 1,2,3,3-tetrafluoropropene ou CHF=CF-CHF₂
- HFO-1234zc: 1,1,3,3-tetrafluoropropene ou CF₂=CH-CHF₂
- HCFO-1242 : chlorodifluoropropène, ou C₃H₃F₂Cl
- HFO-1243yc : 1,1,2-trifluoropropene ou CH₃-CF=CF₂
- HFO-1243ye : 1,2,3-trifluoropropene ou CH₂F-CF=CHF
- HFO-1243yf : 2,3,3-trifluoropropene ou CHF₂-CF=CH₂
- HFO-1243zc : 1,1,3-trifluoropropene ou CH₂F-CH=CF₂
- HFO-1243ze : 1,3,3-trifluoropropene ou CHF₂-CH=CHF
- HCFO-1251 : chlorofluoropropène, ou C₃H₄FCl
- HFO-1252 : difluoropropène, ou C₃H₄F₂
- HFO-216 : hexafluoropropene, ou C₃F₆Cl₂
- HCFO-217 : chloroheptafluoropropane, ou C₃F₇Cl
- HFC-218 : octafluoropropane, ou C₃F₈
- HCFC-225 : dichloropentafluoropropane, ou C₃HF₅Cl₂
- HCFC-226 : chlorohexafluoropropane, ou C₃HF₆Cl
- HFC-227: heptafluoropropane, ou C₃HF₇
- HCFC-234 : dichlorotétrafluoropropane, ou C₃H₂F₄Cl₂
- HCFC-235 : chloropentafluoropropane, ou C₃H₂F₅Cl
- HFC-236 : hexafluoropropane, ou C₃H₂F₆
- HCFC-243 : dichlorotrifluoropropane, ou C₃H₃F₃Cl₂
- HCFC-244 : chlorotétrafluoropropane, ou C₃H₃F₄Cl
- HCFC-244bb : 2-chloro, 1,1,1,2-tetrafluoropropane ou CF₃-CFCl-CH₃
- HFC-245fa : 1,1,1,3,3-pentafluoropropane ou CF₃-CH₂-CHF₂
- HFC-245ea : 1,1,2,3,3-pentafluoropropane ou CHF₂-CHF-CHF₂
- HFC-245eb : 1,1,1,2,3-pentafluoropropane ou CF₃-CHF-CH₂F
- HFC-245ca : 1,1,2,2,3-pentafluoropropane ou CHF₂-CF₂-CH₂F
- HCFC-253 : chlorotrifluoropropane, ou C₃H₄F₃Cl
- HFC-254 : tétrafluoropropane, ou C₃H₄F₄
- HCFC-262 : Chlorodifluoropropane, ou C₃H₅F₂Cl
- HFC-263 : trifluoropropane, ou C₃H₅F₃
- Trifluoropropyne : CF₃-C≡CH

La composition selon l'invention peut éventuellement être un mélange d'un ou de plusieurs azéotrope et/ou hétéro-azéotrope de systèmes ternaires, quaternaires, pentanaires, système à six composés, système à sept composés, système à huit composés ou supérieur.

Le composé 1,3,3,3-tetrafluoropropene comprend soit le composé Z-1,3,3,3-tetrafluoropropene soit le mélange des composés E-1,3,3,3-tetrafluoropropene et Z-1,3,3,3-tetrafluoropropene.

Selon un mode de mise en œuvre de l'invention le 1,3,3,3-tetrafluoropropene est le Z-1,3,3,3-tetrafluoropropene.

Ladite composition peut également comprendre un ou des composé(s) à 1 et/ou 2 atome(s) de carbone choisi(s) parmi le chlorométhane, le chloropentafluoroéthane, le 1-chloro-1,2,2,2-tétrafluoroéthane, le 1-chloro-1,1,2,2-tétrafluoroéthane, le pentafluoroéthane, le 1-chloro-1,2,2-trifluoroéthane, le 1-chloro-2,2,2-trifluoroéthane, le 1,1,2,2-tétrafluoroéthane, le 1,1,1,2-tétrafluoroéthane, le 1-chloro-1,2-difluoroéthane, le 1-chloro-1,1-difluoroéthane, le 1,1,2-trifluoroéthane, le 1,1,1-trifluoroéthane, le 1,1,2-trifluoroéthane, le 1,1-difluoroéthane, le 1,2-difluoroéthylène et le fluoroéthylène.

Ladite composition peut également comprendre un ou des composé(s) ayant 3 atomes de carbone choisi(s) parmi le 1,2-dichloro-1,1,2,3,3,3-hexafluoropropane, le 1,3-dichloro-1,1,2,2,3,3-hexafluoropropane, le 1,1-dichloro-1,2,2,3,3,3-hexafluoropropane, le 2,2-dichloro-1,1,1,3,3,3-hexafluoropropane, le 1-chloro-1,1,2,2,3,3,3-heptafluoropropane, le 2-chloro-1,1,1,2,3,3,3-heptafluoropropane, octafluoropropane, le dichloropentafluoropropane, le 2,2-dichloro-1,1,1,3,3-pentafluoropropane, le 2,3-dichloro-1,1,1,2,3-pentafluoropropane, le 1,2-dichloro-1,1,2,3,3-pentafluoropropane, le 3,3-dichloro-1,1,1,2,2-pentafluoropropane, le 1,3-dichloro-1,1,2,2,3-pentafluoropropane, le 1,1-dichloro-1,2,2,3,3-pentafluoropropane, le 1,2-dichloro-1,1,3,3,3-pentafluoropropane, le 1,3-dichloro-1,1,2,3,3-pentafluoropropane, le 1,1-dichloro-1,2,3,3,3-pentafluoropropane, chlorohexafluoropropane, le 2-chloro-1,1,1,2,3,3-hexafluoropropane, le 3-chloro-1,1,1,2,2,3-hexafluoropropane, le 1-chloro-1,1,2,2,3,3-hexafluoropropane, le 2-chloro-1,1,1,3,3,3-hexafluoropropane, le 1-chloro-1,1,2,3,3,3-hexafluoropropane, 1,1,2,2,3,3,3-heptafluoropropane, le 1,1,1,2,3,3,3-Heptafluoropropane, dichlorotétrafluoropropane, le 2,2-dichloro-1,1,3,3-tétrafluoropropane, le 2,2-dichloro-1,1,1,3-tétrafluoropropane, le 1,2-dichloro-1,2,3,3-tétrafluoropropane, le 2,3-dichloro-1,1,1,2-tétrafluoropropane, le 1,2-dichloro-1,1,2,3-tétrafluoropropane, le 1,3-dichloro-1,2,2,3-tétrafluoropropane, le 1,1-dichloro-2,2,3,3-tétrafluoropropane, le 1,3-dichloro-1,1,2,2-tétrafluoropropane, le 1,1-dichloro-1,2,2,3-tétrafluoropropane, le 2,3-dichloro-1,1,1,3-tétrafluoropropane, le 1,3-dichloro-1,1,3,3-tétrafluoropropane, le 1,3-dichloro-1,1,3,3-tétrafluoropropane, le 1,1-dichloro-1,3,3,3-tétrafluoropropane, le 1,1-dichloro-2,3,3,3-tétrafluoropropane, le 1,3-dichloro-1,1,2,3-tétrafluoropropane, le 1,1-dichloro-1,2,3,3-tétrafluoropropane le chloropentafluoropropane, le 1-chloro-1,2,2,3,3-pentafluoropropane, le 3-chloro-1,1,1,2,3-pentafluoropropane, le 1-chloro-1,1,2,2,3-pentafluoropropane, le 2-chloro-1,1,1,3,3-pentafluoropropane, le 1-chloro-1,1,3,3,3-pentafluoropropane, le 1-chloro-1,1,2,3,3-pentafluoropropane, le 3-chloro-1,1,1,2,2-pentafluoropropane, le 2-chloro-1,1,2,3,3-pentafluoropropane, le 2-chloro-1,1,1,2,3-pentafluoropropane,le 1,1,1,2,2,3-hexafluoropropane, le 1,1,1,2,3,3-hexafluoropropane, le 1,1,1,3,3,3-hexafluoropropane, le 1,1,2,2,3,3-hexafluoropropane, le dichlorotrifluoropropane, le 1,1-dichloro-3,3,3-trifluoropropane, le 1,3-dichloro-1,1,3-trifluoropropane, le 1,1-dichloro-1,3,3-trifluoropropane, le 1,3-dichloro-1,2,3-trifluoropropane, le 1,1-dichloro-2,3,3-trifluoropropane, le 1,3-dichloro-1,1,2-trifluoropropane, le 1,1-dichloro-1,2,3-trifluoropropane, le 1,2-dichloro-1,3,3-trifluoropropane, le 2,3-dichloro-1,1,1-trifluoropropane, le 1,2-dichloro-1,1,3-trifluoropropane, le 1,3-dichloro-1,2,2-trifluoropropane, le 1,1-dichloro-2,2,3-trifluoropropane, le 1,1-dichloro-1,2,2-trifluoropropane, le 2,3-dichloro-1,1,2-trifluoropropane, le 1,2-dichloro-1,2,3-trifluoropropane, le 1,2-dichloro-1,1,2-trifluoropropane, le 2,2-dichloro-1,1,3-trifluoropropane, le 2,2-dichloro-3,3,3-trifluoropropane, le chlorotétrafluoropropane, le 2-chloro-1,2,3,3-tétrafluoropropane, le 3-chloro-1,1,2,2-tétrafluoropropane, le 1-chloro-1,2,2,3-tétrafluoropropane, le 1-chloro-1,1,2,2-tétrafluoropropane, le 2-chloro-1,1,3,3-tétrafluoropropane, le 2-chloro-1,1,1,3-tétrafluoropropane, le 3-chloro-1,1,2,3-tétrafluoropropane, le 3-chloro-1,1,1,2-tétrafluoropropane, le 1-chloro-1,1,2,3-tétrafluoropropane, le 3-chloro-1,1,1,3-tétrafluoropropane, le 1-chloro-1,1,3,3-tétrafluoropropane, le pentafluoropropane, le 1,1,1,3,3-pentafluoropropane, le 1,1,2,2,3-pentafluoropropane, le 1,1,2,3,3-pentafluoropropane, le 1,1,1,2,3-pentafluoropropane, le chlorotrifluoropropane, le 2-chloro-1,2,3-trifluoropropane, le 2-chloro-1,1,2-trifluoropropane, le 1-chloro-2,2,3-trifluoropropane, le 1-chloro-1,2,2-trifluoropropane, le 3-chloro-1,1,2-trifluoropropane, le 1-chloro-1,2,3-trifluoropropane, le 1-chloro-1,1,2-trifluoropropane, le 3-chloro-1,3,3-trifluoropropane, le 3-chloro-1,1,1-trifluoropropane, le 1-chloro-1,1,3-trifluoropropane, le 2-chloro-1,1,3-trifluoropropane, le 2-chloro-1,1,1-trifluoropropane, le 1,1,2,2-tétrafluoropropane, le 1,1,1,3-tétrafluoropropane, le 1,1,2,3-tétrafluoropropane, le 1,1,1,2-tétrafluoropropane, le 1,2,2,3-tétrafluoropropane, le 1,1,3,3-tétrafluoropropane,le chlorodifluoropropane, le 1-chloro-2,2-difluoropropane, le 3-chloro-1,1-difluoropropane, le 1-chloro-1,3-difluoropropane, le 1-chloro-1,1-difluoropropane, le 1-chloro-2,3-difluoropropane, le 1-chloro-1,2-difluoropropane, le 2-chloro-1,3-difluoropropane, le 2-chloro-1,1-difluoropropane, le 2-chloro-1,2-difluoropropane, le trifluoropropane, le 1,1,1-trifluoropropane, le 1,1,3-trifluoropropane, le 1,2,3-trifluoropropane, le 1,1,2-trifluoropropane, le 1,2,2-trifluoropropane, le dichlorotetrafluoropropène le 1,2-dichloro-1,3,3,3-tetrafluoropropène, le 1,1-dichloro-2,3,3,3tetrafluoropropène, le 1,3-dichloro-1,2,3,3tetrafluoropropène, le 2,3-dichloro-1,1,3,3-tetrafluoropropène, le 3,3-dichloro-1,1,2,3tetrafluoropropène, le chloropentafluoropropène, le 1-chloropentafluoropropène, le 2-chloropentafluoropropène, le 3-chloropentafluoropropène, le hexafluoropropène, dichlorotrifluoropropène, le 1,1-dichloro-3,3,3-trifluoropropène, le 1,2-dichloro-3,3,3-trifluoropropène, le 2,3-dichloro-1,3,3-trifluoropropène, le 1,3-dichloro-2,3,3-trifluoropropène, le 1,2-dichloro-1,3,3-trifluoropropène, le 2,3-dichloro-1,1,3-trifluoropropène, le 1,1-dichloro-2,3,3-trifluoropropène, le 1,3-dichloro-1,2,3-trifluoropropène, le 3,3-dichloro-1,1,2-trifluoropropène, le 3,3-dichloro-1,2,3-trifluoropropène, le 1,3-dichloro-1,3,3-trifluoropropène, le 3,3-dichloro-1,1,3-trifluoropropène,le 1-chloro-2,3,3,3-tetrafluoropropène, le 1-chloro-1,3,3,3-tetrafluoropropène, le 2-chloro-1,3,3,3-tetrafluoropropène, le 3-chloro-1,2,3,3-tetrafluoropropène, le 3-chloro-1,1,3,3-tetrafluoropropène, le 2-chloro-1,1,3,3-tetrafluoropropène, le 1-chloro-1,2,3,3-tetrafluoropropène, le 3-chloro-1,1,2,3-tetrafluoropropène, le 1,1,2,3,3-pentafluoropropène, le dichlorodifluoropropène, le 2,3-dichloro-3,3-difluoropropène, le 1,2-dichloro-1,3-difluoropropène, le 2,3-dichloro-1,1-difluoropropène, le 1,2-dichloro-3,3-difluoropropène, le 2,3-dichloro-1,3-difluoropropène, le 1,1-dichloro-2,3-difluoropropène, le 1,3-dichloro-1,2-difluoropropène, le 1,3-dichloro-2,3-difluoropropène, le 3,3-dichloro-1,2-difluoropropène, le 3,3-dichloro-2,3-difluoropropène, le 1,1-dichloro-3,3-difluoropropène, le 1,3-dichloro-1,3-difluoropropène, le 3,3-dichloro-1,1-difluoropropène, le 1,3-dichloro-3,3-difluoropropène, le 3,3-dichloro-1,3-difluoropropène, le chlorotrifluoropropene, le 2-chloro-1,1,3-trifluoropropène, le 2-chloro-1,3,3-trifluoropropène, le 1-chloro-1,2,3-trifluoropropène, le 3-chloro-1,1,2-trifluoropropène, le 1-chloro-2,3,3-trifluoropropène, le 3-chloro-1,2,3-trifluoropropène, le 3-chloro-2,3,3-trifluoropropène, le 1-chloro-1,3,3-trifluoropropène, le 3-chloro-1,1,3-trifluoropropène, le 3-chloro-1,3,3-trifluoropropène, le 1,1,2,3-tetrafluoropropene le 1,2,3,3-tetrafluoropropene, le 1,1,3,3-tetrafluoropropene, le chlorodifluoropropene, le 3-chloro-3,3-difluoropropène, le 3-chloro-1,3-difluoropropène, le 2-chloro-1,1-difluoropropène, le 2-chloro-1,3-difluoropropène, le 2-chloro-3,3-difluoropropène, le 1-chloro-1,2-difluoropropène, le 1-chloro-2,3-difluoropropène, le 3-chloro-1,2-difluoropropène, le 3-chloro-2,3-difluoropropène, le 1-chloro-1,3-difluoropropène, le 3-chloro-1,1-difluoropropène, le 1-chloro-3,3-difluoropropène, le trifluoropropene, le 1,1,2-trifluoropropene, le 1,2,3-trifluoropropene, le 2,3,3-trifluoropropene, le 1,1,3-trifluoropropene, le 1,3,3-trifluoropropene, le chlorofluoropropène, le 1-chloro-3-fluoropropène, le 1-chloro-1-fluoropropène, le 1-chloro-2-fluoropropène, le 2-chloro-1-fluoropropène, le 2-chloro-3-fluoropropène, le 3-chloro-2-fluoropropène, le 3-chloro-1-fluoropropène, le 3-chloro-3-fluoropropène, le difluoropropène, le 1,2-difluoropropène, le 2,3-difluoropropène, le 1,1-difluoropropène, le 1,3-difluoropropène, le 3,3-difluoropropène, le E-1,3,3,3-tetrafluoropropene, le Z-1,3,3,3-tetrafluoropropene le Z-3,3,3-trifluoro-1-chloropropene.

De préférence, les compositions ternaires constituées essentiellement de HF-HFO-1234ze-HFC-245fa et HF-HFO-1234zeE-HFO-1234zeZ sont exclues de la présente invention.

La présente invention a en outre pour objet une composition azéotropique ou quasi- azéotropique comprenant du fluorure d'hydrogène, du Z-1,3,3,3-tetrafluoropropene et au moins un ou plusieurs composé(s) organique(s) choisi parmi le 3,3,3-trifluoro-2-chloropropene, le E-3,3,3-trifluoro-1-chloropropene, le 3,3,3-trifluoropropene, le 1,1,1,2,2-pentafluoropropane et le 2,3,3,3-tetrafluoropropene.

Selon un mode de réalisation, la composition selon l'invention comprend du fluorure d'hydrogène, du Z-1,3,3,3-tetrafluoropropene, du 1,1,1,2,2-pentafluoropropane et éventuellement un ou plusieurs composé(s) choisi(s) parmi le 2,3,3,3-tetrafluoropropene, le 3,3,3-trifluoropropene, le 3,3,3-trifluoro-2-chloropropene, le E-3,3,3-trifluoro-1-chloropropene, le trifluoropropyne, le 1,1,1,3,3-pentafluoropropane, le 1,1,1,3,3-pentafluoropropène, le 1,1,1,2,3-pentafluoropropène et le 2-chloro,1,1,1,2-tetrafluoropropane.

Selon un autre mode de réalisation, la composition selon l'invention comprend du fluorure d'hydrogène, du Z-1,3,3,3-tetrafluoropropene, du 2,3,3,3-tetrafluoropropene et éventuellement un ou plusieurs composé(s) choisi(s) parmi le 3,3,3-trifluoropropene, le 3,3,3-trifluoro-2-chloropropene, le E-3,3,3-trifluoro-1-chloropropene, le trifluoropropyne, le 1,1,1,3,3-pentafluoropropane, le 1,1,1,3,3-pentafluoropropène, le 1,1,1,2,3-pentafluoropropène et le 2-chloro,1,1,1,2-tetrafluoropropane.

Selon un mode de mise en œuvre, la composition selon l'invention comprend du fluorure d'hydrogène, du Z-1,3,3,3-tetrafluoropropene, du 3,3,3-trifluoropropene et éventuellement un ou plusieurs composé(s) choisi(s) parmi le 3,3,3-trifluoro-2-chloropropene, le E-3,3,3-trifluoro-1-chloropropene, le trifluoropropyne, le 1,1,1,3,3-pentafluoropropane, le 1,1,1,3,3-pentafluoropropène, le 1,1,1,2,3-pentafluoropropène et le 2-chloro, 1,1,1,2-tetrafluoropropane.

Selon un autre mode de réalisation, la composition selon l'invention comprend du fluorure d'hydrogène, du Z-1,3,3,3-tetrafluoropropene, du 3,3,3-trifluoro-2-chloropropene et éventuellement un ou plusieurs composé(s) choisi(s) parmi le E-3,3,3-trifluoro-1-chloropropene, le trifluoropropyne, le 1,1,1,3,3-pentafluoropropane, le 1,1,1,3,3-pentafluoropropène, le 1,1,1,2,3-pentafluoropropène et le 2-chloro,1,1,1,2-tetrafluoropropane.

Selon un mode de mise en oeuvre, la composition selon l'invention comprend du fluorure d'hydrogène, du Z-1,3,3,3-tetrafluoropropene, du E-3,3,3-trifluoro-1-chloropropene et éventuellement un ou plusieurs composé(s) choisi(s) parmi le trifluoropropyne, le 1,1,1,3,3-pentafluoropropane, le 1,1,1,3,3-pentafluoropropène, le 1,1,1,2,3-pentafluoropropène et le 2-chloro, 1,1,1,2-tetrafluoropropane.

Selon un autre mode de mise en oeuvre, la composition selon l'invention comprend du fluorure d'hydrogène, du Z-1,3,3,3-tetrafluoropropene, de la trifluoropropyne et éventuellement un ou plusieurs composé(s) choisi(s) parmi le 1,1,1,3,3-pentafluoropropène, le 1,1,1,3,3-pentafluoropropane, le 1,1,1,2,3-pentafluoropropène et le 2-chloro,1,1,1,2-tetrafluoropropane.

Selon un mode de réalisation, la composition selon l'invention comprend du fluorure d'hydrogène, du Z-1,3,3,3-tetrafluoropropene, du 1,1,1,3,3-pentafluoropropène et éventuellement un ou plusieurs composé(s) choisi(s) parmi le 1,1,1,3,3-pentafluoropropane, le 1,1,1,2,3-pentafluoropropène et le 2-chloro,1,1,1,2-tetrafluoropropane.

Selon un autre mode de réalisation, la composition selon l'invention comprend du fluorure d'hydrogène, du Z-1,3,3,3-tetrafluoropropene, du 1,1,1,2,3-pentafluoropropène et éventuellement ou plusieurs composé(s) choisi(s) parmi le 1,1,1,3,3-pentafluoropropane, le 2-chloro, 1,1,1,2-tetrafluoropropane.

Selon un autre mode de réalisation, la composition selon l'invention comprend du fluorure d'hydrogène, du Z-1,3,3,3-tetrafluoropropene, du 3,3,3-trifluoro-2-chloropropene et éventuellement un ou plusieurs composé(s) choisi(s) parmi le E-3,3,3-trifluoro-1-chloropropene, le 3,3,3-trifluoropropene, le 1,1,1,2,2-pentafluoropropane, le 2,3,3,3-tetrafluoropropene, le trifluoropropyne, le 1,1,1,3,3-pentafluoropropane, le 1,1,1,3,3-pentafluoropropène, le 1,1,1,2,3-pentafluoropropène et le 2-chloro, 1,1,1,2-tetrafluoropropane.

Selon un mode de mise en œuvre selon l'invention, la composition comprend du fluorure d'hydrogène, du Z-1,3,3,3-tetrafluoropropene, du E-3,3,3-trifluoro-1-chloropropene et éventuellement un ou plusieurs composé(s) choisi(s) parmi le 3,3,3-trifluoropropene, le 1,1,1,2,2-pentafluoropropane le 2,3,3,3-tetrafluoropropene, le trifluoropropyne, le 1,1,1,3,3-pentafluoropropène, le 1,1,1,2,3-pentafluoropropène et le 2-chloro,1,1,1,2-tetrafluoropropane.

Selon un autre mode de mise en œuvre, la composition selon l'invention comprend du fluorure d'hydrogène, du Z-1,3,3,3-tetrafluoropropene, du 3,3,3-trifluoropropene et éventuellement un ou plusieurs composé(s) choisi(s) parmi le 1,1,1,2,2-pentafluoropropane, le 2,3,3,3-tetrafluoropropene, le trifluoropropyne, le 1,1,1,3,3-pentafluoropropène, le 1,1,1,3,3-pentafluoropropane, le 1,1,1,2,3-pentafluoropropène et le 2-chloro,1,1,1,2-tetrafluoropropane.

Selon un mode de réalisation de l'invention, la composition comprend du fluorure d'hydrogène, du Z-1,3,3,3-tetrafluoropropene, du 1,1,1,2,2-pentafluoropropane et éventuellement un ou plusieurs composé(s) choisi(s) parmi le 2,3,3,3-tetrafluoropropene, le trifluoropropyne, le 1,1,1,3,3-pentafluoropropane, le 1,1,1,3,3-pentafluoropropène, le 1,1,1,2,3-pentafluoropropène et le 2-chloro, 1,1,1,2-tetrafluoropropane.

Quelque soit le mode de mise en œuvre, la composition comprend de 1 à 95 %, et avantageusement de 5 à 80 % en poids de fluorure d'hydrogène et de 99 à 5 %, et avantageusement de 20 à 95% en poids de la somme des composés organiques, plus particulièrement la composition comprend de 1 à 85 % en poids de fluorure d'hydrogène et de 99 à 15 % en poids de la somme des composés organiques (HFO-1234ze et les composés (hydro)halogénocarbonés).

Quelque soit le mode de réalisation, le point d'ébullition de la composition selon l'invention est compris entre -20 °C et 80 °C, et à une pression comprise entre 0,1 et 44 bars absolue, préférentiellement compris entre 0 °C et 40 °C et préférentiellement à une pression comprise entre 0,7 et 18 bars absolue, avantageusement entre 0,9 et 12,5 bars absolue.

La demanderesse a découvert que les compositions selon l'invention présentent des propriétés intéressantes en particulier pour le recyclage de l'HF à l'étape réactionnelle. Ainsi, la phase condensée de ces compositions, éventuellement lorsqu'elles sont soumises à une étape de distillation et/ou une étape de séparation liquide/liquide, telle que par décantation, forme deux phases liquides non miscibles.

A titre d'exemple, pour les composés ternaires contenant du fluorure d'hydrogène, du Z-1,3,3,3-tetrafluoropropene et un composé choisi parmi le 1,1,1,2,2-pentafluoropropane, le 2,3,3,3-tetrafluoropropene, le 3,3,3-trifluoropropene, le 3,3,3-trifluoro-2-chloropropene, le E-3,3,3-trifluoro-1-chloropropene, l'apparition d'un hétéro-azéotrope caractérisé par deux phases liquides, l'une riche en HF et l'autre appauvrie en HF dépend de la quantité d'HF dans la composition. Ces plages de décantation en fonction de la teneur en HF dans les compositions ont été caractérisées pour au moins des isothermes à 0 °C, 25 °C et 40 °C.

De même, les plages de décantation pour les composés ternaires contenant du fluorure d'hydrogène, du 1,3,3,3-tetrafluoropropene et un composé choisi parmi le trifluoropropyne, le 1,1,1,3,3-pentafluoropropène, le Z-1,1,1,2,3-pentafluoropropène et le 2-chloro,1,1,1,2-tetrafluoropropane sont caractérisées par une phase appauvrie en HF et une phase enrichie en HF pour au moins des isothermes à 0 °C, 25 °C et 40 °C.

La demanderesse a observé le même phénomène pour des compositions de fluorure d'hydrogène, Z-1,3,3,3-tetrafluoropropene comprenant plusieurs composés choisis parmi le 1,1,1,2,2-pentafluoropropane, le 2,3,3,3-tetrafluoropropene, le 3,3,3-trifluoropropene, le 3,3,3-trifluoro-2-chloropropene, le E-3,3,3-trifluoro-1-chloropropene, le trifluoropropyne, le 1,1,1,3,3-pentafluoropropène, le 1,1,1,2,3-pentafluoropropène et le 2-chloro, 1,1,1,2-tetrafluoropropane.

Une composition azéotropique ou quasi-azéotropique comprend de 1 à 80 % en poids de fluorure d'hydrogène, et de 99 à 20 % en poids de la somme de HFO-1234zeE et de HCFO-1233xf, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 75 % en poids de fluorure d'hydrogène, et de 95 à 25 % en poids de la somme de HFO-1234zeE et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234zeZ et de HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234zeZ et de HCFO-1233xf, le point d'ébullition de cette composition préférée est compris entre 0 et 40 °C à une pression comprise entre 0,9 et 11,6 bars absolue.

Une composition azéotropique ou quasi-azéotropique comprend de 1 à 80 % en poids de fluorure d'hydrogène, et de 99 à 20 % en poids de la somme de HFO-1234zeE et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 75 % en poids de fluorure d'hydrogène, et de 95 à 25 % en poids de la somme de HFO-1234zeE et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234zeZ et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234zeZ et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de 1 à 85 % en poids de fluorure d'hydrogène, et de 99 à 15 % en poids de la somme de HFO-1234zeE et de HFC-245cb, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 80 % en poids de fluorure d'hydrogène, et de 95 à 20 % en poids de la somme de HFO-1234zeE et de HFC-245cb, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234zeZ et de HFC-245cb, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234zeZ et de HFC-245cb, le point d'ébullition de cette composition préférée est compris entre 0 et 40 °C à une pression comprise entre 0,9 et 11,6 bars absolue.

Une composition azéotropique ou quasi-azéotropique comprend de 1 à 80 % en poids de fluorure d'hydrogène, et de 99 à 20 % en poids de la somme de HFO-1234zeE et de HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 75 % en poids de fluorure d'hydrogène, et de 95 à 25 % en poids de la somme de HFO-1234zeE et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234zeZ et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234zeZ et de HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de 1 à 85 % en poids de fluorure d'hydrogène, et de 99 à 15 % en poids de la somme de HFO-1234zeE, de HCFO-1233xf et HFC-245cb, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 80 % en poids de fluorure d'hydrogène, et de 95 à 20 % en poids de la somme de HFO-1234zeE, de HCFO-1233xf et HFC-245cb, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234zeZ, de HCFO-1233xf et HFC-245cb, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234zeZ, de HCFO-1233xf et HFC-245cb, le point d'ébullition de cette composition préférée est compris entre 0 et 40 °C à une pression comprise entre 0,9 et 11,6 bars absolue.

Une composition azéotropique ou quasi-azéotropique comprend de 1 à 80 % en poids de fluorure d'hydrogène, et de 99 à 20 % en poids de la somme de HFO-1234zeE, de HCFO-1233zdE et HCFO-1233xf, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 75 % en poids de fluorure d'hydrogène, et de 95 à 25 % en poids de la somme de HFO-1234zeE, de HCFO-1233zdE et HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234zeZ, de HCFO-1233zdE et HCFO-1233xf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234zeZ, de HCFO-1233zdE et HCFO-1233xf, le point d'ébullition de cette composition préférée est compris entre 0 et 40 °C à une pression comprise entre 0,9 et 11,6 bars absolue.

Une composition azéotropique ou quasi-azéotropique comprend de 1 à 80 % en poids de fluorure d'hydrogène, et de 99 à 20 % en poids de la somme de HFO-1234zeE, de HCFO-1233xf et HFO-1234zeZ, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 75 % en poids de fluorure d'hydrogène, et de 95 à 25 % en poids de la somme de HFO-1234zeE, de HCFO-1233xf et HFO-1234zeZ, le point d'ébullition de cette composition préférée est compris entre 0 et 40 °C à une pression comprise entre 0,9 et 11,6 bars absolue.

Une composition azéotropique ou quasi-azéotropique comprend de 1 à 80 % en poids de fluorure d'hydrogène, et de 99 à 20 % en poids de la somme de HFO-1234zeE, de HCFO-1233xf et HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 75 % en poids de fluorure d'hydrogène, et de 95 à 25 % en poids de la somme de HFO-1234zeE, de HCFO-1233xf et HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234zeZ, de HCFO-1233xf et HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234zeZ, de HCFO-1233xf et HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de 1 à 85 % en poids de fluorure d'hydrogène, et de 99 à 15 % en poids de la somme de HFO-1234zeE, HFC-245cb et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 80 % en poids de fluorure d'hydrogène, et de 95 à 20 % en poids de la somme de HFO-1234zeE, HFC-245cb et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234zeZ, HFC-245cb et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234zeZ, HFC-245cb et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de 1 à 80 % en poids de fluorure d'hydrogène, et de 99 à 20 % en poids de la somme de HFC-1234zeE, HFO-1234zeZ et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 75 % en poids de fluorure d'hydrogène, et de 95 à 25 % en poids de la somme de HFO-1234zeE, HFO-1234zeZ et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de 1 à 80 % en poids de fluorure d'hydrogène, et de 99 à 20 % en poids de la somme de HFO-1234zeE, HFO-1243zf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 75 % en poids de fluorure d'hydrogène, et de 95 à 25 % en poids de la somme de HFO-1234zeE, HFO-1243zf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234zeZ, HFO-1243zf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234zeZ, HFO-1243zf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234zeZ, HFC-245cb et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234zeZ, HFC-245cb et de HFO-1234zeE, le point d'ébullition de cette composition préférée est compris entre 0 et 40 °C à une pression comprise entre 0,9 et 11,6 bars absolue.

Une composition azéotropique ou quasi-azéotropique comprend de 1 à 85 % en poids de fluorure d'hydrogène, et de 99 à 15 % en poids de la somme de HFO-1234zeE, HFC-245cb et de HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 80 % en poids de fluorure d'hydrogène, et de 95 à 20 % en poids de la somme de HFO-1234zeE, HFC-245cb et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234zeZ, HFC-245cb et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234zeZ, HFC-245cb et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234zeZ, HFO-1243zf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234zeZ, HFO-1243zf et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234zeZ, de HFC-245cb, de HCFO-1233xf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234zeZ, de HFC-245cb, de HCFO-1233xf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de 1 à 85 % en poids de fluorure d'hydrogène, et de 99 à 15 % en poids de la somme de HFO-1234zeE, de HFC-245cb, de HCFO-1233xf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 80 % en poids de fluorure d'hydrogène, et de 95 à 20 % en poids de la somme de HFO-1234zeE, de HFC-245cb, de HCFO-1233xf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de 1 à 85 % en poids de fluorure d'hydrogène, et de 99 à 15 % en poids de la somme de HFO-1234zeE, de HFC-245cb, de HCFO-1233xf et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 80 % en poids de fluorure d'hydrogène, et de 95 à 20 % en poids de la somme de HFO-1234zeE, de HFC-245cb, de HCFO-1233xf et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique comprend de 1 à 85 % en poids de fluorure d'hydrogène, et de 99 à 15 % en poids de la somme de HFO-1234zeE, de HFC-245cb, de HCFO-1233xf et de HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 80 % en poids de fluorure d'hydrogène, et de 95 à 20 % en poids de la somme de HFO-1234zeE, de HFC-245cb, de HCFO-1233xf et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234zeZ, de HFC-245cb, de HCFO-1233xf et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234zeZ, de HFC-245cb, de HCFO-1233xf et de HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de 1 à 80 % en poids de fluorure d'hydrogène, et de 99 à 20 % en poids de la somme de HFO-1234zeE, de HCFO-1233zdE, de HCFO-1233xf et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 75 % en poids de fluorure d'hydrogène, et de 95 à 25 % en poids de la somme de HFO-1234zeE, de HCFO-1233zdE, de HCFO-1233xf et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique comprend de 1 à 80 % en poids de fluorure d'hydrogène, et de 99 à 20 % en poids de la somme de HFO-1234zeE, de HCFO-1233xf, de HCFO-1233zdE et de HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 75 % en poids de fluorure d'hydrogène, et de 95 à 25 % en poids de la somme de HFO-1234zeE, de HCFO-1233zdE, de HCFO-1233xf et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 80 % en poids de fluorure d'hydrogène, et de 99 à 20 % en poids de la somme de HFO-1234zeZ, de HCFO-1233zdE, de HCFO-1233xf et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234zeZ, de HCFO-1233zdE, de HCFO-1233xf et de HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de 1 à 80 % en poids de fluorure d'hydrogène, et de 99 à 20 % en poids de la somme de HFO-1234zeE, de HCFO-1233xf, de HFO-1234zeZ et de HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 75 % en poids de fluorure d'hydrogène, et de 95 à 25 % en poids de la somme de HFO-1234zeE, de HCFO-1233xf, de HFO-1234zeZ et de HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de préférence de 1 à 85 % en poids de fluorure d'hydrogène, et de 99 à 15 % en poids de la somme de HFO-1234zeE, HFC-245cb, HFO-1234zeZ et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 80 % en poids de fluorure d'hydrogène, et de 95 à 20 % en poids de la somme de HFO-1234zeE, HFC-245cb, HFO-1234zeZ et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de 1 à 85 % en poids de fluorure d'hydrogène, et de 99 à 15 % en poids de la somme de HFO-1234zeE, HFC-245cb, HFO-1243zf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 80 % en poids de fluorure d'hydrogène, et de 95 à 20 % en poids de la somme de HFO-1234zeE, HFC-245cb, HFO-1243zf et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234zeZ, de HFC-245cb, de HCFO-1233zdE et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234zeZ, de HFC-245cb, de HCFO-1233zdE et de HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de 1 à 80 % en poids de fluorure d'hydrogène, et de 99 à 20 % en poids de la somme de HFO-1234zeE, de HFO-1243zf, de HCFO-1233zdE et de HFO-1234zeZ, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 75 % en poids de fluorure d'hydrogène, et de 95 à 25 % en poids de la somme de HFO-1234zeE, de HFO-1243zf, de HCFO-1233zdE et de HFO-1234zeZ, le point d'ébullition de cette composition préférée est compris entre 0 et 40 °C à une pression comprise entre 0,9 et 11,6 bars absolue. ()

Une composition azéotropique ou quasi-azéotropique préférée selon l'invention comprend de 1 à 85 % en poids de fluorure d'hydrogène, et de 99 à 15 % en poids de la somme de HFO-1234zeZ, de HFC-245cb, de HCFO-1234zeE et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234zeZ, de HFC-245cb, de HCFO-1234zeE et de HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de 1 à 85 % en poids de fluorure d'hydrogène, et de 99 à 15 % en poids de la somme de HFO-1234zeE, de HFC-245cb, de HCFO-1233xf, de HFO-1234zeZ, et de HCFO-1233zdE le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 80 % en poids de fluorure d'hydrogène, et de 95 à 20 % en poids de la somme de HFO-1234zeE, de HFC-245cb, de HCFO-1233xf, de HFO-1234zeZ, et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de 1 à 85 % en poids de fluorure d'hydrogène, et de 99 à 15 % en poids de la somme de HFO-1234zeE, de HFC-245cb, de HCFO-1233xf, de HFO-1234zeZ, et de HFO-1243zf le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 80 % en poids de fluorure d'hydrogène, et de 95 à 20 % en poids de la somme de HFO-1234zeE, de HFC-245cb, de HCFO-1233xf, de HFO-1234zeZ, et de HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de 1 à 85 % en poids de fluorure d'hydrogène, et de 99 à 15 % en poids de la somme de HFO-1234zeE, de HFC-245cb, de HCFO-1233xf, de HCFO-1233zdE, et de HFO-1243zf le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 80 % en poids de fluorure d'hydrogène, et de 95 à 20 % en poids de la somme de HFO-1234zeE, de HFC-245cb, de HCFO-1233xf, de HCFO-1233zdE, et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234zeZ, de HFC-245cb, de HCFO-1233xf, de HCFO-1233zdE, et de HFO-1243zf le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234zeZ, de HFC-245cb, de HCFO-1233xf, de HCFO-1233zdE, et de HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de 1 à 80 % en poids de fluorure d'hydrogène, et de 99 à 20 % en poids de la somme de HFO-1234zeE, de HCFO-1233xf, de HFO-1234zeZ, de HCFO-1233zdE, et de HFO-1243zf le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 75 % en poids de fluorure d'hydrogène, et de 95% à 25 % en poids de la somme de HFO-1234zeE, de HFO-1234zeZ, de HCFO-1233xf, de HCFO-1233zdE, et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234zeZ, de HFC-245cb, de HCFO-1233zdE, de HFO-1234zeE et de HFO-1243zf, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234zeZ, de HFC-245cb, de HCFO-1233zdE, de HFO-1234zeE et de HFO-1243zf, le point d'ébullition de cette composition préférée est compris entre 0 et 40 °C à une pression comprise entre 0,9 et 11,6 bars absolue.

Une composition azéotropique ou quasi-azéotropique comprend de 1 à 85 % en poids de fluorure d'hydrogène, et de 99 à 15 % en poids de la somme de HFO-1234zeE, de HFC-245cb, de HCFO-1233xf, de HFO-1234zeZ, de HCFO-1233zdE et de HFO1243zf, le point d'ébullition de cette composition préférée est compris entre 0 et 40 °C à une pression comprise entre 0,9 et 11,6 bars absolue.

Une composition azéotropique ou quasi-azéotropique comprend de préférence de 5 à 80 % en poids de fluorure d'hydrogène, et de 95 à 20 % en poids de la somme de HFO-1234zeE, de HFC-245cb, de HCFO-1233xf, de HFO-1234zeZ, et de HCFO-1233zdE, le point d'ébullition de cette composition préférée est compris entre 0 et 40 °C à une pression comprise entre 0,9 et 11,6 bars absolue.

Une composition azéotropique ou quasi-azéotropique comprend de 1 à 80 % en poids de fluorure d'hydrogène, et de 99 à 20 % en poids de la somme de HFO-1234zeE, de HFC-245fa, de HCFC-244bb, de trifluoropropyne, de HFO-1225yeZ et de HFO-1225zc, le point d'ébullition de cette composition préférée est compris entre 0 et 40 °C à une pression comprise entre 0,7 et 18 bars absolue.

Une composition azéotropique ou quasi-azéotropique comprend de préférence de 5 à 75 % en poids de fluorure d'hydrogène, et de 95 à 25 % en poids de la somme de HFO-1234zeE, de HFC-245fa, de HCFC-244bb, de trifluoropropyne, de HFO-1225yeZ et de HFO-1225zc, le point d'ébullition de cette composition préférée est compris entre 0 et 40 °C à une pression comprise entre 0,7 et 18 bars absolue.

Une composition azéotropique ou quasi-azéotropique préférée selon l'invention comprend de 1 à 80 % en poids de fluorure d'hydrogène, et de 99 à 20 % en poids de la somme de HFO-1234zeZ, de HFC-245fa, de HCFC-244bb, de trifluoropropyne, de HFO-1225yeZ et de HFO-1225zc, le point d'ébullition de cette composition préférée est compris entre 0 et 40 °C à une pression comprise entre 0,7 et 18 bars absolue.

Une composition azéotropique ou quasi-azéotropique préférée selon l'invention comprend de préférence de 5 à 75 % en poids de fluorure d'hydrogène, et de 95 à 25 % en poids de la somme de HFO-1234zeZ, de HFC-245fa, de HCFC-244bb, de trifluoropropyne, de HFO-1225yeZ et de HFO-1225zc, le point d'ébullition de cette composition préférée est compris entre 0 et 40 °C à une pression comprise entre 0,7 et 18 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb, de HCFO-1233zdE, de HCFO-1233xf, de HCFC-244bb et de HFO-1234zeE le point d'ébullition de cette composition préférée est compris entre 0 et 40 °C à une pression comprise entre 0,7 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb, de HCFO-1233zdE, de HCFO-1233xf, de HCFC-244bb et de HFO-1234zeE, le point d'ébullition de cette composition préférée est compris entre 0 et 40 °C à une pression comprise entre 0,7 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb, de HCFO-1233zdE, de HCFO-1233xf, de trifluoropropyne et de HFO-1234zeE le point d'ébullition de cette composition préférée est compris entre 0 et 40 °C à une pression comprise entre 0,9 et 18 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb, de HCFO-1233zdE, de HCFO-1233xf, de trifluoropropyne et de HFO-1234zeE, le point d'ébullition de cette composition préférée est compris entre 0 et 40 °C à une pression comprise entre 0,9 et 18 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb, de HCFO-1233zdE, de HCFO-1233xf, de HFC-245fa et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb, de HCFO-1233zdE, de HCFO-1233xf, de HFC-245fa et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb, de HCFO-1233zdE, de HCFO-1233xf, de HFO-1225yeZ et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb, de HCFO-1233zdE, de HCFO-1233xf, de HFO-1225yeZ et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb, de HCFO-1233zdE, de HCFO-1233xf, de HFO-1225zc et de HFO-1234zeE le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb, de HCFO-1233zdE, de HCFO-1233xf, de HFO-1225zc et de HFO-1234zeE, le point d'ébullition de cette composition préférée est compris entre 0 et 40 °C à une pression comprise entre 0,9 et 11,6 bars absolue.

Une composition azéotropique ou quasi-azéotropique préférée selon l'invention comprend de 1 à 85 % en poids de fluorure d'hydrogène, et de 99 à 15 % en poids de la somme de HFO-1234yf, de HFO-1234zeZ, de HFC-245cb, de HCFO-1233zdE, de HCFO-1233xf, de HFO-1225zc, de trifluoropropyne, de HCFC-244bb, de HFC-245fa, de HFO-1225yeZ, de HFO-1243zf et de HFO-1234zeE, le point d'ébullition de cette composition préférée est compris entre 0 et 40 °C à une pression comprise entre 0,7 et 18,0 bars absolue.

Une composition azéotropique ou quasi-azéotropique préférée selon l'invention comprend de préférence de 5 à 80 % en poids de fluorure d'hydrogène, et de 95 à 20 % en poids de la somme de HFO-1234yf, de HFO-1234zeZ, de HFC-245cb, de HCFO-1233zdE, de HCFO-1233xf, de HFO-1225zc, de trifluoropropyne, de HCFC-244bb, de HFC-245fa, de HFO-1225yeZ, de HFO-1243zf et de HFO-1234zeE, le point d'ébullition de cette composition préférée est compris entre 0 et 40 °C à une pression comprise entre 0,7 et 18,0 bars absolue.

Les caractéristiques de pression des mélanges des exemples 1, 4, 7, 10, 13, 16 et 19 ont été calculées pour une isotherme à 25 °C.

Les exemples 2, 5, 8, 11, 14, 17 et 20 représentent les plages de température d'ébullition et de pression des mélanges et les exemples 3, 6, 9, 12, 15, 18 et 21 représentent les plages de décantation des mélanges des exemples 1, 4, 7, 10, 13, 16 et 19 en fonction du pourcentage massique d'HF caractérisées pour des isothermes à 0 °C, 25 °C et 40 °C. Les plages de décantations des exemples 3, 6, 9, 12, 15, 18 et 21 sont calculées pour des mélanges de composés organiques ayant des teneurs equimassiques. A titre d'exemple, pour un mélange ternaire, un mélange à 50 % en poids de chacun des deux composés organiques est considéré ; pour un mélange pentanaire, un mélange à 25% en poids de chacun des quatre composés organiques est considéré, la fraction massique d'HF variant de 0 à 1. Ces calculs sont réalisés à l'équilibre liquide-vapeur, dans des conditions azéotropiques Les mélanges contenant HFO-1234zeZ illustrent l'invention.

### Exemple 1 : Mélanges ternaires, isotherme à 25 °C

**HF - HFO-1234zeE - HF-1233xf**

| Organics 0,95 F1234zeE +0,05 F1233xf | | Organics 0,5 F1234zeE +0,5 F1233xf | | Organics 0,05 F1234zeE +0,95 F1233xf | |
|---|---|---|---|---|---|
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 4,7 | 0 | 3,3 | 0 | 1,7 |
| 0,05 | 5,7 | 0,05 | 4,3 | 0,05 | 2,8 |
| 0,1 | 5,7 | 0,1 | 4,3 | 0,1 | 2,8 |
| 0,15 | 5,7 | 0,15 | 4,3 | 0,15 | 2,8 |
| 0,2 | 5,7 | 0,2 | 4,3 | 0,2 | 2,8 |
| 0,25 | 5,7 | 0,25 | 4,3 | 0,25 | 2,8 |
| 0,3 | 5,7 | 0,3 | 4,3 | 0,3 | 2,8 |
| 0,35 | 5,7 | 0,35 | 4,3 | 0,35 | 2,8 |
| 0,4 | 5,7 | 0,4 | 4,3 | 0,4 | 2,8 |
| 0,45 | 5,7 | 0,45 | 4,3 | 0,45 | 2,8 |
| 0,5 | 5,6 | 0,5 | 4,3 | 0,5 | 2,8 |
| 0,55 | 5,6 | 0,55 | 4,2 | 0,55 | 2,8 |
| 0,6 | 5,5 | 0,6 | 4,2 | 0,6 | 2,8 |
| 0,65 | 5,4 | 0,65 | 4,2 | 0,65 | 2,8 |
| 0,7 | 5,2 | 0,7 | 4,1 | 0,7 | 2,8 |
| 0,75 | 5,0 | 0,75 | 3,9 | 0,75 | 2,8 |
| 0,8 | 4,7 | 0,8 | 3,7 | 0,8 | 2,6 |
| 0,85 | 4,2 | 0,85 | 3,3 | 0,85 | 2,4 |
| 0,9 | 3,5 | 0,9 | 2,8 | 0,9 | 2,2 |
| 0,95 | 2,5 | 0,95 | 2,1 | 0,95 | 1,7 |
| 1 | 1,2 | 1 | 1,2 | 1 | 1,2 |

**HF - HFO-1234zeZ - HCFO-1233xf**

| Organics 0,95 F1234zeZ +0,05 F1233xf | | Organics 0,5 F1234zeZ +0,5 F1233xf | | Organics 0,05 F1234zeZ +0,95 F1233xf | |
|---|---|---|---|---|---|
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 1,8 | 0 | 1,7 | 0 | 1,6 |
| 0,05 | 2,9 | 0,05 | 2,8 | 0,05 | 2,7 |
| 0,1 | 2,9 | 0,1 | 2,8 | 0,1 | 2,7 |
| 0,15 | 2,9 | 0,15 | 2,8 | 0,15 | 2,7 |
| 0,2 | 2,9 | 0,2 | 2,8 | 0,2 | 2,7 |
| 0,25 | 2,9 | 0,25 | 2,8 | 0,25 | 2,7 |
| 0,3 | 2,9 | 0,3 | 2,8 | 0,3 | 2,7 |
| 0,35 | 2,9 | 0,35 | 2,8 | 0,35 | 2,7 |
| 0,4 | 2,9 | 0,4 | 2,8 | 0,4 | 2,7 |
| 0,45 | 2,9 | 0,45 | 2,8 | 0,45 | 2,7 |
| 0,5 | 2,9 | 0,5 | 2,8 | 0,5 | 2,7 |
| 0,55 | 2,9 | 0,55 | 2,8 | 0,55 | 2,7 |
| 0,6 | 2,9 | 0,6 | 2,8 | 0,6 | 2,7 |
| 0,65 | 2,9 | 0,65 | 2,8 | 0,65 | 2,7 |
| 0,7 | 2,9 | 0,7 | 2,8 | 0,7 | 2,7 |
| 0,75 | 2,9 | 0,75 | 2,8 | 0,75 | 2,7 |
| 0,8 | 2,8 | 0,8 | 2,7 | 0,8 | 2,5 |
| 0,85 | 2,6 | 0,85 | 2,5 | 0,85 | 2,4 |
| 0,9 | 2,3 | 0,9 | 2,2 | 0,9 | 2,1 |
| 0,95 | 1,8 | 0,95 | 1,8 | 0,95 | 1,7 |
| 1 | 1,2 | 1 | 1,2 | 1 | 1,2 |

**HF - HFO-1234zeE - HCFO-1233zdE**

| Organics 0,95 F1233zdE +0,05 F1234zeE | | Organics 0,5 F1233zdE +0,5 F1234zeE | | Organics 0,05 F1233zdE +0,95 F1234zeE | |
|---|---|---|---|---|---|
| MASSFRAC | TOTAL | MASSFRAC | TOTAL | MASSFRAC | TOTAL |
| HF | PRES | HF | PRES | HF | PRES |
| | bar | | bar | | bar |
| 0 | 1,5 | 0 | 3,2 | 0 | 4,7 |
| 0,05 | 2,6 | 0,05 | 4,2 | 0,05 | 5,7 |
| 0,1 | 2,6 | 0,1 | 4,2 | 0,1 | 5,7 |
| 0,15 | 2,6 | 0,15 | 4,2 | 0,15 | 5,7 |
| 0,2 | 2,6 | 0,2 | 4,2 | 0,2 | 5,7 |
| 0,25 | 2,6 | 0,25 | 4,2 | 0,25 | 5,7 |
| 0,3 | 2,6 | 0,3 | 4,2 | 0,3 | 5,7 |
| 0,35 | 2,6 | 0,35 | 4,2 | 0,35 | 5,7 |
| 0,4 | 2,6 | 0,4 | 4,2 | 0,4 | 5,7 |
| 0,45 | 2,6 | 0,45 | 4,2 | 0,45 | 5,7 |
| 0,5 | 2,6 | 0,5 | 4,1 | 0,5 | 5,6 |
| 0,55 | 2,6 | 0,55 | 4,1 | 0,55 | 5,6 |
| 0,6 | 2,6 | 0,6 | 4,1 | 0,6 | 5,5 |
| 0,65 | 2,6 | 0,65 | 4,1 | 0,65 | 5,4 |
| 0,7 | 2,6 | 0,7 | 4,0 | 0,7 | 5,2 |
| 0,75 | 2,6 | 0,75 | 3,8 | 0,75 | 5,0 |
| 0,8 | 2,4 | 0,8 | 3,6 | 0,8 | 4,7 |
| 0,85 | 2,3 | 0,85 | 3,2 | 0,85 | 4,2 |
| 0,9 | 2,0 | 0,9 | 2,8 | 0,9 | 3,5 |
| 0,95 | 1,7 | 0,95 | 2,1 | 0,95 | 2,5 |
| 1 | 1,2 | 1 | 1,2 | 1 | 1,2 |

**HF - HFO-1234zeZ - HCFO-1233zdE**

| Organics 0,95 F1233zdE +0,05 F1234zeZ | | Organics 0,5 F1233zdE +0,5 F1234zeZ | | Organics 0,05 F1233zdE +0,95 F1234zeZ | |
|---|---|---|---|---|---|
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 1,3 | 0 | 1,6 | 0 | 1,8 |
| 0,05 | 2,4 | 0,05 | 2,7 | 0,05 | 2,9 |
| 0,1 | 2,4 | 0,1 | 2,7 | 0,1 | 2,9 |
| 0,15 | 2,4 | 0,15 | 2,7 | 0,15 | 2,9 |
| 0,2 | 2,4 | 0,2 | 2,7 | 0,2 | 2,9 |
| 0,25 | 2,4 | 0,25 | 2,7 | 0,25 | 2,9 |
| 0,3 | 2,4 | 0,3 | 2,7 | 0,3 | 2,9 |
| 0,35 | 2,4 | 0,35 | 2,7 | 0,35 | 2,9 |
| 0,4 | 2,4 | 0,4 | 2,7 | 0,4 | 2,9 |
| 0,45 | 2,4 | 0,45 | 2,7 | 0,45 | 2,9 |
| 0,5 | 2,4 | 0,5 | 2,7 | 0,5 | 2,9 |
| 0,55 | 2,4 | 0,55 | 2,7 | 0,55 | 2,9 |
| 0,6 | 2,4 | 0,6 | 2,7 | 0,6 | 2,9 |
| 0,65 | 2,4 | 0,65 | 2,7 | 0,65 | 2,9 |
| 0,7 | 2,4 | 0,7 | 2,7 | 0,7 | 2,9 |
| 0,75 | 2,4 | 0,75 | 2,7 | 0,75 | 2,9 |
| 0,8 | 2,3 | 0,8 | 2,6 | 0,8 | 2,8 |
| 0,85 | 2,2 | 0,85 | 2,4 | 0,85 | 2,6 |
| 0,9 | 2,0 | 0,9 | 2,1 | 0,9 | 2,3 |
| 0,95 | 1,6 | 0,95 | 1,7 | 0,95 | 1,8 |
| 1 | 1,2 | 1 | 1,2 | 1 | 1,2 |

**HF - HFO-1234zeE - HFO-1234f**

| Organics 0,95 F1234zeE +0,05 F1234yf | | Organics 0,5 F1234zeE +0,5 F1234yf | | Organics 0,05 F1234zeE +0,95 F1234yf | |
|---|---|---|---|---|---|
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 5,0 | 0 | 5,9 | 0 | 6,7 |
| 0,05 | 5,9 | 0,05 | 6,8 | 0,05 | 7,7 |
| 0,1 | 5,9 | 0,1 | 6,8 | 0,1 | 7,7 |
| 0,15 | 5,9 | 0,15 | 6,8 | 0,15 | 7,7 |
| 0,2 | 5,9 | 0,2 | 6,8 | 0,2 | 7,7 |
| 0,25 | 5,9 | 0,25 | 6,8 | 0,25 | 7,6 |
| 0,3 | 5,9 | 0,3 | 6,8 | 0,3 | 7,7 |
| 0,35 | 5,9 | 0,35 | 6,8 | 0,35 | 7,7 |
| 0,4 | 5,9 | 0,4 | 6,8 | 0,4 | 7,7 |
| 0,45 | 5,9 | 0,45 | 6,8 | 0,45 | 7,7 |
| 0,5 | 5,9 | 0,5 | 6,8 | 0,5 | 7,7 |
| 0,55 | 5,8 | 0,55 | 6,8 | 0,55 | 7,7 |
| 0,6 | 5,8 | 0,6 | 6,8 | 0,6 | 7,7 |
| 0,65 | 5,7 | 0,65 | 6,7 | 0,65 | 7,7 |
| 0,7 | 5,5 | 0,7 | 6,6 | 0,7 | 7,6 |
| 0,75 | 5,3 | 0,75 | 6,4 | 0,75 | 7,5 |
| 0,8 | 4,9 | 0,8 | 6,1 | 0,8 | 7,1 |
| 0,85 | 4,4 | 0,85 | 5,5 | 0,85 | 6,5 |
| 0,9 | 3,7 | 0,9 | 4,6 | 0,9 | 5,5 |
| 0,95 | 2,6 | 0,95 | 3,2 | 0,95 | 3,8 |
| 1 | 1,2 | 1 | 1,2 | 1 | 1,2 |

**HF - HFO-1234zeZ -HFO-1234yf**

| Organics 0,95 F1234zeZ +0,05 F1234yf | | Organics 0,5 F1234zeZ +0,5 F1234yf | | Organics 0,05 F1234zeZ +0,95 F1234yf | |
|---|---|---|---|---|---|
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 2,1 | 0 | 4,3 | 0 | 6,5 |
| 0,05 | 3,2 | 0,05 | 5,4 | 0,05 | 7,5 |
| 0,1 | 3,2 | 0,1 | 5,4 | 0,1 | 7,5 |
| 0,15 | 3,2 | 0,15 | 5,4 | 0,15 | 7,5 |
| 0,2 | 3,2 | 0,2 | 5,4 | 0,2 | 7,5 |
| 0,25 | 3,2 | 0,25 | 5,4 | 0,25 | 7,5 |
| 0,3 | 3,2 | 0,3 | 5,4 | 0,3 | 7,5 |
| 0,35 | 3,2 | 0,35 | 5,4 | 0,35 | 7,5 |
| 0,4 | 3,2 | 0,4 | 5,4 | 0,4 | 7,5 |
| 0,45 | 3,2 | 0,45 | 5,4 | 0,45 | 7,5 |
| 0,5 | 3,2 | 0,5 | 5,4 | 0,5 | 7,5 |
| 0,55 | 3,2 | 0,55 | 5,4 | 0,55 | 7,5 |
| 0,6 | 3,2 | 0,6 | 5,4 | 0,6 | 7,5 |
| 0,65 | 3,2 | 0,65 | 5,4 | 0,65 | 7,5 |
| 0,7 | 3,2 | 0,7 | 5,4 | 0,7 | 7,5 |
| 0,75 | 3,2 | 0,75 | 5,3 | 0,75 | 7,3 |
| 0,8 | 3,0 | 0,8 | 5,0 | 0,8 | 7,0 |
| 0,85 | 2,8 | 0,85 | 4,6 | 0,85 | 6,5 |
| 0,9 | 2,5 | 0,9 | 3,9 | 0,9 | 5,4 |
| 0,95 | 1,9 | 0,95 | 2,8 | 0,95 | 3,8 |
| 1 | 1,2 | 1 | 1,2 | 1 | 1,2 |

**HF - HFO-1234zeE - HFC-245cb**

| Organics 0,95 F1234zeE +0,05 F245cb | | Organics 0,5 F1234zeE +0,5 F245cb | | Organics 0,05 F1234zeE +0,95 F245cb | |
|---|---|---|---|---|---|
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 4,9 | 0 | 4,8 | 0 | 4,7 |
| 0,05 | 5,8 | 0,05 | 5,9 | 0,05 | 5,8 |
| 0,1 | 5,9 | 0,1 | 5,9 | 0,1 | 5,8 |
| 0,15 | 5,9 | 0,15 | 5,9 | 0,15 | 5,8 |
| 0,2 | 5,9 | 0,2 | 5,9 | 0,2 | 5,8 |
| 0,25 | 5,9 | 0,25 | 5,9 | 0,25 | 5,8 |
| 0,3 | 5,9 | 0,3 | 5,9 | 0,3 | 5,8 |
| 0,35 | 5,9 | 0,35 | 5,9 | 0,35 | 5,8 |
| 0,4 | 5,8 | 0,4 | 5,9 | 0,4 | 5,8 |
| 0,45 | 5,8 | 0,45 | 5,9 | 0,45 | 5,8 |
| 0,5 | 5,8 | 0,5 | 5,9 | 0,5 | 5,8 |
| 0,55 | 5,8 | 0,55 | 5,9 | 0,55 | 5,8 |
| 0,6 | 5,7 | 0,6 | 5,9 | 0,6 | 5,8 |
| 0,65 | 5,6 | 0,65 | 5,9 | 0,65 | 5,8 |
| 0,7 | 5,4 | 0,7 | 5,9 | 0,7 | 5,8 |
| 0,75 | 5,2 | 0,75 | 5,8 | 0,75 | 5,8 |
| 0,8 | 4,9 | 0,8 | 5,6 | 0,8 | 5,8 |
| 0,85 | 4,4 | 0,85 | 5,1 | 0,85 | 5,8 |
| 0,9 | 3,7 | 0,9 | 4,3 | 0,9 | 5,0 |
| 0,95 | 2,6 | 0,95 | 3,1 | 0,95 | 3,5 |
| 1 | 1,2 | 1 | 1,2 | 1 | 1,2 |

**HF - HFO-1234zeZ - HFC-245cb**

| Organics 0,95 F1234zeZ +0,05 F245cb | | Organics 0,5 F1234zeZ +0,5 F245cb | | Organics 0,05 F1234zeZ +0,95 F245cb | |
|---|---|---|---|---|---|
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 1,9 | 0 | 3,2 | 0 | 4,5 |
| 0,05 | 3,1 | 0,05 | 4,3 | 0,05 | 5,7 |
| 0,1 | 3,1 | 0,1 | 4,3 | 0,1 | 5,7 |
| 0,15 | 3,1 | 0,15 | 4,3 | 0,15 | 5,7 |
| 0,2 | 3,1 | 0,2 | 4,3 | 0,2 | 5,7 |
| 0,25 | 3,1 | 0,25 | 4,3 | 0,25 | 5,7 |
| 0,3 | 3,1 | 0,3 | 4,3 | 0,3 | 5,7 |
| 0,35 | 3,1 | 0,35 | 4,3 | 0,35 | 5,7 |
| 0,4 | 3,1 | 0,4 | 4,3 | 0,4 | 5,7 |
| 0,45 | 3,1 | 0,45 | 4,4 | 0,45 | 5,7 |
| 0,5 | 3,1 | 0,5 | 4,4 | 0,5 | 5,7 |
| 0,55 | 3,1 | 0,55 | 4,4 | 0,55 | 5,7 |
| 0,6 | 3,1 | 0,6 | 4,4 | 0,6 | 5,7 |
| 0,65 | 3,1 | 0,65 | 4,4 | 0,65 | 5,7 |
| 0,7 | 3,1 | 0,7 | 4,5 | 0,7 | 5,7 |
| 0,75 | 3,1 | 0,75 | 4,5 | 0,75 | 5,7 |
| 0,8 | 3,0 | 0,8 | 4,5 | 0,8 | 5,7 |
| 0,85 | 2,8 | 0,85 | 4,2 | 0,85 | 5,7 |
| 0,9 | 2,4 | 0,9 | 3,6 | 0,9 | 4,9 |
| 0,95 | 1,9 | 0,95 | 2,7 | 0,95 | 3,5 |
| 1 | 1,2 | 1 | 1,2 | 1 | 1,2 |

**HF - HFO-1234zeE - HFO-1243zf**

| Organics 0,95 F1243zf +0,05 F1234zeE | | Organics 0,5 F1243zf +0,5 F1234zeE | | Organics 0,05 F1243zf +0,95 F1234zeE | |
|---|---|---|---|---|---|
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 5,8 | 0 | 5,4 | 0 | 4,9 |
| 0,05 | 6,7 | 0,05 | 6,3 | 0,05 | 5,9 |
| 0,1 | 6,8 | 0,1 | 6,4 | 0,1 | 5,9 |
| 0,15 | 6,8 | 0,15 | 6,4 | 0,15 | 5,9 |
| 0,2 | 6,8 | 0,2 | 6,4 | 0,2 | 5,9 |
| 0,25 | 6,8 | 0,25 | 6,4 | 0,25 | 5,9 |
| 0,3 | 6,8 | 0,3 | 6,4 | 0,3 | 5,9 |
| 0,35 | 6,8 | 0,35 | 6,4 | 0,35 | 5,9 |
| 0,4 | 6,7 | 0,4 | 6,4 | 0,4 | 5,9 |
| 0,45 | 6,7 | 0,45 | 6,4 | 0,45 | 5,9 |
| 0,5 | 6,7 | 0,5 | 6,3 | 0,5 | 5,8 |
| 0,55 | 6,7 | 0,55 | 6,3 | 0,55 | 5,8 |
| 0,6 | 6,7 | 0,6 | 6,3 | 0,6 | 5,7 |
| 0,65 | 6,6 | 0,65 | 6,2 | 0,65 | 5,6 |
| 0,7 | 6,5 | 0,7 | 6,1 | 0,7 | 5,5 |
| 0,75 | 6,4 | 0,75 | 5,8 | 0,75 | 5,2 |
| 0,8 | 6,1 | 0,8 | 5,5 | 0,8 | 4,9 |
| 0,85 | 5,6 | 0,85 | 5,0 | 0,85 | 4,4 |
| 0,9 | 4,7 | 0,9 | 4,2 | 0,9 | 3,6 |
| 0,95 | 3,4 | 0,95 | 3,0 | 0,95 | 2,6 |
| 1 | 1,2 | 1 | 1,2 | 1 | 1,2 |

**HF - HFO-1234zeZ - HFO-1243zf**

| Organics 0,95 F1243zf +0,05 F1234zeZ | | Organics 0,5 F1243zf +0,5 F1234zeZ | | Organics 0,05 F1243zf +0,95 F1234zeZ | |
|---|---|---|---|---|---|
| MASSFRAC | TOTAL | MASSFRAC | TOTAL | MASSFRAC | TOTAL |
| HF | PRESSURE | HF | PRESSURE | HF | PRESSURE |
| | bar | | bar | | bar |
| 0 | 5,7 | 0 | 4,0 | 0 | 2,0 |
| 0,05 | 6,6 | 0,05 | 5,0 | 0,05 | 3,1 |
| 0,1 | 6,6 | 0,1 | 5,0 | 0,1 | 3,1 |
| 0,15 | 6,6 | 0,15 | 5,0 | 0,15 | 3,1 |
| 0,2 | 6,6 | 0,2 | 5,0 | 0,2 | 3,1 |
| 0,25 | 6,6 | 0,25 | 5,0 | 0,25 | 3,1 |
| 0,3 | 6,6 | 0,3 | 5,0 | 0,3 | 3,1 |
| 0,35 | 6,6 | 0,35 | 5,0 | 0,35 | 3,1 |
| 0,4 | 6,6 | 0,4 | 5,0 | 0,4 | 3,1 |
| 0,45 | 6,6 | 0,45 | 5,0 | 0,45 | 3,1 |
| 0,5 | 6,6 | 0,5 | 5,0 | 0,5 | 3,1 |
| 0,55 | 6,6 | 0,55 | 5,0 | 0,55 | 3,1 |
| 0,6 | 6,6 | 0,6 | 5,0 | 0,6 | 3,1 |
| 0,65 | 6,5 | 0,65 | 5,0 | 0,65 | 3,1 |
| 0,7 | 6,4 | 0,7 | 4,9 | 0,7 | 3,1 |
| 0,75 | 6,3 | 0,75 | 4,8 | 0,75 | 3,1 |
| 0,8 | 6,0 | 0,8 | 4,5 | 0,8 | 3,0 |
| 0,85 | 5,5 | 0,85 | 4,2 | 0,85 | 2,8 |
| 0,9 | 4,7 | 0,9 | 3,6 | 0,9 | 2,4 |
| 0,95 | 3,3 | 0,95 | 2,6 | 0,95 | 1,9 |
| 1 | 1,2 | 1 | 1,2 | 1 | 1,2 |

### Exemple 2 : Plage de température et de pression de mélanges ternaires

| | Enveloppe de points d'ébullition | |
|---|---|---|
| Ternaire | Température °C | Pression bar abs |
| HF-HFO-1234zeE-HFC-245cb | 0 à 40 | ∼2.5 à ∼9.1 |
| H F-H FO-1234zeZ - H FC-245cb | 0 à 40 | ∼1.2 à ∼8.9 |
| HF-HFO-1234yf-HFO-1234zeE | 0 à 40 | ∼2.6 à ∼11.6 |
| HF-HFO-1234yf-HFO-1234zeZ | 0 à 40 | ∼1.3 à ∼11.4 |
| HF-HCFO-1233xf-HFO-1234zeE | 0 à 40 | ∼1.1 à ∼8.8 |
| HF-HCFO-1233xf-HFO-1234zeZ | 0 à 40 | ∼1.0 à ∼4.8 |
| HF-HFO-1234zeE-HCFO-1233zdE | 0 à 40 | ∼1.0 à ∼8.8 |
| HF-HFO-1234zeE-HFO-1243zf | 0 à 40 | ∼2.5 à ∼10.4 |
| HF-HFO-1234zeZ-HCFO-1233zdE | 0 à 40 | ∼0.9 à ∼4.8 |
| HF-HFO-1234zeZ-HFO-1243zf | 0 à 40 | ∼1.2 à ∼10.2 |

### Exemple 3 : Plage de décantation de mélanges ternaires

| | Plages de décantation Pourcentage massique d'HF | | |
|---|---|---|---|
| Ternaire | Isotherme 0°C | Isotherme 25°C | Isotherme 40°C |
| HF-HFO-1234zeE-HFC-245cb | 5-75 | 10-70 | 40-65 |
| H F-HFO-1234zeZ-HFC-245cb | 5-80 | 5-75 | 10-75 |
| HF-HFO-1234yf-HFO-1234zeE | 5-65 | * | * |
| HF-HFO-1234yf-HFO-1234zeZ | 5-75 | 10-70 | 15-30 |
| HF-HCFO-1233xf-HFO-1234zeE | 5-70 | 5-60 | 10-45 |
| HF-HCFO-1233xf-HFO-1234zeZ | 5-80 | 5-70 | 5-65 |
| HF-HFO-1234zeE-HCFO-1233zdE | 5-70 | 5-65 | 10-50 |
| HF-HFO-1234zeE-HFO-1243zf | 5-65 | * | * |
| HF-HFO-1234zeZ-HCFO-1233zdE | 5-80 | 5-75 | 5-65 |
| HF-HFO-1234zeZ-HFO-1243zf | 5-75 | 10-65 | * |

### Exemple 4 : Mélanges quaternaires, isotherme à 25 °C

**HF - HCFO-1233xf - HFO-1234zeE - HFC-245cb**

| Organics 0,9 F1233xf + 0,05 F1234zeE +0,05 F245cb | | Organics 0,4 F1233xf + 0,3 F1234zeE +0,3 F245cb | | Organics 0,05 F1233xf + 0,9 F1234zeE +0,05 F245cb | | Organics 0,05 F1233xf + 0,05 F1234zeE +0,9 F245cb | |
|---|---|---|---|---|---|---|---|
| MASSFRAC | TOTAL | MASSFRAC | TOTAL | MASSFRAC | TOTAL | MASSFRAC | TOTAL |
| HF | PRES | HF | PRES | HF | PRES | HF | PRES |
| | bar | | bar | | bar | | bar |
| 0 | 3,2 | 0 | 3,5 | 0 | 4,7 | 0 | 4,5 |
| 0,05 | 4,3 | 0,05 | 4,6 | 0,05 | 5,7 | 0,05 | 5,7 |
| 0,1 | 4,3 | 0,1 | 4,6 | 0,1 | 5,7 | 0,1 | 5,7 |
| 0,15 | 4,3 | 0,15 | 4,6 | 0,15 | 5,7 | 0,15 | 5,7 |
| 0,2 | 4,3 | 0,2 | 4,6 | 0,2 | 5,7 | 0,2 | 5,7 |
| 0,25 | 4,3 | 0,25 | 4,6 | 0,25 | 5,7 | 0,25 | 5,7 |
| 0,3 | 4,3 | 0,3 | 4,6 | 0,3 | 5,7 | 0,3 | 5,7 |
| 0,35 | 4,3 | 0,35 | 4,6 | 0,35 | 5,7 | 0,35 | 5,7 |
| 0,4 | 4,4 | 0,4 | 4,6 | 0,4 | 5,7 | 0,4 | 5,7 |
| 0,45 | 4,4 | 0,45 | 4,6 | 0,45 | 5,7 | 0,45 | 5,7 |
| 0,5 | 4,4 | 0,5 | 4,6 | 0,5 | 5,7 | 0,5 | 5,7 |
| 0,55 | 4,4 | 0,55 | 4,6 | 0,55 | 5,6 | 0,55 | 5,7 |
| 0,6 | 4,4 | 0,6 | 4,6 | 0,6 | 5,6 | 0,6 | 5,7 |
| 0,65 | 4,4 | 0,65 | 4,7 | 0,65 | 5,5 | 0,65 | 5,7 |
| 0,7 | 4,5 | 0,7 | 4,7 | 0,7 | 5,3 | 0,7 | 5,7 |
| 0,75 | 4,5 | 0,75 | 4,6 | 0,75 | 5,1 | 0,75 | 5,7 |
| 0,8 | 4,5 | 0,8 | 4,4 | 0,8 | 4,8 | 0,8 | 5,7 |
| 0,85 | 4,2 | 0,85 | 4,0 | 0,85 | 4,3 | 0,85 | 5,6 |
| 0,9 | 3,6 | 0,9 | 3,4 | 0,9 | 3,6 | 0,9 | 4,8 |
| 0,95 | 2,6 | 0,95 | 2,5 | 0,95 | 2,6 | 0,95 | 3,4 |
| 1 | 1,2 | 1 | 1,2 | 1 | 1,2 | 1 | 1,2 |

**HF - HCFO-1233xf - HFO-1234zeZ - HFC-245cb**

| Organics 0,9 F1233xf + 0,05 F1234zeZ +0,05 F245cb | | Organics 0,4 F1233xf + 0,3 F1234zeZ +0,3 F245cb | | Organics 0,05 F1233xf + 0,9 F1234zeZ +0,05 F245cb | | Organics 0,05 F1233xf + 0,05 F1234zeZ +0,9 F245cb | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 1,7 | 0 | 2,6 | 0 | 1,9 | 0 | 4,3 |
| 0,05 | 2,8 | 0,05 | 3,7 | 0,05 | 3,0 | 0,05 | 5,5 |
| 0,1 | 2,8 | 0,1 | 3,7 | 0,1 | 3,0 | 0,1 | 5,5 |
| 0,15 | 2,8 | 0,15 | 3,7 | 0,15 | 3,0 | 0,15 | 5,5 |
| 0,2 | 2,8 | 0,2 | 3,7 | 0,2 | 3,0 | 0,2 | 5,5 |
| 0,25 | 2,8 | 0,25 | 3,7 | 0,25 | 3,0 | 0,25 | 5,5 |
| 0,3 | 2,8 | 0,3 | 3,7 | 0,3 | 3,0 | 0,3 | 5,5 |
| 0,35 | 2,8 | 0,35 | 3,7 | 0,35 | 3,0 | 0,35 | 5,5 |
| 0,4 | 2,8 | 0,4 | 3,7 | 0,4 | 3,0 | 0,4 | 5,5 |
| 0,45 | 2,8 | 0,45 | 3,7 | 0,45 | 3,1 | 0,45 | 5,5 |
| 0,5 | 2,8 | 0,5 | 3,7 | 0,5 | 3,1 | 0,5 | 5,5 |
| 0,55 | 2,9 | 0,55 | 3,7 | 0,55 | 3,1 | 0,55 | 5,5 |
| 0,6 | 2,9 | 0,6 | 3,8 | 0,6 | 3,1 | 0,6 | 5,5 |
| 0,65 | 2,9 | 0,65 | 3,8 | 0,65 | 3,1 | 0,65 | 5,6 |
| 0,7 | 2,9 | 0,7 | 3,8 | 0,7 | 3,1 | 0,7 | 5,6 |
| 0,75 | 2,9 | 0,75 | 3,8 | 0,75 | 3,1 | 0,75 | 5,6 |
| 0,8 | 2,7 | 0,8 | 3,8 | 0,8 | 2,9 | 0,8 | 5,6 |
| 0,85 | 2,5 | 0,85 | 3,5 | 0,85 | 2,7 | 0,85 | 5,5 |
| 0,9 | 2,2 | 0,9 | 3,0 | 0,9 | 2,4 | 0,9 | 4,8 |
| 0,95 | 1,8 | 0,95 | 2,3 | 0,95 | 1,9 | 0,95 | 3,4 |
| 1 | 1,2 | 1 | 1,2 | 1 | 1,2 | 1 | 1,2 |

**HF - HCFO-1233xf - HCFO-1233zdE - HFO-1234zeE**

| Organics 0,9 F1233xf + 0,05 F1233zdE +0,05 F1234zeE | | Organics 0,05 F1233xf + 0,9 F1233zdE +0,05 F1234zeE | | Organics 0,05 F1233xf + 0,05 F1233zdE +0,9 F1234zeE | | Organics 0,4 F1233xf + 0,3 F1233zdE +0,3 F1234zeE | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 1,7 | 0 | 1,5 | 0 | 4,6 | 0 | 2,5 |
| 0,05 | 2,8 | 0,05 | 2,6 | 0,05 | 5,5 | 0,05 | 3,6 |
| 0,1 | 2,8 | 0,1 | 2,6 | 0,1 | 5,5 | 0,1 | 3,6 |
| 0,15 | 2,8 | 0,15 | 2,6 | 0,15 | 5,5 | 0,15 | 3,6 |
| 0,2 | 2,8 | 0,2 | 2,6 | 0,2 | 5,5 | 0,2 | 3,6 |
| 0,25 | 2,8 | 0,25 | 2,6 | 0,25 | 5,5 | 0,25 | 3,6 |
| 0,3 | 2,8 | 0,3 | 2,6 | 0,3 | 5,5 | 0,3 | 3,6 |
| 0,35 | 2,8 | 0,35 | 2,6 | 0,35 | 5,5 | 0,35 | 3,6 |
| 0,4 | 2,8 | 0,4 | 2,6 | 0,4 | 5,5 | 0,4 | 3,6 |
| 0,45 | 2,8 | 0,45 | 2,6 | 0,45 | 5,5 | 0,45 | 3,6 |
| 0,5 | 2,8 | 0,5 | 2,6 | 0,5 | 5,5 | 0,5 | 3,6 |
| 0,55 | 2,8 | 0,55 | 2,6 | 0,55 | 5,4 | 0,55 | 3,5 |
| 0,6 | 2,8 | 0,6 | 2,6 | 0,6 | 5,4 | 0,6 | 3,5 |
| 0,65 | 2,8 | 0,65 | 2,6 | 0,65 | 5,3 | 0,65 | 3,5 |
| 0,7 | 2,8 | 0,7 | 2,6 | 0,7 | 5,1 | 0,7 | 3,5 |
| 0,75 | 2,8 | 0,75 | 2,6 | 0,75 | 4,9 | 0,75 | 3,3 |
| 0,8 | 2,6 | 0,8 | 2,5 | 0,8 | 4,6 | 0,8 | 3,2 |
| 0,85 | 2,4 | 0,85 | 2,3 | 0,85 | 4,1 | 0,85 | 2,9 |
| 0,9 | 2,2 | 0,9 | 2,0 | 0,9 | 3,4 | 0,9 | 2,5 |
| 0,95 | 1,7 | 0,95 | 1,7 | 0,95 | 2,5 | 0,95 | 1,9 |
| 1 | 1,2 | 1 | 1,2 | 1 | 1,2 | 1 | 1,2 |

**HF - HCFO-1233xf - HCFO-1233zdE - HFO-1234zeZ**

| Organics 0,9 F1233xf + 0,05 F1233zdE +0,05 F1234zeZ | | Organics 0,05 F1233xf + 0,9 F1233zdE +0,05 F1234zeZ | | Organics 0,05 F1233xf + 0,05 F1233zdE +0,9 F1234zeZ | | Organics 0,4 F1233xf + 0,3 F1233zdE +0,3 F1234zeZ | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 1,5 | 0 | 1,4 | 0 | 1,8 | 0 | 1,6 |
| 0,05 | 2,7 | 0,05 | 2,5 | 0,05 | 2,9 | 0,05 | 2,7 |
| 0,1 | 2,7 | 0,1 | 2,5 | 0,1 | 2,9 | 0,1 | 2,7 |
| 0,15 | 2,7 | 0,15 | 2,5 | 0,15 | 2,9 | 0,15 | 2,7 |
| 0,2 | 2,7 | 0,2 | 2,5 | 0,2 | 2,9 | 0,2 | 2,7 |
| 0,25 | 2,7 | 0,25 | 2,5 | 0,25 | 2,9 | 0,25 | 2,7 |
| 0,3 | 2,7 | 0,3 | 2,5 | 0,3 | 2,9 | 0,3 | 2,7 |
| 0,35 | 2,7 | 0,35 | 2,5 | 0,35 | 2,9 | 0,35 | 2,7 |
| 0,4 | 2,7 | 0,4 | 2,5 | 0,4 | 2,9 | 0,4 | 2,7 |
| 0,45 | 2,7 | 0,45 | 2,5 | 0,45 | 2,9 | 0,45 | 2,7 |
| 0,5 | 2,7 | 0,5 | 2,5 | 0,5 | 2,9 | 0,5 | 2,7 |
| 0,55 | 2,7 | 0,55 | 2,5 | 0,55 | 2,9 | 0,55 | 2,7 |
| 0,6 | 2,7 | 0,6 | 2,5 | 0,6 | 2,9 | 0,6 | 2,7 |
| 0,65 | 2,7 | 0,65 | 2,5 | 0,65 | 2,9 | 0,65 | 2,7 |
| 0,7 | 2,7 | 0,7 | 2,5 | 0,7 | 2,9 | 0,7 | 2,7 |
| 0,75 | 2,7 | 0,75 | 2,5 | 0,75 | 2,9 | 0,75 | 2,7 |
| 0,8 | 2,5 | 0,8 | 2,4 | 0,8 | 2,8 | 0,8 | 2,6 |
| 0,85 | 2,4 | 0,85 | 2,2 | 0,85 | 2,6 | 0,85 | 2,4 |
| 0,9 | 2,1 | 0,9 | 2,0 | 0,9 | 2,3 | 0,9 | 2,1 |
| 0,95 | 1,7 | 0,95 | 1,6 | 0,95 | 1,8 | 0,95 | 1,7 |
| 1 | 1,2 | 1 | 1,2 | 1 | 1,2 | 1 | 1,2 |

**HF - HCFO-1233xf - HFO-1234zeE - HFO-1234zeZ**

| Organics 0,9 F1233xf + 0,05 F1234zeE +0,05 F1234zeZ | | Organics 0,05 F1233xf + 0,9 F1234zeE +0,05 F1234zeZ | | Organics 0,05 F1233xf + 0,05 F1234zeE +0,9 F1234zeZ | | Organics 0,4 F1233xf + 0,3 F1234zeE +0,3 F1234zeZ | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 1,7 | 0 | 4,6 | 0 | 1,9 | 0 | 2,7 |
| 0,05 | 2,8 | 0,05 | 5,5 | 0,05 | 3,1 | 0,05 | 3,7 |
| 0,1 | 2,8 | 0,1 | 5,5 | 0,1 | 3,0 | 0,1 | 3,7 |
| 0,15 | 2,8 | 0,15 | 5,5 | 0,15 | 3,0 | 0,15 | 3,7 |
| 0,2 | 2,8 | 0,2 | 5,5 | 0,2 | 3,0 | 0,2 | 3,7 |
| 0,25 | 2,8 | 0,25 | 5,5 | 0,25 | 3,0 | 0,25 | 3,7 |
| 0,3 | 2,8 | 0,3 | 5,5 | 0,3 | 3,0 | 0,3 | 3,7 |
| 0,35 | 2,8 | 0,35 | 5,5 | 0,35 | 3,0 | 0,35 | 3,7 |
| 0,4 | 2,8 | 0,4 | 5,5 | 0,4 | 3,0 | 0,4 | 3,7 |
| 0,45 | 2,8 | 0,45 | 5,5 | 0,45 | 3,0 | 0,45 | 3,7 |
| 0,5 | 2,8 | 0,5 | 5,5 | 0,5 | 3,0 | 0,5 | 3,7 |
| 0,55 | 2,8 | 0,55 | 5,4 | 0,55 | 3,0 | 0,55 | 3,7 |
| 0,6 | 2,8 | 0,6 | 5,4 | 0,6 | 3,0 | 0,6 | 3,6 |
| 0,65 | 2,8 | 0,65 | 5,3 | 0,65 | 3,0 | 0,65 | 3,6 |
| 0,7 | 2,8 | 0,7 | 5,1 | 0,7 | 3,0 | 0,7 | 3,6 |
| 0,75 | 2,8 | 0,75 | 4,9 | 0,75 | 3,0 | 0,75 | 3,5 |
| 0,8 | 2,7 | 0,8 | 4,6 | 0,8 | 2,9 | 0,8 | 3,3 |
| 0,85 | 2,5 | 0,85 | 4,1 | 0,85 | 2,7 | 0,85 | 3,0 |
| 0,9 | 2,2 | 0,9 | 3,4 | 0,9 | 2,3 | 0,9 | 2,6 |
| 0,95 | 1,8 | 0,95 | 2,5 | 0,95 | 1,9 | 0,95 | 2,0 |
| 1 | 1,2 | 1 | 1,2 | 1 | 1,2 | 1 | 1,2 |

**HF - HCFO-1233xf - HFO-1234zeE - HFO-1243zf**

| Organics 0,9 F1233xf + 0,05 F1234zeE +0,05 F1243zf | | Organics 0,05 F1233xf + 0,9 F1234zeE +0,05 F1243zf | | Organics 0,05 F1233xf + 0,05 F1234zeE +0,9 F1243zf | | Organics 0,4 F1233xf + 0,3 F1234zeE +0,3 F1243zf | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 2,0 | 0 | 4,8 | 0 | 5,6 | 0 | 4,0 |
| 0,05 | 3,1 | 0,05 | 5,7 | 0,05 | 6,6 | 0,05 | 5,1 |
| 0,1 | 3,1 | 0,1 | 5,8 | 0,1 | 6,6 | 0,1 | 5,1 |
| 0,15 | 3,1 | 0,15 | 5,8 | 0,15 | 6,6 | 0,15 | 5,1 |
| 0,2 | 3,1 | 0,2 | 5,8 | 0,2 | 6,6 | 0,2 | 5,1 |
| 0,25 | 3,1 | 0,25 | 5,8 | 0,25 | 6,6 | 0,25 | 5,1 |
| 0,3 | 3,1 | 0,3 | 5,8 | 0,3 | 6,6 | 0,3 | 5,0 |
| 0,35 | 3,1 | 0,35 | 5,7 | 0,35 | 6,6 | 0,35 | 5,0 |
| 0,4 | 3,1 | 0,4 | 5,7 | 0,4 | 6,6 | 0,4 | 5,0 |
| 0,45 | 3,1 | 0,45 | 5,7 | 0,45 | 6,6 | 0,45 | 5,0 |
| 0,5 | 3,1 | 0,5 | 5,7 | 0,5 | 6,6 | 0,5 | 5,0 |
| 0,55 | 3,1 | 0,55 | 5,7 | 0,55 | 6,6 | 0,55 | 5,0 |
| 0,6 | 3,1 | 0,6 | 5,6 | 0,6 | 6,5 | 0,6 | 5,0 |
| 0,65 | 3,1 | 0,65 | 5,5 | 0,65 | 6,5 | 0,65 | 4,9 |
| 0,7 | 3,0 | 0,7 | 5,3 | 0,7 | 6,4 | 0,7 | 4,8 |
| 0,75 | 3,0 | 0,75 | 5,1 | 0,75 | 6,2 | 0,75 | 4,7 |
| 0,8 | 2,9 | 0,8 | 4,8 | 0,8 | 5,9 | 0,8 | 4,4 |
| 0,85 | 2,6 | 0,85 | 4,3 | 0,85 | 5,4 | 0,85 | 4,0 |
| 0,9 | 2,3 | 0,9 | 3,6 | 0,9 | 4,6 | 0,9 | 3,4 |
| 0,95 | 1,8 | 0,95 | 2,6 | 0,95 | 3,3 | 0,95 | 2,5 |
| 1 | 1,2 | 1 | 1,2 | 1 | 1,2 | 1 | 1,2 |

**HF - HCFO-1233xf - HFO-1234zeZ - HFO-1243zf**

| Organics 0,9 F1233xf + 0,05 F1234zeZ +0,05 F1243zf | | Organics 0,05 F1233xf + 0,9 F1234zeZ +0,05 F1243zf | | Organics 0,05 F1233xf + 0,05 F1234zeZ +0,9 F1243zf | | Organics 0,4 F1233xf + 0,3 F1234zeZ +0,3 F1243zf | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 1,8 | 0 | 2,0 | 0 | 5,5 | 0 | 3,1 |
| 0,05 | 2,9 | 0,05 | 3,1 | 0,05 | 6,4 | 0,05 | 4,2 |
| 0,1 | 2,9 | 0,1 | 3,1 | 0,1 | 6,5 | 0,1 | 4,2 |
| 0,15 | 2,9 | 0,15 | 3,1 | 0,15 | 6,5 | 0,15 | 4,2 |
| 0,2 | 2,9 | 0,2 | 3,1 | 0,2 | 6,5 | 0,2 | 4,2 |
| 0,25 | 2,9 | 0,25 | 3,1 | 0,25 | 6,5 | 0,25 | 4,2 |
| 0,3 | 2,9 | 0,3 | 3,1 | 0,3 | 6,5 | 0,3 | 4,2 |
| 0,35 | 2,9 | 0,35 | 3,1 | 0,35 | 6,5 | 0,35 | 4,2 |
| 0,4 | 2,9 | 0,4 | 3,1 | 0,4 | 6,5 | 0,4 | 4,2 |
| 0,45 | 2,9 | 0,45 | 3,1 | 0,45 | 6,5 | 0,45 | 4,2 |
| 0,5 | 2,9 | 0,5 | 3,1 | 0,5 | 6,4 | 0,5 | 4,2 |
| 0,55 | 2,9 | 0,55 | 3,1 | 0,55 | 6,4 | 0,55 | 4,2 |
| 0,6 | 2,9 | 0,6 | 3,1 | 0,6 | 6,4 | 0,6 | 4,1 |
| 0,65 | 2,9 | 0,65 | 3,1 | 0,65 | 6,4 | 0,65 | 4,1 |
| 0,7 | 2,9 | 0,7 | 3,1 | 0,7 | 6,3 | 0,7 | 4,1 |
| 0,75 | 2,9 | 0,75 | 3,1 | 0,75 | 6,1 | 0,75 | 4,0 |
| 0,8 | 2,8 | 0,8 | 3,0 | 0,8 | 5,8 | 0,8 | 3,8 |
| 0,85 | 2,5 | 0,85 | 2,7 | 0,85 | 5,3 | 0,85 | 3,5 |
| 0,9 | 2,2 | 0,9 | 2,4 | 0,9 | 4,5 | 0,9 | 3,0 |
| 0,95 | 1,8 | 0,95 | 1,9 | 0,95 | 3,2 | 0,95 | 2,3 |
| 1 | 1,2 | 1 | 1,2 | 1 | 1,2 | 1 | 1,2 |

**HF - HCFO-1233xf - HFO-1234f - HFO-1234zeE**

| Organics 0,9 F1233xf + 0,05 F1234yf +0,05 F1234zeE | | Organics 0,05 F1233xf + 0,9 F1234yf +0,05 F1234zeE | | Organics 0,05 F1233xf + 0,05 F1234yf +0,9 F1234zeE | | Organics 0,4 F1233xf + 0,3 F1234yf +0,3 F1234zeE | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 2,0 | 0 | 6,5 | 0 | 4,8 | 0 | 4,2 |
| 0,05 | 3,1 | 0,05 | 7,4 | 0,05 | 5,8 | 0,05 | 5,3 |
| 0,1 | 3,1 | 0,1 | 7,4 | 0,1 | 5,8 | 0,1 | 5,3 |
| 0,15 | 3,1 | 0,15 | 7,4 | 0,15 | 5,8 | 0,15 | 5,3 |
| 0,2 | 3,1 | 0,2 | 7,4 | 0,2 | 5,8 | 0,2 | 5,3 |
| 0,25 | 3,1 | 0,25 | 7,4 | 0,25 | 5,8 | 0,25 | 5,3 |
| 0,3 | 3,1 | 0,3 | 7,4 | 0,3 | 5,8 | 0,3 | 5,3 |
| 0,35 | 3,1 | 0,35 | 7,4 | 0,35 | 5,8 | 0,35 | 5,3 |
| 0,4 | 3,1 | 0,4 | 7,4 | 0,4 | 5,8 | 0,4 | 5,3 |
| 0,45 | 3,1 | 0,45 | 7,4 | 0,45 | 5,8 | 0,45 | 5,3 |
| 0,5 | 3,1 | 0,5 | 7,4 | 0,5 | 5,8 | 0,5 | 5,3 |
| 0,55 | 3,1 | 0,55 | 7,4 | 0,55 | 5,7 | 0,55 | 5,3 |
| 0,6 | 3,1 | 0,6 | 7,4 | 0,6 | 5,6 | 0,6 | 5,3 |
| 0,65 | 3,1 | 0,65 | 7,5 | 0,65 | 5,5 | 0,65 | 5,3 |
| 0,7 | 3,1 | 0,7 | 7,4 | 0,7 | 5,4 | 0,7 | 5,2 |
| 0,75 | 3,1 | 0,75 | 7,2 | 0,75 | 5,2 | 0,75 | 5,0 |
| 0,8 | 2,9 | 0,8 | 6,9 | 0,8 | 4,8 | 0,8 | 4,7 |
| 0,85 | 2,7 | 0,85 | 6,3 | 0,85 | 4,3 | 0,85 | 4,3 |
| 0,9 | 2,3 | 0,9 | 5,3 | 0,9 | 3,6 | 0,9 | 3,6 |
| 0,95 | 1,9 | 0,95 | 3,7 | 0,95 | 2,6 | 0,95 | 2,6 |
| 1 | 1,2 | 1 | 1,2 | 1 | 1,2 | 1 | 1,2 |

**HF - HCFO-1233xf - HFO-1234f - HFO-1234zeZ**

| Organics 0,9 F1233xf + 0,05 F1234yf +0,05 F1234zeZ | | Organics 0,05 F1233xf + 0,9 F1234yf +0,05 F1234zeZ | | Organics 0,05 F1233xf + 0,05 F1234yf +0,9 F1234zeZ | | Organics 0,4 F1233xf + 0,3 F1234yf +0,3 F1234zeZ | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 1,9 | 0 | 6,3 | 0 | 2,1 | 0 | 3,3 |
| 0,05 | 3,0 | 0,05 | 7,3 | 0,05 | 3,2 | 0,05 | 4,4 |
| 0,1 | 3,0 | 0,1 | 7,3 | 0,1 | 3,2 | 0,1 | 4,4 |
| 0,15 | 3,0 | 0,15 | 7,3 | 0,15 | 3,2 | 0,15 | 4,4 |
| 0,2 | 3,0 | 0,2 | 7,3 | 0,2 | 3,2 | 0,2 | 4,4 |
| 0,25 | 3,0 | 0,25 | 7,3 | 0,25 | 3,2 | 0,25 | 4,4 |
| 0,3 | 3,0 | 0,3 | 7,3 | 0,3 | 3,2 | 0,3 | 4,4 |
| 0,35 | 3,0 | 0,35 | 7,3 | 0,35 | 3,2 | 0,35 | 4,4 |
| 0,4 | 3,0 | 0,4 | 7,3 | 0,4 | 3,2 | 0,4 | 4,4 |
| 0,45 | 3,0 | 0,45 | 7,3 | 0,45 | 3,2 | 0,45 | 4,4 |
| 0,5 | 3,0 | 0,5 | 7,3 | 0,5 | 3,2 | 0,5 | 4,4 |
| 0,55 | 3,0 | 0,55 | 7,3 | 0,55 | 3,2 | 0,55 | 4,4 |
| 0,6 | 3,0 | 0,6 | 7,3 | 0,6 | 3,2 | 0,6 | 4,4 |
| 0,65 | 3,0 | 0,65 | 7,3 | 0,65 | 3,2 | 0,65 | 4,4 |
| 0,7 | 3,0 | 0,7 | 7,3 | 0,7 | 3,2 | 0,7 | 4,4 |
| 0,75 | 2,9 | 0,75 | 7,1 | 0,75 | 3,1 | 0,75 | 4,3 |
| 0,8 | 2,8 | 0,8 | 6,8 | 0,8 | 3,0 | 0,8 | 4,1 |
| 0,85 | 2,6 | 0,85 | 6,3 | 0,85 | 2,8 | 0,85 | 3,7 |
| 0,9 | 2,3 | 0,9 | 5,3 | 0,9 | 2,4 | 0,9 | 3,2 |
| 0,95 | 1,8 | 0,95 | 3,7 | 0,95 | 1,9 | 0,95 | 2,4 |
| 1 | 1,2 | 1 | 1,2 | 1 | 1,2 | 1 | 1,2 |

**HF - HFO-1234f - HFO-1234zeE - HFC-245cb**

| Organics 0,9 F1234yf + 0,05 F1234zeE +0,05 F245cb | | Organics 0,4 F1234yf + 0,3 F1234zeE +0,3 F245cb | | Organics 0,05 F1234yf + 0,9 F1234zeE +0,05 F245cb | | Organics 0,05 F1234yf + 0,05 F1234zeE +0,9 F245cb | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,6 | 0 | 5,6 | 0 | 5,0 | 0 | 4,8 |
| 0,05 | 7,6 | 0,05 | 6,7 | 0,05 | 5,9 | 0,05 | 6,0 |
| 0,1 | 7,6 | 0,1 | 6,7 | 0,1 | 6,0 | 0,1 | 6,0 |
| 0,15 | 7,6 | 0,15 | 6,7 | 0,15 | 6,0 | 0,15 | 6,0 |
| 0,2 | 7,6 | 0,2 | 6,7 | 0,2 | 6,0 | 0,2 | 6,0 |
| 0,25 | 7,6 | 0,25 | 6,7 | 0,25 | 6,0 | 0,25 | 6,0 |
| 0,3 | 7,6 | 0,3 | 6,7 | 0,3 | 6,0 | 0,3 | 6,0 |
| 0,35 | 7,6 | 0,35 | 6,7 | 0,35 | 6,0 | 0,35 | 6,0 |
| 0,4 | 7,6 | 0,4 | 6,7 | 0,4 | 6,0 | 0,4 | 6,0 |
| 0,45 | 7,6 | 0,45 | 6,7 | 0,45 | 5,9 | 0,45 | 6,0 |
| 0,5 | 7,6 | 0,5 | 6,7 | 0,5 | 5,9 | 0,5 | 6,0 |
| 0,55 | 7,6 | 0,55 | 6,7 | 0,55 | 5,9 | 0,55 | 5,9 |
| 0,6 | 7,6 | 0,6 | 6,7 | 0,6 | 5,8 | 0,6 | 5,9 |
| 0,65 | 7,6 | 0,65 | 6,7 | 0,65 | 5,7 | 0,65 | 5,9 |
| 0,7 | 7,6 | 0,7 | 6,7 | 0,7 | 5,6 | 0,7 | 5,9 |
| 0,75 | 7,4 | 0,75 | 6,6 | 0,75 | 5,3 | 0,75 | 5,9 |
| 0,8 | 7,1 | 0,8 | 6,3 | 0,8 | 5,0 | 0,8 | 5,9 |
| 0,85 | 6,5 | 0,85 | 5,7 | 0,85 | 4,5 | 0,85 | 5,8 |
| 0,9 | 5,5 | 0,9 | 4,8 | 0,9 | 3,8 | 0,9 | 5,0 |
| 0,95 | 3,8 | 0,95 | 3,4 | 0,95 | 2,7 | 0,95 | 3,5 |
| 1 | 1,2 | 1 | 1,2 | 1 | 1,2 | 1 | 1,2 |

**HF - HFO-1234f - HFO-1234zeZ - HFC-245cb**

| Organics 0,9 F1234yf + 0,05 F1234zeZ +0,05 F245cb | | Organics 0,4 F1234yf + 0,3 F1234zeZ +0,3 F245cb | | Organics 0,05 F1234yf + 0,9 F1234zeZ +0,05 F245cb | | Organics 0,05 F1234yf + 0,05 F1234zeZ +0,9 F245cb | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,4 | 0 | 4,6 | 0 | 2,2 | 0 | 4,6 |
| 0,05 | 7,4 | 0,05 | 5,7 | 0,05 | 3,3 | 0,05 | 5,8 |
| 0,1 | 7,4 | 0,1 | 5,7 | 0,1 | 3,3 | 0,1 | 5,8 |
| 0,15 | 7,4 | 0,15 | 5,7 | 0,15 | 3,3 | 0,15 | 5,8 |
| 0,2 | 7,4 | 0,2 | 5,7 | 0,2 | 3,3 | 0,2 | 5,8 |
| 0,25 | 7,4 | 0,25 | 5,7 | 0,25 | 3,3 | 0,25 | 5,8 |
| 0,3 | 7,4 | 0,3 | 5,7 | 0,3 | 3,3 | 0,3 | 5,8 |
| 0,35 | 7,4 | 0,35 | 5,7 | 0,35 | 3,3 | 0,35 | 5,8 |
| 0,4 | 7,4 | 0,4 | 5,8 | 0,4 | 3,3 | 0,4 | 5,8 |
| 0,45 | 7,4 | 0,45 | 5,8 | 0,45 | 3,3 | 0,45 | 5,8 |
| 0,5 | 7,4 | 0,5 | 5,8 | 0,5 | 3,3 | 0,5 | 5,8 |
| 0,55 | 7,4 | 0,55 | 5,8 | 0,55 | 3,3 | 0,55 | 5,8 |
| 0,6 | 7,4 | 0,6 | 5,8 | 0,6 | 3,3 | 0,6 | 5,8 |
| 0,65 | 7,4 | 0,65 | 5,8 | 0,65 | 3,3 | 0,65 | 5,8 |
| 0,7 | 7,4 | 0,7 | 5,8 | 0,7 | 3,3 | 0,7 | 5,8 |
| 0,75 | 7,3 | 0,75 | 5,8 | 0,75 | 3,3 | 0,75 | 5,8 |
| 0,8 | 7,0 | 0,8 | 5,6 | 0,8 | 3,2 | 0,8 | 5,8 |
| 0,85 | 6,4 | 0,85 | 5,2 | 0,85 | 3,0 | 0,85 | 5,7 |
| 0,9 | 5,4 | 0,9 | 4,4 | 0,9 | 2,6 | 0,9 | 4,9 |
| 0,95 | 3,8 | 0,95 | 3,2 | 0,95 | 2,0 | 0,95 | 3,5 |
| 1 | 1,2 | 1 | 1,2 | 1 | 1,2 | 1 | 1,2 |

**HF - HFO-1234f - HFO-1234zeE - HFO-1234zeZ**

| Organics 0,9 F1234yf + 0,05 F1234zeE +0,05 F1234zeZ | | Organics 0,05 F1234yf + 0,9 F1234zeE +0,05 F1234zeZ | | Organics 0,05 F1234yf + 0,05 F1234zeE +0,9 F1234zeZ | | Organics 0,4 F1234yf + 0,3 F1234zeE +0,3 F1234zeZ | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,4 | 0 | 4,8 | 0 | 2,2 | 0 | 4,7 |
| 0,05 | 7,4 | 0,05 | 5,8 | 0,05 | 3,3 | 0,05 | 5,7 |
| 0,1 | 7,4 | 0,1 | 5,8 | 0,1 | 3,3 | 0,1 | 5,7 |
| 0,15 | 7,4 | 0,15 | 5,8 | 0,15 | 3,3 | 0,15 | 5,7 |
| 0,2 | 7,4 | 0,2 | 5,8 | 0,2 | 3,3 | 0,2 | 5,7 |
| 0,25 | 7,4 | 0,25 | 5,8 | 0,25 | 3,3 | 0,25 | 5,8 |
| 0,3 | 7,4 | 0,3 | 5,8 | 0,3 | 3,3 | 0,3 | 5,8 |
| 0,35 | 7,4 | 0,35 | 5,8 | 0,35 | 3,3 | 0,35 | 5,8 |
| 0,4 | 7,4 | 0,4 | 5,8 | 0,4 | 3,3 | 0,4 | 5,8 |
| 0,45 | 7,4 | 0,45 | 5,8 | 0,45 | 3,3 | 0,45 | 5,8 |
| 0,5 | 7,4 | 0,5 | 5,7 | 0,5 | 3,3 | 0,5 | 5,8 |
| 0,55 | 7,4 | 0,55 | 5,7 | 0,55 | 3,3 | 0,55 | 5,8 |
| 0,6 | 7,4 | 0,6 | 5,6 | 0,6 | 3,3 | 0,6 | 5,8 |
| 0,65 | 7,4 | 0,65 | 5,5 | 0,65 | 3,3 | 0,65 | 5,7 |
| 0,7 | 7,4 | 0,7 | 5,4 | 0,7 | 3,3 | 0,7 | 5,6 |
| 0,75 | 7,2 | 0,75 | 5,2 | 0,75 | 3,3 | 0,75 | 5,5 |
| 0,8 | 6,9 | 0,8 | 4,8 | 0,8 | 3,1 | 0,8 | 5,2 |
| 0,85 | 6,3 | 0,85 | 4,3 | 0,85 | 2,9 | 0,85 | 4,7 |
| 0,9 | 5,3 | 0,9 | 3,6 | 0,9 | 2,5 | 0,9 | 4,0 |
| 0,95 | 3,7 | 0,95 | 2,6 | 0,95 | 2,0 | 0,95 | 2,9 |
| 1 | 1,2 | 1 | 1,2 | 1 | 1,2 | 1 | 1,2 |

**HF - HFO-1234f - HFO-1234zeE - HCFO-1233zdE**

| Organics 0,9 F1234yf + 0,05 F1234zeE +0,05 F1233zdE | | Organics 0,05 F1234yf + 0,9 F1234zeE +0,05 F1233zdE | | Organics 0,05 F1234yf + 0,05 F1234zeE +0,9 F1233zdE | | Organics 0,4 F1234yf + 0,3 F1234zeE +0,3 F1233zdE | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,4 | 0 | 4,8 | 0 | 1,8 | 0 | 4,7 |
| 0,05 | 7,4 | 0,05 | 5,8 | 0,05 | 2,9 | 0,05 | 5,7 |
| 0,1 | 7,4 | 0,1 | 5,8 | 0,1 | 2,9 | 0,1 | 5,7 |
| 0,15 | 7,4 | 0,15 | 5,8 | 0,15 | 2,9 | 0,15 | 5,7 |
| 0,2 | 7,4 | 0,2 | 5,8 | 0,2 | 2,9 | 0,2 | 5,7 |
| 0,25 | 7,4 | 0,25 | 5,8 | 0,25 | 2,9 | 0,25 | 5,7 |
| 0,3 | 7,4 | 0,3 | 5,8 | 0,3 | 2,9 | 0,3 | 5,7 |
| 0,35 | 7,4 | 0,35 | 5,8 | 0,35 | 2,9 | 0,35 | 5,7 |
| 0,4 | 7,4 | 0,4 | 5,8 | 0,4 | 2,9 | 0,4 | 5,7 |
| 0,45 | 7,4 | 0,45 | 5,8 | 0,45 | 2,9 | 0,45 | 5,7 |
| 0,5 | 7,4 | 0,5 | 5,7 | 0,5 | 2,9 | 0,5 | 5,7 |
| 0,55 | 7,4 | 0,55 | 5,7 | 0,55 | 2,9 | 0,55 | 5,7 |
| 0,6 | 7,4 | 0,6 | 5,6 | 0,6 | 2,9 | 0,6 | 5,7 |
| 0,65 | 7,4 | 0,65 | 5,5 | 0,65 | 2,9 | 0,65 | 5,7 |
| 0,7 | 7,4 | 0,7 | 5,4 | 0,7 | 2,9 | 0,7 | 5,6 |
| 0,75 | 7,2 | 0,75 | 5,1 | 0,75 | 2,9 | 0,75 | 5,4 |
| 0,8 | 6,9 | 0,8 | 4,8 | 0,8 | 2,7 | 0,8 | 5,1 |
| 0,85 | 6,3 | 0,85 | 4,3 | 0,85 | 2,5 | 0,85 | 4,7 |
| 0,9 | 5,3 | 0,9 | 3,6 | 0,9 | 2,2 | 0,9 | 3,9 |
| 0,95 | 3,7 | 0,95 | 2,6 | 0,95 | 1,8 | 0,95 | 2,8 |
| 1 | 1,2 | 1 | 1,2 | 1 | 1,2 | 1 | 1,2 |

**HF - HFO-1234f - HFO-1234zeE - HFO-1243zf**

| Organics 0,9 F1234yf + 0,05 F1234zeE +0,05 F1243zf | | Organics 0,05 F1234yf + 0,9 F1234zeE +0,05 F1243zf | | Organics 0,05 F1234yf + 0,05 F1234zeE +0,9 F1243zf | | Organics 0,4 F1234yf + 0,3 F1234zeE +0,3 F1243zf | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,7 | 0 | 5,0 | 0 | 5,8 | 0 | 5,9 |
| 0,05 | 7,6 | 0,05 | 6,0 | 0,05 | 6,8 | 0,05 | 6,9 |
| 0,1 | 7,6 | 0,1 | 6,0 | 0,1 | 6,8 | 0,1 | 6,9 |
| 0,15 | 7,6 | 0,15 | 6,0 | 0,15 | 6,8 | 0,15 | 6,9 |
| 0,2 | 7,6 | 0,2 | 6,0 | 0,2 | 6,8 | 0,2 | 6,9 |
| 0,25 | 7,6 | 0,25 | 6,0 | 0,25 | 6,8 | 0,25 | 6,9 |
| 0,3 | 7,6 | 0,3 | 6,0 | 0,3 | 6,8 | 0,3 | 6,9 |
| 0,35 | 7,6 | 0,35 | 6,0 | 0,35 | 6,8 | 0,35 | 6,9 |
| 0,4 | 7,6 | 0,4 | 6,0 | 0,4 | 6,8 | 0,4 | 6,9 |
| 0,45 | 7,6 | 0,45 | 6,0 | 0,45 | 6,8 | 0,45 | 6,9 |
| 0,5 | 7,6 | 0,5 | 6,0 | 0,5 | 6,8 | 0,5 | 6,9 |
| 0,55 | 7,6 | 0,55 | 5,9 | 0,55 | 6,8 | 0,55 | 6,9 |
| 0,6 | 7,6 | 0,6 | 5,8 | 0,6 | 6,7 | 0,6 | 6,9 |
| 0,65 | 7,6 | 0,65 | 5,7 | 0,65 | 6,7 | 0,65 | 6,8 |
| 0,7 | 7,6 | 0,7 | 5,6 | 0,7 | 6,6 | 0,7 | 6,7 |
| 0,75 | 7,4 | 0,75 | 5,4 | 0,75 | 6,4 | 0,75 | 6,5 |
| 0,8 | 7,1 | 0,8 | 5,0 | 0,8 | 6,1 | 0,8 | 6,2 |
| 0,85 | 6,5 | 0,85 | 4,5 | 0,85 | 5,6 | 0,85 | 5,7 |
| 0,9 | 5,5 | 0,9 | 3,8 | 0,9 | 4,8 | 0,9 | 4,8 |
| 0,95 | 3,8 | 0,95 | 2,7 | 0,95 | 3,4 | 0,95 | 3,3 |
| 1 | 1,2 | 1 | 1,2 | 1 | 1,2 | 1 | 1,2 |

**HF - HFO-1234f - HFO-1234zeZ - HCFO-1233zdE**

| Organics 0,9 F1234yf + 0,05 F1234zeZ +0,05 F1233zdE | | Organics 0,05 F1234yf + 0,9 F1234zeZ +0,05 F1233zdE | | Organics 0,05 F1234yf + 0,05 F1234zeZ +0,9 F1233zdE | | Organics 0,4 F1234yf + 0,3 F1234zeZ +0,3 F1233zdE | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,3 | 0 | 2,0 | 0 | 1,7 | 0 | 3,8 |
| 0,05 | 7,3 | 0,05 | 3,2 | 0,05 | 2,8 | 0,05 | 4,8 |
| 0,1 | 7,3 | 0,1 | 3,2 | 0,1 | 2,8 | 0,1 | 4,8 |
| 0,15 | 7,3 | 0,15 | 3,2 | 0,15 | 2,8 | 0,15 | 4,8 |
| 0,2 | 7,3 | 0,2 | 3,2 | 0,2 | 2,8 | 0,2 | 4,8 |
| 0,25 | 7,3 | 0,25 | 3,2 | 0,25 | 2,8 | 0,25 | 4,8 |
| 0,3 | 7,3 | 0,3 | 3,2 | 0,3 | 2,8 | 0,3 | 4,8 |
| 0,35 | 7,3 | 0,35 | 3,1 | 0,35 | 2,8 | 0,35 | 4,8 |
| 0,4 | 7,3 | 0,4 | 3,1 | 0,4 | 2,8 | 0,4 | 4,8 |
| 0,45 | 7,3 | 0,45 | 3,1 | 0,45 | 2,8 | 0,45 | 4,8 |
| 0,5 | 7,3 | 0,5 | 3,1 | 0,5 | 2,8 | 0,5 | 4,8 |
| 0,55 | 7,3 | 0,55 | 3,1 | 0,55 | 2,8 | 0,55 | 4,8 |
| 0,6 | 7,3 | 0,6 | 3,1 | 0,6 | 2,8 | 0,6 | 4,8 |
| 0,65 | 7,3 | 0,65 | 3,1 | 0,65 | 2,8 | 0,65 | 4,8 |
| 0,7 | 7,3 | 0,7 | 3,1 | 0,7 | 2,7 | 0,7 | 4,8 |
| 0,75 | 7,1 | 0,75 | 3,1 | 0,75 | 2,7 | 0,75 | 4,7 |
| 0,8 | 6,8 | 0,8 | 3,0 | 0,8 | 2,6 | 0,8 | 4,5 |
| 0,85 | 6,2 | 0,85 | 2,8 | 0,85 | 2,4 | 0,85 | 4,1 |
| 0,9 | 5,3 | 0,9 | 2,4 | 0,9 | 2,2 | 0,9 | 3,5 |
| 0,95 | 3,7 | 0,95 | 1,9 | 0,95 | 1,7 | 0,95 | 2,6 |
| 1 | 1,2 | 1 | 1,2 | 1 | 1,2 | 1 | 1,2 |

**HF - HFO-1234yf - HFO-1234zeZ - HFO-1243zf**

| Organics 0,9 F1234yf + 0,05 F1234zeZ +0,05 F1243zf | | Organics 0,05 F1234yf + 0,9 F1234zeZ +0,05 F1243zf | | Organics 0,05 F1234yf + 0,05 F1234zeZ +0,9 F1243zf | | Organics 0,4 F1234yf + 0,3 F1234zeZ +0,3 F1243zf | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 6,5 | 0 | 2,3 | 0 | 5,7 | 0 | 5,0 |
| 0,05 | 7,5 | 0,05 | 3,4 | 0,05 | 6,7 | 0,05 | 6,0 |
| 0,1 | 7,5 | 0,1 | 3,4 | 0,1 | 6,7 | 0,1 | 6,1 |
| 0,15 | 7,5 | 0,15 | 3,4 | 0,15 | 6,7 | 0,15 | 6,1 |
| 0,2 | 7,4 | 0,2 | 3,4 | 0,2 | 6,7 | 0,2 | 6,1 |
| 0,25 | 7,4 | 0,25 | 3,4 | 0,25 | 6,7 | 0,25 | 6,1 |
| 0,3 | 7,4 | 0,3 | 3,4 | 0,3 | 6,7 | 0,3 | 6,1 |
| 0,35 | 7,5 | 0,35 | 3,4 | 0,35 | 6,7 | 0,35 | 6,1 |
| 0,4 | 7,5 | 0,4 | 3,4 | 0,4 | 6,7 | 0,4 | 6,1 |
| 0,45 | 7,5 | 0,45 | 3,4 | 0,45 | 6,7 | 0,45 | 6,1 |
| 0,5 | 7,5 | 0,5 | 3,4 | 0,5 | 6,7 | 0,5 | 6,1 |
| 0,55 | 7,5 | 0,55 | 3,4 | 0,55 | 6,6 | 0,55 | 6,1 |
| 0,6 | 7,5 | 0,6 | 3,4 | 0,6 | 6,6 | 0,6 | 6,1 |
| 0,65 | 7,5 | 0,65 | 3,4 | 0,65 | 6,6 | 0,65 | 6,1 |
| 0,7 | 7,4 | 0,7 | 3,4 | 0,7 | 6,5 | 0,7 | 6,0 |
| 0,75 | 7,3 | 0,75 | 3,3 | 0,75 | 6,3 | 0,75 | 5,9 |
| 0,8 | 7,0 | 0,8 | 3,2 | 0,8 | 6,0 | 0,8 | 5,6 |
| 0,85 | 6,4 | 0,85 | 3,0 | 0,85 | 5,5 | 0,85 | 5,1 |
| 0,9 | 5,4 | 0,9 | 2,6 | 0,9 | 4,7 | 0,9 | 4,4 |
| 0,95 | 3,7 | 0,95 | 2,0 | 0,95 | 3,3 | 0,95 | 3,1 |
| 1 | 1,2 | 1 | 1,2 | 1 | 1,2 | 1 | 1,2 |

**HF - HFC-245cb - HCFO-1233zdE - HFO-1234zeE**

| Organics 0,9 F245cb + 0,05 F1233zdE +0,05 F1234zeE | | Organics 0,05 F245cb + 0,9 F1233zdE +0,05 F1234zeE | | Organics 0,05 F245cb + 0,05 F1233zdE +0,9 F1234zeE | | Organics 0,4 F245cb + 0,3 F1233zdE +0,3 F1234zeE | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 4,5 | 0 | 1,7 | 0 | 4,7 | 0 | 3,8 |
| 0,05 | 5,7 | 0,05 | 2,8 | 0,05 | 5,7 | 0,05 | 4,9 |
| 0,1 | 5,7 | 0,1 | 2,8 | 0,1 | 5,7 | 0,1 | 4,9 |
| 0,15 | 5,7 | 0,15 | 2,8 | 0,15 | 5,7 | 0,15 | 4,9 |
| 0,2 | 5,7 | 0,2 | 2,8 | 0,2 | 5,7 | 0,2 | 4,9 |
| 0,25 | 5,7 | 0,25 | 2,8 | 0,25 | 5,7 | 0,25 | 4,9 |
| 0,3 | 5,7 | 0,3 | 2,8 | 0,3 | 5,7 | 0,3 | 4,9 |
| 0,35 | 5,7 | 0,35 | 2,8 | 0,35 | 5,7 | 0,35 | 4,9 |
| 0,4 | 5,7 | 0,4 | 2,8 | 0,4 | 5,7 | 0,4 | 4,9 |
| 0,45 | 5,7 | 0,45 | 2,8 | 0,45 | 5,7 | 0,45 | 4,9 |
| 0,5 | 5,7 | 0,5 | 2,8 | 0,5 | 5,7 | 0,5 | 4,9 |
| 0,55 | 5,7 | 0,55 | 2,8 | 0,55 | 5,6 | 0,55 | 4,9 |
| 0,6 | 5,7 | 0,6 | 2,8 | 0,6 | 5,5 | 0,6 | 4,9 |
| 0,65 | 5,7 | 0,65 | 2,8 | 0,65 | 5,5 | 0,65 | 4,9 |
| 0,7 | 5,7 | 0,7 | 2,8 | 0,7 | 5,3 | 0,7 | 4,9 |
| 0,75 | 5,7 | 0,75 | 2,8 | 0,75 | 5,1 | 0,75 | 4,9 |
| 0,8 | 5,7 | 0,8 | 2,7 | 0,8 | 4,8 | 0,8 | 4,7 |
| 0,85 | 5,6 | 0,85 | 2,5 | 0,85 | 4,3 | 0,85 | 4,3 |
| 0,9 | 4,8 | 0,9 | 2,2 | 0,9 | 3,6 | 0,9 | 3,7 |
| 0,95 | 3,4 | 0,95 | 1,8 | 0,95 | 2,6 | 0,95 | 2,7 |
| 1 | 1,2 | 1 | 1,2 | 1 | 1,2 | 1 | 1,2 |

**HF - HFC-245cb - HCFO-1233zdE - HFO-1234zeZ**

| Organics 0,9 F245cb + 0,05 F1233zdE +0,05 F1234zeZ | | Organics 0,05 F245cb + 0,9 F1233zdE +0,05 F1234zeZ | | Organics 0,05 F245cb + 0,05 F1233zdE +0,9 F1234zeZ | | Organics 0,4 F245cb + 0,3 F1233zdE +0,3 F1234zeZ | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 4,3 | 0 | 1,5 | 0 | 1,9 | 0 | 2,8 |
| 0,05 | 5,5 | 0,05 | 2,6 | 0,05 | 3,0 | 0,05 | 3,9 |
| 0,1 | 5,5 | 0,1 | 2,6 | 0,1 | 3,0 | 0,1 | 3,9 |
| 0,15 | 5,5 | 0,15 | 2,6 | 0,15 | 3,0 | 0,15 | 3,9 |
| 0,2 | 5,5 | 0,2 | 2,6 | 0,2 | 3,0 | 0,2 | 3,9 |
| 0,25 | 5,5 | 0,25 | 2,6 | 0,25 | 3,0 | 0,25 | 3,9 |
| 0,3 | 5,5 | 0,3 | 2,6 | 0,3 | 3,0 | 0,3 | 4,0 |
| 0,35 | 5,5 | 0,35 | 2,6 | 0,35 | 3,0 | 0,35 | 4,0 |
| 0,4 | 5,5 | 0,4 | 2,6 | 0,4 | 3,0 | 0,4 | 4,0 |
| 0,45 | 5,5 | 0,45 | 2,6 | 0,45 | 3,0 | 0,45 | 4,0 |
| 0,5 | 5,5 | 0,5 | 2,6 | 0,5 | 3,0 | 0,5 | 4,0 |
| 0,55 | 5,5 | 0,55 | 2,6 | 0,55 | 3,0 | 0,55 | 4,0 |
| 0,6 | 5,5 | 0,6 | 2,6 | 0,6 | 3,0 | 0,6 | 4,0 |
| 0,65 | 5,5 | 0,65 | 2,7 | 0,65 | 3,1 | 0,65 | 4,0 |
| 0,7 | 5,6 | 0,7 | 2,7 | 0,7 | 3,1 | 0,7 | 4,1 |
| 0,75 | 5,6 | 0,75 | 2,7 | 0,75 | 3,1 | 0,75 | 4,1 |
| 0,8 | 5,6 | 0,8 | 2,6 | 0,8 | 2,9 | 0,8 | 4,1 |
| 0,85 | 5,5 | 0,85 | 2,4 | 0,85 | 2,7 | 0,85 | 3,8 |
| 0,9 | 4,8 | 0,9 | 2,1 | 0,9 | 2,4 | 0,9 | 3,3 |
| 0,95 | 3,4 | 0,95 | 1,7 | 0,95 | 1,9 | 0,95 | 2,5 |
| 1 | 1,2 | 1 | 1,2 | 1 | 1,2 | 1 | 1,2 |

**HF - HCFO-1233zdE - HFO-1234zeE - HFO-1234zeZ**

| Organics 0,9 1233zdE + 0,05 1234zeE +0,05 1234zeZ | | Organics 0,051233zdE + 0,9 1234zeE +0,05 1234zeZ | | Organics 0,05 1233zdE + 0,05 1234zeE +0,9 1234zeZ | | Organics 0,4 1233zdE + 0,3 1234zeE +0,3 1234zeZ | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 1,5 | 0 | 4,5 | 0 | 1,9 | 0 | 2,6 |
| 0,05 | 2,6 | 0,05 | 5,5 | 0,05 | 3,0 | 0,05 | 3,7 |
| 0,1 | 2,6 | 0,1 | 5,5 | 0,1 | 3,0 | 0,1 | 3,6 |
| 0,15 | 2,6 | 0,15 | 5,5 | 0,15 | 3,0 | 0,15 | 3,6 |
| 0,2 | 2,6 | 0,2 | 5,5 | 0,2 | 3,0 | 0,2 | 3,6 |
| 0,25 | 2,6 | 0,25 | 5,5 | 0,25 | 3,0 | 0,25 | 3,6 |
| 0,3 | 2,6 | 0,3 | 5,5 | 0,3 | 3,0 | 0,3 | 3,6 |
| 0,35 | 2,6 | 0,35 | 5,5 | 0,35 | 3,0 | 0,35 | 3,6 |
| 0,4 | 2,6 | 0,4 | 5,5 | 0,4 | 3,0 | 0,4 | 3,6 |
| 0,45 | 2,6 | 0,45 | 5,5 | 0,45 | 3,0 | 0,45 | 3,6 |
| 0,5 | 2,6 | 0,5 | 5,5 | 0,5 | 3,0 | 0,5 | 3,6 |
| 0,55 | 2,6 | 0,55 | 5,4 | 0,55 | 3,0 | 0,55 | 3,6 |
| 0,6 | 2,6 | 0,6 | 5,4 | 0,6 | 3,0 | 0,6 | 3,6 |
| 0,65 | 2,6 | 0,65 | 5,3 | 0,65 | 3,0 | 0,65 | 3,5 |
| 0,7 | 2,6 | 0,7 | 5,1 | 0,7 | 3,0 | 0,7 | 3,5 |
| 0,75 | 2,6 | 0,75 | 4,9 | 0,75 | 3,0 | 0,75 | 3,4 |
| 0,8 | 2,5 | 0,8 | 4,6 | 0,8 | 2,9 | 0,8 | 3,2 |
| 0,85 | 2,3 | 0,85 | 4,1 | 0,85 | 2,7 | 0,85 | 2,9 |
| 0,9 | 2,0 | 0,9 | 3,4 | 0,9 | 2,3 | 0,9 | 2,5 |
| 0,95 | 1,7 | 0,95 | 2,5 | 0,95 | 1,9 | 0,95 | 2,0 |
| 1 | 1,2 | 1 | 1,2 | 1 | 1,2 | 1 | 1,2 |

**HF - HCFO-1233zdE - HFO-1234zeE - HFO-1243zf**

| Organics 0,9 F1233zdE + 0,05 F1234zeE +0,05 F1243zf | | Organics 0,05 F1233zdE + 0,9 F1234zeE +0,05 F1243zf | | Organics 0,05 F1233zdE + 0,05 F1234zeE +0,9 F1243zf | | Organics 0,4 F1233zdE + 0,3 F1234zeE +0,3 F1243zf | |
|---|---|---|---|---|---|---|---|
| HF | PRESSURE | HF | PRESSURE | HF | PRESSURE | HF | PRESSURE |
| HF | PRES | HF | PRES | HF | PRES | HF | PRES |
| | bar | | bar | | bar | | bar |
| 0 | 1,8 | 0 | 4,8 | 0 | 5,6 | 0 | 3,9 |
| 0,05 | 2,9 | 0,05 | 5,7 | 0,05 | 6,6 | 0,05 | 5,0 |
| 0,1 | 2,9 | 0,1 | 5,7 | 0,1 | 6,6 | 0,1 | 5,0 |
| 0,15 | 2,9 | 0,15 | 5,7 | 0,15 | 6,6 | 0,15 | 5,0 |
| 0,2 | 2,9 | 0,2 | 5,7 | 0,2 | 6,6 | 0,2 | 5,0 |
| 0,25 | 2,9 | 0,25 | 5,7 | 0,25 | 6,6 | 0,25 | 5,0 |
| 0,3 | 2,9 | 0,3 | 5,7 | 0,3 | 6,6 | 0,3 | 5,0 |
| 0,35 | 2,9 | 0,35 | 5,7 | 0,35 | 6,6 | 0,35 | 5,0 |
| 0,4 | 2,9 | 0,4 | 5,7 | 0,4 | 6,6 | 0,4 | 4,9 |
| 0,45 | 2,9 | 0,45 | 5,7 | 0,45 | 6,6 | 0,45 | 4,9 |
| 0,5 | 2,9 | 0,5 | 5,7 | 0,5 | 6,6 | 0,5 | 4,9 |
| 0,55 | 2,9 | 0,55 | 5,6 | 0,55 | 6,5 | 0,55 | 4,9 |
| 0,6 | 2,9 | 0,6 | 5,6 | 0,6 | 6,5 | 0,6 | 4,9 |
| 0,65 | 2,9 | 0,65 | 5,5 | 0,65 | 6,5 | 0,65 | 4,9 |
| 0,7 | 2,8 | 0,7 | 5,3 | 0,7 | 6,4 | 0,7 | 4,8 |
| 0,75 | 2,8 | 0,75 | 5,1 | 0,75 | 6,2 | 0,75 | 4,6 |
| 0,8 | 2,7 | 0,8 | 4,8 | 0,8 | 5,9 | 0,8 | 4,3 |
| 0,85 | 2,5 | 0,85 | 4,3 | 0,85 | 5,4 | 0,85 | 3,9 |
| 0,9 | 2,2 | 0,9 | 3,6 | 0,9 | 4,6 | 0,9 | 3,3 |
| 0,95 | 1,8 | 0,95 | 2,6 | 0,95 | 3,3 | 0,95 | 2,5 |
| 1 | 1,2 | 1 | 1,2 | 1 | 1,2 | 1 | 1,2 |

**HF - HCFO-1233zdE - HFO-1234zeZ - HFO-1243zf**

| Organics 0,9 F1233zdE + 0,05 F1234zeZ +0,05 F1243zf | | Organics 0,05 F1233zdE + 0,9 F1234zeZ +0,05 F1243zf | | Organics 0,05 F1233zdE + 0,05 F1234zeZ +0,9 F1243zf | | Organics 0,4 F1233zdE + 0,3 F1234zeZ +0,3 F1243zf | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 1,6 | 0 | 2,0 | 0 | 5,5 | 0 | 3,0 |
| 0,05 | 2,7 | 0,05 | 3,1 | 0,05 | 6,4 | 0,05 | 4,1 |
| 0,1 | 2,7 | 0,1 | 3,1 | 0,1 | 6,5 | 0,1 | 4,1 |
| 0,15 | 2,7 | 0,15 | 3,1 | 0,15 | 6,5 | 0,15 | 4,1 |
| 0,2 | 2,7 | 0,2 | 3,1 | 0,2 | 6,5 | 0,2 | 4,1 |
| 0,25 | 2,7 | 0,25 | 3,1 | 0,25 | 6,5 | 0,25 | 4,1 |
| 0,3 | 2,7 | 0,3 | 3,1 | 0,3 | 6,4 | 0,3 | 4,1 |
| 0,35 | 2,7 | 0,35 | 3,1 | 0,35 | 6,4 | 0,35 | 4,1 |
| 0,4 | 2,7 | 0,4 | 3,1 | 0,4 | 6,4 | 0,4 | 4,1 |
| 0,45 | 2,7 | 0,45 | 3,1 | 0,45 | 6,4 | 0,45 | 4,1 |
| 0,5 | 2,7 | 0,5 | 3,1 | 0,5 | 6,4 | 0,5 | 4,1 |
| 0,55 | 2,7 | 0,55 | 3,1 | 0,55 | 6,4 | 0,55 | 4,1 |
| 0,6 | 2,7 | 0,6 | 3,1 | 0,6 | 6,4 | 0,6 | 4,1 |
| 0,65 | 2,7 | 0,65 | 3,1 | 0,65 | 6,3 | 0,65 | 4,0 |
| 0,7 | 2,7 | 0,7 | 3,1 | 0,7 | 6,3 | 0,7 | 4,0 |
| 0,75 | 2,7 | 0,75 | 3,1 | 0,75 | 6,1 | 0,75 | 3,9 |
| 0,8 | 2,6 | 0,8 | 3,0 | 0,8 | 5,8 | 0,8 | 3,7 |
| 0,85 | 2,4 | 0,85 | 2,7 | 0,85 | 5,3 | 0,85 | 3,4 |
| 0,9 | 2,1 | 0,9 | 2,4 | 0,9 | 4,5 | 0,9 | 2,9 |
| 0,95 | 1,7 | 0,95 | 1,9 | 0,95 | 3,2 | 0,95 | 2,2 |
| 1 | 1,2 | 1 | 1,2 | 1 | 1,2 | 1 | 1,2 |

**HF - HFC-245cb - HFO-1234zeE - HFO-1234zeZ**

| Organics 0,9 F245cb + 0,05 F1234zeE +0,05 F1234zeZ | | Organics 0,05 F245cb + 0,9 F1234zeE +0,05 F1234zeZ | | Organics 0,05 F245cb + 0,05 F1234zeE +0,9 F1234zeZ | | Organics 0,4 F245cb + 0,3 F1234zeE +0,3 F1234zeZ | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 4,5 | 0 | 4,7 | 0 | 2,1 | 0 | 3,8 |
| 0,05 | 5,7 | 0,05 | 5,7 | 0,05 | 3,2 | 0,05 | 4,9 |
| 0,1 | 5,7 | 0,1 | 5,7 | 0,1 | 3,2 | 0,1 | 4,9 |
| 0,15 | 5,7 | 0,15 | 5,7 | 0,15 | 3,2 | 0,15 | 4,9 |
| 0,2 | 5,7 | 0,2 | 5,7 | 0,2 | 3,2 | 0,2 | 4,9 |
| 0,25 | 5,7 | 0,25 | 5,7 | 0,25 | 3,2 | 0,25 | 4,9 |
| 0,3 | 5,7 | 0,3 | 5,7 | 0,3 | 3,2 | 0,3 | 4,9 |
| 0,35 | 5,7 | 0,35 | 5,7 | 0,35 | 3,2 | 0,35 | 5,0 |
| 0,4 | 5,7 | 0,4 | 5,7 | 0,4 | 3,2 | 0,4 | 5,0 |
| 0,45 | 5,7 | 0,45 | 5,7 | 0,45 | 3,2 | 0,45 | 5,0 |
| 0,5 | 5,7 | 0,5 | 5,7 | 0,5 | 3,2 | 0,5 | 5,0 |
| 0,55 | 5,7 | 0,55 | 5,6 | 0,55 | 3,2 | 0,55 | 5,0 |
| 0,6 | 5,7 | 0,6 | 5,6 | 0,6 | 3,2 | 0,6 | 5,0 |
| 0,65 | 5,7 | 0,65 | 5,5 | 0,65 | 3,2 | 0,65 | 5,0 |
| 0,7 | 5,7 | 0,7 | 5,3 | 0,7 | 3,2 | 0,7 | 5,0 |
| 0,75 | 5,7 | 0,75 | 5,1 | 0,75 | 3,2 | 0,75 | 5,0 |
| 0,8 | 5,7 | 0,8 | 4,8 | 0,8 | 3,1 | 0,8 | 4,8 |
| 0,85 | 5,6 | 0,85 | 4,3 | 0,85 | 2,8 | 0,85 | 4,4 |
| 0,9 | 4,8 | 0,9 | 3,6 | 0,9 | 2,5 | 0,9 | 3,8 |
| 0,95 | 3,4 | 0,95 | 2,6 | 0,95 | 2,0 | 0,95 | 2,7 |
| 1 | 1,2 | 1 | 1,2 | 1 | 1,2 | 1 | 1,2 |

**HF - HFC-245cb - HFO-1234zeE - HFO-1243zf**

| Organics 0,9 F245cb + 0,05 F1234zeE +0,05 F1243zf | | Organics 0,05 F245cb + 0,9 F1234zeE +0,05 F1243zf | | Organics 0,05 F245cb + 0,05 F1234zeE +0,9 F1243zf | | Organics 0,4 F245cb + 0,3 F1234zeE +0,3 F1243zf | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 4,8 | 0 | 4,9 | 0 | 5,8 | 0 | 5,2 |
| 0,05 | 5,9 | 0,05 | 5,9 | 0,05 | 6,7 | 0,05 | 6,2 |
| 0,1 | 5,9 | 0,1 | 5,9 | 0,1 | 6,7 | 0,1 | 6,2 |
| 0,15 | 5,9 | 0,15 | 5,9 | 0,15 | 6,7 | 0,15 | 6,2 |
| 0,2 | 5,9 | 0,2 | 5,9 | 0,2 | 6,7 | 0,2 | 6,2 |
| 0,25 | 5,9 | 0,25 | 5,9 | 0,25 | 6,7 | 0,25 | 6,2 |
| 0,3 | 5,9 | 0,3 | 5,9 | 0,3 | 6,7 | 0,3 | 6,2 |
| 0,35 | 5,9 | 0,35 | 5,9 | 0,35 | 6,7 | 0,35 | 6,2 |
| 0,4 | 5,9 | 0,4 | 5,9 | 0,4 | 6,7 | 0,4 | 6,2 |
| 0,45 | 5,9 | 0,45 | 5,9 | 0,45 | 6,7 | 0,45 | 6,2 |
| 0,5 | 5,9 | 0,5 | 5,9 | 0,5 | 6,7 | 0,5 | 6,2 |
| 0,55 | 5,9 | 0,55 | 5,8 | 0,55 | 6,7 | 0,55 | 6,2 |
| 0,6 | 5,9 | 0,6 | 5,8 | 0,6 | 6,7 | 0,6 | 6,2 |
| 0,65 | 5,9 | 0,65 | 5,7 | 0,65 | 6,6 | 0,65 | 6,2 |
| 0,7 | 5,9 | 0,7 | 5,5 | 0,7 | 6,5 | 0,7 | 6,2 |
| 0,75 | 5,9 | 0,75 | 5,3 | 0,75 | 6,4 | 0,75 | 6,1 |
| 0,8 | 5,9 | 0,8 | 5,0 | 0,8 | 6,1 | 0,8 | 5,8 |
| 0,85 | 5,8 | 0,85 | 4,5 | 0,85 | 5,6 | 0,85 | 5,3 |
| 0,9 | 5,0 | 0,9 | 3,7 | 0,9 | 4,7 | 0,9 | 4,5 |
| 0,95 | 3,5 | 0,95 | 2,7 | 0,95 | 3,4 | 0,95 | 3,2 |
| 1 | 1,2 | 1 | 1,2 | 1 | 1,2 | 1 | 1,2 |

**HF - HFC-245cb - HFO-1234zeZ - HFO-1243zf**

| Organics 0,9 F245cb + 0,05 F1234zeZ +0,05 F1243zf | | Organics 0,05 F245cb + 0,9 F1234zeZ +0,05 F1243zf | | Organics 0,05 F245cb + 0,05 F1234zeZ +0,9 F1243zf | | Organics 0,4 F245cb + 0,3 F1234zeZ +0,3 F1243zf | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 4,6 | 0 | 2,2 | 0 | 5,6 | 0 | 4,2 |
| 0,05 | 5,7 | 0,05 | 3,3 | 0,05 | 6,6 | 0,05 | 5,3 |
| 0,1 | 5,7 | 0,1 | 3,3 | 0,1 | 6,6 | 0,1 | 5,3 |
| 0,15 | 5,7 | 0,15 | 3,3 | 0,15 | 6,6 | 0,15 | 5,3 |
| 0,2 | 5,7 | 0,2 | 3,3 | 0,2 | 6,6 | 0,2 | 5,3 |
| 0,25 | 5,7 | 0,25 | 3,3 | 0,25 | 6,6 | 0,25 | 5,3 |
| 0,3 | 5,7 | 0,3 | 3,3 | 0,3 | 6,6 | 0,3 | 5,3 |
| 0,35 | 5,7 | 0,35 | 3,3 | 0,35 | 6,6 | 0,35 | 5,3 |
| 0,4 | 5,7 | 0,4 | 3,3 | 0,4 | 6,6 | 0,4 | 5,3 |
| 0,45 | 5,7 | 0,45 | 3,3 | 0,45 | 6,6 | 0,45 | 5,3 |
| 0,5 | 5,7 | 0,5 | 3,3 | 0,5 | 6,6 | 0,5 | 5,3 |
| 0,55 | 5,8 | 0,55 | 3,3 | 0,55 | 6,6 | 0,55 | 5,3 |
| 0,6 | 5,8 | 0,6 | 3,3 | 0,6 | 6,5 | 0,6 | 5,3 |
| 0,65 | 5,8 | 0,65 | 3,3 | 0,65 | 6,5 | 0,65 | 5,3 |
| 0,7 | 5,8 | 0,7 | 3,3 | 0,7 | 6,4 | 0,7 | 5,4 |
| 0,75 | 5,8 | 0,75 | 3,3 | 0,75 | 6,3 | 0,75 | 5,4 |
| 0,8 | 5,8 | 0,8 | 3,1 | 0,8 | 6,0 | 0,8 | 5,2 |
| 0,85 | 5,7 | 0,85 | 2,9 | 0,85 | 5,5 | 0,85 | 4,8 |
| 0,9 | 4,9 | 0,9 | 2,6 | 0,9 | 4,7 | 0,9 | 4,1 |
| 0,95 | 3,5 | 0,95 | 2,0 | 0,95 | 3,3 | 0,95 | 3,0 |
| 1 | 1,2 | 1 | 1,2 | 1 | 1,2 | 1 | 1,2 |

**HF - HFO-1234zeE - HFO-1234zeZ - HFO-1243zf**

| Organics 0,9 F1234zeE + 0,05 F1234zeZ +0,05 F1243zf | | Organics 0,05 F1234zeE + 0,9 F1234zeZ +0,05 F1243zf | | Organics 0,05 F1234zeE + 0,05 F1234zeZ +0,9 F1243zf | | Organics 0,4 F1234zeE + 0,3 F1234zeZ +0,3 F1243zf | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 4,8 | 0 | 2,2 | 0 | 5,6 | 0 | 4,3 |
| 0,05 | 5,7 | 0,05 | 3,3 | 0,05 | 6,6 | 0,05 | 5,3 |
| 0,1 | 5,8 | 0,1 | 3,3 | 0,1 | 6,6 | 0,1 | 5,3 |
| 0,15 | 5,8 | 0,15 | 3,3 | 0,15 | 6,6 | 0,15 | 5,3 |
| 0,2 | 5,7 | 0,2 | 3,3 | 0,2 | 6,6 | 0,2 | 5,3 |
| 0,25 | 5,7 | 0,25 | 3,3 | 0,25 | 6,6 | 0,25 | 5,3 |
| 0,3 | 5,7 | 0,3 | 3,3 | 0,3 | 6,6 | 0,3 | 5,3 |
| 0,35 | 5,7 | 0,35 | 3,3 | 0,35 | 6,6 | 0,35 | 5,3 |
| 0,4 | 5,7 | 0,4 | 3,3 | 0,4 | 6,6 | 0,4 | 5,3 |
| 0,45 | 5,7 | 0,45 | 3,3 | 0,45 | 6,6 | 0,45 | 5,3 |
| 0,5 | 5,7 | 0,5 | 3,3 | 0,5 | 6,6 | 0,5 | 5,3 |
| 0,55 | 5,7 | 0,55 | 3,3 | 0,55 | 6,5 | 0,55 | 5,3 |
| 0,6 | 5,6 | 0,6 | 3,3 | 0,6 | 6,5 | 0,6 | 5,3 |
| 0,65 | 5,5 | 0,65 | 3,3 | 0,65 | 6,5 | 0,65 | 5,2 |
| 0,7 | 5,3 | 0,7 | 3,3 | 0,7 | 6,4 | 0,7 | 5,1 |
| 0,75 | 5,1 | 0,75 | 3,2 | 0,75 | 6,2 | 0,75 | 4,9 |
| 0,8 | 4,8 | 0,8 | 3,1 | 0,8 | 5,9 | 0,8 | 4,6 |
| 0,85 | 4,3 | 0,85 | 2,8 | 0,85 | 5,4 | 0,85 | 4,2 |
| 0,9 | 3,6 | 0,9 | 2,5 | 0,9 | 4,6 | 0,9 | 3,6 |
| 0,95 | 2,6 | 0,95 | 2,0 | 0,95 | 3,3 | 0,95 | 2,6 |
| 1 | 1,2 | 1 | 1,2 | 1 | 1,2 | 1 | 1,2 |

**HF - HFO-1234f - HFO-1234zeE - Trifluoropropyne**

| **Organics 0,34 F1234yf + 0,33 F1234zeE + 0,33 TFP** | | **Organics 0,99 F1234yf + 0,005 F1234zeE + 0,005 TFP** | | **Organics 0,005 F1234yf + 0,99 F1234zeE + 0,005 TFP** | | **Organics 0,005 F1234yf + 0,005 F1234zeE + 0,99 TFP** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 8,0 | 0 | 6,8 | 0 | 4,9 | 0 | 11,6 |
| 0,05 | 8,8 | 0,05 | 7,8 | 0,05 | 5,9 | 0,05 | 12,4 |
| 0,1 | 8,8 | 0,1 | 7,8 | 0,1 | 5,9 | 0,1 | 12,3 |
| 0,15 | 8,8 | 0,15 | 7,8 | 0,15 | 5,9 | 0,15 | 12,2 |
| 0,2 | 8,8 | 0,2 | 7,8 | 0,2 | 5,9 | 0,2 | 12,1 |
| 0,25 | 8,7 | 0,25 | 7,8 | 0,25 | 5,9 | 0,25 | 12,0 |
| 0,3 | 8,7 | 0,3 | 7,7 | 0,3 | 5,9 | 0,3 | 11,9 |
| 0,35 | 8,7 | 0,35 | 7,8 | 0,35 | 5,9 | 0,35 | 11,8 |
| 0,4 | 8,7 | 0,4 | 7,8 | 0,4 | 5,9 | 0,4 | 11,8 |
| 0,45 | 8,7 | 0,45 | 7,8 | 0,45 | 5,9 | 0,45 | 11,8 |
| 0,5 | 8,7 | 0,5 | 7,8 | 0,5 | 5,8 | 0,5 | 11,8 |
| 0,55 | 8,7 | 0,55 | 7,8 | 0,55 | 5,8 | 0,55 | 11,8 |
| 0,6 | 8,7 | 0,6 | 7,8 | 0,6 | 5,7 | 0,6 | 11,8 |
| 0,65 | 8,7 | 0,65 | 7,8 | 0,65 | 5,6 | 0,65 | 11,8 |
| 0,7 | 8,6 | 0,7 | 7,7 | 0,7 | 5,4 | 0,7 | 11,8 |
| 0,75 | 8,5 | 0,75 | 7,6 | 0,75 | 5,2 | 0,75 | 11,8 |
| 0,8 | 8,1 | 0,8 | 7,3 | 0,8 | 4,8 | 0,8 | 11,6 |
| 0,85 | 7,4 | 0,85 | 6,7 | 0,85 | 4,3 | 0,85 | 10,8 |
| 0,9 | 6,2 | 0,9 | 5,6 | 0,9 | 3,6 | 0,9 | 9,1 |
| 0,95 | 4,2 | 0,95 | 3,9 | 0,95 | 2,6 | 0,95 | 6,1 |
| 1 | 1,2 | 1 | 1,2 | 1 | 1,2 | 1 | 1,2 |

**HF - HFO-1234f - HFO-1234zeE - HCFC-244bb**

| **Organics 0,34 F1234yf + 0,33 F1234zeE + 0,33 F244bb** | | **Organics 0,99 F1234yf + 0,005 F1234zeE + 0,005 F244bb** | | **Organics 0,005 F1234yf + 0,99 F1234zeE + 0,005 F244bb** | | **Organics 0,005 F1234yf + 0,005 F1234zeE + 0,99 F244bb** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 4,4 | 0 | 6,8 | 0 | 4,9 | 0 | 0,7 |
| 0,05 | 5,4 | 0,05 | 7,7 | 0,05 | 58 | 0,05 | 1,8 |
| 0,1 | 5,4 | 0,1 | 7,7 | 0,1 | 5,8 | 0,1 | 1,8 |
| 0,15 | 5,4 | 0,15 | 7,7 | 0,15 | 5,8 | 0,15 | 1,8 |
| 0,2 | 5,4 | 0,2 | 7,7 | 0,2 | 5,8 | 0,2 | 1,8 |
| 0,25 | 5,4 | 0,25 | 7,7 | 0,25 | 5,8 | 0,25 | 1,8 |
| 0,3 | 5,4 | 0,3 | 7,7 | 0,3 | 5,8 | 0,3 | 1,8 |
| 0,35 | 5,4 | 0,35 | 7,7 | 0,35 | 5,8 | 0,35 | 1,8 |
| 0,4 | 5,4 | 0,4 | 7,7 | 0,4 | 5,8 | 0,4 | 1,8 |
| 0,45 | 5,3 | 0,45 | 7,7 | 0,45 | 5,8 | 0,45 | 1,8 |
| 0,5 | 5,3 | 0,5 | 7,7 | 0,5 | 5,8 | 0,5 | 1,8 |
| 0,55 | 5,3 | 0,55 | 7,7 | 0,55 | 5,7 | 0,55 | 1,8 |
| 0,6 | 5,2 | 0,6 | 7,7 | 0,6 | 5,6 | 0,6 | 1,8 |
| 0,65 | 5,2 | 0,65 | 7,7 | 0,65 | 5,5 | 0,65 | 1,8 |
| 0,7 | 5,1 | 0,7 | 7,7 | 0,7 | 5,4 | 0,7 | 1,8 |
| 0,75 | 5,0 | 0,75 | 7,5 | 1 0,75 | 5,1 | 0,75 | 1,8 |
| 0,8 | 4,8 | 0,8 | 7,2 | 0,8 | 4,8 | 0,8 | 1,8 |
| 0,85 | 4,3 | 0,85 | 6,6 | 0,85 | 4,3 | 0,85 | 1,8 |
| 0,9 | 3,7 | 0,9 | 5,6 | 0,9 | 3,6 | 0,9 | 1,7 |
| 0,95 | 2,7 | 0,95 | 3,8 | 0,95 | 2,6 | 0,95 | 1,5 |
| 1 | 1,2 | 1 | 1,2 | 1 | 1,2 | 1 | 1,2 |

**HF - HFO-1234yf - HFO-1234zeE - HFC-245fa**

| **Organics 0,34 F1234yf + 0,33 F1234zeE + 0,33 F245fa** | | **Organics 0,99 F1234yf + 0,005 F1234zeE + 0,005 F245fa** | | **Organics 0,005 F1234yf + 0,99 F1234zeE + 0,005 F245fa** | | **Organics 0,005 F1234yf + 0,005 F1234zeE + 0,99 F245fa** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 4,5 | 0 | 6,8 | 0 | 4,9 | 0 | 1,6 |
| 0,05 | 5,6 | 0,05 | 7,7 | 0,05 | 5,8 | 0,05 | 2,7 |
| 0,1 | 5,6 | 0,1 | 7,7 | 0,1 | 5,8 | 0.1 | 2,7 |
| 0,15 | 5,6 | 0,15 | 7,7 | 0,15 | 5,8 | 0,15 | 2,7 |
| 0,2 | 5,6 | 0,2 | 7,7 | 0,2 | 5,8 | 0,2 | 2,7 |
| 0,25 | 5,6 | 0,25 | 7,7 | 0,25 | 5,8 | 0,25 | 2,7 |
| 0,3 | 5,6 | 0,3 | 7,7 | 0,3 | 5,8 | 0,3 | 2,7 |
| 0,35 | 5,6 | 0,35 | 7,7 | 0,35 | 5,8 | 0,35 | 2,7 |
| 0,4 | 5,6 | 0,4 | 7,7 | 0,4 | 5,8 | 0,4 | 2,7 |
| 0,45 | 5,6 | 0,45 | 7,7 | 0,45 | 5,8 | 0,45 | 2,7 |
| 0,5 | 5,6 | 0,5 | 7,7 | 0,5 | 5,8 | 0,5 | 2,7 |
| 0,55 | 5,5 | 0,55 | 7,7 | 0,55 | 5,7 | 0,55 | 2,7 |
| 0,6 | 5,5 | 0,6 | 7,7 | 0,6 | 5,6 | 0,6 | 2,7 |
| 0,65 | 5,5 | 0,65 | 7,7 | 0,65 | 5,5 | 0,65 | 2,7 |
| 0,7 | 5,4 | 0,7 | 7,7 | 0,7 | 5,4 | 0,7 | 2,7 |
| 0,75 | 5,3 | 0,75 | 7,5 | 0,75 | 5,1 | 0,75 | 2,7 |
| 0,8 | 5,0 | 0,8 | 7,2 | 0,8 | 4,8 | 0,8 | 2,6 |
| 0,85 | 4,5 | 0,85 | 6,6 | 0,85 | 4,3 | 0,85 | 2,4 |
| 0,9 | 3,8 | 0,9 | 5,6 | 0,9 | 3,6 | 0,9 | 2,1 |
| 0,95 | 2,7 | 0,95 | 3,9 | 0,95 | 2,6 | 0,95 | 1,7 |
| 1 | 1,2 | 1 | 1,2 | 1 | 1,2 | 1 | 1,2 |

**HF - HFO-1234f - HFO-1234zeE - HFO-1225eZ**

| **Organics 0,34 F1234yf + 0,33 F1234zeE + 0,33** | | **Organics 0,99 F1234yf + 0,005 F1234zeE + 0,005** | | **Organics 0,005 F1234yf + 0,99 F1234zeE + 0,005** | | **Organics 0,005 F1234yf + 0,005 F1234zeE + 0,99** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | bar | | bar | | bar | | bar |
| 0 | 5,7 | 0 | 6,8 | 0 | 4,9 | 0 | 5,2 |
| 0,05 | 6,6 | 0,05 | 7,7 | 0,05 | 5,8 | 0,05 | 6,2 |
| 0,1 | 6,7 | 0,1 | 7,8 | 0,1 | 5,9 | 0,1 | 6,2 |
| 0,15 | 6,7 | 0,15 | 7,7 | 0,15 | 5,9 | 0,15 | 6,2 |
| 0,2 | 6,7 | 0,2 | 7,7 | 0,2 | 5,9 | 0,2 | 6,2 |
| 0,25 | 6,7 | 0,25 | 7,7 | 0,25 | 5,9 | 0,25 | 6,2 |
| 0,3 | 6,7 | 0,3 | 7,7 | 0,3 | 5,9 | 0,3 | 6,2 |
| 0,35 | 6,7 | 0,35 | 7,7 | 0,35 | 5,9 | 0,35 | 6,2 |
| 0,4 | 6,7 | 0,4 | 7,7 | 0,4 | 5,8 | 0,4 | 6,2 |
| 0,45 | 6,7 | 0,45 | 7,7 | 0,45 | 5,8 | 0,45 | 6,2 |
| 0,5 | 6,7 | 0,5 | 7,7 | 0,5 | 5,8 | 0,5 | 6,2 |
| 0,55 | 6,7 | 0,55 | 7,7 | 0,55 | 5,7 | 0,55 | 6,2 |
| 0,6 | 6,6 | 0,6 | 7,7 | 0,6 | 5,7 | 0,6 | 6,2 |
| 0,65 | 6,6 | 0,65 | 7,7 | 0,65 | 5,6 | 0,65 | 6,2 |
| 0,7 | 6,5 | 0,7 | 7,7 | 0,7 | 5,4 | 0,7 | 6,1 |
| 0,75 | 6,3 | 0,75 | 7,6 | 0,75 | 5,2 | 0,75 | 5,9 |
| 0,8 | 5,9 | 0,8 | 7,2 | 0,8 | 4,8 | 0,8 | 5,6 |
| 0,85 | 5,4 | 0,85 | 6,6 | 0,85 | 4,3 | 0,85 | 5,1 |
| 0,9 | 4,5 | 0,9 | 5,6 | 0,9 | 3,6 | 0,9 | 4,2 |
| 0,95 | 3,1 | 0,95 | 3,9 | 0,95 | 2,6 | 0,95 | 3,0 |
| 1 | 1,2 | 1 | 1,2 | 1 | 1,2 | 1 | 1,2 |

**HF - HFO-1234yf - HFO-1234zeE - HFO-1225zc**

| **Organics 0,34 F1234yf + 0,33 F1234zeE + 0,33 F1225zc** | | **Organics 0,99 F1234yf + 0,005 F1234zeE + 0,005 F1225zc** | | **Organics 0,005 F1234yf + 0,99 F1234zeE + 0,005 F1225zc** | | **Organics 0,005 F1234yf + 0,005 F1234zeE + 0,99 F1225zc** | |
|---|---|---|---|---|---|---|---|
| MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE |
| | | | | | | | |

| | bar | | bar | | bar | | bar |
|---|---|---|---|---|---|---|---|
| 0 | 5,7 | 0 | 6,8 | 0 | 4,9 | 0 | 5,3 |
| 0,05 | 6,7 | 0,05 | 7,7 | 0,05 | 5,8 | 0,05 | 6,3 |
| 0,1 | 6,7 | 0,1 | 7,8 | 0,1 | 5,9 | 0,1 | 6,3 |
| 0,15 | 6,7 | 0,15 | 7,7 | 0,15 | 5,9 | 0,15 | 6,3 |
| 0,2 | 6,7 | 0,2 | 7,7 | 0,2 | 5,9 | 0,2 | 6,3 |
| 0,25 | 6,7 | 0,25 | 7,7 | 0,25 | 5,9 | 0,25 | 6,3 |
| 0,3 | 6,7 | 0,3 | 7,7 | 0,3 | 5,9 | 0,3 | 6,3 |
| 0,35 | 6,7 | 0,35 | 7,7 | 0,35 | 5,9 | 0,35 | 6,3 |
| 0,4 | 6,7 | 0,4 | 7,7 | 0,4 | 5,8 | 0,4 | 6,3 |
| 0,45 | 6,7 | 0,45 | 7,7 | 0,45 | 5,8 | 0,45 | 6,3 |
| 0,5 | 6,7 | 0,5 | 7,7 | 0,5 | 5,8 | 0,5 | 6,2 |
| 0,55 | 6,7 | 0,55 | 7,7 | 0,55 | 5,7 | 0,55 | 6,2 |
| 0,6 | 6,6 | 0,6 | 7,7 | 0,6 | 5,7 | 0,6 | 6,1 |
| 0,65 | 6,5 | 0,65 | 7,7 | 0,65 | 5,6 | 0,65 | 6,0 |
| 0,7 | 6,4 | 0,7 | 7,7 | 0,7 | 5,4 | 0,7 | 5,9 |
| 0,75 | 6,2 | 0,75 | 7,6 | 0,75 | 5,2 | 0,75 | 5,6 |
| 0,8 | 5,8 | 0,8 | 7,2 | 0,8 | 4,8 | 0,8 | 5,2 |
| 0,85 | 5,3 | 0,85 | 6,6 | 0,85 | 4,3 | 0,85 | 4,6 |
| 0,9 | 4,4 | 0,9 | 5,6 | 0,9 | 3,6 | 0,9 | 3,8 |
| 0,95 | 3,1 | 0,95 | 3,9 | 0,95 | 2,6 | 0,95 | 2,7 |
| 1 | 1,2 | 1 | 1,2 | 1 | 1,2 | 1 | 1,2 |

### Exemple 5 : Plage de température et de pression de mélanges quaternaires

| | Enveloope de points d'ébullition | |
|---|---|---|
| Quaternaire | Température °C | Pression bar abs |
| HF - HCFO-1233xf - HFC-245cb - HFO-1234zeE | 0 à 40 | ∼1.1 à ∼8.8 |
| HF - HCFO-1233xf - HFC-245cb - HFO-1234zeZ | 0 à 40 | ∼1,1 à ∼8.8 |
| HF - HFO-1234yf - HFC-245cb - HFO-1234zeE | 0 à 40 | ∼2.5 à ∼11.5 |
| HF - HFO-1234yf - HFC-245cb - HFO-1234zeZ | 0 à 40 | ∼1.3 à ∼11.3 |
| HF - HCFO-1233xf - HFO-1234yf - HFO-1234zeE | 0 à 40 | ∼1.2 à ∼11.3 |
| HF - HCFO-1233xf - HFO-1234yf - HFO-1234zeZ | 0 à 40 | ∼1.0 à ∼11.0 |
| HF - HCFO-1233xf - HCFO-1233zdE - HFO-1234zeE | 0 à 40 | ∼1.0 à ∼8.6 |
| HF - HCFO-1233xf - HCFO-1233zdE - HFO-1234zeZ | 0 à 40 | ∼0.9 à ∼4.8 |
| HF - HCFO-1233xf - HFO-1234zeE - HFO-1234zeZ | 0 à 40 | ∼1.1 à ∼8.6 |
| HF - HCFO-1233xf - HFO-1234zeE - HFO-1243zf | 0 à 40 | ∼1.2 à ∼10.1 |
| HF - HCFO-1233xf - HFO-1234zeZ - HFO-1243zf | 0 à 40 | ∼1.1 à ∼9.9 |
| HF - HFO-1234yf - HFO-1234zeE - HFO-1234zeZ | 0 à 40 | ∼1.3 à ∼11.3 |
| HF - HFO-1234yf - HFO-1234zeE - HCFO-1233zdE | 0 à 40 | ∼1.1 à ∼11.3 |
| HF - HFO-1234yf - HFO-1234zeE - HFO-1243zf | 0 à 40 | ∼3.0 à ∼11.6 |
| HF - HFO-1234yf - HFO-1234zeZ - HCFO-1233zdE | 0 à 40 | ∼1.0 à ∼11.0 |
| HF - HFO-1234yf - HFO-1234zeZ - HFO-1243zf | 0 à 40 | ∼1.3 à ∼11.3 |
| HF - HFC-245cb - HCFO-1233zdE - HFO-1234zeE | 0 à 40 | ∼1.0 à ∼8.9 |
| HF - HFC-245cb - HCFO-1233zdE - HFO-1234zeZ | 0 à 40 | ∼1.0 à ∼8.9 |
| HF - HFC-245cb - HFO-1234zeE - HFO-1234zeZ | 0 à 40 | ∼1.2 à ∼9.0 |
| HF - HFC-245cb - HFO-1234zeE - HFO-1243zf | 0 à 40 | ∼2.5 à ∼10.3 |
| HF - HFC-245cb - HFO-1234zeZ - HFO-1243zf | 0 à 40 | ∼1.3 à ∼10.1 |
| HF - HCFO-1233zdE - F 1234zeE - HFO-1234zeZ | 0 à 40 | ∼1.0 à ∼8.6 |
| HF - HCFO-1233zdE - F 1234zeE - HFO-1243zf | 0 à 40 | ∼1.1 à ∼10.1 |
| HF - HCFO-1233zdE - F 1234zeZ - HFO-1243zf | 0 à 40 | ∼1.0 à ∼9.9 |
| HF - HFO-1234zeE - HFO-1234zeZ - HFO-1243zf | 0 à 40 | ∼1.3 à ∼10.1 |
| HF - HFO-1234yf - HFO-1234zeE - Trifluoropropyne | 0 à 40 | ∼5.9 à ∼12.4 |
| HF - HFO-1234yf - HFO-1234zeE - HCFC-244bb | 0 à 40 | ∼1.8 à ∼7.7 |
| HF - HFO-1234yf - HFO-1234zeE - HFC-245fa | 0 à 40 | ∼2.7 à ∼7.7 |
| HF - HFO-1234yf - HFO-1234zeE - HFO-1225yeZ | 0 à 40 | ∼5.2 à ∼7.7 |
| HF - HFO-1234yf - HFO-1234zeE - HFO-1225zc | 0 à 40 | ∼5.2 à ∼7.7 |

### Exemple 6 : Plage de décantation de mélanges quaternaires

| | Plages de décantation | | |
|---|---|---|---|
| | Pourcentage massique d'HF | | |
| Quaternaire | Isotherme | Isotherme | Isotherme |
| HF-Orga1 Orqa2 Orqa3 | 0°C | 25°C | 40°C |
| HF - HCFO-1233xf - HFC-245cb - HFO-1234zeE | 5-75 | 5-70 | 15-60 |
| HF - HCFO-1233xf - HFC-245cb - HFO-1234zeZ | 5-80 | 5-75 | 5-70 |
| HF - HFO-1234yf - HFC-245cb - HFO-1234zeE | 5-75 | 10-70 | * |
| HF - HFO-1234yf - HFC-245cb - HFO-1234zeZ | 5-75 | 10-75 | 20-70 |
| HF - HCFO-1233xf - HFO-1234yf - HFO-1234zeE | 5-70 | 10-60 | * |
| HF - HCFO-1233xf - HFO-1234yf - HFO-1234zeZ | 5-75 | 5-70 | 10-60 |
| HF - HCFO-1233xf - HCFO-1233zdE - HFO-1234zeE | 5-75 | 5-65 | 5-55 |
| HF - HCFO-1233xf - HCFO-1233zdE - HFO-1234zeZ | 5-80 | 5-75 | 5-65 |
| HF - HCFO-1233xf - HFO-1234zeE - HFO-1234zeZ | 5-75 | 5-65 | 10-55 |
| HF - HCFO-1233xf - HFO-1234zeE - HFO-1243zf | 5-70 | 10-60 | * |
| HF - HCFO-1233xf - HFO-1234zeZ - HFO-1243zf | 5-75 | 5-70 | 10-55 |
| HF - HFO-1234yf - HFO-1234zeE - HFO-1234zeZ | 5-70 | 10-60 | * |
| HF - HFO-1234yf - HFO-1234zeE - HCFO-1233zdE | 5-70 | 10-60 | * |
| HF - HFO-1234yf - HFO-1234zeE - HFO-1243zf | 5-65 | * | * |
| HF - HFO-1234yf - HFO-1234zeZ - HCFO-1233zdE | 5-75 | 5-70 | 10-60 |
| HF - HFO-1234yf - HFO-1234zeZ - HFO-1243zf | 5-75 | 10-65 | * |
| HF - HFC-245cb - HCFO-1233zdE - HFO-1234zeE | 5-75 | 5-70 | 10-65 |
| HF - HFC-245cb - HCFO-1233zdE - HFO-1234zeZ | 5-80 | 5-75 | 5-70 |
| HF - HFC-245cb - HFO-1234zeE - HFO-1234zeZ | 5-75 | 5-70 | 15-55 |
| HF - HFC-245cb - HFO-1234zeE - HFO-1243zf | 5-75 | 15-65 | * |
| HF - HFC-245cb - HFO-1234zeZ - HFO-1243zf | 5-75 | 10-70 | 20-60 |
| HF - HCFO-1233zdE - F 1234zeE - HFO-1234zeZ | 5-75 | 5-65 | 10-55 |
| HF - HCFO-1233zdE - F 1234zeE - HFO-1243zf | 5-70 | 10-60 | * |
| HF - HCFO-1233zdE - F 1234zeZ - HFO-1243zf | 5-75 | 5-70 | 10-55 |
| HF - HFO-1234zeE - HFO-1234zeZ - HFO-1243zf | 5-70 | 10-60 | * |
| HF - HFO-1234yf - HFO-1234zeE - Trifluoropropyne | 10-65 | * | * |
| HF - HFO-1234yf - HFO-1234zeE - HCFC-244bb | 5-80 | 5-75 | 10-65 |
| HF - HFO-1234yf - HFO-1234zeE - HFC-245fa | 5-70 | 10-65 | * |
| HF - HFO-1234yf - HFO-1234zeE - HFO-1225yeZ | 5-70 | 20-45 | * |
| HF - HFO-1234yf - HFO-1234zeE - HFO-1225zc | 5-65 | * | * |

### Exemple 7 : Mélanges pentanaires, isotherme à 25 °C

**HF - HCFO-1233xf - HFC-245cb - HCFO-1233zdE - HFO-1234zeE**

| Organics 0,9 F1233xf + 0,033 F1233zdE +0,033 F245cb +0,034 F1234zeE | | Organics 0,033 F1233xf + 0,9 F1233zdE +0,033 F245cb +0,034 F1234zeE | | Organics 0,033 F1233xf + 0,033 F1233zdE +0,9 245cb +0,034 F1234zeE | | Organics 0,034 F1233xf + 0,033 F1233zdE +0,033 F245cb +0,9 F1234zeE | | Organics 0,25 F1233xf + 0,25 F1233zdE +0,25 F245cb +0,25 F1234zeE | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,8 | 0 | 1,6 | 0 | 4,4 | 0 | 4,7 | 0 | 3,2 |
| 0,05 | 2,9 | 0,05 | 2,7 | 0,05 | 5,6 | 0,05 | 5,6 | 0,05 | 4,3 |
| 0,1 | 2,9 | 0,1 | 2,7 | 0,1 | 5,6 | 0,1 | 5,6 | 0,1 | 4,3 |
| 0,15 | 2,9 | 0,15 | 2,7 | 0,15 | 5,6 | 0,15 | 5,6 | 0,15 | 4,3 |
| 0,2 | 2,9 | 0,2 | 2,7 | 0,2 | 5,6 | 0,2 | 5,6 | 0,2 | 4,3 |
| 0,25 | 2,9 | 0,25 | 2,7 | 0,25 | 5,6 | 0,25 | 5,6 | 0,25 | 4,3 |
| 0,3 | 2,9 | 0,3 | 2,7 | 0,3 | 5,6 | 0,3 | 5,6 | 0,3 | 4,3 |
| 0,35 | 2,9 | 0,35 | 2,7 | 0,35 | 5,6 | 0,35 | 5,6 | 0,35 | 4,3 |
| 0,4 | 2,9 | 0,4 | 2,7 | 0,4 | 5,6 | 0,4 | 5,6 | 0,4 | 4,3 |
| 0,45 | 2,9 | 0,45 | 2,7 | 0,45 | 5,6 | 0,45 | 5,6 | 0,45 | 4,3 |
| 0,5 | 2,9 | 0,5 | 2,7 | 0,5 | 5,6 | 0,5 | 5,6 | 0,5 | 4,3 |
| 0,55 | 2,9 | 0,55 | 2,7 | 0,55 | 5,6 | 0,55 | 5,6 | 0,55 | 4,3 |
| 0,6 | 2,9 | 0,6 | 2,7 | 0,6 | 5,6 | 0,6 | 5,5 | 0,6 | 4,3 |
| 0,65 | 2,9 | 0,65 | 2,7 | 0,65 | 5,6 | 0,65 | 5,4 | 0,65 | 4,3 |
| 0,7 | 2,9 | 0,7 | 2,7 | 0,7 | 5,7 | 0,7 | 5,2 | 0,7 | 4,3 |
| 0,75 | 2,9 | 0,75 | 2,7 | 0,75 | 5,7 | 0,75 | 5,0 | 0,75 | 4,2 |
| 0,8 | 2,7 | 0,8 | 2,6 | 0,8 | 5,7 | 0,8 | 4,7 | 0,8 | 4,0 |
| 0,85 | 2,5 | 0,85 | 2,4 | 0,85 | 5,6 | 0,85 | 4,2 | 0,85 | 3,7 |
| 0,9 | 2,2 | 0,9 | 2,1 | 0,9 | 4,8 | 0,9 | 3,5 | 0,9 | 3,2 |
| 0,95 | 1,8 | 0,95 | 1,7 | 0,95 | 3,4 | 0,95 | 2,5 | 0,95 | 2,4 |
| 1 | 1,2 | 1 | 1,2 | 1 | 1,2 | 1 | 1,2 | 1 | 1,2 |

**HF - HCFO-1233xf - HFC-245cb - HCFO-1233zdE - HFO-1234zeZ**

| Organics 0,9 F1233xf + 0,033 F1233zdE +0,033 F245cb +0,034 F1234zeZ | | Organics 0,033 F1233xf + 0,9 F1233zdE +0,033 F245cb +0,034 F1234zeZ | | Organics 0,033 F1233xf + 0,033 F1233zdE +0,9 F245cb +0,034 F1234zeZ | | Organics 0,034 F1233xf + 0,033 F1233zdE +0,033 F245cb +0,9 F1234zeZ | | Organics 0,25 F1233xf + 0,25 F1233zdE +0,25 F245cb +0,25 F1234zeZ | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,7 | 0 | 1,5 | 0 | 4,3 | 0 | 1,9 | 0 | 2,3 |
| 0,05 | 2,8 | 0,05 | 2,6 | 0,05 | 5,5 | 0,05 | 3,0 | 0,05 | 3,5 |
| 0,1 | 2,8 | 0,1 | 2,6 | 0,1 | 5,5 | 0,1 | 3,0 | 0,1 | 3,5 |
| 0,15 | 2,8 | 0,15 | 2,6 | 0,15 | 5,5 | 0,15 | 3,0 | 0,15 | 3,5 |
| 0,2 | 2,8 | 0,2 | 2,6 | 0,2 | 5,5 | 0,2 | 3,0 | 0,2 | 3,5 |
| 0,25 | 2,8 | 0,25 | 2,6 | 0,25 | 5,5 | 0,25 | 3,0 | 0,25 | 3,5 |
| 0,3 | 2,8 | 0,3 | 2,6 | 0,3 | 5,5 | 0,3 | 3,0 | 0,3 | 3,5 |
| 0,35 | 2,8 | 0,35 | 2,6 | 0,35 | 5,5 | 0,35 | 3,0 | 0,35 | 3,5 |
| 0,4 | 2,8 | 0,4 | 2,6 | 0,4 | 5,5 | 0,4 | 3,0 | 0,4 | 3,5 |
| 0,45 | 2,8 | 0,45 | 2,6 | 0,45 | 5,5 | 0,45 | 3,0 | 0,45 | 3,5 |
| 0,5 | 2,8 | 0,5 | 2,6 | 0,5 | 5,5 | 0,5 | 3,0 | 0,5 | 3,5 |
| 0,55 | 2,8 | 0,55 | 2,6 | 0,55 | 5,5 | 0,55 | 3,0 | 0,55 | 3,5 |
| 0,6 | 2,8 | 0,6 | 2,6 | 0,6 | 5,5 | 0,6 | 3,0 | 0,6 | 3,5 |
| 0,65 | 2,8 | 0,65 | 2,6 | 0,65 | 5,5 | 0,65 | 3,0 | 0,65 | 3,6 |
| 0,7 | 2,8 | 0,7 | 2,6 | 0,7 | 5,6 | 0,7 | 3,0 | 0,7 | 3,6 |
| 0,75 | 2,8 | 0,75 | 2,6 | 0,75 | 5,6 | 0,75 | 3,0 | 0,75 | 3,6 |
| 0,8 | 2,7 | 0,8 | 2,5 | 0,8 | 5,6 | 0,8 | 2,9 | 0,8 | 3,5 |
| 0,85 | 2,5 | 0,85 | 2,3 | 0,85 | 5,5 | 0,85 | 2,7 | 0,85 | 3,3 |
| 0,9 | 2,2 | 0,9 | 2,1 | 0,9 | 4,8 | 0,9 | 2,4 | 0,9 | 2,8 |
| 0,95 | 1,8 | 0,95 | 1,7 | 0,95 | 3,4 | 0,95 | 1,9 | 0,95 | 2,2 |
| 1 | 1,2 | 1 | 1,2 | 1 | 1,2 | 1 | 1,2 | 1 | 1,2 |

**HF - HCFO-1233xf - HFC-245cb - HFO-1234zeE - HFO-1234zeZ**

| Organics 0,9 F1233xf + 0,033 F1234zeE +0,033 F245cb +0,034 F1234zeZ | | Organics 0,033 F1233xf + 0,9 1234zeE +0,033 F245cb +0,034 F1234zeZ | | Organics 0,033 F1233xf + 0,033 F1234zeE +0,9 F245cb +0,034 F1234zeZ | | Organics 0,034 F1233xf + 0,033 F1234zeE +0,033 F245cb +0,9 F1234zeZ | | Organics 0,25 F1233xf + 0,25 F1234zeE +0,25 F245cb +0,25 F1234zeZ | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,8 | 0 | 4,7 | 0 | 4,5 | 0 | 2,0 | 0 | 3,2 |
| 0,05 | 2,9 | 0,05 | 5,6 | 0,05 | 5,6 | 0,05 | 3,1 | 0,05 | 4,3 |
| 0,1 | 2,9 | 0,1 | 5,7 | 0,1 | 5,6 | 0,1 | 3,1 | 0,1 | 4,3 |
| 0,15 | 2,9 | 0,15 | 5,6 | 0,15 | 5,6 | 0,15 | 3,1 | 0,15 | 4,3 |
| 0,2 | 2,9 | 0,2 | 5,6 | 0,2 | 5,6 | 0,2 | 3,1 | 0,2 | 4,3 |
| 0,25 | 2,9 | 0,25 | 5,6 | 0,25 | 5,6 | 0,25 | 3,1 | 0,25 | 4,3 |
| 0,3 | 2,9 | 0,3 | 5,6 | 0,3 | 5,6 | 0,3 | 3,1 | 0,3 | 4,3 |
| 0,35 | 2,9 | 0,35 | 5,6 | 0,35 | 5,6 | 0,35 | 3,1 | 0,35 | 4,3 |
| 0,4 | 2,9 | 0,4 | 5,6 | 0,4 | 5,6 | 0,4 | 3,1 | 0,4 | 4,3 |
| 0,45 | 2,9 | 0,45 | 5,6 | 0,45 | 5,6 | 0,45 | 3,1 | 0,45 | 4,3 |
| 0,5 | 2,9 | 0,5 | 5,6 | 0,5 | 5,6 | 0,5 | 3,1 | 0,5 | 4,3 |
| 0,55 | 2,9 | 0,55 | 5,6 | 0,55 | 5,6 | 0,55 | 3,1 | 0,55 | 4,3 |
| 0,6 | 2,9 | 0,6 | 5,5 | 0,6 | 5,6 | 0,6 | 3,1 | 0,6 | 4,3 |
| 0,65 | 2,9 | 0,65 | 5,4 | 0,65 | 5,7 | 0,65 | 3,1 | 0,65 | 4,4 |
| 0,7 | 2,9 | 0,7 | 5,2 | 0,7 | 5,7 | 0,7 | 3,1 | 0,7 | 4,4 |
| 0,75 | 2,9 | 0,75 | 5,0 | 0,75 | 5,7 | 0,75 | 3,1 | 0,75 | 4,3 |
| 0,8 | 2,7 | 0,8 | 4,7 | 0,8 | 5,7 | 0,8 | 3,0 | 0,8 | 4,1 |
| 0,85 | 2,5 | 0,85 | 4,2 | 0,85 | 5,6 | 0,85 | 2,7 | 0,85 | 3,8 |
| 0,9 | 2,2 | 0,9 | 3,5 | 0,9 | 4,8 | 0,9 | 2,4 | 0,9 | 3,3 |
| 0,95 | 1,8 | 0,95 | 2,6 | 0,95 | 3,4 | 0,95 | 1,9 | 0,95 | 2,4 |
| 1 | 1,2 | 1 | 1,2 | 1 | 1,2 | 1 | 1,2 | 1 | 1,2 |

**HF - HCFO-1233xf - HFC-245cb - HFO-1234zeE - HFO-1243zf**

| Organics 0,9 F1233xf + 0,033 F1234zeE +0,033 F245cb +0,034 F1243zf | | Organics 0,033 F1233xf + 0,9 F1234zeE +0,033 F245cb +0,034 F1243zf | | Organics 0,033 F1233xf + 0,033 F1234zeE +0,9 F245cb +0,034 F1243zf | | Organics 0,034 F1233xf + 0,033 F1234zeE +0,033 F245cb +0,9 F1243zf | | Organics 0,25 F1233xf + 0,25 F1234zeE +0,25 F245cb +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 2,0 | 0 | 4,8 | 0 | 4,6 | 0 | 5,7 | 0 | 4,4 |
| 0,05 | 3,1 | 0,05 | 5,8 | 0,05 | 5,8 | 0,05 | 6,6 | 0,05 | 5,4 |
| 0,1 | 3,1 | 0,1 | 5,8 | 0,1 | 5,8 | 0,1 | 6,7 | 0,1 | 5,4 |
| 0,15 | 3,1 | 0,15 | 5,8 | 0,15 | 5,8 | 0,15 | 6,6 | 0,15 | 5,4 |
| 0,2 | 3,1 | 0,2 | 5,8 | 0,2 | 5,8 | 0,2 | 6,6 | 0,2 | 5,4 |
| 0,25 | 3,1 | 0,25 | 5,8 | 0,25 | 5,8 | 0,25 | 6,6 | 0,25 | 5,4 |
| 0,3 | 3,1 | 0,3 | 5,8 | 0,3 | 5,8 | 0,3 | 6,6 | 0,3 | 5,4 |
| 0,35 | 3,1 | 0,35 | 5,8 | 0,35 | 5,8 | 0,35 | 6,6 | 0,35 | 5,4 |
| 0,4 | 3,1 | 0,4 | 5,8 | 0,4 | 5,8 | 0,4 | 6,6 | 0,4 | 5,4 |
| 0,45 | 3,1 | 0,45 | 5,8 | 0,45 | 5,8 | 0,45 | 6,6 | 0,45 | 5,4 |
| 0,5 | 3,1 | 0,5 | 5,7 | 0,5 | 5,8 | 0,5 | 6,6 | 0,5 | 5,4 |
| 0,55 | 3,1 | 0,55 | 5,7 | 0,55 | 5,8 | 0,55 | 6,6 | 0,55 | 5,4 |
| 0,6 | 3,1 | 0,6 | 5,6 | 0,6 | 5,8 | 0,6 | 6,6 | 0,6 | 5,4 |
| 0,65 | 3,1 | 0,65 | 5,5 | 0,65 | 5,8 | 0,65 | 6,5 | 0,65 | 5,4 |
| 0,7 | 3,1 | 0,7 | 5,4 | 0,7 | 5,8 | 0,7 | 6,4 | 0,7 | 5,4 |
| 0,75 | 3,0 | 0,75 | 5,2 | 0,75 | 5,8 | 0,75 | 6,3 | 0,75 | 5,3 |
| 0,8 | 2,9 | 0,8 | 4,8 | 0,8 | 5,8 | 0,8 | 6,0 | 0,8 | 5,0 |
| 0,85 | 2,7 | 0,85 | 4,3 | 0,85 | 5,7 | 0,85 | 5,5 | 0,85 | 4,6 |
| 0,9 | 2,3 | 0,9 | 3,6 | 0,9 | 4,9 | 0,9 | 4,7 | 0,9 | 3,9 |
| 0,95 | 1,9 | 0,95 | 2,6 | 0,95 | 3,5 | 0,95 | 3,3 | 0,95 | 2,8 |
| 1 | 1,2 | 1 | 1,2 | 1 | 1,2 | 1 | 1,2 | 1 | 1,2 |

**HF - HCFO-1233xf - HFC-245cb - HFO-1234zeE - HFO-1234yf**

| Organics 0,9 F1233xf + 0,033 F1234zeE +0,033 F245cb +0,034 F1234yf | | Organics 0,033 F1233xf + 0,9 F1234zeE +0,033 F245cb +0,034 F1234yf | | Organics 0,033 F1233xf + 0,033 F1234zeE +0,9 F245cb +0,034 F1234yf | | Organics 0,034 F1233xf + 0,033 F1234zeE +0,033 F245cb +0,9 F1234yf | | Organics 0,25 F1233xf + 0,25 F1234zeE +0,25 F245cb +0,25 F1234yf | |
|---|---|---|---|---|---|---|---|---|---|
| MASSFRAC TOTAL | | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 2,0 | 0 | 4,8 | 0 | 4,6 | 0 | 6,5 | 0 | 4,6 |
| 0,05 | 3,1 | 0,05 | 5,8 | 0,05 | 5,8 | 0,05 | 7,5 | 0,05 | 5,6 |
| 0,1 | 3,1 | 0,1 | 5,8 | 0,1 | 5,8 | 0,1 | 7,5 | 0,1 | 5,6 |
| 0,15 | 3,1 | 0,15 | 5,8 | 0,15 | 5,8 | 0,15 | 7,5 | 0,15 | 5,6 |
| 0,2 | 3,1 | 0,2 | 5,8 | 0,2 | 5,8 | 0,2 | 7,5 | 0,2 | 5,6 |
| 0,25 | 3,1 | 0,25 | 5,8 | 0,25 | 5,8 | 0,25 | 7,5 | 0,25 | 5,6 |
| 0,3 | 3,1 | 0,3 | 5,8 | 0,3 | 5,8 | 0,3 | 7,5 | 0,3 | 5,6 |
| 0,35 | 3,1 | 0,35 | 5,8 | 0,35 | 5,8 | 0,35 | 7,5 | 0,35 | 5,6 |
| 0,4 | 3,1 | 0,4 | 5,8 | 0,4 | 5,8 | 0,4 | 7,5 | 0,4 | 5,6 |
| 0,45 | 3,1 | 0,45 | 5,8 | 0,45 | 5,8 | 0,45 | 7,5 | 0,45 | 5,6 |
| 0,5 | 3,1 | 0,5 | 5,8 | 0,5 | 5,8 | 0,5 | 7,5 | 0,5 | 5,6 |
| 0,55 | 3,1 | 0,55 | 5,7 | 0,55 | 5,8 | 0,55 | 7,5 | 0,55 | 5,6 |
| 0,6 | 3,1 | 0,6 | 5,7 | 0,6 | 5,8 | 0,6 | 7,5 | 0,6 | 5,6 |
| 0,65 | 3,1 | 0,65 | 5,6 | 0,65 | 5,8 | 0,65 | 7,5 | 0,65 | 5,7 |
| 0,7 | 3,1 | 0,7 | 5,4 | 0,7 | 5,8 | 0,7 | 7,5 | 0,7 | 5,7 |
| 0,75 | 3,1 | 0,75 | 5,2 | 0,75 | 5,8 | 0,75 | 7,3 | 0,75 | 5,5 |
| 0,8 | 2,9 | 0,8 | 4,9 | 0,8 | 5,8 | 0,8 | 7,0 | 0,8 | 5,3 |
| 0,85 | 2,7 | 0,85 | 4,4 | 0,85 | 5,7 | 0,85 | 6,4 | 0,85 | 4,8 |
| 0,9 | 2,4 | 0,9 | 3,7 | 0,9 | 4,9 | 0,9 | 5,4 | 0,9 | 4,1 |
| 0,95 | 1,9 | 0,95 | 2,6 | 0,95 | 3,5 | 0,95 | 3,7 | 0,95 | 2,9 |
| 1 | 1,2 | 1 | 1,2 | 1 | 1,2 | 1 | 1,2 | 1 | 1,2 |

**HF - HCFO-1233xf - HFC-245cb - HFO-1234zeZ - HFO-1243zf**

| Organics 0,9 F1233xf + 0,033 F1234zeZ +0,033 F245cb +0,034 F1243zf | | Organics 0,033 F1233xf + 0,9 F1234zeZ +0,033 F245cb +0,034 F1243zf | | Organics 0,033 F1233xf + 0,033 F1234zeZ +0,9 F245cb +0,034 F1243zf | | Organics 0,034 F1233xf + 0,033 F1234zeZ +0,033 F245cb +0,9 F1243zf | | Organics 0,25 F1233xf + 0,25 F1234zeZ +0,25 F245cb +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,9 | 0 | 2,1 | 0 | 4,5 | 0 | 5,6 | 0 | 3,6 |
| 0,05 | 3,0 | 0,05 | 3,2 | 0,05 | 5,7 | 0,05 | 6,5 | 0,05 | 4,7 |
| 0,1 | 3,0 | 0,1 | 3,2 | 0,1 | 5,7 | 0,1 | 6,6 | 0,1 | 4,7 |
| 0,15 | 3,0 | 0,15 | 3,2 | 0,15 | 5,7 | 0,15 | 6,6 | 0,15 | 4,7 |
| 0,2 | 3,0 | 0,2 | 3,2 | 0,2 | 5,7 | 0,2 | 6,6 | 0,2 | 4,7 |
| 0,25 | 3,0 | 0,25 | 3,2 | 0,25 | 5,7 | 0,25 | 6,5 | 0,25 | 4,7 |
| 0,3 | 3,0 | 0,3 | 3,2 | 0,3 | 5,7 | 0,3 | 6,5 | 0,3 | 4,7 |
| 0,35 | 3,0 | 0,35 | 3,2 | 0,35 | 5,7 | 0,35 | 6,5 | 0,35 | 4,7 |
| 0,4 | 3,0 | 0,4 | 3,2 | 0,4 | 5,7 | 0,4 | 6,5 | 0,4 | 4,7 |
| 0,45 | 3,0 | 0,45 | 3,2 | 0,45 | 5,7 | 0,45 | 6,5 | 0,45 | 4,7 |
| 0,5 | 3,0 | 0,5 | 3,2 | 0,5 | 5,7 | 0,5 | 6,5 | 0,5 | 4,7 |
| 0,55 | 3,0 | 0,55 | 3,2 | 0,55 | 5,7 | 0,55 | 6,5 | 0,55 | 4,7 |
| 0,6 | 3,0 | 0,6 | 3,2 | 0,6 | 5,7 | 0,6 | 6,5 | 0,6 | 4,7 |
| 0,65 | 3,0 | 0,65 | 3,2 | 0,65 | 5,7 | 0,65 | 6,5 | 0,65 | 4,7 |
| 0,7 | 3,0 | 0,7 | 3,2 | 0,7 | 5,7 | 0,7 | 6,4 | 0,7 | 4,7 |
| 0,75 | 3,0 | 0,75 | 3,2 | 0,75 | 5,7 | 0,75 | 6,2 | 0,75 | 4,7 |
| 0,8 | 2,8 | 0,8 | 3,0 | 0,8 | 5,7 | 0,8 | 5,9 | 0,8 | 4,5 |
| 0,85 | 2,6 | 0,85 | 2,8 | 0,85 | 5,6 | 0,85 | 5,4 | 0,85 | 4,2 |
| 0,9 | 2,3 | 0,9 | 2,5 | 0,9 | 4,8 | 0,9 | 4,6 | 0,9 | 3,6 |
| 0,95 | 1,8 | 0,95 | 1,9 | 0,95 | 3,4 | 0,95 | 3,3 | 0,95 | 2,6 |
| 1 | 1,2 | 1 | 1,2 | 1 | 1,2 | 1 | 1,2 | 1 | 1,2 |

**HF - HCFO-1233xf - HFC-245cb - HFO-1234zeZ - HFO-1234f**

| Organics 0,9 F1233xf + 0,033 F1234zeZ +0,033 F245cb +0,034 F1234yf | | Organics 0,033 F1233xf + 0,9 F1234zeZ +0,033 F245cb +0,034 F1234yf | | Organics 0,033 F1233xf + 0,033 F1234zeZ +0,9 F245cb +0,034 F1234yf | | Organics 0,034 F1233xf + 0,033 F1234zeZ +0,033 F245cb +0,9 F1234yf | | Organics 0,25 F1233xf + 0,25 F1234zeZ +0,25 F245cb +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,9 | 0 | 2,1 | 0 | 4,5 | 0 | 6,4 | 0 | 3,7 |
| 0,05 | 3,0 | 0,05 | 3,2 | 0,05 | 5,7 | 0,05 | 7,4 | 0,05 | 4,9 |
| 0,1 | 3,0 | 0,1 | 3,2 | 0,1 | 5,7 | 0,1 | 7,4 | 0,1 | 4,9 |
| 0,15 | 3,0 | 0,15 | 3,2 | 0,15 | 5,7 | 0,15 | 7,4 | 0,15 | 4,9 |
| 0,2 | 3,0 | 0,2 | 3,2 | 0,2 | 5,7 | 0,2 | 7,4 | 0,2 | 4,9 |
| 0,25 | 3,0 | 0,25 | 3,2 | 0,25 | 5,7 | 0,25 | 7,4 | 0,25 | 4,9 |
| 0,3 | 3,0 | 0,3 | 3,2 | 0,3 | 5,7 | 0,3 | 7,4 | 0,3 | 4,9 |
| 0,35 | 3,0 | 0,35 | 3,2 | 0,35 | 5,7 | 0,35 | 7,4 | 0,35 | 4,9 |
| 0,4 | 3,0 | 0,4 | 3,2 | 0,4 | 5,7 | 0,4 | 7,4 | 0,4 | 4,9 |
| 0,45 | 3,0 | 0,45 | 3,2 | 0,45 | 5,7 | 0,45 | 7,4 | 0,45 | 4,9 |
| 0,5 | 3,0 | 0,5 | 3,2 | 0,5 | 5,7 | 0,5 | 7,4 | 0,5 | 4,9 |
| 0,55 | 3,0 | 0,55 | 3,2 | 0,55 | 5,7 | 0,55 | 7,4 | 0,55 | 4,9 |
| 0,6 | 3,0 | 0,6 | 3,2 | 0,6 | 5,7 | 0,6 | 7,4 | 0,6 | 4,9 |
| 0,65 | 3,0 | 0,65 | 3,2 | 0,65 | 5,7 | 0,65 | 7,4 | 0,65 | 4,9 |
| 0,7 | 3,0 | 0,7 | 3,2 | 0,7 | 5,7 | 0,7 | 7,4 | 0,7 | 4,9 |
| 0,75 | 3,0 | 0,75 | 3,2 | 0,75 | 5,7 | 0,75 | 7,2 | 0,75 | 5,0 |
| 0,8 | 2,8 | 0,8 | 3,0 | 0,8 | 5,7 | 0,8 | 6,9 | 0,8 | 4,8 |
| 0,85 | 2,6 | 0,85 | 2,8 | 0,85 | 5,7 | 0,85 | 6,4 | 0,85 | 4,4 |
| 0,9 | 2,3 | 0,9 | 2,5 | 0,9 | 4,9 | 0,9 | 5,4 | 0,9 | 3,8 |
| 0,95 | 1,8 | 0,95 | 2,0 | 0,95 | 3,5 | 0,95 | 3,7 | 0,95 | 2,7 |
| 1 | 1,2 | 1 | 1,2 | 1 | 1,2 | 1 | 1,2 | 1 | 1,2 |

**HF - HCFO-1233xf - HCFO-1233zdE - HFO-1234zeE - HFO-1234zeZ**

| Organics 0,9 F1233xf + 0,033 F1234zeE +0,033 F1233zdE +0,034 F1234zeZ | | Organics 0,033 F1233xf + 0,9 F1234zeE +0,033 F1233zdE +0,034 F1234zeZ | | Organics 0,033 F1233xf + 0,033 F1234zeE +0,9 F1233zdE +0,034 F1234zeZ | | Organics 0,034 F1233xf + 0,033 F1234zeE +0,033 F1233zdE +0,9 F1234zeZ | | Organics 0,25 F1233xf + 0,25 F1234zeE +0,25 F1233zdE +0,25 F1234zeZ | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,7 | 0 | 4,6 | 0 | 1,5 | 0 | 1,9 | 0 | 2,4 |
| 0,05 | 2,8 | 0,05 | 5,5 | 0,05 | 2,6 | 0,05 | 3,0 | 0,05 | 3,5 |
| 0,1 | 2,8 | 0,1 | 5,5 | 0,1 | 2,6 | 0,1 | 3,0 | 0,1 | 3,5 |
| 0,15 | 2,8 | 0,15 | 5,5 | 0,15 | 2,6 | 0,15 | 3,0 | 0,15 | 3,5 |
| 0,2 | 2,8 | 0,2 | 5,5 | 0,2 | 2,6 | 0,2 | 3,0 | 0,2 | 3,5 |
| 0,25 | 2,8 | 0,25 | 5,5 | 0,25 | 2,6 | 0,25 | 3,0 | 0,25 | 3,5 |
| 0,3 | 2,8 | 0,3 | 5,5 | 0,3 | 2,6 | 0,3 | 3,0 | 0,3 | 3,5 |
| 0,35 | 2,8 | 0,35 | 5,5 | 0,35 | 2,6 | 0,35 | 3,0 | 0,35 | 3,5 |
| 0,4 | 2,8 | 0,4 | 5,5 | 0,4 | 2,6 | 0,4 | 3,0 | 0,4 | 3,5 |
| 0,45 | 2,8 | 0,45 | 5,5 | 0,45 | 2,6 | 0,45 | 3,0 | 0,45 | 3,5 |
| 0,5 | 2,8 | 0,5 | 5,5 | 0,5 | 2,6 | 0,5 | 3,0 | 0,5 | 3,5 |
| 0,55 | 2,8 | 0,55 | 5,4 | 0,55 | 2,6 | 0,55 | 3,0 | 0,55 | 3,4 |
| 0,6 | 2,8 | 0,6 | 5,4 | 0,6 | 2,6 | 0,6 | 3,0 | 0,6 | 3,4 |
| 0,65 | 2,8 | 0,65 | 5,3 | 0,65 | 2,6 | 0,65 | 3,0 | 0,65 | 3,4 |
| 0,7 | 2,8 | 0,7 | 5,1 | 0,7 | 2,5 | 0,7 | 3,0 | 0,7 | 3,4 |
| 0,75 | 2,7 | 0,75 | 4,9 | 0,75 | 2,5 | 0,75 | 3,0 | 0,75 | 3,3 |
| 0,8 | 2,6 | 0,8 | 4,6 | 0,8 | 2,4 | 0,8 | 2,8 | 0,8 | 3,1 |
| 0,85 | 2,4 | 0,85 | 4,1 | 0,85 | 2,3 | 0,85 | 2,6 | 0,85 | 2,9 |
| 0,9 | 2,1 | 0,9 | 3,4 | 0,9 | 2,0 | 0,9 | 2,3 | 0,9 | 2,5 |
| 0,95 | 1,7 | 0,95 | 2,5 | 0,95 | 1,7 | 0,95 | 1,9 | 0,95 | 1,9 |
| 1 | 1,2 | 1 | 1,2 | 1 | 1,2 | 1 | 1,2 | 1 | 1,2 |

**HF - HCFO-1233xf - HCFO-1233zdE - HFO-1234zeE - HFO-1243zf**

| Organics 0,9 F1233xf + 0,033 F1234zeE +0,033 F1233zdE +0,034 F1243zf | | Organics 0,033 F1233xf + 0,9 F1234zeE +0,033 F1233zdE +0,034 F1243zf | | Organics 0,033 F1233xf + 0,033 F1234zeE +0,9 F1233zdE +0,034 F1243zf | | Organics 0,034 F1233xf + 0,033 F1234zeE +0,033 F1233zdE +0,9 F1243zf | | Organics 0,25 F1233xf + 0,25 1234zeE +0,25 F1233zdE +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,9 | 0 | 4,7 | 0 | 1,7 | 0 | 5,6 | 0 | 3,6 |
| 0,05 | 3,0 | 0,05 | 5,7 | 0,05 | 2,8 | 0,05 | 6,5 | 0,05 | 4,7 |
| 0,1 | 3,0 | 0,1 | 5,7 | 0,1 | 2,8 | 0,1 | 6,6 | 0,1 | 4,7 |
| 0,15 | 3,0 | 0,15 | 5,7 | 0,15 | 2,8 | 0,15 | 6,6 | 0,15 | 4,7 |
| 0,2 | 3,0 | 0,2 | 5,7 | 0,2 | 2,8 | 0,2 | 6,5 | 0,2 | 4,7 |
| 0,25 | 3,0 | 0,25 | 5,7 | 0,25 | 2,8 | 0,25 | 6,5 | 0,25 | 4,6 |
| 0,3 | 2,9 | 0,3 | 5,7 | 0,3 | 2,7 | 0,3 | 6,5 | 0,3 | 4,6 |
| 0,35 | 2,9 | 0,35 | 5,7 | 0,35 | 2,7 | 0,35 | 6,5 | 0,35 | 4,6 |
| 0,4 | 2,9 | 0,4 | 5,7 | 0,4 | 2,7 | 0,4 | 6,5 | 0,4 | 4,6 |
| 0,45 | 2,9 | 0,45 | 5,7 | 0,45 | 2,7 | 0,45 | 6,5 | 0,45 | 4,6 |
| 0,5 | 2,9 | 0,5 | 5,6 | 0,5 | 2,7 | 0,5 | 6,5 | 0,5 | 4,6 |
| 0,55 | 2,9 | 0,55 | 5,6 | 0,55 | 2,7 | 0,55 | 6,5 | 0,55 | 4,6 |
| 0,6 | 2,9 | 0,6 | 5,5 | 0,6 | 2,7 | 0,6 | 6,5 | 0,6 | 4,6 |
| 0,65 | 2,9 | 0,65 | 5,4 | 0,65 | 2,7 | 0,65 | 6,4 | 0,65 | 4,5 |
| 0,7 | 2,9 | 0,7 | 5,3 | 0,7 | 2,7 | 0,7 | 6,3 | 0,7 | 4,5 |
| 0,75 | 2,9 | 0,75 | 5,0 | 0,75 | 2,7 | 0,75 | 6,2 | 0,75 | 4,3 |
| 0,8 | 2,7 | 0,8 | 4,7 | 0,8 | 2,6 | 0,8 | 5,9 | 0,8 | 4,1 |
| 0,85 | 2,5 | 0,85 | 4,2 | 0,85 | 2,4 | 0,85 | 5,4 | 0,85 | 3,7 |
| 0,9 | 2,2 | 0,9 | 3,5 | 0,9 | 2,1 | 0,9 | 4,6 | 0,9 | 3,1 |
| 0,95 | 1,8 | 0,95 | 2,5 | 0,95 | 1,7 | 0,95 | 3,3 | 0,95 | 2,3 |
| 1 | 1,2 | 1 | 1,2 | 1 | 1,2 | 1 | 1,2 | 1 | 1,2 |

**HF - HCFO-1233xf - HCFO-1233zdE - HFO-1234zeZ - HFO-1243zf**

| Organics 0,9 F1233xf + 0,033 F1234zeZ +0,033 F1233zdE +0,034 F1243zf | | Organics 0,033 F1233xf + 0,9 F1234zeZ +0,033 F1233zdE +0,034 F1243zf | | Organics 0,033 F1233xf + 0,033 F1234zeZ +0,9 F1233zdE +0,034 F1243zf | | Organics 0,034 F1233xf + 0,033 F1234zeZ +0,033 F1233zdE +0,9 F1243zf | | Organics 0,25 F1233xf + 0,25 F1234zeZ +0,25 F1233zdE +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,7 | 0 | 1,9 | 0 | 1,5 | 0 | 5,5 | 0 | 2,8 |
| 0,05 | 2,8 | 0,05 | 3,0 | 0,05 | 2,6 | 0,05 | 6,4 | 0,05 | 3,9 |
| 0,1 | 2,8 | 0,1 | 3,0 | 0,1 | 2,6 | 0,1 | 6,5 | 0,1 | 3,9 |
| 0,15 | 2,8 | 0,15 | 3,0 | 0,15 | 2,6 | 0,15 | 6,5 | 0,15 | 3,9 |
| 0,2 | 2,8 | 0,2 | 3,0 | 0,2 | 2,6 | 0,2 | 6,5 | 0,2 | 3,9 |
| 0,25 | 2,8 | 0,25 | 3,0 | 0,25 | 2,6 | 0,25 | 6,5 | 0,25 | 3,9 |
| 0,3 | 2,8 | 0,3 | 3,0 | 0,3 | 2,6 | 0,3 | 6,5 | 0,3 | 3,9 |
| 0,35 | 2,8 | 0,35 | 3,0 | 0,35 | 2,6 | 0,35 | 6,5 | 0,35 | 3,9 |
| 0,4 | 2,8 | 0,4 | 3,0 | 0,4 | 2,6 | 0,4 | 6,5 | 0,4 | 3,9 |
| 0,45 | 2,8 | 0,45 | 3,0 | 0,45 | 2,6 | 0,45 | 6,4 | 0,45 | 3,9 |
| 0,5 | 2,8 | 0,5 | 3,0 | 0,5 | 2,6 | 0,5 | 6,4 | 0,5 | 3,9 |
| 0,55 | 2,8 | 0,55 | 3,0 | 0,55 | 2,6 | 0,55 | 6,4 | 0,55 | 3,9 |
| 0,6 | 2,8 | 0,6 | 3,0 | 0,6 | 2,6 | 0,6 | 6,4 | 0,6 | 3,9 |
| 0,65 | 2,8 | 0,65 | 3,0 | 0,65 | 2,6 | 0,65 | 6,4 | 0,65 | 3,8 |
| 0,7 | 2,8 | 0,7 | 3,0 | 0,7 | 2,6 | 0,7 | 6,3 | 0,7 | 3,8 |
| 0,75 | 2,8 | 0,75 | 3,0 | 0,75 | 2,6 | 0,75 | 6,1 | 0,75 | 3,7 |
| 0,8 | 2,7 | 0,8 | 2,9 | 0,8 | 2,5 | 0,8 | 5,8 | 0,8 | 3,6 |
| 0,85 | 2,5 | 0,85 | 2,7 | 0,85 | 2,3 | 0,85 | 5,3 | 0,85 | 3,3 |
| 0,9 | 2,2 | 0,9 | 2,4 | 0,9 | 2,1 | 0,9 | 4,5 | 0,9 | 2,8 |
| 0,95 | 1,8 | 0,95 | 1,9 | 0,95 | 1,7 | 0,95 | 3,2 | 0,95 | 2,2 |
| 1 | 1,2 | 1 | 1,2 | 1 | 1,2 | 1 | 1,2 | 1 | 1,2 |

**HF - HCFO-1233xf - HFO-1234zeE - HFO-1234zeZ - HFO-1243zf**

| Organics 0,9 F1233xf + 0,033 F1234zeE +0,033 F1234zeZ +0,034 F1243zf | | Organics 0,033 F1233xf + 0,9 F1234zeE +0,033 F1234zeZ +0,034 F1243zf | | Organics 0,033 F1233xf + 0,033 F1234zeE +0,9 F1234zeZ +0,034 F1243zf | | Organics 0,034 F1233xf + 0,033 F1234zeE +0,033 F1234zeZ +0,9 F1243zf | | Organics 0,25 F1233xf + 0,25 F1234zeE +0,25 F1234zeZ +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,9 | 0 | 4,7 | 0 | 2,1 | 0 | 5,6 | 0 | 3,7 |
| 0,05 | 3,0 | 0,05 | 5,7 | 0,05 | 3,2 | 0,05 | 6,5 | 0,05 | 4,7 |
| 0,1 | 3,0 | 0,1 | 5,7 | 0,1 | 3,2 | 0,1 | 6,6 | 0,1 | 4,7 |
| 0,15 | 3,0 | 0,15 | 5,7 | 0,15 | 3,2 | 0,15 | 6,6 | 0,15 | 4,7 |
| 0,2 | 3,0 | 0,2 | 5,7 | 0,2 | 3,2 | 0,2 | 6,5 | 0,2 | 4,7 |
| 0,25 | 3,0 | 0,25 | 5,7 | 0,25 | 3,2 | 0,25 | 6,5 | 0,25 | 4,7 |
| 0,3 | 3,0 | 0,3 | 5,7 | 0,3 | 3,2 | 0,3 | 6,5 | 0,3 | 4,7 |
| 0,35 | 3,0 | 0,35 | 5,7 | 0,35 | 3,2 | 0,35 | 6,5 | 0,35 | 4,7 |
| 0,4 | 3,0 | 0,4 | 5,7 | 0,4 | 3,2 | 0,4 | 6,5 | 0,4 | 4,7 |
| 0,45 | 3,0 | 0,45 | 5,7 | 0,45 | 3,2 | 0,45 | 6,5 | 0,45 | 4,7 |
| 0,5 | 3,0 | 0,5 | 5,6 | 0,5 | 3,2 | 0,5 | 6,5 | 0,5 | 4,7 |
| 0,55 | 2,9 | 0,55 | 5,6 | 0,55 | 3,1 | 0,55 | 6,5 | 0,55 | 4,6 |
| 0,6 | 2,9 | 0,6 | 5,5 | 0,6 | 3,1 | 0,6 | 6,5 | 0,6 | 4,6 |
| 0,65 | 2,9 | 0,65 | 5,4 | 0,65 | 3,1 | 0,65 | 6,4 | 0,65 | 4,6 |
| 0,7 | 2,9 | 0,7 | 5,3 | 0,7 | 3,1 | 0,7 | 6,3 | 0,7 | 4,5 |
| 0,75 | 2,9 | 0,75 | 5,0 | 0,75 | 3,1 | 0,75 | 6,2 | 0,75 | 4,4 |
| 0,8 | 2,8 | 0,8 | 4,7 | 0,8 | 3,0 | 0,8 | 5,9 | 0,8 | 4,1 |
| 0,85 | 2,6 | 0,85 | 4,2 | 0,85 | 2,7 | 0,85 | 5,4 | 0,85 | 3,8 |
| 0,9 | 2,2 | 0,9 | 3,5 | 0,9 | 2,4 | 0,9 | 4,6 | 0,9 | 3,2 |
| 0,95 | 1,8 | 0,95 | 2,5 | 0,95 | 1,9 | 0,95 | 3,3 | 0,95 | 2,4 |
| 1 | 1,2 | 1 | 1,2 | 1 | 1,2 | 1 | 1,2 | 1 | 1,2 |

**HF - HCFO-1233xf -HFO-1234yf - HFO-1234zeE - HFO-1234zeZ**

| Organics 0,9 F1233xf + 0,033 F1234zeE +0,033 F1234zeZ +0,034 F1234yf | | Organics 0,033 F1233xf + 0,9 F1234zeE +0,033 F1234zeZ +0,034 F1234yf | | Organics 0,033 F1233xf + 0,033 F1234zeE +0,9 F1234zeZ +0,034 F1234yf | | Organics 0,034 F1233xf + 0,033 F1234zeE +0,033 F1234zeZ +0,9 F1234yf | | Organics 0,25 F1233xf + 0,25 F1234zeE +0,25 F1234zeZ +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,9 | 0 | 4,7 | 0 | 2,1 | 0 | 6,4 | 0 | 3,8 |
| 0,05 | 3,0 | 0,05 | 5,7 | 0,05 | 3,2 | 0,05 | 7,4 | 0,05 | 4,9 |
| 0,1 | 3,0 | 0,1 | 5,7 | 0,1 | 3,2 | 0,1 | 7,4 | 0,1 | 4,9 |
| 0,15 | 3,0 | 0,15 | 5,7 | 0,15 | 3,2 | 0,15 | 7,4 | 0,15 | 4,9 |
| 0,2 | 3,0 | 0,2 | 5,7 | 0,2 | 3,2 | 0,2 | 7,4 | 0,2 | 4,9 |
| 0,25 | 3,0 | 0,25 | 5,7 | 0,25 | 3,2 | 0,25 | 7,4 | 0,25 | 4,9 |
| 0,3 | 3,0 | 0,3 | 5,7 | 0,3 | 3,2 | 0,3 | 7,4 | 0,3 | 4,9 |
| 0,35 | 3,0 | 0,35 | 5,7 | 0,35 | 3,2 | 0,35 | 7,4 | 0,35 | 4,9 |
| 0,4 | 3,0 | 0,4 | 5,7 | 0,4 | 3,2 | 0,4 | 7,4 | 0,4 | 4,9 |
| 0,45 | 3,0 | 0,45 | 5,7 | 0,45 | 3,2 | 0,45 | 7,4 | 0,45 | 4,9 |
| 0,5 | 3,0 | 0,5 | 5,7 | 0,5 | 3,2 | 0,5 | 7,4 | 0,5 | 4,9 |
| 0,55 | 3,0 | 0,55 | 5,6 | 0,55 | 3,2 | 0,55 | 7,4 | 0,55 | 4,9 |
| 0,6 | 3,0 | 0,6 | 5,6 | 0,6 | 3,2 | 0,6 | 7,4 | 0,6 | 4,8 |
| 0,65 | 3,0 | 0,65 | 5,5 | 0,65 | 3,2 | 0,65 | 7,4 | 0,65 | 4,8 |
| 0,7 | 3,0 | 0,7 | 5,3 | 0,7 | 3,2 | 0,7 | 7,4 | 0,7 | 4,8 |
| 0,75 | 2,9 | 0,75 | 5,1 | 0,75 | 3,1 | 0,75 | 7,2 | 0,75 | 4,6 |
| 0,8 | 2,8 | 0,8 | 4,7 | 0,8 | 3,0 | 0,8 | 6,9 | 0,8 | 4,4 |
| 0,85 | 2,6 | 0,85 | 4,3 | 0,85 | 2,8 | 0,85 | 6,3 | 0,85 | 4,0 |
| 0,9 | 2,3 | 0,9 | 3,6 | 0,9 | 2,4 | 0,9 | 5,3 | 0,9 | 3,4 |
| 0,95 | 1,8 | 0,95 | 2,6 | 0,95 | 1,9 | 0,95 | 3,7 | 0,95 | 2,5 |
| 1 | 1,2 | 1 | 1,2 | 1 | 1,2 | 1 | 1,2 | 1 | 1,2 |

**HF - HCFO-1233xf - HFO-1234f - HFO-1234zeE - HFO-1243zf**

| Organics 0,9 F1233xf + 0,033 F1234zeE +0,033 F1243zf +0,034 F1234yf | | Organics 0,033 F1233xf + 0,9 F1234zeE +0,033 F1243zf +0,034 F1234yf | | Organics 0,033 F1233xf + 0,033 F1234zeE +0,9 F1243zf +0,034 F1234yf | | Organics 0,034 F1233xf + 0,033 F1234zeE +0,033 F1243zf +0,9 F1234yf | | Organics 0,25 F1233xf + 0,25 F1234zeE +0,25 F1243zf +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 2,1 | 0 | 4,9 | 0 | 5,7 | 0 | 6,5 | 0 | 4,9 |
| 0,05 | 3,2 | 0,05 | 5,8 | 0,05 | 6,7 | 0,05 | 7,5 | 0,05 | 5,9 |
| 0,1 | 3,2 | 0,1 | 5,9 | 0,1 | 6,7 | 0,1 | 7,5 | 0,1 | 5,9 |
| 0,15 | 3,2 | 0,15 | 5,9 | 0,15 | 6,7 | 0,15 | 7,5 | 0,15 | 5,9 |
| 0,2 | 3,2 | 0,2 | 5,9 | 0,2 | 6,7 | 0,2 | 7,5 | 0,2 | 5,9 |
| 0,25 | 3,2 | 0,25 | 5,9 | 0,25 | 6,7 | 0,25 | 7,5 | 0,25 | 5,9 |
| 0,3 | 3,2 | 0,3 | 5,9 | 0,3 | 6,7 | 0,3 | 7,5 | 0,3 | 5,9 |
| 0,35 | 3,2 | 0,35 | 5,9 | 0,35 | 6,7 | 0,35 | 7,5 | 0,35 | 5,9 |
| 0,4 | 3,1 | 0,4 | 5,8 | 0,4 | 6,7 | 0,4 | 7,5 | 0,4 | 5,9 |
| 0,45 | 3,1 | 0,45 | 5,8 | 0,45 | 6,7 | 0,45 | 7,5 | 0,45 | 5,9 |
| 0,5 | 3,1 | 0,5 | 5,8 | 0,5 | 6,7 | 0,5 | 7,5 | 0,5 | 5,9 |
| 0,55 | 3,1 | 0,55 | 5,8 | 0,55 | 6,7 | 0,55 | 7,5 | 0,55 | 5,9 |
| 0,6 | 3,1 | 0,6 | 5,7 | 0,6 | 6,6 | 0,6 | 7,5 | 0,6 | 5,9 |
| 0,65 | 3,1 | 0,65 | 5,6 | 0,65 | 6,6 | 0,65 | 7,5 | 0,65 | 5,8 |
| 0,7 | 3,1 | 0,7 | 5,4 | 0,7 | 6,5 | 0,7 | 7,5 | 0,7 | 5,7 |
| 0,75 | 3,1 | 0,75 | 5,2 | 0,75 | 6,3 | 0,75 | 7,3 | 0,75 | 5,6 |
| 0,8 | 2,9 | 0,8 | 4,9 | 0,8 | 6,0 | 0,8 | 7,0 | 0,8 | 5,3 |
| 0,85 | 2,7 | 0,85 | 4,4 | 0,85 | 5,5 | 0,85 | 6,4 | 0,85 | 4,8 |
| 0,9 | 2,4 | 0,9 | 3,6 | 0,9 | 4,7 | 0,9 | 5,4 | 0,9 | 4,0 |
| 0,95 | 1,9 | 0,95 | 2,6 | 0,95 | 3,3 | 0,95 | 3,7 | 0,95 | 2,9 |
| 1 | 1,2 | 1 | 1,2 | 1 | 1,2 | 1 | 1,2 | 1 | 1,2 |

**HF - HCFO-1233xf - HFO-1234f - HFO-1234zeE - HCFO-1233zdE**

| Organics 0,9 F1233xf + 0,033 F1234zeE +0,033 F1233zdE +0,034 F1234yf | | Organics 0,033 F1233xf + 0,9 F1234zeE +0,033 F1233zdE +0,034 F1234yf | | Organics 0,033 F1233xf + 0,033 F1234zeE +0,9 F1233zdE +0,034 F1234yf | | Organics 0,034 F1233xf + 0,033 F1234zeE +0,033 F1233zdE +0,9 F1234yf | | Organics 0,25 F1233xf + 0,25 F1234zeE +0,25 F1233zdE +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,9 | 0 | 4,7 | 0 | 1,7 | 0 | 6,4 | 0 | 3,8 |
| 0,05 | 3,0 | 0,05 | 5,7 | 0,05 | 2,8 | 0,05 | 7,4 | 0,05 | 4,8 |
| 0,1 | 3,0 | 0,1 | 5,7 | 0,1 | 2,8 | 0,1 | 7,4 | 0,1 | 4,8 |
| 0,15 | 3,0 | 0,15 | 5,7 | 0,15 | 2,8 | 0,15 | 7,4 | 0,15 | 4,8 |
| 0,2 | 3,0 | 0,2 | 5,7 | 0,2 | 2,8 | 0,2 | 7,4 | 0,2 | 4,8 |
| 0,25 | 3,0 | 0,25 | 5,7 | 0,25 | 2,8 | 0,25 | 7,4 | 0,25 | 4,8 |
| 0,3 | 3,0 | 0,3 | 5,7 | 0,3 | 2,8 | 0,3 | 7,4 | 0,3 | 4,8 |
| 0,35 | 3,0 | 0,35 | 5,7 | 0,35 | 2,8 | 0,35 | 7,4 | 0,35 | 4,8 |
| 0,4 | 3,0 | 0,4 | 5,7 | 0,4 | 2,8 | 0,4 | 7,4 | 0,4 | 4,8 |
| 0,45 | 3,0 | 0,45 | 5,7 | 0,45 | 2,8 | 0,45 | 7,4 | 0,45 | 4,8 |
| 0,5 | 3,0 | 0,5 | 5,7 | 0,5 | 2,8 | 0,5 | 7,4 | 0,5 | 4,8 |
| 0,55 | 3,0 | 0,55 | 5,6 | 0,55 | 2,8 | 0,55 | 7,4 | 0,55 | 4,8 |
| 0,6 | 3,0 | 0,6 | 5,5 | 0,6 | 2,8 | 0,6 | 7,4 | 0,6 | 4,8 |
| 0,65 | 2,9 | 0,65 | 5,4 | 0,65 | 2,7 | 0,65 | 7,4 | 0,65 | 4,8 |
| 0,7 | 2,9 | 0,7 | 5,3 | 0,7 | 2,7 | 0,7 | 7,4 | 0,7 | 4,7 |
| 0,75 | 2,9 | 0,75 | 5,1 | 0,75 | 2,7 | 0,75 | 7,2 | 0,75 | 4,6 |
| 0,8 | 2,8 | 0,8 | 4,7 | 0,8 | 2,6 | 0,8 | 6,9 | 0,8 | 4,3 |
| 0,85 | 2,6 | 0,85 | 4,2 | 0,85 | 2,4 | 0,85 | 6,3 | 0,85 | 3,9 |
| 0,9 | 2,3 | 0,9 | 3,6 | 0,9 | 2,1 | 0,9 | 5,3 | 0,9 | 3,3 |
| 0,95 | 1,8 | 0,95 | 2,6 | 0,95 | 1,7 | 0,95 | 3,7 | 0,95 | 2,4 |
| 1 | 1,2 | 1 | 1,2 | 1 | 1,2 | 1 | 1,2 | 1 | 1,2 |

**HF - HCFO-1233xf - HFO-1234f - HFO-1234zeZ - HFO-1243zf**

| Organics 0,9 F1233xf + 0,033 F1234zeZ +0,033 F1243zf +0,034 F1234yf | | Organics 0,033 F1233xf + 0,9 F1234zeZ +0,033 F1243zf +0,034 F1234yf | | Organics 0,033 F1233xf + 0,033 F1234zeZ +0,9 F1243zf +0,034 F1234yf | | Organics 0,034 F1233xf + 0,033 F1234zeZ +0,033 F1243zf +0,9 F1234yf | | Organics 0,25 F1233xf + 0,25 F1234zeZ +0,25 F1243zf +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,9 | 0 | 2,1 | 0 | 5,6 | 0 | 6,4 | 0 | 4,1 |
| 0,05 | 3,1 | 0,05 | 3,2 | 0,05 | 6,6 | 0,05 | 7,4 | 0,05 | 5,2 |
| 0,1 | 3,1 | 0,1 | 3,2 | 0,1 | 6,6 | 0,1 | 7,4 | 0,1 | 5,2 |
| 0,15 | 3,1 | 0,15 | 3,2 | 0,15 | 6,6 | 0,15 | 7,4 | 0,15 | 5,2 |
| 0,2 | 3,1 | 0,2 | 3,2 | 0,2 | 6,6 | 0,2 | 7,4 | 0,2 | 5,2 |
| 0,25 | 3,1 | 0,25 | 3,2 | 0,25 | 6,6 | 0,25 | 7,4 | 0,25 | 5,2 |
| 0,3 | 3,1 | 0,3 | 3,2 | 0,3 | 6,6 | 0,3 | 7,4 | 0,3 | 5,2 |
| 0,35 | 3,0 | 0,35 | 3,2 | 0,35 | 6,6 | 0,35 | 7,4 | 0,35 | 5,2 |
| 0,4 | 3,0 | 0,4 | 3,2 | 0,4 | 6,6 | 0,4 | 7,4 | 0,4 | 5,2 |
| 0,45 | 3,0 | 0,45 | 3,2 | 0,45 | 6,6 | 0,45 | 7,4 | 0,45 | 5,2 |
| 0,5 | 3,0 | 0,5 | 3,2 | 0,5 | 6,6 | 0,5 | 7,4 | 0,5 | 5,2 |
| 0,55 | 3,0 | 0,55 | 3,2 | 0,55 | 6,6 | 0,55 | 7,4 | 0,55 | 5,2 |
| 0,6 | 3,0 | 0,6 | 3,2 | 0,6 | 6,5 | 0,6 | 7,4 | 0,6 | 5,2 |
| 0,65 | 3,0 | 0,65 | 3,2 | 0,65 | 6,5 | 0,65 | 7,4 | 0,65 | 5,2 |
| 0,7 | 3,0 | 0,7 | 3,2 | 0,7 | 6,4 | 0,7 | 7,4 | 0,7 | 5,1 |
| 0,75 | 3,0 | 0,75 | 3,2 | 0,75 | 6,2 | 0,75 | 7,2 | 0,75 | 5,0 |
| 0,8 | 2,9 | 0,8 | 3,1 | 0,8 | 6,0 | 0,8 | 6,9 | 0,8 | 4,8 |
| 0,85 | 2,6 | 0,85 | 2,8 | 0,85 | 5,5 | 0,85 | 6,3 | 0,85 | 4,4 |
| 0,9 | 2,3 | 0,9 | 2,5 | 0,9 | 4,6 | 0,9 | 5,3 | 0,9 | 3,7 |
| 0,95 | 1,8 | 0,95 | 2,0 | 0,95 | 3,3 | 0,95 | 3,7 | 0,95 | 2,7 |
| 1 | 1,2 | 1 | 1,2 | 1 | 1,2 | 1 | 1,2 | 1 | 1,2 |

**HF - HCFO-1233xf - HFO-1234f - HFO-1234zeZ - HCFO-1233zdE**

| Organics 0,9 F1233xf + 0,033 F1234zeZ +0,033 F1233zdE +0,034 F1234yf | | Organics 0,033 F1233xf + 0,9 F1234zeZ +0,033 F1233zdE +0,034 F1234yf | | Organics 0,033 F1233xf + 0,033 F1234zeZ +0,9 F1233zdE +0,034 F1234yf | | Organics 0,034 F1233xf + 0,033 F1234zeZ +0,033 F1233zdE +0,9 F1234yf | | Organics 0,25 F1233xf + 0,25 F1234zeZ +0,25 F1233zdE +0,25 F1234yf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 1,8 | 0 | 2,0 | 0 | 1,6 | 0 | 6,3 | 0 | 3,0 |
| 0,05 | 2,9 | 0,05 | 3,1 | 0,05 | 2,7 | 0,05 | 7,3 | 0,05 | 4,1 |
| 0,1 | 2,9 | 0,1 | 3,1 | 0,1 | 2,7 | 0,1 | 7,3 | 0,1 | 4,1 |
| 0,15 | 2,9 | 0,15 | 3,1 | 0,15 | 2,7 | 0,15 | 7,3 | 0,15 | 4,1 |
| 0,2 | 2,9 | 0,2 | 3,1 | 0,2 | 2,7 | 0,2 | 7,3 | 0,2 | 4,1 |
| 0,25 | 2,9 | 0,25 | 3,1 | 0,25 | 2,7 | 0,25 | 7,3 | 0,25 | 4,1 |
| 0,3 | 2,9 | 0,3 | 3,1 | 0,3 | 2,7 | 0,3 | 7,3 | 0,3 | 4,1 |
| 0,35 | 2,9 | 0,35 | 3,1 | 0,35 | 2,7 | 0,35 | 7,3 | 0,35 | 4,1 |
| 0,4 | 2,9 | 0,4 | 3,1 | 0,4 | 2,7 | 0,4 | 7,3 | 0,4 | 4,1 |
| 0,45 | 2,9 | 0,45 | 3,1 | 0,45 | 2,7 | 0,45 | 7,3 | 0,45 | 4,1 |
| 0,5 | 2,9 | 0,5 | 3,1 | 0,5 | 2,7 | 0,5 | 7,3 | 0,5 | 4,1 |
| 0,55 | 2,9 | 0,55 | 3,1 | 0,55 | 2,7 | 0,55 | 7,3 | 0,55 | 4,1 |
| 0,6 | 2,9 | 0,6 | 3,1 | 0,6 | 2,7 | 0,6 | 7,3 | 0,6 | 4,1 |
| 0,65 | 2,9 | 0,65 | 3,1 | 0,65 | 2,7 | 0,65 | 7,3 | 0,65 | 4,1 |
| 0,7 | 2,9 | 0,7 | 3,1 | 0,7 | 2,7 | 0,7 | 7,3 | 0,7 | 4,0 |
| 0,75 | 2,8 | 0,75 | 3,1 | 0,75 | 2,6 | 0,75 | 7,1 | 0,75 | 4,0 |
| 0,8 | 2,7 | 0,8 | 2,9 | 0,8 | 2,5 | 0,8 | 6,8 | 0,8 | 3,8 |
| 0,85 | 2,5 | 0,85 | 2,7 | 0,85 | 2,4 | 0,85 | 6,2 | 0,85 | 3,5 |
| 0,9 | 2,2 | 0,9 | 2,4 | 0,9 | 2,1 | 0,9 | 5,3 | 0,9 | 3,0 |
| 0,95 | 1,8 | 0,95 | 1,9 | 0,95 | 1,7 | 0,95 | 3,7 | 0,95 | 2,3 |
| 1 | 1,2 | 1 | 1,2 | 1 | 1,2 | 1 | 1,2 | 1 | 1,2 |

**HF - HFO-1234f - HFO-1234zeE - HFO-1234zeZ - HCFO-1233zdE**

| Organics 0,9 F1234yf + 0,033 F1233zdE +0,033 F1234zeE +0,034 F1234zeZ | | Organics 0,033 F1234yf + 0,9 F1233zdE +0,033 F1234zeE +0,034 F1234zeZ | | Organics 0,033 F1234yf + 0,033 F1233zdE +0,9 F1234zeE +0,034 F1234zeZ | | Organics 0,034 F1234yf + 0,033 F1233zdE +0,033 F1234zeE +0,9 F1234zeZ | | Organics 0,25 F1234yf + 0,25 F1233zdE +0,25 F1234zeE +0,25 F1234zeZ | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,4 | 0 | 1,7 | 0 | 4,7 | 0 | 2,1 | 0 | 3,8 |
| 0,05 | 7,4 | 0,05 | 2,8 | 0,05 | 5,7 | 0,05 | 3,2 | 0,05 | 4,8 |
| 0,1 | 7,4 | 0,1 | 2,8 | 0,1 | 5,7 | 0,1 | 3,2 | 0,1 | 4,8 |
| 0,15 | 7,4 | 0,15 | 2,8 | 0,15 | 5,7 | 0,15 | 3,2 | 0,15 | 4,8 |
| 0,2 | 7,4 | 0,2 | 2,8 | 0,2 | 5,7 | 0,2 | 3,2 | 0,2 | 4,8 |
| 0,25 | 7,4 | 0,25 | 2,8 | 0,25 | 5,7 | 0,25 | 3,2 | 0,25 | 4,8 |
| 0,3 | 7,4 | 0,3 | 2,8 | 0,3 | 5,7 | 0,3 | 3,2 | 0,3 | 4,8 |
| 0,35 | 7,4 | 0,35 | 2,8 | 0,35 | 5,7 | 0,35 | 3,2 | 0,35 | 4,8 |
| 0,4 | 7,4 | 0,4 | 2,8 | 0,4 | 5,7 | 0,4 | 3,2 | 0,4 | 4,8 |
| 0,45 | 7,4 | 0,45 | 2,8 | 0,45 | 5,7 | 0,45 | 3,2 | 0,45 | 4,8 |
| 0,5 | 7,4 | 0,5 | 2,8 | 0,5 | 5,7 | 0,5 | 3,2 | 0,5 | 4,8 |
| 0,55 | 7,4 | 0,55 | 2,8 | 0,55 | 5,6 | 0,55 | 3,2 | 0,55 | 4,8 |
| 0,6 | 7,4 | 0,6 | 2,8 | 0,6 | 5,5 | 0,6 | 3,2 | 0,6 | 4,8 |
| 0,65 | 7,4 | 0,65 | 2,7 | 0,65 | 5,4 | 0,65 | 3,2 | 0,65 | 4,8 |
| 0,7 | 7,4 | 0,7 | 2,7 | 0,7 | 5,3 | 0,7 | 3,2 | 0,7 | 4,7 |
| 0,75 | 7,2 | 0,75 | 2,7 | 0,75 | 5,1 | 0,75 | 3,1 | 0,75 | 4,6 |
| 0,8 | 6,9 | 0,8 | 2,6 | 0,8 | 4,7 | 0,8 | 3,0 | 0,8 | 4,3 |
| 0,85 | 6,3 | 0,85 | 2,4 | 0,85 | 4,2 | 0,85 | 2,8 | 0,85 | 4,0 |
| 0,9 | 5,3 | 0,9 | 2,1 | 0,9 | 3,6 | 0,9 | 2,4 | 0,9 | 3,4 |
| 0,95 | 3,7 | 0,95 | 1,7 | 0,95 | 2,6 | 0,95 | 1,9 | 0,95 | 2,5 |
| 1 | 1,2 | 1 | 1,2 | 1 | 1,2 | 1 | 1,2 | 1 | 1,2 |

**HF - HFO-1234f - HFO-1234zeE - HFO-1234zeZ - HFO-1243zf**

| Organics 0,9 F1234yf + 0,033 F1234zeZ +0,033 F1234zeE +0,034 F1243zf | | Organics 0,033 F1234yf + 0,9 F1234zeZ +0,033 F1234zeE +0,034 F1243zf | | Organics 0,033 F1234yf + 0,033 F1234zeZ +0,9 F1234zeE +0,034 F1243zf | | Organics 0,034 F1234yf + 0,033 F1234zeZ +0,033 F1234zeE +0,9 F1243zf | | Organics 0,25 F1234yf + 0,25 F1234zeZ +0,25 F1234zeE +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,5 | 0 | 2,2 | 0 | 4,9 | 0 | 5,7 | 0 | 4,9 |
| 0,05 | 7,5 | 0,05 | 3,3 | 0,05 | 5,8 | 0,05 | 6,7 | 0,05 | 5,9 |
| 0,1 | 7,5 | 0,1 | 3,3 | 0,1 | 5,9 | 0,1 | 6,7 | 0,1 | 5,9 |
| 0,15 | 7,5 | 0,15 | 3,3 | 0,15 | 5,8 | 0,15 | 6,7 | 0,15 | 5,9 |
| 0,2 | 7,5 | 0,2 | 3,3 | 0,2 | 5,8 | 0,2 | 6,7 | 0,2 | 5,9 |
| 0,25 | 7,5 | 0,25 | 3,3 | 0,25 | 5,8 | 0,25 | 6,7 | 0,25 | 5,9 |
| 0,3 | 7,5 | 0,3 | 3,3 | 0,3 | 5,8 | 0,3 | 6,7 | 0,3 | 5,9 |
| 0,35 | 7,5 | 0,35 | 3,3 | 0,35 | 5,8 | 0,35 | 6,7 | 0,35 | 5,9 |
| 0,4 | 7,5 | 0,4 | 3,3 | 0,4 | 5,8 | 0,4 | 6,7 | 0,4 | 5,9 |
| 0,45 | 7,5 | 0,45 | 3,3 | 0,45 | 5,8 | 0,45 | 6,7 | 0,45 | 5,9 |
| 0,5 | 7,5 | 0,5 | 3,3 | 0,5 | 5,8 | 0,5 | 6,7 | 0,5 | 5,9 |
| 0,55 | 7,5 | 0,55 | 3,3 | 0,55 | 5,8 | 0,55 | 6,7 | 0,55 | 5,9 |
| 0,6 | 7,5 | 0,6 | 3,3 | 0,6 | 5,7 | 0,6 | 6,6 | 0,6 | 5,9 |
| 0,65 | 7,5 | 0,65 | 3,3 | 0,65 | 5,6 | 0,65 | 6,6 | 0,65 | 5,8 |
| 0,7 | 7,5 | 0,7 | 3,3 | 0,7 | 5,4 | 0,7 | 6,5 | 0,7 | 5,7 |
| 0,75 | 7,3 | 0,75 | 3,3 | 0,75 | 5,2 | 0,75 | 6,3 | 0,75 | 5,6 |
| 0,8 | 7,0 | 0,8 | 3,1 | 0,8 | 4,9 | 0,8 | 6,0 | 0,8 | 5,3 |
| 0,85 | 6,4 | 0,85 | 2,9 | 0,85 | 4,4 | 0,85 | 5,5 | 0,85 | 4,8 |
| 0,9 | 5,4 | 0,9 | 2,5 | 0,9 | 3,7 | 0,9 | 4,7 | 0,9 | 4,1 |
| 0,95 | 3,7 | 0,95 | 2,0 | 0,95 | 2,6 | 0,95 | 3,3 | 0,95 | 2,9 |
| 1 | 1,2 | 1 | 1,2 | 1 | 1,2 | 1 | 1,2 | 1 | 1,2 |

**HF - HFO-1234f - HFO-1234zeE - HCFO-1233zdE - HFO-1243zf**

| Organics 0,9 F1234yf + 0,033 F1233zdE +0,033 F1234zeE +0,034 F1243zf | | Organics 0,033 F1234yf + 0,9 F1233zdE +0,033 F1234zeE +0,034 F1243zf | | Organics 0,033 F1234yf + 0,033 F1233zdE +0,9 F1234zeE +0,034 F1243zf | | Organics 0,034 F1234yf + 0,033 F1233zdE +0,033 F1234zeE +0,9 F1243zf | | Organics 0,25 F1234yf + 0,25 F1233zdE +0,25 F1234zeE +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,5 | 0 | 1,9 | 0 | 4,9 | 0 | 5,7 | 0 | 4,8 |
| 0,05 | 7,5 | 0,05 | 3,0 | 0,05 | 5,8 | 0,05 | 6,7 | 0,05 | 5,8 |
| 0,1 | 7,5 | 0,1 | 3,0 | 0,1 | 5,9 | 0,1 | 6,7 | 0,1 | 5,9 |
| 0,15 | 7,5 | 0,15 | 3,0 | 0,15 | 5,8 | 0,15 | 6,7 | 0,15 | 5,9 |
| 0,2 | 7,5 | 0,2 | 3,0 | 0,2 | 5,8 | 0,2 | 6,7 | 0,2 | 5,9 |
| 0,25 | 7,5 | 0,25 | 3,0 | 0,25 | 5,8 | 0,25 | 6,7 | 0,25 | 5,9 |
| 0,3 | 7,5 | 0,3 | 3,0 | 0,3 | 5,8 | 0,3 | 6,7 | 0,3 | 5,9 |
| 0,35 | 7,5 | 0,35 | 2,9 | 0,35 | 5,8 | 0,35 | 6,7 | 0,35 | 5,9 |
| 0,4 | 7,5 | 0,4 | 2,9 | 0,4 | 5,8 | 0,4 | 6,7 | 0,4 | 5,9 |
| 0,45 | 7,5 | 0,45 | 2,9 | 0,45 | 5,8 | 0,45 | 6,7 | 0,45 | 5,9 |
| 0,5 | 7,5 | 0,5 | 2,9 | 0,5 | 5,8 | 0,5 | 6,7 | 0,5 | 5,8 |
| 0,55 | 7,5 | 0,55 | 2,9 | 0,55 | 5,8 | 0,55 | 6,6 | 0,55 | 5,8 |
| 0,6 | 7,5 | 0,6 | 2,9 | 0,6 | 5,7 | 0,6 | 6,6 | 0,6 | 5,8 |
| 0,65 | 7,5 | 0,65 | 2,9 | 0,65 | 5,6 | 0,65 | 6,6 | 0,65 | 5,8 |
| 0,7 | 7,5 | 0,7 | 2,9 | 0,7 | 5,4 | 0,7 | 6,5 | 0,7 | 5,7 |
| 0,75 | 7,3 | 0,75 | 2,9 | 0,75 | 5,2 | 0,75 | 6,3 | 0,75 | 5,5 |
| 0,8 | 7,0 | 0,8 | 2,7 | 0,8 | 4,9 | 0,8 | 6,0 | 0,8 | 5,2 |
| 0,85 | 6,4 | 0,85 | 2,5 | 0,85 | 4,4 | 0,85 | 5,5 | 0,85 | 4,8 |
| 0,9 | 5,4 | 0,9 | 2,2 | 0,9 | 3,6 | 0,9 | 4,7 | 0,9 | 4,0 |
| 0,95 | 3,7 | 0,95 | 1,8 | 0,95 | 2,6 | 0,95 | 3,3 | 0,95 | 2,9 |
| 1 | 1,2 | 1 | 1,2 | 1 | 1,2 | 1 | 1,2 | 1 | 1,2 |

**HF - HFO-1234f - HFO-1234zeZ - HCFO-1233zdE - HFO-1243zf**

| Organics 0,9 F1234yf + 0,033 F1233zdE +0,033 F1234zeZ +0,034 F1243zf | | Organics 0,033 F1234yf + 0,9 F1233zdE+0,033 F1234zeZ +0,034 F1243zf | | Organics 0,033 F1234yf + 0,033 F1233zdE +0,9 F1234zeZ +0,034 F1243zf | | Organics 0,034 F1234yf + 0,033 F1233zdE +0,033 F1234zeZ +0,9 F1243zf | | Organics 0,25 F1234yf + 0,25 F1233zdE +0,25 F1234zeZ +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,4 | 0 | 1,7 | 0 | 2,1 | 0 | 5,6 | 0 | 4,1 |
| 0,05 | 7,4 | 0,05 | 2,9 | 0,05 | 3,2 | 0,05 | 6,6 | 0,05 | 5,2 |
| 0,1 | 7,4 | 0,1 | 2,9 | 0,1 | 3,2 | 0,1 | 6,6 | 0,1 | 5,2 |
| 0,15 | 7,4 | 0,15 | 2,9 | 0,15 | 3,2 | 0,15 | 6,6 | 0,15 | 5,2 |
| 0,2 | 7,4 | 0,2 | 2,9 | 0,2 | 3,2 | 0,2 | 6,6 | 0,2 | 5,2 |
| 0,25 | 7,4 | 0,25 | 2,8 | 0,25 | 3,2 | 0,25 | 6,6 | 0,25 | 5,2 |
| 0,3 | 7,4 | 0,3 | 2,8 | 0,3 | 3,2 | 0,3 | 6,6 | 0,3 | 5,2 |
| 0,35 | 7,4 | 0,35 | 2,8 | 0,35 | 3,2 | 0,35 | 6,6 | 0,35 | 5,1 |
| 0,4 | 7,4 | 0,4 | 2,8 | 0,4 | 3,2 | 0,4 | 6,6 | 0,4 | 5,1 |
| 0,45 | 7,4 | 0,45 | 2,8 | 0,45 | 3,2 | 0,45 | 6,6 | 0,45 | 5,1 |
| 0,5 | 7,4 | 0,5 | 2,8 | 0,5 | 3,2 | 0,5 | 6,6 | 0,5 | 5,1 |
| 0,55 | 7,4 | 0,55 | 2,8 | 0,55 | 3,2 | 0,55 | 6,6 | 0,55 | 5,1 |
| 0,6 | 7,4 | 0,6 | 2,8 | 0,6 | 3,2 | 0,6 | 6,5 | 0,6 | 5,1 |
| 0,65 | 7,4 | 0,65 | 2,8 | 0,65 | 3,2 | 0,65 | 6,5 | 0,65 | 5,1 |
| 0,7 | 7,4 | 0,7 | 2,8 | 0,7 | 3,2 | 0,7 | 6,4 | 0,7 | 5,1 |
| 0,75 | 7,2 | 0,75 | 2,8 | 0,75 | 3,2 | 0,75 | 6,2 | 0,75 | 5,0 |
| 0,8 | 6,9 | 0,8 | 2,7 | 0,8 | 3,0 | 0,8 | 5,9 | 0,8 | 4,7 |
| 0,85 | 6,3 | 0,85 | 2,5 | 0,85 | 2,8 | 0,85 | 5,5 | 0,85 | 4,3 |
| 0,9 | 5,3 | 0,9 | 2,2 | 0,9 | 2,5 | 0,9 | 4,6 | 0,9 | 3,7 |
| 0,95 | 3,7 | 0,95 | 1,8 | 0,95 | 2,0 | 0,95 | 3,3 | 0,95 | 2,7 |
| 1 | 1,2 | 1 | 1,2 | 1 | 1,2 | 1 | 1,2 | 1 | 1,2 |

**HF - HFO-1234yf - HFC-245cb - HFO-1234zeE - HCFO-1233zdE**

| Organics 0,9 F1234yf + 0,033 F1234zeE +0,033 F245cb +0,034 F1233zdE | | Organics 0,033 F1234yf + 0,9 F1234zeE +0,033 F245cb +0,034 F1233zdE | | Organics 0,033 F1234yf + 0,033 F1234zeE +0,9 F245cb +0,034 F1233zdE | | Organics 0,034 F1234yf + 0,033 F1234zeE +0,033 F245cb +0,9 F1233zdE | | Organics 0,25 F1234yf + 0,25 F1234zeE +0,25 F245cb +0,25 F1233zdE | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,5 | 0 | 4,8 | 0 | 4,6 | 0 | 1,8 | 0 | 4,5 |
| 0,05 | 7,5 | 0,05 | 5,8 | 0,05 | 5,8 | 0,05 | 2,9 | 0,05 | 5,6 |
| 0,1 | 7,5 | 0,1 | 5,8 | 0,1 | 5,8 | 0,1 | 2,9 | 0,1 | 5,6 |
| 0,15 | 7,5 | 0,15 | 5,8 | 0,15 | 5,8 | 0,15 | 2,9 | 0,15 | 5,6 |
| 0,2 | 7,5 | 0,2 | 5,8 | 0,2 | 5,8 | 0,2 | 2,9 | 0,2 | 5,6 |
| 0,25 | 7,5 | 0,25 | 5,8 | 0,25 | 5,8 | 0,25 | 2,9 | 0,25 | 5,6 |
| 0,3 | 7,5 | 0,3 | 5,8 | 0,3 | 5,8 | 0,3 | 2,9 | 0,3 | 5,6 |
| 0,35 | 7,5 | 0,35 | 5,8 | 0,35 | 5,8 | 0,35 | 2,9 | 0,35 | 5,6 |
| 0,4 | 7,5 | 0,4 | 5,8 | 0,4 | 5,8 | 0,4 | 2,9 | 0,4 | 5,6 |
| 0,45 | 7,5 | 0,45 | 5,8 | 0,45 | 5,8 | 0,45 | 2,9 | 0,45 | 5,6 |
| 0,5 | 7,5 | 0,5 | 5,8 | 0,5 | 5,8 | 0,5 | 2,9 | 0,5 | 5,6 |
| 0,55 | 7,5 | 0,55 | 5,7 | 0,55 | 5,8 | 0,55 | 2,9 | 0,55 | 5,6 |
| 0,6 | 7,5 | 0,6 | 5,7 | 0,6 | 5,8 | 0,6 | 2,9 | 0,6 | 5,6 |
| 0,65 | 7,5 | 0,65 | 5,6 | 0,65 | 5,8 | 0,65 | 2,9 | 0,65 | 5,6 |
| 0,7 | 7,5 | 0,7 | 5,4 | 0,7 | 5,8 | 0,7 | 2,9 | 0,7 | 5,6 |
| 0,75 | 7,3 | 0,75 | 5,2 | 0,75 | 5,8 | 0,75 | 2,9 | 0,75 | 5,5 |
| 0,8 | 7,0 | 0,8 | 4,9 | 0,8 | 5,8 | 0,8 | 2,7 | 0,8 | 5,2 |
| 0,85 | 6,4 | 0,85 | 4,4 | 0,85 | 5,7 | 0,85 | 2,5 | 0,85 | 4,8 |
| 0,9 | 5,4 | 0,9 | 3,6 | 0,9 | 4,9 | 0,9 | 2,2 | 0,9 | 4,1 |
| 0,95 | 3,7 | 0,95 | 2,6 | 0,95 | 3,5 | 0,95 | 1,8 | 0,95 | 2,9 |
| 1 | 1,2 | 1 | 1,2 | 1 | 1,2 | 1 | 1,2 | 1 | 1,2 |

**HF - HFO-1234f - HFC-245cb - HFO-1234zeE - HFO-1234zeZ**

| Organics 0,9 F1234yf + 0,033 F1234zeE +0,033 F245cb +0,034 F1234zeZ | | Organics 0,033 F1234yf + 0,9 F1234zeE +0,033 F245cb +0,034 F1234zeZ | | Organics 0,033 F1234yf + 0,033 F1234zeE +0,9 F245cb +0,034 F1234zeZ | | Organics 0,034 F1234yf + 0,033 F1234zeE +0,033 F245cb +0,9 F1234zeZ | | Organics 0,25 F1234yf + 0,25 F1234zeE +0,25 F245cb +0,25 F1234zeZ | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,5 | 0 | 4,8 | 0 | 4,6 | 0 | 2,2 | 0 | 4,5 |
| 0,05 | 7,5 | 0,05 | 5,8 | 0,05 | 5,8 | 0,05 | 3,3 | 0,05 | 5,6 |
| 0,1 | 7,5 | 0,1 | 5,8 | 0,1 | 5,8 | 0,1 | 3,3 | 0,1 | 5,6 |
| 0,15 | 7,5 | 0,15 | 5,8 | 0,15 | 5,8 | 0,15 | 3,3 | 0,15 | 5,6 |
| 0,2 | 7,5 | 0,2 | 5,8 | 0,2 | 5,8 | 0,2 | 3,3 | 0,2 | 5,6 |
| 0,25 | 7,5 | 0,25 | 5,8 | 0,25 | 5,8 | 0,25 | 3,3 | 0,25 | 5,6 |
| 0,3 | 7,5 | 03 | 5,8 | 0,3 | 5,8 | 0,3 | 3,3 | 0,3 | 5,6 |
| 0,35 | 7,5 | 0,35 | 5,8 | 0,35 | 5,8 | 0,35 | 3,3 | 0,35 | 5,6 |
| 0,4 | 7,5 | 0,4 | 5,8 | 0,4 | 5,8 | 0,4 | 3,3 | 0,4 | 5,6 |
| 0,45 | 7,5 | 0,45 | 5,8 | 0,45 | 5,8 | 0,45 | 3,3 | 0,45 | 5,6 |
| 0,5 | 7,5 | 0,5 | 5,8 | 0,5 | 5,8 | 0,5 | 3,3 | 0,5 | 5,6 |
| 0,55 | 7,5 | 0,55 | 5,7 | 0,55 | 5,8 | 0,55 | 3,3 | 0,55 | 5,6 |
| 0,6 | 7,5 | 0,6 | 5,7 | 0,6 | 5,8 | 0,6 | 3,3 | 0,6 | 5,6 |
| 0,65 | 7,5 | 0,65 | 5,6 | 0,65 | 5,8 | 0,65 | 3,3 | 0,65 | 5,6 |
| 0,7 | 7,5 | 0,7 | 5,4 | 0,7 | 5,8 | 0,7 | 3,3 | 0,7 | 5,6 |
| 0,75 | 7,3 | 0,75 | 5,2 | 0,75 | 5,8 | 0,75 | 3,3 | 0,75 | 5,5 |
| 0,8 | 7,0 | 0,8 | 4,9 | 0,8 | 5,8 | 0,8 | 3,1 | 0,8 | 5,3 |
| 0,85 | 6,4 | 0,85 | 4,4 | 0,85 | 5,7 | 0,85 | 2,9 | 0,85 | 4,9 |
| 0,9 | 5,4 | 0,9 | 3,7 | 0,9 | 4,9 | 0,9 | 2,5 | 0,9 | 4,1 |
| 0,95 | 3,7 | 0,95 | 2,6 | 0,95 | 3,5 | 0,95 | 2,0 | 0,95 | 3,0 |
| 1 | 1,2 | 1 | 1,2 | 1 | 1,2 | 1 | 1,2 | 1 | 1,2 |

**HF - HFO-1234yf - HFC-245cb - HFO-1234zeE - HFO-1243zf**

| Organics 0,9 F1234yf + 0,033 F1234zeE +0,033 F245cb +0,034 F1243zf | | Organics 0,033 F1234yf + 0,9 F1234zeE +0,033 F245cb +0,034 F1243zf | | Organics 0,033 F1234yf + 0,033 F1234zeE +0,9 F245cb +0,034 F1243zf | | Organics 0,034 F1234yf + 0,033 F1234zeE +0,033 F245cb +0,9 F1243zf | | Organics 0,25 F1234yf + 0,25 F1234zeE +0,25 F245cb +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,6 | 0 | 5,0 | 0 | 4,8 | 0 | 5,8 | 0 | 5,6 |
| 0,05 | 7,6 | 0,05 | 5,9 | 0,05 | 6,0 | 0,05 | 6,8 | 0,05 | 6,6 |
| 0,1 | 7,6 | 0,1 | 6,0 | 0,1 | 6,0 | 0,1 | 6,8 | 0,1 | 6,6 |
| 0,15 | 7,6 | 0,15 | 6,0 | 0,15 | 6,0 | 0,15 | 6,8 | 0,15 | 6,6 |
| 0,2 | 7,6 | 0,2 | 6,0 | 0,2 | 6,0 | 0,2 | 6,8 | 0,2 | 6,6 |
| 0,25 | 7,6 | 0,25 | 6,0 | 0,25 | 6,0 | 0,25 | 6,8 | 0,25 | 6,6 |
| 0,3 | 7,6 | 0,3 | 6,0 | 0,3 | 6,0 | 0,3 | 6,8 | 0,3 | 6,6 |
| 0,35 | 7,6 | 0,35 | 6,0 | 0,35 | 6,0 | 0,35 | 6,8 | 0,35 | 6,6 |
| 0,4 | 7,6 | 0,4 | 6,0 | 0,4 | 6,0 | 0,4 | 6,8 | 0,4 | 6,6 |
| 0,45 | 7,6 | 0,45 | 5,9 | 0,45 | 6,0 | 0,45 | 6,8 | 0,45 | 6,6 |
| 0,5 | 7,6 | 0,5 | 5,9 | 0,5 | 6,0 | 0,5 | 6,8 | 0,5 | 6,6 |
| 0,55 | 7,6 | 0,55 | 5,9 | 0,55 | 6,0 | 0,55 | 6,7 | 0,55 | 6,6 |
| 0,6 | 7,6 | 0,6 | 5,8 | 0,6 | 6,0 | 0,6 | 6,7 | 0,6 | 6,6 |
| 0,65 | 7,6 | 0,65 | 5,7 | 0,65 | 5,9 | 0,65 | 6,7 | 0,65 | 6,6 |
| 0,7 | 7,6 | 0,7 | 5,6 | 0,7 | 5,9 | 0,7 | 6,6 | 0,7 | 6,6 |
| 0,75 | 7,4 | 0,75 | 5,3 | 0,75 | 5,9 | 0,75 | 6,4 | 0,75 | 6,4 |
| 0,8 | 7,1 | 0,8 | 5,0 | 0,8 | 5,9 | 0,8 | 6,1 | 0,8 | 6,1 |
| 0,85 | 6,5 | 0,85 | 4,5 | 0,85 | 5,8 | 0,85 | 5,6 | 0,85 | 5,6 |
| 0,9 | 5,5 | 0,9 | 3,7 | 0,9 | 5,0 | 0,9 | 4,8 | 0,9 | 4,8 |
| 0,95 | 3,8 | 0,95 | 2,7 | 0,95 | 3,5 | 0,95 | 3,4 | 0,95 | 3,4 |
| 1 | 1,2 | 1 | 1,2 | 1 | 1,2 | 1 | 1,2 | 1 | 1,2 |

**HF - HFO-1234f - HFC-245cb - HFO-1234zeZ - HCFO-1233zdE**

| Organics 0,9 F1234yf + 0,033 F1234zeZ +0,033 F245cb +0,034 F1233zdE | | Organics 0,033 F1234yf + 0,9 F1234zeZ +0,033 F245cb +0,034 F1233zdE | | Organics 0,033 F1234yf + 0,033 F1234zeZ +0,9 F245cb +0,034 F1233zdE | | Organics 0,034 F1234yf + 0,033 F1234zeZ +0,033 F245cb +0,9 F1233zdE | | Organics 0,25 F1234yf + 0,25 F1234zeZ +0,25 F245cb +0,25 F1233zdE | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,4 | 0 | 2,1 | 0 | 4,5 | 0 | 1,7 | 0 | 3,7 |
| 0,05 | 7,4 | 0,05 | 3,2 | 0,05 | 5,7 | 0,05 | 2,8 | 0,05 | 4,8 |
| 0,1 | 7,4 | 0,1 | 3,2 | 0,1 | 5,7 | 0,1 | 2,8 | 0,1 | 4,8 |
| 0,15 | 7,4 | 0,15 | 3,2 | 0,15 | 5,7 | 0,15 | 2,8 | 0,15 | 4,8 |
| 0,2 | 7,4 | 0,2 | 3,2 | 0,2 | 5,7 | 0,2 | 2,8 | 0,2 | 4,8 |
| 0,25 | 7,4 | 0,25 | 3,2 | 0,25 | 5,7 | 0,25 | 2,8 | 0,25 | 4,8 |
| 0,3 | 7,4 | 0,3 | 3,2 | 0,3 | 5,7 | 0,3 | 2,8 | 0,3 | 4,8 |
| 0,35 | 7,4 | 0,35 | 3,2 | 0,35 | 5,7 | 0,35 | 2,8 | 0,35 | 4,8 |
| 0,4 | 7,4 | 0,4 | 3,2 | 0,4 | 5,7 | 0,4 | 2,8 | 0,4 | 4,8 |
| 0,45 | 7,4 | 0,45 | 3,2 | 0,45 | 5,7 | 0,45 | 2,8 | 0,45 | 4,8 |
| 0,5 | 7,4 | 0,5 | 3,2 | 0,5 | 5,7 | 0,5 | 2,8 | 0,5 | 4,8 |
| 0,55 | 7,4 | 0,55 | 3,2 | 0,55 | 5,7 | 0,55 | 2,8 | 0,55 | 4,8 |
| 0,6 | 7,4 | 0,6 | 3,2 | 0,6 | 5,7 | 0,6 | 2,8 | 0,6 | 4,9 |
| 0,65 | 7,4 | 0,65 | 3,2 | 0,65 | 5,7 | 0,65 | 2,8 | 0,65 | 4,9 |
| 0,7 | 7,4 | 0,7 | 3,2 | 0,7 | 5,7 | 0,7 | 2,8 | 0,7 | 4,9 |
| 0,75 | 7,2 | 0,75 | 3,2 | 0,75 | 5,7 | 0,75 | 2,8 | 0,75 | 4,9 |
| 0,8 | 6,9 | 0,8 | 3,0 | 0,8 | 5,7 | 0,8 | 2,7 | 0,8 | 4,7 |
| 0,85 | 6,4 | 0,85 | 2,8 | 0,85 | 5,7 | 0,85 | 2,5 | 0,85 | 4,3 |
| 0,9 | 5,4 | 0,9 | 2,5 | 0,9 | 4,9 | 0,9 | 2,2 | 0,9 | 3,7 |
| 0,95 | 3,7 | 0,95 | 2,0 | 0,95 | 3,5 | 0,95 | 1,8 | 0,95 | 2,7 |
| 1 | 1,2 | 1 | 1,2 | 1 | 1,2 | 1 | 1,2 | 1 | 1,2 |

**HF - HFO-1234yf - HFC-245cb - HFO-1234zeZ - HFO-1243zf**

| Organics 0,9 F1234yf + 0,033 F1234zeZ +0,033 F245cb +0,034 F1243zf | | Organics 0,033 F1234yf + 0,9 F1234zeZ +0,033 F245cb +0,034 F1243zf | | Organics 0,033 F1234yf + 0,033 F1234zeZ +0,9 F245cb +0,034 F1243zf | | Organics 0,034 F1234yf + 0,033 F1234zeZ +0,033 F245cb +0,9 F1243zf | | Organics 0,25 F1234yf + 0,25 F1234zeZ +0,25 F245cb +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 6,5 | 0 | 2,2 | 0 | 4,7 | 0 | 5,7 | 0 | 4,8 |
| 0,05 | 7,5 | 0,05 | 3,3 | 0,05 | 5,8 | 0,05 | 6,7 | 0,05 | 5,9 |
| 0,1 | 7,5 | 0,1 | 3,3 | 0,1 | 5,8 | 0,1 | 6,7 | 0,1 | 5,9 |
| 0,15 | 7,5 | 0,15 | 3,3 | 0,15 | 5,8 | 0,15 | 6,7 | 0,15 | 5,9 |
| 0,2 | 7,5 | 0,2 | 3,3 | 0,2 | 5,8 | 0,2 | 6,7 | 0,2 | 5,9 |
| 0,25 | 7,5 | 0,25 | 3,3 | 0,25 | 5,8 | 0,25 | 6,7 | 0,25 | 5,9 |
| 0,3 | 7,5 | 0,3 | 3,3 | 0,3 | 5,8 | 0,3 | 6,7 | 0,3 | 5,9 |
| 0,35 | 7,5 | 0,35 | 3,3 | 0,35 | 5,8 | 0,35 | 6,7 | 0,35 | 5,9 |
| 0,4 | 7,5 | 0,4 | 3,3 | 0,4 | 5,8 | 0,4 | 6,7 | 0,4 | 5,9 |
| 0,45 | 7,5 | 0,45 | 3,3 | 0,45 | 5,8 | 0,45 | 6,7 | 0,45 | 5,9 |
| 0,5 | 7,5 | 0,5 | 3,3 | 0,5 | 5,8 | 0,5 | 6,7 | 0,5 | 5,9 |
| 0,55 | 7,5 | 0,55 | 3,3 | 0,55 | 5,8 | 0,55 | 6,7 | 0,55 | 5,9 |
| 0,6 | 7,5 | 0,6 | 3,3 | 0,6 | 5,8 | 0,6 | 6,6 | 0,6 | 5,9 |
| 0,65 | 7,5 | 0,65 | 3,3 | 0,65 | 5,8 | 0,65 | 6,6 | 0,65 | 5,9 |
| 0,7 | 7,5 | 0,7 | 3,3 | 0,7 | 5,9 | 0,7 | 6,5 | 0,7 | 5,9 |
| 0,75 | 7,3 | 0,75 | 3,3 | 0,75 | 5,9 | 0,75 | 6,3 | 0,75 | 5,9 |
| 0,8 | 7,0 | 0,8 | 3,2 | 0,8 | 5,9 | 0,8 | 6,1 | 0,8 | 5,6 |
| 0,85 | 6,5 | 0,85 | 2,9 | 0,85 | 5,8 | 0,85 | 5,6 | 0,85 | 5,2 |
| 0,9 | 5,4 | 0,9 | 2,6 | 0,9 | 5,0 | 0,9 | 4,7 | 0,9 | 4,4 |
| 0,95 | 3,8 | 0,95 | 2,0 | 0,95 | 3,5 | 0,95 | 3,4 | 0,95 | 3,2 |
| 1 | 1,2 | 1 | 1,2 | 1 | 1,2 | 1 | 1,2 | 1 | 1,2 |

**HF -HFC-245cb - HCFO-1233zdE - HFO-1234zeE - HFO-1234zeZ**

| Organics 0,9 F245cb + 0,033 F1234zeE +0,033 F1233zdE +0,034 F1234zeZ | | Organics 0,033 F245cb + 0,9 F1234zeE +0,033 F1233zdE +0,034 F1234zeZ | | Organics 0,033 F245cb + 0,033 F1234zeE +0,9 F1233zdE +0,034 F1234zeZ | | Organics 0,034 F245cb + 0,033 F1234zeE +0,033 F1233zdE +0,9 F1234zeZ | | Organics 0,25 F245cb + 0,25 F1234zeE +0,25 F1233zdE +0,25 F1234zeZ | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 4,4 | 0 | 4,7 | 0 | 1,6 | 0 | 2,0 | 0 | 3,2 |
| 0,05 | 5,6 | 0,05 | 5,6 | 0,05 | 2,7 | 0,05 | 3,1 | 0,05 | 4,3 |
| 0,1 | 5,6 | 0,1 | 5,6 | 0,1 | 2,7 | 0,1 | 3,1 | 0,1 | 4,3 |
| 0,15 | 5,6 | 0,15 | 5,6 | 0,15 | 2,7 | 0,15 | 3,1 | 0,15 | 4,3 |
| 0,2 | 5,6 | 0,2 | 5,6 | 0,2 | 2,7 | 0,2 | 3,1 | 0,2 | 4,3 |
| 0,25 | 5,6 | 0,25 | 5,6 | 0,25 | 2,7 | 0,25 | 3,1 | 0,25 | 4,3 |
| 0,3 | 5,6 | 0,3 | 5,6 | 0,3 | 2,7 | 0,3 | 3,1 | 0,3 | 4,3 |
| 0,35 | 5,6 | 0,35 | 5,6 | 0,35 | 2,7 | 0,35 | 3,1 | 0,35 | 4,3 |
| 0,4 | 5,6 | 0,4 | 5,6 | 0,4 | 2,7 | 0,4 | 3,1 | 0,4 | 4,3 |
| 0,45 | 5,6 | 0,45 | 5,6 | 0,45 | 2,7 | 0,45 | 3,1 | 0,45 | 4,3 |
| 0,5 | 5,6 | 0,5 | 5,6 | 0,5 | 2,7 | 0,5 | 3,1 | 0,5 | 4,3 |
| 0,55 | 5,6 | 0,55 | 5,6 | 0,55 | 2,7 | 0,55 | 3,1 | 0,55 | 4,3 |
| 0,6 | 5,6 | 0,6 | 5,5 | 0,6 | 2,7 | 0,6 | 3,1 | 0,6 | 4,3 |
| 0,65 | 5,6 | 0,65 | 5,4 | 0,65 | 2,7 | 0,65 | 3,1 | 0,65 | 4,3 |
| 0,7 | 5,7 | 0,7 | 5,2 | 0,7 | 2,7 | 0,7 | 3,1 | 0,7 | 4,3 |
| 0,75 | 5,7 | 0,75 | 5,0 | 0,75 | 2,7 | 0,75 | 3,1 | 0,75 | 4,3 |
| 0,8 | 5,7 | 0,8 | 4,7 | 0,8 | 2,6 | 0,8 | 3,0 | 0,8 | 4,1 |
| 0,85 | 5,6 | 0,85 | 4,2 | 0,85 | 2,4 | 0,85 | 2,7 | 0,85 | 3,7 |
| 0,9 | 4,8 | 0,9 | 3,5 | 0,9 | 2,1 | 0,9 | 2,4 | 0,9 | 3,2 |
| 0,95 | 3,4 | 0,95 | 2,5 | 0,95 | 1,7 | 0,95 | 1,9 | 0,95 | 2,4 |
| 1 | 1,2 | 1 | 1,2 | 1 | 1,2 | 1 | 1,2 | 1 | 1,2 |

**HF -HFC-245cb - HCFO-1233zdE - HFO-1234zeE - HFO-1243zf**

| Organics 0,9 F245cb + 0,033 F1234zeE +0,033 F1233zdE +0,034 F1243zf | | Organics 0,033 F245cb + 0,9 F1234zeE +0,033 F1233zdE +0,034 F1243zf | | Organics 0,033 F245cb + 0,033 F1234zeE +0,9 F1233zdE +0,034 F1243zf | | Organics 0,034 F245cb + 0,033 F1234zeE +0,033 F1233zdE +0,9 F1243zf | | Organics 0,25 F245cb + 0,25 F1234zeE +0,25 F1233zdE +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 4,6 | 0 | 4,8 | 0 | 1,8 | 0 | 5,7 | 0 | 4,3 |
| 0,05 | 5,8 | 0,05 | 5,8 | 0,05 | 2,9 | 0,05 | 6,6 | 0,05 | 5,4 |
| 0,1 | 5,8 | 0,1 | 5,8 | 0,1 | 2,9 | 0,1 | 6,6 | 0,1 | 5,4 |
| 0,15 | 5,8 | 0,15 | 5,8 | 0,15 | 2,9 | 0,15 | 6,6 | 0,15 | 5,4 |
| 0,2 | 5,8 | 0,2 | 5,8 | 0,2 | 2,9 | 0,2 | 6,6 | 0,2 | 5,4 |
| 0,25 | 5,8 | 0,25 | 5,8 | 0,25 | 2,9 | 0,25 | 6,6 | 0,25 | 5,4 |
| 0,3 | 5,8 | 0,3 | 5,8 | 0,3 | 2,9 | 0,3 | 6,6 | 0,3 | 5,4 |
| 0,35 | 5,8 | 0,35 | 5,8 | 0,35 | 2,9 | 0,35 | 6,6 | 0,35 | 5,4 |
| 0,4 | 5,8 | 0,4 | 5,8 | 0,4 | 2,9 | 0,4 | 6,6 | 0,4 | 5,4 |
| 0,45 | 5,8 | 0,45 | 5,8 | 0,45 | 2,9 | 0,45 | 6,6 | 0,45 | 5,4 |
| 0,5 | 5,8 | 0,5 | 5,7 | 0,5 | 2,9 | 0,5 | 6,6 | 0,5 | 5,4 |
| 0,55 | 5,8 | 0,55 | 5,7 | 0,55 | 2,9 | 0,55 | 6,6 | 0,55 | 5,4 |
| 0,6 | 5,8 | 0,6 | 5,6 | 0,6 | 2,9 | 0,6 | 6,6 | 0,6 | 5,4 |
| 0,65 | 5,8 | 0,65 | 5,5 | 0,65 | 2,8 | 0,65 | 6,5 | 0,65 | 5,4 |
| 0,7 | 5,8 | 0,7 | 5,4 | 0,7 | 2,8 | 0,7 | 6,4 | 0,7 | 5,4 |
| 0,75 | 5,8 | 0,75 | 5,2 | 0,75 | 2,8 | 0,75 | 6,3 | 0,75 | 5,2 |
| 0,8 | 5,8 | 0,8 | 4,8 | 0,8 | 2,7 | 0,8 | 6,0 | 0,8 | 5,0 |
| 0,85 | 5,7 | 0,85 | 4,3 | 0,85 | 2,5 | 0,85 | 5,5 | 0,85 | 4,5 |
| 0,9 | 4,9 | 0,9 | 3,6 | 0,9 | 2,2 | 0,9 | 4,7 | 0,9 | 3,9 |
| 0,95 | 3,5 | 0,95 | 2,6 | 0,95 | 1,8 | 0,95 | 3,3 | 0,95 | 2,8 |
| 1 | 1,2 | 1 | 1,2 | 1 | 1,2 | 1 | 1,2 | 1 | 1,2 |

**HF -HFC-245cb - HCFO-1233zdE - HFO-1234zeZ - HFO-1243zf**

| Organics 0,9 F245cb + 0,033 F1234zeZ +0,033 F1233zdE +0,034 F1243zf | | Organics 0,033 F245cb + 0,9 F1234zeZ +0,033 F1233zdE +0,034 F1243zf | | Organics 0,033 F245cb + 0,033 F1234zeZ +0,9 F1233zdE +0,034 F1243zf | | Organics 0,034 F245cb + 0,033 F1234zeZ +0,033 F1233zdE +0,9 F1243zf | | Organics 0,25 F245cb + 0,25 F1234zeZ +0,25 F1233zdE +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 4,5 | 0 | 2,0 | 0 | 1,7 | 0 | 5,6 | 0 | 3,5 |
| 0,05 | 5,7 | 0,05 | 3,2 | 0,05 | 2,8 | 0,05 | 6,5 | 0,05 | 4,6 |
| 0,1 | 5,7 | 0,1 | 3,2 | 0,1 | 2,8 | 0,1 | 6,6 | 0,1 | 4,6 |
| 0,15 | 5,7 | 0,15 | 3,2 | 0,15 | 2,8 | 0,15 | 6,6 | 0,15 | 4,6 |
| 0,2 | 5,7 | 0,2 | 3,2 | 0,2 | 2,8 | 0,2 | 6,5 | 0,2 | 4,6 |
| 0,25 | 5,7 | 0,25 | 3,2 | 0,25 | 2,8 | 0,25 | 6,5 | 0,25 | 4,6 |
| 0,3 | 5,7 | 0,3 | 3,2 | 0,3 | 2,8 | 0,3 | 6,5 | 0,3 | 4,6 |
| 0,35 | 5,7 | 0,35 | 3,2 | 0,35 | 2,8 | 0,35 | 6,5 | 0,35 | 4,6 |
| 0,4 | 5,7 | 0,4 | 3,2 | 0,4 | 2,8 | 0,4 | 6,5 | 0,4 | 4,6 |
| 0,45 | 5,7 | 0,45 | 3,2 | 0,45 | 2,8 | 0,45 | 6,5 | 0,45 | 4,6 |
| 0,5 | 5,7 | 0,5 | 3,2 | 0,5 | 2,8 | 0,5 | 6,5 | 0,5 | 4,7 |
| 0,55 | 5,7 | 0,55 | 3,2 | 0,55 | 2,8 | 0,55 | 6,5 | 0,55 | 4,7 |
| 0,6 | 5,7 | 0,6 | 3,2 | 0,6 | 2,8 | 0,6 | 6,5 | 0,6 | 4,7 |
| 0,65 | 5,7 | 0,65 | 3,2 | 0,65 | 2,8 | 0,65 | 6,4 | 0,65 | 4,7 |
| 0,7 | 5,7 | 0,7 | 3,2 | 0,7 | 2,8 | 0,7 | 6,4 | 0,7 | 4,7 |
| 0,75 | 5,7 | 0,75 | 3,1 | 0,75 | 2,8 | 0,75 | 6,2 | 0,75 | 4,7 |
| 0,8 | 5,7 | 0,8 | 3,0 | 0,8 | 2,6 | 0,8 | 5,9 | 0,8 | 4,5 |
| 0,85 | 5,6 | 0,85 | 2,8 | 0,85 | 2,4 | 0,85 | 5,4 | 0,85 | 4,1 |
| 0,9 | 4,8 | 0,9 | 2,5 | 0,9 | 2,2 | 0,9 | 4,6 | 0,9 | 3,5 |
| 0,95 | 3,4 | 0,95 | 1,9 | 0,95 | 1,8 | 0,95 | 3,3 | 0,95 | 2,6 |
| 1 | 1,2 | 1 | 1,2 | 1 | 1,2 | 1 | 1,2 | 1 | 1,2 |

**HF - HCFO-1233zdE - HFO-1234zeE - HFO-1234zeZ - HFO-1243zf**

| Organics 0,9 F1234zeE + 0,033 F1234zeZ +0,033 F1233zdE +0,034 F1243zf | | Organics 0,033 F1234zeE + 0,9 F1234zeZ +0,033 F1233zdE +0,034 F1243zf | | Organics 0,033 F1234zeE + 0,033 F1234zeZ +0,9 F1233zdE +0,034 F1243zf | | Organics 0,034 F1234zeE + 0,033 F1234zeZ +0,033 F1233zdE +0,9 F1243zf | | Organics 0,25 F1234zeE+ 0,25 F1234zeZ +0,25 F1233zdE +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRES | HF | PRES | HF | PRES | HF | PRES | HF | PRES |
| | bar | | bar | | bar | | bar | | bar |
| 0 | 4,7 | 0 | 2,1 | 0 | 1,7 | 0 | 5,6 | 0 | 3,6 |
| 0,05 | 5,7 | 0,05 | 3,2 | 0,05 | 2,8 | 0,05 | 6,5 | 0,05 | 4,7 |
| 0,1 | 5,7 | 0,1 | 3,2 | 0,1 | 2,8 | 0,1 | 6,6 | 0,1 | 4,7 |
| 0,15 | 5,7 | 0,15 | 3,2 | 0,15 | 2,8 | 0,15 | 6,5 | 0,15 | 4,7 |
| 0,2 | 5,7 | 0,2 | 3,2 | 0,2 | 2,8 | 0,2 | 6,5 | 0,2 | 4,7 |
| 0,25 | 5,7 | 0,25 | 3,2 | 0,25 | 2,8 | 0,25 | 6,5 | 0,25 | 4,7 |
| 0,3 | 5,7 | 0,3 | 3,2 | 0,3 | 2,8 | 0,3 | 6,5 | 0,3 | 4,6 |
| 0,35 | 5,7 | 0,35 | 3,1 | 0,35 | 2,8 | 0,35 | 6,5 | 0,35 | 4,6 |
| 0,4 | 5,7 | 0,4 | 3,1 | 0,4 | 2,8 | 0,4 | 6,5 | 0,4 | 4,6 |
| 0,45 | 5,7 | 0,45 | 3,1 | 0,45 | 2,7 | 0,45 | 6,5 | 0,45 | 4,6 |
| 0,5 | 5,6 | 0,5 | 3,1 | 0,5 | 2,7 | 0,5 | 6,5 | 0,5 | 4,6 |
| 0,55 | 5,6 | 0,55 | 3,1 | 0,55 | 2,7 | 0,55 | 6,5 | 0,55 | 4,6 |
| 0,6 | 5,5 | 0,6 | 3,1 | 0,6 | 2,7 | 0,6 | 6,5 | 0,6 | 4,6 |
| 0,65 | 5,4 | 0,65 | 3,1 | 0,65 | 2,7 | 0,65 | 6,4 | 0,65 | 4,6 |
| 0,7 | 5,3 | 0,7 | 3,1 | 0,7 | 2,7 | 0,7 | 6,3 | 0,7 | 4,5 |
| 0,75 | 5,0 | 0,75 | 3,1 | 0,75 | 2,7 | 0,75 | 6,2 | 0,75 | 4,3 |
| 0,8 | 4,7 | 0,8 | 3,0 | 0,8 | 2,6 | 0,8 | 5,9 | 0,8 | 4,1 |
| 0,85 | 4,2 | 0,85 | 2,7 | 0,85 | 2,4 | 0,85 | 5,4 | 0,85 | 3,7 |
| 0,9 | 3,5 | 0,9 | 2,4 | 0,9 | 2,1 | 0,9 | 4,6 | 0,9 | 3,2 |
| 0,95 | 2,5 | 0,95 | 1,9 | 0,95 | 1,7 | 0,95 | 3,3 | 0,95 | 2,4 |
| 1 | 1,2 | 1 | 1,2 | 1 | 1,2 | 1 | 1,2 | 1 | 1,2 |

**HF -HFC-245cb - HFO-1234zeE - HFO-1234zeZ - HFO-1243zf**

| Organics 0,9 F245cb + 0,033 F1234zeE +0,033 F1234zeZ + 0,034 F1243zf | | Organics 0,033 F245cb + 0,9 F1234zeE +0,033 F1234zeZ + 0,034 F1243zf | | Organics 0,033 F245cb+ 0,033 F1234zeE +0,9 F1234zeZ +0,034 F1243zf | | Organics 0,034 F245cb+ 0,033 F1234zeE +0,033 F1234zeZ +0,9 F1243zf | | Organics 0,25 F245cb + 0,25 F1234zeE +0,25 F1234zeZ +0,25 F1243zf | |
|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE |
| | bar | | bar | | bar | | bar | | bar |
| 0 | 4,6 | 0 | 4,8 | 0 | 2,2 | 0 | 5,7 | 0 | 4,4 |
| 0,05 | 5,8 | 0,05 | 5,8 | 0,05 | 3,3 | 0,05 | 6,6 | 0,05 | 5,4 |
| 0,1 | 5,8 | 0,1 | 5,8 | 0,1 | 3,3 | 0,1 | 6,7 | 0,1 | 5,4 |
| 0,15 | 5,8 | 0,15 | 5,8 | 0,15 | 3,3 | 0,15 | 6,6 | 0,15 | 5,4 |
| 0,2 | 5,8 | 0,2 | 5,8 | 0,2 | 3,3 | 0,2 | 6,6 | 0,2 | 5,4 |
| 0,25 | 5,8 | 0,25 | 5,8 | 0,25 | 3,3 | 0,25 | 6,6 | 0,25 | 5,4 |
| 0,3 | 5,8 | 0,3 | 5,8 | 0,3 | 3,3 | 0,3 | 6,6 | 0,3 | 5,4 |
| 0,35 | 5,8 | 0,35 | 5,8 | 0,35 | 3,3 | 0,35 | 6,6 | 0,35 | 5,4 |
| 0,4 | 5,8 | 0,4 | 5,8 | 0,4 | 3,3 | 0,4 | 6,6 | 0,4 | 5,4 |
| 0,45 | 5,8 | 0,45 | 5,8 | 0,45 | 3,3 | 0,45 | 6,6 | 0,45 | 5,4 |
| 0,5 | 5,8 | 0,5 | 5,7 | 0,5 | 3,3 | 0,5 | 6,6 | 0,5 | 5,4 |
| 0,55 | 5,8 | 0,55 | 5,7 | 0,55 | 3,3 | 0,55 | 6,6 | 0,55 | 5,4 |
| 0,6 | 5,8 | 0,6 | 5,6 | 0,6 | 3,3 | 0,6 | 6, 6 | 0,6 | 5,4 |
| 0,65 | 5,8 | 0,65 | 5,5 | 0,65 | 3,3 | 0,65 | 6,5 | 0,65 | 5,4 |
| 0,7 | 5,8 | 0,7 | 5,4 | 0,7 | 3,2 | 0,7 | 6,4 | 0,7 | 5,4 |
| 0,75 | 5,8 | 0,75 | 5,2 | 0,75 | 3,2 | 0,75 | 6,3 | 0,75 | 5,3 |
| 0,8 | 5,8 | 0,8 | 4,8 | 0,8 | 3,1 | 0,8 | 6,0 | 0,8 | 5,0 |
| 0,85 | 5,7 | 0,85 | 4,3 | 0,85 | 2,9 | 0,85 | 5,5 | 0,85 | 4,6 |
| 0,9 | 4,9 | 0,9 | 3,6 | 0,9 | 2,5 | 0,9 | 4,7 | 0,9 | 3,9 |
| 0,95 | 3,5 | 0,95 | 2,6 | 0,95 | 2,0 | 0,95 | 3,3 | 0,95 | 2,8 |
| 1 | 1,2 | 1 | 1,2 | 1 | 1,2 | 1 | 1,2 | 1 | 1,2 |

### Exemple 8 : Plage de température et de pression de mélanges pentanaires

| | Enveloppe de points d'ébullition | |
|---|---|---|
| Système à 5 composés | Température °C | Pression bar abs |
| HF - HCFO-1233xf - HFC-245cb - HCFO-1233zdE - HFO-1234zeE | 0 à 40 | ∼1.0 ∼8.9 |
| HF - HCFO-1233xf - HFC-245cb - HCFO-1233zdE - HFO-1234zeZ | 0 à 40 | ∼1.0 à ∼8.8 |
| HF - HCFO-1233xf - HFC-245cb - HFO-1234zeE - HFO-1234zeZ | 0 à 40 | ∼1.1 à ∼8.8 |
| HF - HCFO-1233xf - HFC-245cb - HFO-1234zeE - HFO-1243zf | 0 à 40 | ∼1.0 à ∼10.2 |
| HF - HCFO-1233xf - HFC-245cb - HFO-1234zeE - HFO-1234yf | 0 à 40 | ∼1.0 à ∼11.4 |
| HF - HCFO-1233xf - HFC-245cb - HFO-1234zeZ - HFO-1243zf | 0 à 40 | ∼1.1 à ∼10.1 |
| HF - HCFO-1233xf - HFC-245cb - HFO-1234zeZ - HFO-1234yf | 0 à 40 | ∼1.1 à ∼11.2 |
| HF - HCFO-1233xf - HFO-1234yf - HFO-1234zeE - HFO-1234zeZ | 0 à 40 | ∼1.1 à ∼11.2 |
| HF - HCFO-1233xf - HFO-1234yf - HFO-1234zeE - HFO-1243zf | 0 à 40 | ∼1.2 à ∼11.4 |
| HF - HCFO-1233xf - HFO-1234yf - HFO-1234zeE - HCFO-1233zdE | 0 à 40 | ∼1.0 à ∼11.2 |
| HF - HCFO-1233xf - HFO-1234yf - HFO-1234zeZ - HFO-1243zf | 0 à 40 | ∼1.2 à ∼11.2 |
| HF - HCFO-1233xf - HFO-1234yf - HFO-1234zeZ - HCFO-1233zdE | 0 à 40 | ∼1.0 ∼11.1 |
| HF - HCFO-1233xf - HCFO-1233zdE - HFO-1234zeE - HFO-1234zeZ | 0 à 40 | ∼0.9 à ∼8.6 |
| HF - HCFO-1233xf - HCFO-1233zdE - HFO-1234zeE - HFO-1243zf | 0 à 40 | ∼1.0 à ∼10.1 |
| HF - HCFO-1233xf - HCFO-1233zdE - HFO-1234zeZ - HFO-1243zf | 0 à 40 | ∼1.0 à ∼9.9 |
| HF - HCFO-1233xf - HFO-1234zeE - HFO-1234zeZ - HFO-1243zf | 0 à 40 | ∼1.0 à ∼10.1 |
| HF - HFO-1234yf - HFO-1234zeE - HFO-1234zeZ - HCFO-1233zdE | 0 à 40 | ∼1.0 à ∼11.2 |
| HF - HFO-1234yf - HFO-1234zeE - HFO-1234zeZ - HFO-1243zf | 0 à 40 | ∼1.3 à ∼11.4 |
| HF - HFO-1234yf - HFO-1234zeE - HCFO-1233zdE - HFO-1243zf | 0 à 40 | ∼1.0 à ∼11.4 |
| HF - HFO-1234yf - HFO-1234zeZ - HCFO-1233zdE - HFO-1243zf | 0 à 40 | ∼1.1 à ∼11.2 |
| HF - HFO-1234yf - HFC-245cb - HFO-1234zeE - HCFO-1233zdE | 0 à 40 | ∼1.1 à ∼11.4 |
| HF - HFO-1234yf - HFC-245cb - HFO-1234zeE - HFO-1234zeZ | 0 à 40 | ∼1.3 à ∼9.1 |
| HF - HFO-1234yf - HFC-245cb - HFO-1234zeE - HFO-1243zf | 0 à 40 | ∼2.5 à ∼11.6 |
| HF - HFO-1234yf - HFC-245cb - HFO-1234zeZ - HCFO-1233zdE | 0 à 40 | ∼1.0 à ∼11.2 |
| HF - HFO-1234yf - HFC-245cb - HFO-1234zeZ- HFO-1243zf | 0 à 40 | ∼1.3 à ∼10.3 |
| HF - HFC-245cb - HCFO-1233zdE -HFO-1234zeE - HFO-1234zeZ | 0 à 40 | ∼1.0 à ∼8.9 |
| HF - HFC-245cb - HCFO-1233zdE - F 1234zeE - HFO-1243zf | 0 à 40 | ∼1.1 à ∼10.2 |
| HF - HFC-245cb - HCFO-1233zdE - F 1234zeZ - HFO-1243zf | 0 à 40 | ∼1.0 à ∼10.1 |
| HF - HFC-245cb - HFO-1234zeE - F 1234zeZ - HFO-1243zf | 0 à 40 | ∼1.2 à ∼10.2 |
| HF - HCFO-1233zdE - HFO-1234zeE - HFO-1234zeZ - HFO-1243zf | 0 à 40 | ∼1.0 à ∼10.1 |

### Exemple 9 : Plage de décantation de mélanges pentanaires

| | Plages de décantation | | |
|---|---|---|---|
| | Pourcentage massique d'HF | | |
| Système à 5 composés | Isotherme 0°C | Isotherme 25°C | Isotherme 40°C |
| HF - HCFO-1233xf - HFC-245cb - HCFO-1233zdE - HFO-1234zeE | 5-75 | 5-70 | 10-60 |
| HF - HCFO-1233xf - HFC-245cb - HCFO-1233zdE - HFO-1234zeZ | 5-80 | 5-75 | 5-70 |
| HF - HCFO-1233xf - HFC-245cb - HFO-1234zeE - HFO-1234zeZ | 5-75 | 5-70 | 10-60 |
| HF - HCFO-1233xf - HFC-245cb - HFO-1234zeE - HFO-1243zf | 10-75 | 10-65 | * |
| HF - HCFO-1233xf - HFC-245cb - HFO-1234zeE - HFO-1234yf | 5-75 | 10-70 | * |
| HF - HCFO-1233xf - HFC-245cb - HFO-1234zeZ - HFO-1243zf | 5-75 | 5-70 | 10-60 |
| HF - HCFO-1233xf - HFC-245cb - HFO-1234zeZ - HFO-1234yf | 5-75 | 5-75 | 10-65 |
| HF - HCFO-1233xf - HFO-1234yf - HFO-1234zeE - HFO-1234zeZ | 5-75 | 5-65 | 15-45 |
| HF - HCFO-1233xf - HFO-1234yf - HFO-1234zeE - HFO-1243zf | 5-70 | 10-60 | * |
| HF - HCFO-1233xf - HFO-1234yf - HFO-1234zeE - HCFO-1233zdE | 5-75 | 5-65 | 10-50 |
| HF - HCFO-1233xf - HFO-1234yf - HFO-1234zeZ - HFO-1243zf | 5-75 | 10-65 | 20-40 |
| HF - HCFO-1233xf - HFO-1234yf - HFO-1234zeZ - HCFO-1233zdE | 5-75 | 5-70 | 10-60 |
| HF - HCFO-1233xf - HCFO-1233zdE - HFO-1234zeE - HFO-1234zeZ | 5-75 | 5-70 | 5-60 |
| HF - HCFO-1233xf - HCFO-1233zdE - HFO-1234zeE - HFO-1243zf | 5-75 | 5-65 | 15-45 |
| HF - HCFO-1233xf - HCFO-1233zdE - HFO-1234zeZ - HFO-1243zf | 5-75 | 5-70 | 10-60 |
| HF - HCFO-1233xf - HFO-1234zeE - HFO-1234zeZ - HFO-1243zf | 5-75 | 10-65 | 15-40 |
| HF - HFO-1234yf - HFO-1234zeE - HFO-1234zeZ - HCFO-1233zdE | 5-75 | 5-65 | 15-45 |
| HF - HFO-1234yf - HFO-1234zeE - HFO-1234zeZ - HFO-1243zf | 5-70 | 10-60 | * |
| HF - HFO-1234yf - HFO-1234zeE - HCFO-1233zdE - HFO-1243zf | 5-70 | 10-60 | * |
| HF - HFO-1234yf - HFO-1234zeZ - HCFO-1233zdE - HFO-1243zf | 5-75 | 10-65 | 15-45 |
| HF - HFO-1234yf - HFC-245cb - HFO-1234zeE - HCFO-1233zdE | 5-75 | 10-70 | * |
| HF - HFO-1234yf - HFC-245cb - HFO-1234zeE - HFO-1234zeZ | 5-75 | 10-70 | * |
| HF - HFO-1234yf - HFC-245cb - HFO-1234zeE - HFO-1243zf | 5-75 | 15-65 | * |
| HF - HFO-1234yf - HFC-245cb - HFO-1234zeZ - HCFO-1233zdE | 5-75 | 5-75 | 10-65 |
| HF - HFO-1234yf - HFC-245cb - HFO-1234zeZ- HFO-1243zf | 5-75 | 10-70 | * |
| HF - HFC-245cb - HCFO-1233zdE -HFO-1234zeE - HFO-1234zeZ | 5-75 | 5-70 | 10-60 |
| HF - HFC-245cb - HCFO-1233zdE - F 1234zeE - HFO-1243zf | 5-75 | 10-65 | * |
| HF - HFC-245cb - HCFO-1233zdE - F 1234zeZ - HFO-1243zf | 5-75 | 5-70 | 10-60 |
| HF - HFC-245cb - HFO-1234zeE - F 1234zeZ - HFO-1243zf | 5-75 | 10-65 | * |
| HF - HCFO-1233zdE - HFO-1234zeE - HFO-1234zeZ - HFO-1243zf | 5-75 | 5-65 | 15-50 |

### Exemple 10 : Systèmes à six composés, isotherme à 25 °C

**HF - HCFO-1233xf - HFO-1234f - HFC-245cb - HFO-1234zeE- HFO-1234zeZ**

| **Organics 0,2 F1233xf + 0,2 F1234yf + 0,2 F245cb 0,2 F1234zeE + 0,2 F1234zeZ** | | **Organics 0,96 F1233xf + 0,01 F1234yf + 0,01 F245cb + 0,01 F1234zeE + 0,01 F1234zeZ** | | **Organics 0,01 F1233xf + 0,96 F1234yf + 0,01 F245cb + 0,01 F1234zeE + 0,01 F1234zeZ** | | **Organics 0,01 F1233xf + 0,01 F1234yf + 0,96 F245cb + 0,01 F1234zeE + 0,01 F1234zeZ** | | **Organics 0,01 F1233xf + 0,01 F1234yf + 0,01 F245cb + 0,96 F1234zeE + 0,01 F1234zeZ** | | **Organics 0,01 F1233xf + 0,01 F1234yf + 0,01 F245cb + 0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,0 | 0 | 1,7 | 0 | 6,7 | 0 | 4,6 | 0 | 4,8 | 0 | 1,9 |
| 0,05 | 5,1 | 0,05 | 2,8 | 0,05 | 7,6 | 0,05 | 5,8 | 0,05 | 5,8 | 0,05 | 3,0 |
| 0,1 | 5,1 | 0,1 | 2,8 | 0,1 | 7,6 | 0,1 | 5,8 | 0,1 | 5,8 | 0,1 | 3,0 |
| 0,15 | 5,1 | 0,15 | 2,8 | 0,15 | 7,6 | 0,15 | 5,8 | 0,15 | 5,8 | 0,15 | 3,0 |
| 0,2 | 5,1 | 0,2 | 2,8 | 0,2 | 7,6 | 0,2 | 5,8 | 0,2 | 5,8 | 0,2 | 3,0 |
| 0,25 | 5,1 | 0,25 | 2,8 | 0,25 | 7,6 | 0,25 | 5,8 | 0,25 | 5,8 | 0,25 | 3,0 |
| 0,3 | 5,1 | 0,3 | 2,8 | 0,3 | 7,6 | 0,3 | 5,8 | 0,3 | 5,8 | 0,3 | 3,0 |
| 0,35 | 5,1 | 0,35 | 2,8 | 0,35 | 7,6 | 0,35 | 5,8 | 0,35 | 5,8 | 0,35 | 3,0 |
| 0,4 | 5,1 | 0,4 | 2,8 | 0,4 | 7,6 | 0,4 | 5,8 | 0,4 | 5,8 | 0,4 | 3,0 |
| 0,45 | 5,1 | 0,45 | 2,8 | 0,45 | 7,6 | 0,45 | 5,8 | 0,45 | 5,8 | 0,45 | 3,0 |
| 0,5 | 5,1 | 0,5 | 2,8 | 0,5 | 7,6 | 0,5 | 5,8 | 0,5 | 5,7 | 0,5 | 3,0 |
| 0,55 | 5,1 | 0,55 | 2,8 | 0,55 | 7,6 | 0,55 | 5,8 | 0,55 | 5,7 | 0,55 | 3,0 |
| 0,6 | 5,1 | 0,6 | 2,8 | 0,6 | 7,6 | 0,6 | 5,8 | 0,6 | 5,6 | 0,6 | 3,0 |
| 0,65 | 5,1 | 0,65 | 2,8 | 0,65 | 7,6 | 0,65 | 5,8 | 0,65 | 5,5 | 0,65 | 3,0 |
| 0,7 | 5,1 | 0,7 | 2,8 | 0,7 | 7,6 | 0,7 | 5,8 | 0,7 | 5,4 | 0,7 | 3,0 |
| 0,75 | 5,0 | 0,75 | 2,8 | 0,75 | 7,5 | 0,75 | 5,8 | 0,75 | 5,1 | 0,75 | 3,0 |
| 0,8 | 4,8 | 0,8 | 2,6 | 0,8 | 7,1 | 0,8 | 5,8 | 0,8 | 4,8 | 0,8 | 2,9 |
| 0,85 | 4,4 | 0,85 | 2,5 | 0,85 | 6,5 | 0,85 | 5,8 | 0,85 | 4,3 | 0,85 | 2,7 |
| 0,9 | 3,7 | 0,9 | 2,2 | 0,9 | 5,5 | 0,9 | 5,0 | 0,9 | 3,6 | 0,9 | 2,4 |
| 0,95 | 2,7 | 0,95 | 1,8 | 0,95 | 3,8 | 0,95 | 3,5 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HCFO-1233xf - HFO-1234f - HFC-245cb - HFO-1234zeE- HCFO-1233zdE**

| **Organics 0,2 F1233xf + 0,2 F1234yf + 0,2 F245cb 0,2 F1234zeE + 0,2 F1233zdE** | | **Organics 0,96 F1233xf + 0,01 F1234yf + 0,01 F245cb + 0,01 F1234zeE + 0,01 F1233zdE** | | **Organics 0,01 F1233xf + 0,96 F1234yf + 0,01 F245cb + 0,01 F1234zeE + 0,01 F1233zdE** | | **Organics 0,01 F1233xf + 0,01 F1234yf + 0,96 F245cb + 0,01 F1234zeE + 0,01 F1233zdE** | | **Organics 0,01 F1233xf + 0,01 F1234yf + 0,01 F245cb + 0,96 F1234zeE + 0,01 F1233zdE** | | **Organics 0,01 F1233xf + 0,01 F1234yf + 0,01 F245cb + 0,01 F1234zeE + 0,96 F1233zdE** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,9 | 0 | 1,7 | 0 | 6,7 | 0 | 4,6 | 0 | 4,8 | 0 | 1,5 |
| 0,05 | 5,0 | 0,05 | 2,8 | 0,05 | 7,6 | 0,05 | 5,8 | 0,05 | 5,8 | 0,05 | 2,6 |
| 0,1 | 5,0 | 0,1 | 2,8 | 0,1 | 7,6 | 0,1 | 5,8 | 0,1 | 5,8 | 0,1 | 2,6 |
| 0,15 | 5,0 | 0,15 | 2,8 | 0,15 | 7,6 | 0,15 | 5,8 | 0,15 | 5,8 | 0,15 | 2,6 |
| 0,2 | 5,0 | 0,2 | 2,8 | 0,2 | 7,6 | 0,2 | 5,8 | 0,2 | 5,8 | 0,2 | 2,6 |
| 0,25 | 5,0 | 0,25 | 2,8 | 0,25 | 7,6 | 0,25 | 5,8 | 0,25 | 5,8 | 0,25 | 2,6 |
| 0,3 | 5,0 | 0,3 | 2,8 | 0,3 | 7,6 | 0,3 | 5,8 | 0,3 | 5,8 | 0,3 | 2,6 |
| 0,35 | 5,0 | 0,35 | 2,8 | 0,35 | 7,6 | 0,35 | 5,8 | 0,35 | 5,8 | 0,35 | 2,6 |
| 0,4 | 5,0 | 0,4 | 2,8 | 0,4 | 7,6 | 0,4 | 5,8 | 0,4 | 5,8 | 0,4 | 2,6 |
| 0,45 | 5,0 | 0,45 | 2,8 | 0,45 | 7,6 | 0,45 | 5,8 | 0,45 | 5,8 | 0,45 | 2,6 |
| 0,5 | 5,0 | 0,5 | 2,8 | 0,5 | 7,6 | 0,5 | 5,8 | 0,5 | 5,7 | 0,5 | 2,6 |
| 0,55 | 5,0 | 0,55 | 2,8 | 0,55 | 7,6 | 0,55 | 5,8 | 0,55 | 5,7 | 0,55 | 2,6 |
| 0,6 | 5,0 | 0,6 | 2,8 | 0,6 | 7,6 | 0,6 | 5,8 | 0,6 | 5,6 | 0,6 | 2,6 |
| 0,65 | 5,0 | 0,65 | 2,8 | 0,65 | 7,6 | 0,65 | 5,8 | 0,65 | 5,5 | 0,65 | 2,6 |
| 0,7 | 5,1 | 0,7 | 2,8 | 0,7 | 7,6 | 0,7 | 5,8 | 0,7 | 5,4 | 0,7 | 2,6 |
| 0,75 | 5,0 | 0,75 | 2,8 | 0,75 | 7,5 | 0,75 | 5,8 | 0,75 | 5,1 | 0,75 | 2,6 |
| 0,8 | 4,7 | 0,8 | 2,6 | 0,8 | 7,1 | 0,8 | 5,8 | 0,8 | 4,8 | 0,8 | 2,4 |
| 0,85 | 4,3 | 0,85 | 2,5 | 0,85 | 6,5 | 0,85 | 5,8 | 0,85 | 4,3 | 0,85 | 2,3 |
| 0,9 | 3,7 | 0,9 | 2,2 | 0,9 | 5,5 | 0,9 | 5,0 | 0,9 | 3,6 | 0,9 | 2,0 |
| 0,95 | 2,7 | 0,95 | 1,8 | 0,95 | 3,8 | 0,95 | 3,5 | 0,95 | 2,6 | 0,95 | 1,7 |
| 1 | 1,2 | 1 | 1,2 | 1 | 1,2 | 1 | 1,2 | 1 | 1,2 | 1 | 1,2 |

**HF - HCFO-1233xf - HFO-1234yf - HFC-245cb - HFO-1234zeE- HFO-1243zf**

| **Organics 0,2 F1233xf + 0,2 F1234yf + 0,2 F245cb 0,2 F1234zeE + 0,2 F1243zf** | | **Organics 0,96 F1233xf + 0,01 F1234yf + 0,01 F245cb + 0,01 F1234zeE + 0,01 F1243zf** | | **Organics 0,01 F1233xf + 0,96 F1234yf + 0,01 F245cb + 0,01 F1234zeE + 0,01 F1243zf** | | **Organics 0,01 F1233xf + 0,01 F1234yf + 0,96 F245cb + 0,01 F1234zeE + 0,01 F1243zf** | | **Organics 0,01 F1233xf + 0,01 F1234yf + 0,01 F245cb + 0,96 F1234zeE + 0,01 F1243zf** | | **Organics 0,01 F1233xf + 0,01 F1234yf + 0,01 F245cb + 0,01 F1234zeE + 0,96 F1243zf** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,9 | 0 | 1,7 | 0 | 6,7 | 0 | 4,7 | 0 | 4,9 | 0 | 5,8 |
| 0,05 | 5,9 | 0,05 | 2,8 | 0,05 | 7,7 | 0,05 | 5,8 | 0,05 | 5,8 | 0,05 | 6,7 |
| 0,1 | 5,9 | 0,1 | 2,8 | 0,1 | 7,7 | 0,1 | 5,8 | 0,1 | 5,9 | 0,1 | 6,8 |
| 0,15 | 5,9 | 0,15 | 2,8 | 0,15 | 7,7 | 0,15 | 5,8 | 0,15 | 5,8 | 0,15 | 6,8 |
| 0,2 | 5,9 | 0,2 | 2,8 | 0,2 | 7,7 | 0,2 | 5,8 | 0,2 | 5,8 | 0,2 | 6,8 |
| 0,25 | 5,9 | 0,25 | 2,8 | 0,25 | 7,7 | 0,25 | 5,8 | 0,25 | 5,8 | 0,25 | 6,8 |
| 0,3 | 5,9 | 0,3 | 2,8 | 0,3 | 7,7 | 0,3 | 5,8 | 0,3 | 5,8 | 0,3 | 6,8 |
| 0,35 | 5,9 | 0,35 | 2,8 | 0,35 | 7,7 | 0,35 | 5,8 | 0,35 | 5,8 | 0,35 | 6,8 |
| 0,4 | 5,9 | 0,4 | 2,8 | 0,4 | 7,7 | 0,4 | 5,8 | 0,4 | 5,8 | 0,4 | 6,8 |
| 0,45 | 5,9 | 0,45 | 2,8 | 0,45 | 7,7 | 0,45 | 5,8 | 0,45 | 5,8 | 0,45 | 6,7 |
| 0,5 | 5,9 | 0,5 | 2,8 | 0,5 | 7,7 | 0,5 | 5,8 | 0,5 | 5,8 | 0,5 | 6,7 |
| 0,55 | 5,9 | 0,55 | 2,8 | 0,55 | 7,7 | 0,55 | 5,8 | 0,55 | 5,7 | 0,55 | 6,7 |
| 0,6 | 5,9 | 0,6 | 2,8 | 0,6 | 7,7 | 0,6 | 5,8 | 0,6 | 5,7 | 0,6 | 6,7 |
| 0,65 | 5,9 | 0,65 | 2,8 | 0,65 | 7,7 | 0,65 | 5,8 | 0,65 | 5,6 | 0,65 | 6,6 |
| 0,7 | 5,9 | 0,7 | 2,8 | 0,7 | 7,6 | 0,7 | 5,8 | 0,7 | 5,4 | 0,7 | 6,5 |
| 0,75 | 5,7 | 0,75 | 2,8 | 0,75 | 7,5 | 0,75 | 5,8 | 0,75 | 5,2 | 0,75 | 6,4 |
| 0,8 | 5,5 | 0,8 | 2,7 | 0,8 | 7,2 | 0,8 | 5,8 | 0,8 | 4,8 | 0,8 | 6,1 |
| 0,85 | 5,0 | 0,85 | 2,5 | 0,85 | 6,6 | 0,85 | 5,8 | 0,85 | 4,3 | 0,85 | 5,6 |
| 0,9 | 4,2 | 0,9 | 2,2 | 0,9 | 5,5 | 0,9 | 5,0 | 0,9 | 3,6 | 0,9 | 4,8 |
| 0,95 | 3,0 | 0,95 | 1,8 | 0,95 | 3,8 | 0,95 | 3,5 | 0,95 | 2,6 | 0,95 | 3,4 |
| 1 | 1,2 | 1 | 1,2 | 1 | 1,2 | 1 | 1,2 | 1 | 1,2 | 1 | 1,2 |

**HF - HCFO-1233xf - HFO-1234f - HFC-245cb - HFO-1234zeZ- HCFO-1233zdE**

| **Organics 0,2 F1233xf + 0,2 F1234yf + 0,2 F245cb 0,2 F1234zeZ + 0,2 F1233zdE** | | **Organics 0,96 F1233xf + 0,01 F1234yf + 0,01 F245cb + 0,01 F1234zeZ + 0,01 F1233zdE** | | **Organics 0,01 F1233xf + 0,96 F1234yf + 0,01 F245cb + 0,01 F1234zeZ + 0,01 F1233zdE** | | **Organics 0,01 F1233xf + 0,01 F1234yf + 0,96 F245cb + 0,01 F1234zeZ + 0,01 F1233zdE** | | **Organics 0,01 F1233xf + 0,01 F1234yf + 0,01 F245cb + 0,96 F1234zeZ + 0,01 F1233zdE** | | **Organics 0,01 F1233xf + 0,01 F1234yf + 0,01 F245cb + 0,01 F1234zeZ + 0,96 F1233zdE** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,3 | 0 | 1,6 | 0 | 6,6 | 0 | 4,6 | 0 | 1,9 | 0 | 1,4 |
| 0,05 | 4,4 | 0,05 | 2,8 | 0,05 | 7,6 | 0,05 | 5,8 | 0,05 | 3,0 | 0,05 | 2,5 |
| 0,1 | 4,4 | 0,1 | 2,8 | 0,1 | 7,6 | 0,1 | 5,8 | 0,1 | 3,0 | 0,1 | 2,5 |
| 0,15 | 4,4 | 0,15 | 2,8 | 0,15 | 7,6 | 0,15 | 5,8 | 0,15 | 3,0 | 0,15 | 2,5 |
| 0,2 | 4,4 | 0,2 | 2,8 | 0,2 | 7,6 | 0,2 | 5,8 | 0,2 | 3,0 | 0,2 | 2,5 |
| 0,25 | 4,4 | 0,25 | 2,8 | 0,25 | 7,6 | 0,25 | 5,8 | 0,25 | 3,0 | 0,25 | 2,5 |
| 0,3 | 4,4 | 0,3 | 2,8 | 0,3 | 7,6 | 0,3 | 5,8 | 0,3 | 3,0 | 0,3 | 2,5 |
| 0,35 | 4,4 | 0,35 | 2,8 | 0,35 | 7,6 | 0,35 | 5,8 | 0,35 | 3,0 | 0,35 | 2,5 |
| 0,4 | 4,4 | 0,4 | 2,8 | 0,4 | 7,6 | 0,4 | 5,8 | 0,4 | 3,0 | 0,4 | 2,5 |
| 0,45 | 4,4 | 0,45 | 2,8 | 0,45 | 7,6 | 0,45 | 5,8 | 0,45 | 3,0 | 0,45 | 2,5 |
| 0,5 | 4,4 | 0,5 | 2,8 | 0,5 | 7,6 | 0,5 | 5,8 | 0,5 | 3,0 | 0,5 | 2,5 |
| 0,55 | 4,4 | 0,55 | 2,8 | 0,55 | 7,6 | 0,55 | 5,8 | 0,55 | 3,0 | 0,55 | 2,5 |
| 0,6 | 4,4 | 0,6 | 2,8 | 0,6 | 7,6 | 0,6 | 5,8 | 0,6 | 3,0 | 0,6 | 2,5 |
| 0,65 | 4,5 | 0,65 | 2,8 | 0,65 | 7,6 | 0,65 | 5,8 | 0,65 | 3,0 | 0,65 | 2,5 |
| 0,7 | 4,5 | 0,7 | 2,8 | 0,7 | 7,6 | 0,7 | 5,8 | 0,7 | 3,0 | 0,7 | 2,5 |
| 0,75 | 4,5 | 0,75 | 2,8 | 0,75 | 7,4 | 0,75 | 5,8 | 0,75 | 3,0 | 0,75 | 2,5 |
| 0,8 | 4,3 | 0,8 | 2,6 | 0,8 | 7,1 | 0,8 | 5,8 | 0,8 | 2,9 | 0,8 | 2,4 |
| 0,85 | 4,0 | 0,85 | 2,4 | 0,85 | 6,5 | 0,85 | 5,8 | 0,85 | 2,7 | 0,85 | 2,3 |
| 0,9 | 3,4 | 0,9 | 2,2 | 0,9 | 5,5 | 0,9 | 5,0 | 0,9 | 2,3 | 0,9 | 2,0 |
| 0,95 | 2,5 | 0,95 | 1,7 | 0,95 | 3,8 | 0,95 | 3,5 | 0,95 | 1,9 | 0,95 | 1,7 |
| 1 | 1,2 | 1 | 1,2 | 1 | 1,2 | 1 | 1,2 | 1 | 1,2 | 1 | 1,2 |

**HF - HCFO-1233xf - HFO-1234yf - HFC-245cb - HFO-1234zeZ- HFO-1243zf**

| **Organics 0,2 F1233xf + 0,2 F1234yf + 0,2 F245cb 0,2 F1234zeZ + 0,2 F1243zf** | | **Organics 0,96 F1233xf + 0,01 F1234yf + 0,01 F245cb + 0,01 F1234zeZ + 0,01 F1243zf** | | **Organics 0,01 F1233xf + 0,96 F1234yf + 0,01 F245cb + 0,01 F1234zeZ + 0,01 F1243zf** | | **Organics 0,01 F1233xf + 0,01 F1234yf + 0,96 F245cb + 0,01 F1234zeZ + 0,01 F1243zf** | | **Organics 0,01 F1233xf + 0,01 F1234yf + 0,01 F245cb + 0,96 F1234zeZ + 0,01 F1243zf** | | **Organics 0,01 F1233xf + 0,01 F1234yf + 0,01 F245cb + 0,01 F1234zeZ + 0,96 F1243zf** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |

| HF | PRESSURE | HF | PRESSURE | HF | PRESSURE | HF | PRESSURE | HF | PRESSURE | HF | PRESSURE |
|---|---|---|---|---|---|---|---|---|---|---|---|
| | bar | | bar | | bar | | bar | | bar | | bar |
| 0 | 4,2 | 0 | 1,7 | 0 | 6,7 | 0 | 4,6 | 0 | 1,9 | 0 | 5,8 |
| 0,05 | 5,3 | 0,05 | 2,8 | 0,05 | 7,6 | 0,05 | 5,8 | 0,05 | 3,0 | 0,05 | 6,7 |
| 0,1 | 5,3 | 0,1 | 2,8 | 0,1 | 7,7 | 0,1 | 5,8 | 0,1 | 3,0 | 0,1 | 6,8 |
| 0,15 | 5,3 | 0,15 | 2,8 | 0,15 | 7,6 | 0,15 | 5,8 | 0,15 | 3,0 | 0,15 | 6,7 |
| 0,2 | 5,3 | 0,2 | 2,8 | 0,2 | 7,6 | 0,2 | 5,8 | 0,2 | 3,0 | 0,2 | 6,7 |
| 0,25 | 5,3 | 0,25 | 2,8 | 0,25 | 7,6 | 0,25 | 5,8 | 0,25 | 3,0 | 0,25 | 6,7 |
| 0,3 | 5,3 | 0,3 | 2,8 | 0,3 | 7,6 | 0,3 | 5,8 | 0,3 | 3,0 | 0,3 | 6,7 |
| 0,35 | 5,3 | 0,35 | 2,8 | 0,35 | 7,6 | 0,35 | 5,8 | 0,35 | 3,0 | 0,35 | 6,7 |
| 0,4 | 5,3 | 0,4 | 2,8 | 0,4 | 7,6 | 0,4 | 5,8 | 0,4 | 3,0 | 0,4 | 6,7 |
| 0,45 | 5,3 | 0,45 | 2,8 | 0,45 | 7,6 | 0,45 | 5,8 | 0,45 | 3,0 | 0,45 | 6,7 |
| 0,5 | 5,3 | 0,5 | 2,8 | 0,5 | 7,6 | 0,5 | 5,8 | 0,5 | 3,0 | 0,5 | 6,7 |
| 0,55 | 5,3 | 0,55 | 2,8 | 0,55 | 7,6 | 0,55 | 5,8 | 0,55 | 3,0 | 0,55 | 6,7 |
| 0,6 | 5,3 | 0,6 | 2,8 | 0,6 | 7,6 | 0,6 | 5,8 | 0,6 | 3,0 | 0,6 | 6,7 |
| 0,65 | 5,3 | 0,65 | 2,8 | 0,65 | 7,6 | 0,65 | 5,8 | 0,65 | 3,0 | 0,65 | 6,6 |
| 0,7 | 5,3 | 0,7 | 2,8 | 0,7 | 7,6 | 0,7 | 5,8 | 0,7 | 3,0 | 0,7 | 6,5 |
| 0,75 | 5,3 | 0,75 | 2,8 | 0,75 | 7,5 | 0,75 | 5,8 | 0,75 | 3,0 | 0,75 | 6,4 |
| 0,8 | 5,1 | 0,8 | 2,7 | 0,8 | 7,2 | 0,8 | 5,8 | 0,8 | 2,9 | 0,8 | 6,1 |
| 0,85 | 4,7 | 0,85 | 2,5 | 0,85 | 6,6 | 0,85 | 5,8 | 0,85 | 2,7 | 0,85 | 5,6 |
| 0,9 | 4,0 | 0,9 | 2,2 | 0,9 | 5,5 | 0,9 | 5,0 | 0,9 | 2,4 | 0,9 | 4,7 |
| 0,95 | 2,9 | 0,95 | 1,8 | 0,95 | 3,8 | 0,95 | 3,5 | 0,95 | 1,9 | 0,95 | 3,4 |
| 1 | 1,2 | 1 | 1,2 | 1 | 1,2 | 1 | 1,2 | 1 | 1,2 | 1 | 1,2 |

**HF - HCFO-1233xf - HFO-1234f - HFO-1234zeE - HFO-1234zeZ - HCFO-1233zdE**

| **Organics 0,2 F1233xf + 0,2 F1234yf + 0,2 F1233zdE 0,2 F1234zeE + 0,2 F1234zeZ** | | **Organics 0,96 F1233xf + 0,01 F1234yf + 0,01 F1233zdE + 0,01 F1234zeE + 0,01 F1234zeZ** | | **Organics 0,01 F1233xf + 0,96 F1234yf + 0,01 F1233zdE + 0,01 F1234zeE + 0,01 F1234zeZ** | | **Organics 0,01 F1233xf + 0,01 F1234yf + 0,96 F1233zdE + 0,01 F1234zeE + 0,01 F1234zeZ** | | **Organics 0,01 F1233xf + 0,01 F1234yf + 0,01 F1233zdE + 0,96 F1234zeE + 0,01 F1234zeZ** | | **Organics 0,01 F1233xf + 0,01 F1234yf + 0,01 F1233zdE + 0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,4 | 0 | 1,6 | 0 | 6,6 | 0 | 1,4 | 0 | 4,8 | 0 | 1,9 |
| 0,05 | 4,4 | 0,05 | 2,8 | 0,05 | 7,6 | 0,05 | 2,5 | 0,05 | 5,8 | 0,05 | 3,0 |
| 0,1 | 4,4 | 0,1 | 2,8 | 0,1 | 7,6 | 0,1 | 2,5 | 0,1 | 5,8 | 0,1 | 3,0 |
| 0,15 | 4,4 | 0,15 | 2,8 | 0,15 | 7,6 | 0,15 | 2,5 | 0,15 | 5,8 | 0,15 | 3,0 |
| 0,2 | 4,4 | 0,2 | 2,8 | 0,2 | 7,6 | 0,2 | 2,5 | 0,2 | 5,8 | 0,2 | 3,0 |
| 0,25 | 4,4 | 0,25 | 2,8 | 0,25 | 7,6 | 0,25 | 2,5 | 0,25 | 5,8 | 0,25 | 3,0 |
| 0,3 | 4,4 | 0,3 | 2,8 | 0,3 | 7,6 | 0,3 | 2,5 | 0,3 | 5,8 | 0,3 | 3,0 |
| 0,35 | 4,4 | 0,35 | 2,8 | 0,35 | 7,6 | 0,35 | 2,5 | 0,35 | 5,8 | 0,35 | 3,0 |
| 0,4 | 4,4 | 0,4 | 2,8 | 0,4 | 7,6 | 0,4 | 2,5 | 0,4 | 5,8 | 0,4 | 3,0 |
| 0,45 | 4,4 | 0,45 | 2,7 | 0,45 | 7,6 | 0,45 | 2,5 | 0,45 | 5,7 | 0,45 | 3,0 |
| 0,5 | 4,4 | 0,5 | 2,7 | 0,5 | 7,6 | 0,5 | 2,5 | 0,5 | 5,7 | 0,5 | 3,0 |
| 0,55 | 4,4 | 0,55 | 2,7 | 0,55 | 7,6 | 0,55 | 2,5 | 0,55 | 5,7 | 0,55 | 3,0 |
| 0,6 | 4,4 | 0,6 | 2,7 | 0,6 | 7,6 | 0,6 | 2,5 | 0,6 | 5,6 | 0,6 | 3,0 |
| 0,65 | 4,4 | 0,65 | 2,7 | 0,65 | 7,6 | 0,65 | 2,5 | 0,65 | 5,5 | 0,65 | 3,0 |
| 0,7 | 4,4 | 0,7 | 2,7 | 0,7 | 7,6 | 0,7 | 2,5 | 0,7 | 5,3 | 0,7 | 3,0 |
| 0,75 | 4,2 | 0,75 | 2,7 | 0,75 | 7,4 | 0,75 | 2,5 | 0,75 | 5,1 | 0,75 | 3,0 |
| 0,8 | 4,0 | 0,8 | 2,6 | 0,8 | 7,1 | 0,8 | 2,4 | 0,8 | 4,8 | 0,8 | 2,8 |
| 0,85 | 3,6 | 0,85 | 2,4 | 0,85 | 6,5 | 0,85 | 2,2 | 0,85 | 4,3 | 0,85 | 2,6 |
| 0,9 | 3,1 | 0,9 | 2,1 | 0,9 | 5,5 | 0,9 | 2,0 | 0,9 | 3,6 | 0,9 | 2,3 |
| 0,95 | 2,3 | 0,95 | 1,7 | 0,95 | 3,8 | 0,95 | 1,7 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HCFO-1233xf - HFO-1234f - HFO-1234zeE - HFO-1234zeZ - HFO-1243zf**

| **Organics 0,2 F1233xf + 0,2 F1234yf + 0,2 F1243zf 0,2 F1234zeE + 0,2 F1234zeZ** | | **Organics 0,96 F1233xf + 0,01 F1234yf + 0,01 F1243zf + 0,01 F1234zeE + 0,01 F1234zeZ** | | **Organics 0,01 F1233xf + 0,96 F1234yf + 0,01 F1243zf + 0,01 F1234zeE + 0,01 F1234zeZ** | | **Organics 0,01 F1233xf + 0,01 F1234yf + 0,96 F1243zf + 0,01 F1234zeE + 0,01 F1234zeZ** | | **Organics 0,01 F1233xf + 0,01 F1234yf + 0,01 F1243zf + 0,96 F1234zeE + 0,01 F1234zeZ** | | **Organics 0,01 F1233xf + 0,01 F1234yf + 0,01 F1243zf + 0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,3 | 0 | 1,7 | 0 | 6,7 | 0 | 5,8 | 0 | 4,8 | 0 | 1,9 |
| 0,05 | 5,3 | 0,05 | 2,8 | 0,05 | 7,6 | 0,05 | 6,7 | 0,05 | 5,8 | 0,05 | 3,0 |
| 0,1 | 5,3 | 0,1 | 2,8 | 0,1 | 7,6 | 0,1 | 6,7 | 0,1 | 5,8 | 0,1 | 3,0 |
| 0,15 | 5,3 | 0,15 | 2,8 | 0,15 | 7,6 | 0,15 | 6,7 | 0,15 | 5,8 | 0,15 | 3,0 |
| 0,2 | 5,3 | 0,2 | 2,8 | 0,2 | 7,6 | 0,2 | 6,7 | 0,2 | 5,8 | 0,2 | 3,0 |
| 0,25 | 5,3 | 0,25 | 2,8 | 0,25 | 7,6 | 0,25 | 6,7 | 0,25 | 5,8 | 0,25 | 3,0 |
| 0,3 | 5,3 | 0,3 | 2,8 | 0,3 | 7,6 | 0,3 | 6,7 | 0,3 | 5,8 | 0,3 | 3,0 |
| 0,35 | 5,3 | 0,35 | 2,8 | 0,35 | 7,6 | 0,35 | 6,7 | 0,35 | 5,8 | 0,35 | 3,0 |
| 0,4 | 5,3 | 0,4 | 2,8 | 0,4 | 7,6 | 0,4 | 6,7 | 0,4 | 5,8 | 0,4 | 3,0 |
| 0,45 | 5,3 | 0,45 | 2,8 | 0,45 | 7,6 | 0,45 | 6,7 | 0,45 | 5,8 | 0,45 | 3,0 |
| 0,5 | 5,3 | 0,5 | 2,8 | 0,5 | 7,6 | 0,5 | 6,7 | 0,5 | 5,8 | 0,5 | 3,0 |
| 0,55 | 5,3 | 0,55 | 2,8 | 0,55 | 7,6 | 0,55 | 6,7 | 0,55 | 5,7 | 0,55 | 3,0 |
| 0,6 | 5,3 | 0,6 | 2,8 | 0,6 | 7,6 | 0,6 | 6,7 | 0,6 | 5,6 | 0,6 | 3,0 |
| 0,65 | 5,3 | 0,65 | 2,8 | 0,65 | 7,6 | 0,65 | 6,6 | 0,65 | 5,5 | 0,65 | 3,0 |
| 0,7 | 5,2 | 0,7 | 2,8 | 0,7 | 7,6 | 0,7 | 6,5 | 0,7 | 5,4 | 0,7 | 3,0 |
| 0,75 | 5,0 | 0,75 | 2,8 | 0,75 | 7,5 | 0,75 | 6,3 | 0,75 | 5,1 | 0,75 | 3,0 |
| 0,8 | 4,8 | 0,8 | 2,7 | 0,8 | 7,1 | 0,8 | 6,1 | 0,8 | 4,8 | 0,8 | 2,9 |
| 0,85 | 4,3 | 0,85 | 2,5 | 0,85 | 6,5 | 0,85 | 5,6 | 0,85 | 4,3 | 0,85 | 2,7 |
| 0,9 | 3,7 | 0,9 | 2,2 | 0,9 | 5,5 | 0,9 | 4,7 | 0,9 | 3,6 | 0,9 | 2,4 |
| 0,95 | 2,7 | 0,95 | 1,8 | 0,95 | 3,8 | 0,95 | 3,4 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HCFO-1233xf- HFO-1234yf - HFO-1234zeE - HCFO-1233zdE - HFO-1243zf**

| **Organics 0,2 F1233xf + 0,2 F1234yf + 0,2 F1243zf + 0,2 F1234zeE + 0,2 F1233zdE** | | **Organics 0,96 F1233xf + 0,01 F1234yf + 0,01 F1243zf + 0,01 F1234zeE + 0,01 F1233zdE** | | **Organics 0,01 F1233xf + 0,96 F1234yf + 0,01 F1243zf + 0,01 F1234zeE + 0,01 F1233zdE** | | **Organics 0,01 F1233xf + 0,01 F1234yf + 0,96 F1243zf + 0,01 F1234zeE + 0,01 F1233zdE** | | **Organics 0,01 F1233xf + 0,01 F1234yf + 0,01 F1243zf + 0,96 F1234zeE + 0,01 F1233zdE** | | **Organics 0,01 F1233xf + 0,01 F1234yf + 0,01 F1243zf + 0,01 F1234zeE + 0,96 F1233zdE** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,3 | 0 | 1,7 | 0 | 6,7 | 0 | 5,8 | 0 | 4,8 | 0 | 1,5 |
| 0,05 | 5,3 | 0,05 | 2,8 | 0,05 | 7,6 | 0,05 | 6,7 | 0,05 | 5,8 | 0,05 | 2,6 |
| 0,1 | 5,3 | 0,1 | 2,8 | 0,1 | 7,6 | 0,1 | 6,7 | 0,1 | 5,8 | 0,1 | 2,6 |
| 0,15 | 5,3 | 0,15 | 2,8 | 0,15 | 7,6 | 0,15 | 6,7 | 0,15 | 5,8 | 0,15 | 2,6 |
| 0,2 | 5,3 | 0,2 | 2,8 | 0,2 | 7,6 | 0,2 | 6,7 | 0,2 | 5,8 | 0,2 | 2,6 |
| 0,25 | 5,3 | 0,25 | 2,8 | 0,25 | 7,6 | 0,25 | 6,7 | 0,25 | 5,8 | 0,25 | 2,6 |
| 0,3 | 5,3 | 0,3 | 2,8 | 0,3 | 7,6 | 0,3 | 6,7 | 0,3 | 5,8 | 0,3 | 2,6 |
| 0,35 | 5,3 | 0,35 | 2,8 | 0,35 | 7,6 | 0,35 | 6,7 | 0,35 | 5,8 | 0,35 | 2,6 |
| 0,4 | 5,3 | 0,4 | 2,8 | 0,4 | 7,6 | 0,4 | 6,7 | 0,4 | 5,8 | 0,4 | 2,6 |
| 0,45 | 5,3 | 0,45 | 2,8 | 0,45 | 7,6 | 0,45 | 6,7 | 0,45 | 5,8 | 0,45 | 2,6 |
| 0,5 | 5,3 | 0,5 | 2,8 | 0,5 | 7,6 | 0,5 | 6,7 | 0,5 | 5,8 | 0,5 | 2,6 |
| 0,55 | 5,3 | 0,55 | 2,8 | 0,55 | 7,6 | 0,55 | 6,7 | 0,55 | 5,7 | 0,55 | 2,6 |
| 0,6 | 5,3 | 0,6 | 2,8 | 0,6 | 7,6 | 0,6 | 6,7 | 0,6 | 5,6 | 0,6 | 2,6 |
| 0,65 | 5,2 | 0,65 | 2,8 | 0,65 | 7,6 | 0,65 | 6,6 | 0,65 | 5,5 | 0,65 | 2,6 |
| 0,7 | 5,1 | 0,7 | 2,8 | 0,7 | 7,6 | 0,7 | 6,5 | 0,7 | 5,4 | 0,7 | 2,6 |
| 0,75 | 5,0 | 0,75 | 2,8 | 0,75 | 7,5 | 0,75 | 6,3 | 0,75 | 5,1 | 0,75 | 2,6 |
| 0,8 | 4,7 | 0,8 | 2,6 | 0,8 | 7,1 | 0,8 | 6,1 | 0,8 | 4,8 | 0,8 | 2,4 |
| 0,85 | 4,3 | 0,85 | 2,5 | 0,85 | 6,5 | 0,85 | 5,6 | 0,85 | 4,3 | 0,85 | 2,3 |
| 0,9 | 3,6 | 0,9 | 2,2 | 0,9 | 5,5 | 0,9 | 4,7 | 0,9 | 3,6 | 0,9 | 2,0 |
| 0,95 | 2,6 | 0,95 | 1,8 | 0,95 | 3,8 | 0,95 | 3,4 | 0,95 | 2,6 | 0,95 | 1,7 |
| 1 | 1,2 | 1 | 1,2 | 1 | 1,2 | 1 | 1,2 | 1 | 1,2 | 1 | 1,2 |

**HF - HCFO-1233xf - HFO-1234yf - HFO-1234zeZ - HCFO-1233zdE - HFO-1243zf**

| **Organics 0,2 F1233xf + 0,2 F1234yf + 0,2 F1243zf + 0,2 F1234zeZ + 0,2 F1233zdE** | | **Organics 0,96 F1233xf + 0,01 F1234yf + 0,01 F1243zf + 0,01 F1234zeZ + 0,01 F1233zdE** | | **Organics 0,01 F1233xf + 0,96 F1234yf + 0,01 F1243zf + 0,01 F1234zeZ + 0,01 F1233zdE** | | **Organics 0,01 F1233xf + 0,01 F1234yf + 0,96 F1243zf + 0,01 F1234zeZ + 0,01 F1233zdE** | | **Organics 0,01 F1233xf + 0,01 F1234yf + 0,01 F1243zf + 0,96 F1234zeZ + 0,01 F1233zdE** | | **Organics 0,01 F1233xf + 0,01 F1234yf + 0,01 F1243zf + 0,01 F1234zeZ + 0,96 F1233zdE** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,6 | 0 | 1,7 | 0 | 6,6 | 0 | 5,7 | 0 | 1,9 | 0 | 1,4 |
| 0,05 | 4,7 | 0,05 | 2,8 | 0,05 | 7,6 | 0,05 | 6,7 | 0,05 | 3,0 | 0,05 | 2,6 |
| 0,1 | 4,7 | 0,1 | 2,8 | 0,1 | 7,6 | 0,1 | 6,7 | 0,1 | 3,0 | 0,1 | 2,6 |
| 0,15 | 4,7 | 0,15 | 2,8 | 0,15 | 7,6 | 0,15 | 6,7 | 0,15 | 3,0 | 0,15 | 2,6 |
| 0,2 | 4,7 | 0,2 | 2,8 | 0,2 | 7,6 | 0,2 | 6,7 | 0,2 | 3,0 | 0,2 | 2,6 |
| 0,25 | 4,7 | 0,25 | 2,8 | 0,25 | 7,6 | 0,25 | 6,7 | 0,25 | 3,0 | 0,25 | 2,6 |
| 0,3 | 4,7 | 0,3 | 2,8 | 0,3 | 7,6 | 0,3 | 6,7 | 0,3 | 3,0 | 0,3 | 2,6 |
| 0,35 | 4,7 | 0,35 | 2,8 | 0,35 | 7,6 | 0,35 | 6,7 | 0,35 | 3,0 | 0,35 | 2,5 |
| 0,4 | 4,7 | 0,4 | 2,8 | 0,4 | 7,6 | 0,4 | 6,7 | 0,4 | 3,0 | 0,4 | 2,5 |
| 0,45 | 4,7 | 0,45 | 2,8 | 0,45 | 7,6 | 0,45 | 6,7 | 0,45 | 3,0 | 0,45 | 2,5 |
| 0,5 | 4,7 | 0,5 | 2,8 | 0,5 | 7,6 | 0,5 | 6,7 | 0,5 | 3,0 | 0,5 | 2,5 |
| 0,55 | 4,7 | 0,55 | 2,8 | 0,55 | 7,6 | 0,55 | 6,7 | 0,55 | 3,0 | 0,55 | 2,5 |
| 0,6 | 4,7 | 0,6 | 2,8 | 0,6 | 7,6 | 0,6 | 6,6 | 0,6 | 3,0 | 0,6 | 2,5 |
| 0,65 | 4,7 | 0,65 | 2,8 | 0,65 | 7,6 | 0,65 | 6,6 | 0,65 | 3,0 | 0,65 | 2,5 |
| 0,7 | 4,7 | 0,7 | 2,8 | 0,7 | 7,6 | 0,7 | 6,5 | 0,7 | 3,0 | 0,7 | 2,5 |
| 0,75 | 4,5 | 0,75 | 2,8 | 0,75 | 7,4 | 0,75 | 6,3 | 0,75 | 3,0 | 0,75 | 2,5 |
| 0,8 | 4,3 | 0,8 | 2,6 | 0,8 | 7,1 | 0,8 | 6,0 | 0,8 | 2,9 | 0,8 | 2,4 |
| 0,85 | 3,9 | 0,85 | 2,4 | 0,85 | 6,5 | 0,85 | 5,5 | 0,85 | 2,7 | 0,85 | 2,3 |
| 0,9 | 3,4 | 0,9 | 2,2 | 0,9 | 5,5 | 0,9 | 4,7 | 0,9 | 2,3 | 0,9 | 2,0 |
| 0,95 | 2,5 | 0,95 | 1,7 | 0,95 | 3,8 | 0,95 | 3,3 | 0,95 | 1,9 | 0,95 | 1,7 |
| 1 | 1,2 | 1 | 1,2 | 1 | 1,2 | 1 | 1,2 | 1 | 1,2 | 1 | 1,2 |

**HF - HCFO-1233xf - HFC-245cb - HFO-1234zeE - HFO-1234zeZ - HCFO-1233zdE**

| **Organics 0,2 F1233xf + 0,2 F245cb + 0,2 F1233zdE 0,2 F1234zeE + 0,2 F1234zeZ** | | **Organics 0,96 F1233xf + 0,01 F245cb + 0,01 F1233zdE + 0,01 F1234zeE + 0,01 F1234zeZ** | | **Organics 0,01 F1233xf + 0,96 F245cb + 0,01 F1233zdE + 0,01 F1234zeE + 0,01 F1234zeZ** | | **Organics 0,01 F1233xf + 0,01 F245cb + 0,96 F1233zdE + 0,01 F1234zeE + 0,01 F1234zeZ** | | **Organics 0,01 F1233xf + 0,01 F245cb + 0,01 F1233zdE + 0,96F1234zeE + 0,01 F1234zeZ** | | **Organics 0,01 F1233xf + 0,01 F245cb + 0,01 F1233zdE + 0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 2,9 | 0 | 1,6 | 0 | 4,6 | 0 | 1,4 | 0 | 4,8 | 0 | 1,9 |
| 0,05 | 4,0 | 0,05 | 2,7 | 0,05 | 5,7 | 0,05 | 2,5 | 0,05 | 5,7 | 0,05 | 3,0 |
| 0,1 | 4,0 | 0,1 | 2,7 | 0,1 | 5,7 | 0,1 | 2,5 | 0,1 | 5,8 | 0,1 | 3,0 |
| 0,15 | 4,0 | 0,15 | 2,7 | 0,15 | 5,7 | 0,15 | 2,5 | 0,15 | 5,8 | 0,15 | 3,0 |
| 0,2 | 4,0 | 0,2 | 2,7 | 0,2 | 5,7 | 0,2 | 2,5 | 0,2 | 5,8 | 0,2 | 3,0 |
| 0,25 | 4,0 | 0,25 | 2,7 | 0,25 | 5,7 | 0,25 | 2,5 | 0,25 | 5,8 | 0,25 | 3,0 |
| 0,3 | 4,0 | 0,3 | 2,7 | 0,3 | 5,7 | 0,3 | 2,5 | 0,3 | 5,8 | 0,3 | 3,0 |
| 0,35 | 4,0 | 0,35 | 2,7 | 0,35 | 5,7 | 0,35 | 2,5 | 0,35 | 5,7 | 0,35 | 3,0 |
| 0,4 | 4,0 | 0,4 | 2,7 | 0,4 | 5,7 | 0,4 | 2,5 | 0,4 | 5,7 | 0,4 | 3,0 |
| 0,45 | 4,0 | 0,45 | 2,7 | 0,45 | 5,7 | 0,45 | 2,5 | 0,45 | 5,7 | 0,45 | 3,0 |
| 0,5 | 4,0 | 0,5 | 2,7 | 0,5 | 5,7 | 0,5 | 2,5 | 0,5 | 5,7 | 0,5 | 3,0 |
| 0,55 | 4,0 | 0,55 | 2,7 | 0,55 | 5,7 | 0,55 | 2,5 | 0,55 | 5,6 | 0,55 | 3,0 |
| 0,6 | 4,0 | 0,6 | 2,7 | 0,6 | 5,7 | 0,6 | 2,5 | 0,6 | 5,6 | 0,6 | 3,0 |
| 0,65 | 4,0 | 0,65 | 2,7 | 0,65 | 5,7 | 0,65 | 2,5 | 0,65 | 5,5 | 0,65 | 3,0 |
| 0,7 | 4,0 | 0,7 | 2,7 | 0,7 | 5,8 | 0,7 | 2,5 | 0,7 | 5,3 | 0,7 | 3,0 |
| 0,75 | 4,0 | 0,75 | 2,7 | 0,75 | 5,8 | 0,75 | 2,5 | 0,75 | 5,1 | 0,75 | 3,0 |
| 0,8 | 3,8 | 0,8 | 2,6 | 0,8 | 5,8 | 0,8 | 2,4 | 0,8 | 4,7 | 0,8 | 2,8 |
| 0,85 | 3,5 | 0,85 | 2,4 | 0,85 | 5,8 | 0,85 | 2,2 | 0,85 | 4,3 | 0,85 | 2,6 |
| 0,9 | 3,0 | 0,9 | 2,1 | 0,9 | 5,0 | 0,9 | 2,0 | 0,9 | 3,6 | 0,9 | 2,3 |
| 0,95 | 2,3 | 0,95 | 1,7 | 0,95 | 3,5 | 0,95 | 1,7 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HCFO-1233xf - HFC-245cb - HFO-1234zeE - HFO-1234zeZ - HFO-1243zf**

| **Organics 0,2 F1233xf + 0,2 F245cb + 0,2 F1243zf 0,2 F1234zeE + 0,2 F1234zeZ** | | **Organics 0,96 F1233xf + 0,01 F245cb + 0,01 F1243zf + 0,01 F1234zeE + 0,01 F1234zeZ** | | **Organics 0,01 F1233xf + 0,96 F245cb + 0,01 F1243zf + 0,01 F1234zeE + 0,01 F1234zeZ** | | **Organics 0,01 F1233xf + 0,01 F245cb + 0,96 F1243zf + 0,01 F1234zeE + 0,01 F1234zeZ** | | **Organics 0,01 F1233xf + 0,01 F245cb + 0,01 F1243zf + 0,96 F1234zeE + 0,01 F1234zeZ** | | **Organics 0,01 F1233xf** + **0,01 F245cb** + **0,01 F1243zf** + **0,01 F1234zeE** + **0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,8 | 0 | 1,7 | 0 | 4,6 | 0 | 5,8 | 0 | 4,8 | 0 | 1,9 |
| 0,05 | 4,9 | 0,05 | 2,8 | 0,05 | 5,8 | 0,05 | 6,7 | 0,05 | 5,8 | 0,05 | 3,0 |
| 0,1 | 4,9 | 0,1 | 2,8 | 0,1 | 5,8 | 0,1 | 6,7 | 0,1 | 5,8 | 0,1 | 3,0 |
| 0,15 | 4,9 | 0,15 | 2,8 | 0,15 | 5,8 | 0,15 | 6,7 | 0,15 | 5,8 | 0,15 | 3,0 |
| 0,2 | 4,9 | 0,2 | 2,8 | 0,2 | 5,8 | 0,2 | 6,7 | 0,2 | 5,8 | 0,2 | 3,0 |
| 0,25 | 4,9 | 0,25 | 2,8 | 0,25 | 5,8 | 0,25 | 6,7 | 0,25 | 5,8 | 0,25 | 3,0 |
| 0,3 | 4,9 | 0,3 | 2,8 | 0,3 | 5,8 | 0,3 | 6,7 | 0,3 | 5,8 | 0,3 | 3,0 |
| 0,35 | 4,9 | 0,35 | 2,8 | 0,35 | 5,8 | 0,35 | 6,7 | 0,35 | 5,8 | 0,35 | 3,0 |
| 0,4 | 4,9 | 0,4 | 2,8 | 0,4 | 5,8 | 0,4 | 6,7 | 0,4 | 5,8 | 0,4 | 3,0 |
| 0,45 | 4,9 | 0,45 | 2,8 | 0,45 | 5,8 | 0,45 | 6,7 | 0,45 | 5,8 | 0,45 | 3,0 |
| 0,5 | 4,9 | 0,5 | 2,8 | 0,5 | 5,8 | 0,5 | 6,7 | 0,5 | 5,7 | 0,5 | 3,0 |
| 0,55 | 4,9 | 0,55 | 2,8 | 0,55 | 5,8 | 0,55 | 6,7 | 0,55 | 5,7 | 0,55 | 3,0 |
| 0,6 | 4,9 | 0,6 | 2,8 | 0,6 | 5,8 | 0,6 | 6,6 | 0,6 | 5,6 | 0,6 | 3,0 |
| 0,65 | 4,9 | 0,65 | 2,8 | 0,65 | 5,8 | 0,65 | 6,6 | 0,65 | 5,5 | 0,65 | 3,0 |
| 0,7 | 4,9 | 0,7 | 2,8 | 0,7 | 5,8 | 0,7 | 6,5 | 0,7 | 5,4 | 0,7 | 3,0 |
| 0,75 | 4,8 | 0,75 | 2,8 | 0,75 | 5,8 | 0,75 | 6,3 | 0,75 | 5,1 | 0,75 | 3,0 |
| 0,8 | 4,6 | 0,8 | 2,6 | 0,8 | 5,8 | 0,8 | 6,0 | 0,8 | 4,8 | 0,8 | 2,9 |
| 0,85 | 4,2 | 0,85 | 2,4 | 0,85 | 5,8 | 0,85 | 5,6 | 0,85 | 4,3 | 0,85 | 2,7 |
| 0,9 | 3,6 | 0,9 | 2,2 | 0,9 | 5,0 | 0,9 | 4,7 | 0,9 | 3,6 | 0,9 | 2,4 |
| 0,95 | 2,6 | 0,95 | 1,8 | 0,95 | 3,5 | 0,95 | 3,4 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HCFO-1233xf - HFC-245cb - HFO-1234zeE - HCFO-1233zdE - HFO-1243zf**

| **Organics 0,2 F1233xf + 0,2 F245cb + 0,2 F1243zf + 0,2 F1234zeE + 0,2 F1233zdE** | | **Organics 0,96 F1233xf + 0,01 F245cb + 0,01 F1243zf + 0,01 F1234zeE + 0,01 F1233zdE** | | **Organics 0,01 F1233xf + 0,96 F245cb + 0,01 F1243zf + 0,01 F1234zeE + 0,01 F1233zdE** | | **Organics 0,01 F1233xf + 0,01 F245cb + 0,96 F1243zf + 0,01 F1234zeE + 0,01 F1233zdE** | | **Organics 0,01 F1233xf + 0,01 F245cb + 0,01 F1243zf + 0,96 F1234zeE + 0,01 F1233zdE** | | **Organics 0,01 F1233xf + 0,01 F245cb + 0,01 F1243zf + 0,01 F1234zeE + 0,96 F1233zdE** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,8 | 0 | 1,7 | 0 | 4,6 | 0 | 5,8 | 0 | 4,8 | 0 | 1,5 |
| 0,05 | 4,9 | 0,05 | 2,8 | 0,05 | 5,8 | 0,05 | 6,7 | 0,05 | 5,8 | 0,05 | 2,6 |
| 0,1 | 4,9 | 0,1 | 2,8 | 0,1 | 5,8 | 0,1 | 6,7 | 0,1 | 5,8 | 0,1 | 2,6 |
| 0,15 | 4,9 | 0,15 | 2,8 | 0,15 | 5,8 | 0,15 | 6,7 | 0,15 | 5,8 | 0,15 | 2,6 |
| 0,2 | 4,9 | 0,2 | 2,8 | 0,2 | 5,8 | 0,2 | 6,7 | 0,2 | 5,8 | 0,2 | 2,6 |
| 0,25 | 4,9 | 0,25 | 2,8 | 0,25 | 5,8 | 0,25 | 6,7 | 0,25 | 5,8 | 0,25 | 2,6 |
| 0,3 | 4,9 | 0,3 | 2,8 | 0,3 | 5,8 | 0,3 | 6,7 | 0,3 | 5,8 | 0,3 | 2,6 |
| 0,35 | 4,9 | 0,35 | 2,8 | 0,35 | 5,8 | 0,35 | 6,7 | 0,35 | 5,8 | 0,35 | 2,6 |
| 0,4 | 4,9 | 0,4 | 2,8 | 0,4 | 5,8 | 0,4 | 6,7 | 0,4 | 5,8 | 0,4 | 2,6 |
| 0,45 | 4,9 | 0,45 | 2,8 | 0,45 | 5,8 | 0,45 | 6,7 | 0,45 | 5,8 | 0,45 | 2,6 |
| 0,5 | 4,9 | 0,5 | 2,8 | 0,5 | 5,8 | 0,5 | 6,7 | 0,5 | 5,7 | 0,5 | 2,6 |
| 0,55 | 4,9 | 0,55 | 2,8 | 0,55 | 5,8 | 0,55 | 6,7 | 0,55 | 5,7 | 0,55 | 2,6 |
| 0,6 | 4,9 | 0,6 | 2,8 | 0,6 | 5,8 | 0,6 | 6,6 | 0,6 | 5,6 | 0,6 | 2,6 |
| 0,65 | 4,9 | 0,65 | 2,8 | 0,65 | 5,8 | 0,65 | 6,6 | 0,65 | 5,5 | 0,65 | 2,5 |
| 0,7 | 4,9 | 0,7 | 2,8 | 0,7 | 5,8 | 0,7 | 6,5 | 0,7 | 5,4 | 0,7 | 2,5 |
| 0,75 | 4,7 | 0,75 | 2,8 | 0,75 | 5,8 | 0,75 | 6,3 | 0,75 | 5,1 | 0,75 | 2,5 |
| 0,8 | 4,5 | 0,8 | 2,6 | 0,8 | 5,8 | 0,8 | 6,0 | 0,8 | 4,8 | 0,8 | 2,4 |
| 0,85 | 4,1 | 0,85 | 2,4 | 0,85 | 5,8 | 0,85 | 5,6 | 0,85 | 4,3 | 0,85 | 2,3 |
| 0,9 | 3,5 | 0,9 | 2,2 | 0,9 | 5,0 | 0,9 | 4,7 | 0,9 | 3,6 | 0,9 | 2,0 |
| 0,95 | 2,6 | 0,95 | 1,7 | 0,95 | 3,5 | 0,95 | 3,3 | 0,95 | 2,6 | 0,95 | 1,7 |
| 1 | 1,2 | 1 | 1,2 | 1 | 1,2 | 1 | 1,2 | 1 | 1,2 | 1 | 1,2 |

**HF - HCFO-1233xf - HFC-245cb - HFO-1234zeZ - HCFO-1233zdE - HFO-1243zf**

| **Organics 0,2 F1233xf + 0,2 F245cb + 0,2 F1243zf + 0,2 F1234zeZ + 0,2 F1233zdE** | | **Organics 0,96 F1233xf + 0,01 F245cb + 0,01 F1243zf + 0,01 F1234zeZ + 0,01 F1233zdE** | | **Organics 0,01 F1233xf + 0,96 F245cb + 0,01 F1243zf + 0,01 F1234zeZ + 0,01 F1233zdE** | | **Organics 0,01 F1233xf + 0,01 F245cb + 0,96 F1243zf + 0,01 F1234zeZ + 0,01 F1233zdE** | | **Organics 0,01 F1233xf + 0,01 F245cb + 0,01 F1243zf + 0,96 F1234zeZ + 0,01 F1233zdE** | | **Organics 0,01 F1233xf + 0,01 F245cb + 0,01 F1243zf + 0,01 F1234zeZ + 0,96 F1233zdE** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,2 | 0 | 1,6 | 0 | 4,6 | 0 | 5,7 | 0 | 1,9 | 0 | 1,4 |
| 0,05 | 4,3 | 0,05 | 2,7 | 0,05 | 5,7 | 0,05 | 6,7 | 0,05 | 3,0 | 0,05 | 2,5 |
| 0,1 | 4,3 | 0,1 | 2,7 | 0,1 | 5,7 | 0,1 | 6,7 | 0,1 | 3,0 | 0,1 | 2,5 |
| 0,15 | 4,3 | 0,15 | 2,7 | 0,15 | 5,7 | 0,15 | 6,7 | 0,15 | 3,0 | 0,15 | 2,5 |
| 0,2 | 4,3 | 0,2 | 2,7 | 0,2 | 5,7 | 0,2 | 6,7 | 0,2 | 3,0 | 0,2 | 2,5 |
| 0,25 | 4,3 | 0,25 | 2,7 | 0,25 | 5,7 | 0,25 | 6,7 | 0,25 | 3,0 | 0,25 | 2,5 |
| 0,3 | 4,3 | 0,3 | 2,7 | 0,3 | 5,7 | 0,3 | 6,7 | 0,3 | 3,0 | 0,3 | 2,5 |
| 0,35 | 4,3 | 0,35 | 2,7 | 0,35 | 5,7 | 0,35 | 6,7 | 0,35 | 3,0 | 0,35 | 2,5 |
| 0,4 | 4,3 | 0,4 | 2,7 | 0,4 | 5,8 | 0,4 | 6,7 | 0,4 | 3,0 | 0,4 | 2,5 |
| 0,45 | 4,3 | 0,45 | 2,7 | 0,45 | 5,8 | 0,45 | 6,7 | 0,45 | 3,0 | 0,45 | 2,5 |
| 0,5 | 4,3 | 0,5 | 2,7 | 0,5 | 5,8 | 0,5 | 6,7 | 0,5 | 3,0 | 0,5 | 2,5 |
| 0,55 | 4,3 | 0,55 | 2,7 | 0,55 | 5,8 | 0,55 | 6,7 | 0,55 | 3,0 | 0,55 | 2,5 |
| 0,6 | 4,3 | 0,6 | 2,7 | 0,6 | 5,8 | 0,6 | 6,6 | 0,6 | 3,0 | 0,6 | 2,5 |
| 0,65 | 4,3 | 0,65 | 2,7 | 0,65 | 5,8 | 0,65 | 6,6 | 0,65 | 3,0 | 0,65 | 2,5 |
| 0,7 | 4,3 | 0,7 | 2,7 | 0,7 | 5,8 | 0,7 | 6,5 | 0,7 | 3,0 | 0,7 | 2,5 |
| 0,75 | 4,3 | 0,75 | 2,7 | 0,75 | 5,8 | 0,75 | 6,3 | 0,75 | 3,0 | 0,75 | 2,5 |
| 0,8 | 4,1 | 0,8 | 2,6 | 0,8 | 5,8 | 0,8 | 6,0 | 0,8 | 2,9 | 0,8 | 2,4 |
| 0,85 | 3,8 | 0,85 | 2,4 | 0,85 | 5,8 | 0,85 | 5,5 | 0,85 | 2,7 | 0,85 | 2,3 |
| 0,9 | 3,3 | 0,9 | 2,1 | 0,9 | 5,0 | 0,9 | 4,7 | 0,9 | 2,3 | 0,9 | 2,0 |
| 0,95 | 2,4 | 0,95 | 1,7 | 0,95 | 3,5 | 0,95 | 3,3 | 0,95 | 1,9 | 0,95 | 1,7 |
| 1 | 1,2 | 1 | 1,2 | 1 | 1,2 | 1 | 1,2 | 1 | 1,2 | 1 | 1,2 |

**HF - HCFO-1233xf - HFO-1234zeE- HFO-1234zeZ - HCFO-1233zdE- HFO-1243zf**

| **Organics 0,2 F1233xf + 0,2 F1243zf + 0,2 F1233zdE 0,2 F1234zeE + 0,2 F1234zeZ** | | **Organics 0,96 F1233xf + 0,01 F1243zf + 0,01 F1233zdE + 0,01 F1234zeE + 0,01 F1234zeZ** | | **Organics 0,01 F1233xf + 0,96 F1243zf + 0,01 F1233zdE + 0,01 F1234zeE + 0,01 F1234zeZ** | | **Organics 0,01 F1233xf + 0,01 F1243zf** + **0,96 F1233zdE** + **0,01 F1234zeE + 0,01 F1234zeZ** | | **Organics 0,01 F1233xf + 0,01 F1243zf** + **0,01 F1233zdE** + **0,96 F1234zeE + 0,01 F1234zeZ** | | **Organics 0,01 F1233xf + 0,01 F1243zf** + **0,01 F1233zdE** + **0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,2 | 0 | 1,6 | 0 | 5,7 | 0 | 1,4 | 0 | 4,8 | 0 | 1,9 |
| 0,05 | 4,3 | 0,05 | 2,7 | 0,05 | 6,7 | 0,05 | 2,5 | 0,05 | 5,7 | 0,05 | 3,0 |
| 0,1 | 4,3 | 0,1 | 2,7 | 0,1 | 6,7 | 0,1 | 2,5 | 0,1 | 5,8 | 0,1 | 3,0 |
| 0,15 | 4,3 | 0,15 | 2,7 | 0,15 | 6,7 | 0,15 | 2,5 | 0,15 | 5,8 | 0,15 | 3,0 |
| 0,2 | 4,3 | 0,2 | 2,7 | 0,2 | 6,7 | 0,2 | 2,5 | 0,2 | 5,8 | 0,2 | 3,0 |
| 0,25 | 4,3 | 0,25 | 2,7 | 0,25 | 6,7 | 0,25 | 2,5 | 0,25 | 5,8 | 0,25 | 3,0 |
| 0,3 | 4,3 | 0,3 | 2,7 | 0,3 | 6,7 | 0,3 | 2,5 | 0,3 | 5,8 | 0,3 | 3,0 |
| 0,35 | 4,3 | 0,35 | 2,7 | 0,35 | 6,7 | 0,35 | 2,5 | 0,35 | 5,8 | 0,35 | 3,0 |
| 0,4 | 4,3 | 0,4 | 2,7 | 0,4 | 6,7 | 0,4 | 2,5 | 0,4 | 5,8 | 0,4 | 3,0 |
| 0,45 | 4,3 | 0,45 | 2,7 | 0,45 | 6,7 | 0,45 | 2,5 | 0,45 | 5,7 | 0,45 | 3,0 |
| 0,5 | 4,3 | 0,5 | 2,7 | 0,5 | 6,7 | 0,5 | 2,5 | 0,5 | 5,7 | 0,5 | 3,0 |
| 0,55 | 4,2 | 0,55 | 2,7 | 0,55 | 6,6 | 0,55 | 2,5 | 0,55 | 5,7 | 0,55 | 3,0 |
| 0,6 | 4,2 | 0,6 | 2,7 | 0,6 | 6,6 | 0,6 | 2,5 | 0,6 | 5,6 | 0,6 | 3,0 |
| 0,65 | 4,2 | 0,65 | 2,7 | 0,65 | 6,6 | 0,65 | 2,5 | 0,65 | 5,5 | 0,65 | 3,0 |
| 0,7 | 4,2 | 0,7 | 2,7 | 0,7 | 6,5 | 0,7 | 2,5 | 0,7 | 5,3 | 0,7 | 3,0 |
| 0,75 | 4,0 | 0,75 | 2,7 | 0,75 | 6,3 | 0,75 | 2,5 | 0,75 | 5,1 | 0,75 | 3,0 |
| 0,8 | 3,8 | 0,8 | 2,6 | 0,8 | 6,0 | 0,8 | 2,4 | 0,8 | 4,7 | 0,8 | 2,8 |
| 0,85 | 3,5 | 0,85 | 2,4 | 0,85 | 5,5 | 0,85 | 2,2 | 0,85 | 4,3 | 0,85 | 2,6 |
| 0,9 | 3,0 | 0,9 | 2,1 | 0,9 | 4,7 | 0,9 | 2,0 | 0,9 | 3,6 | 0,9 | 2,3 |
| 0,95 | 2,2 | 0,95 | 1,7 | 0,95 | 3,3 | 0,95 | 1,7 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HFO-1234yf - HFC-245cb - HFO-1234zeE - HFO-1234zeZ - HCFO-1233zdE**

| **Organics 0,2 F1234yf + 0,2 F245cb + 0,2 F1233zdE + 0,2 F1234zeE + 0,2 F1234zeZ** | | **Organics 0,96 F1234yf + 0,01 F245cb + 0,01 F1233zdE + 0,01 F1234zeE + 0,01 F1234zeZ** | | **Organics 0,01 F1234yf + 0,96 F245cb + 0,01 F1233zdE + 0,01 F1234zeE + 0,01 F1234zeZ** | | **Organics 0,01 F1234yf + 0,01 F245cb + 0,96 F1233zdE + 0,01 F1234zeE + 0,01 F1234zeZ** | | **Organics 0,01 F1234yf + 0,01 F245cb + 0,01 F1233zdE + 0,96 F1234zeE + 0,01 F1234zeZ** | | **Organics 0,01 F1234yf + 0,01 F245cb + 0,01 F1233zdE + 0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,9 | 0 | 6,7 | 0 | 4,6 | 0 | 1,5 | 0 | 4,8 | 0 | 1,9 |
| 0,05 | 5,0 | 0,05 | 7,6 | 0,05 | 5,8 | 0,05 | 2,6 | 0,05 | 5,8 | 0,05 | 3,0 |
| 0,1 | 5,0 | 0,1 | 7,6 | 0,1 | 5,8 | 0,1 | 2,6 | 0,1 | 5,8 | 0,1 | 3,0 |
| 0,15 | 5,0 | 0,15 | 7,6 | 0,15 | 5,8 | 0,15 | 2,6 | 0,15 | 5,8 | 0,15 | 3,0 |
| 0,2 | 5,0 | 0,2 | 7,6 | 0,2 | 5,8 | 0,2 | 2,6 | 0,2 | 5,8 | 0,2 | 3,0 |
| 0,25 | 5,0 | 0,25 | 7,6 | 0,25 | 5,8 | 0,25 | 2,6 | 0,25 | 5,8 | 0,25 | 3,0 |
| 0,3 | 5,0 | 0,3 | 7,6 | 0,3 | 5,8 | 0,3 | 2,6 | 0,3 | 5,8 | 0,3 | 3,0 |
| 0,35 | 5,0 | 0,35 | 7,6 | 0,35 | 5,8 | 0,35 | 2,6 | 0,35 | 5,8 | 0,35 | 3,0 |
| 0,4 | 5,0 | 0,4 | 7,6 | 0,4 | 5,8 | 0,4 | 2,6 | 0,4 | 5,8 | 0,4 | 3,0 |
| 0,45 | 5,0 | 0,45 | 7,6 | 0,45 | 5,8 | 0,45 | 2,6 | 0,45 | 5,8 | 0,45 | 3,0 |
| 0,5 | 5,0 | 0,5 | 7,6 | 0,5 | 5,8 | 0,5 | 2,6 | 0,5 | 5,7 | 0,5 | 3,0 |
| 0,55 | 5,0 | 0,55 | 7,6 | 0,55 | 5,8 | 0,55 | 2,6 | 0,55 | 5,7 | 0,55 | 3,0 |
| 0,6 | 5,0 | 0,6 | 7,6 | 0,6 | 5,8 | 0,6 | 2,6 | 0,6 | 5,6 | 0,6 | 3,0 |
| 0,65 | 5,0 | 0,65 | 7,6 | 0,65 | 5,8 | 0,65 | 2,6 | 0,65 | 5,5 | 0,65 | 3,0 |
| 0,7 | 5,1 | 0,7 | 7,6 | 0,7 | 5,8 | 0,7 | 2,6 | 0,7 | 5,4 | 0,7 | 3,0 |
| 0,75 | 5,0 | 0,75 | 7,5 | 0,75 | 5,8 | 0,75 | 2,6 | 0,75 | 5,1 | 0,75 | 3,0 |
| 0,8 | 4,7 | 0,8 | 7,1 | 0,8 | 5,8 | 0,8 | 2,5 | 0,8 | 4,8 | 0,8 | 2,9 |
| 0,85 | 4,3 | 0,85 | 6,5 | 0,85 | 5,8 | 0,85 | 2,3 | 0,85 | 4,3 | 0,85 | 2,7 |
| 0,9 | 3,7 | 0,9 | 5,5 | 0,9 | 5,0 | 0,9 | 2,0 | 0,9 | 3,6 | 0,9 | 2,4 |
| 0,95 | 2,7 | 0,95 | 3,8 | 0,95 | 3,5 | 0,95 | 1,7 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HFO-1234yf - HFC-245cb - HFO-1234zeE - HFO-1234zeZ - HFO-1243zf**

| **Organics 0,2 F1234yf + 0,2 F245cb + 0,2 F1243zf + 0,2 F1234zeE + 0,2 F1234zeZ** | | **Organics 0,96 F1234yf + 0,01 F245cb + 0,01 F1243zf + 0,01 F1234zeE + 0,01 F1234zeZ** | | **Organics 0,01 F1234yf + 0,96 F245cb + 0,01 F1243zf + 0,01 F1234zeE + 0,01 F1234zeZ** | | **Organics 0,01 F1234yf + 0,01 F245cb + 0,96 F1243zf + 0,01 F1234zeE + 0,01 F1234zeZ** | | **Organics 0,01 F1234yf + 0,01 F245cb + 0,01 F1243zf + 0,96 F1234zeE + 0,01 F1234zeZ** | | **Organics 0,01 F1234yf + 0,01 F245cb + 0,01 F1243zf + 0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | | | | | | | | | | | |

| | bar | | bar | | bar | | bar | | bar | | bar |
|---|---|---|---|---|---|---|---|---|---|---|---|
| 0 | 4,8 | 0 | 6,7 | 0 | 4,7 | 0 | 5,8 | 0 | 4,9 | 0 | 2,0 |
| 0,05 | 5,9 | 0,05 | 7,7 | 0,05 | 5,8 | 0,05 | 6,7 | 0,05 | 5,8 | 0,05 | 3,1 |
| 0,1 | 5,9 | 0,1 | 7,7 | 0,1 | 5,8 | 0,1 | 6,8 | 0,1 | 5,9 | 0,1 | 3,1 |
| 0,15 | 5,9 | 0,15 | 7,7 | 0,15 | 5,8 | 0,15 | 6,8 | 0,15 | 5,8 | 0,15 | 3,1 |
| 0,2 | 5,9 | 0,2 | 7,7 | 0,2 | 5,8 | 0,2 | 6,8 | 0,2 | 5,8 | 0,2 | 3,1 |
| 0,25 | 5,9 | 0,25 | 7,6 | 0,25 | 5,8 | 0,25 | 6,8 | 0,25 | 5,8 | 0,25 | 3,1 |
| 0,3 | 5,9 | 0,3 | 7,6 | 0,3 | 5,8 | 0,3 | 6,8 | 0,3 | 5,8 | 0,3 | 3,1 |
| 0,35 | 5,9 | 0,35 | 7,6 | 0,35 | 5,8 | 0,35 | 6,8 | 0,35 | 5,8 | 0,35 | 3,1 |
| 0,4 | 5,9 | 0,4 | 7,6 | 0,4 | 5,8 | 0,4 | 6,7 | 0,4 | 5,8 | 0,4 | 3,1 |
| 0,45 | 5,9 | 0,45 | 7,6 | 0,45 | 5,8 | 0,45 | 6,7 | 0,45 | 5,8 | 0,45 | 3,1 |
| 0,5 | 5,9 | 0,5 | 7,7 | 0,5 | 5,8 | 0,5 | 6,7 | 0,5 | 5,8 | 0,5 | 3,1 |
| 0,55 | 5,9 | 0,55 | 7,7 | 0,55 | 5,8 | 0,55 | 6,7 | 0,55 | 5,7 | 0,55 | 3,1 |
| 0,6 | 5,9 | 0,6 | 7,7 | 0,6 | 5,8 | 0,6 | 6,7 | 0,6 | 5,7 | 0,6 | 3,1 |
| 0,65 | 5,9 | 0,65 | 7,7 | 0,65 | 5,8 | 0,65 | 6,6 | 0,65 | 5,6 | 0,65 | 3,1 |
| 0,7 | 5,9 | 0,7 | 7,6 | 0,7 | 5,8 | 0,7 | 6,5 | 0,7 | 5,4 | 0,7 | 3,1 |
| 0,75 | 5,7 | 0,75 | 7,5 | 0,75 | 5,8 | 0,75 | 6,4 | 0,75 | 5,2 | 0,75 | 3,1 |
| 0,8 | 5,5 | 0,8 | 7,2 | 0,8 | 5,8 | 0,8 | 6,1 | 0,8 | 4,8 | 0,8 | 2,9 |
| 0,85 | 5,0 | 0,85 | 6,6 | 0,85 | 5,8 | 0,85 | 5,6 | 0,85 | 4,3 | 0,85 | 2,7 |
| 0,9 | 4,3 | 0,9 | 5,5 | 0,9 | 5,0 | 0,9 | 4,8 | 0,9 | 3,6 | 0,9 | 2,4 |
| 0,95 | 3,0 | 0,95 | 3,8 | 0,95 | 3,5 | 0,95 | 3,4 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HFO-1234yf - HFC-245cb - HFO-1234zeE - HCFO-1233zdE- HFO-1243zf**

| **Organics 0,2 F1234yf + 0,2 F245cb + 0,2 F1243zf + 0,2 F1234zeE + 0,2 F1233zdE** | | **Organics 0,96 F1234yf + 0,01 F245cb + 0,01 F1243zf + 0,01 F1234zeE + 0,01 F1233zdE** | | **Organics 0,01 F1234yf + 0,96 F245cb + 0,01 F1243zf + 0,01 F1234zeE + 0,01 F1233zdE** | | **Organics 0,01 F1234yf + 0,01 F245cb + 0,96 F1243zf + 0,01 F1234zeE + 0,01 F1233zdE** | | **Organics 0,01 F1234yf + 0,01 F245cb + 0,01 F1243zf + 0,96 F1234zeE + 0,01 F1233zdE** | | **Organics 0,01 F1234yf + 0,01 F245cb + 0,01 F1243zf + 0,01 F1234zeE + 0,96 F1233zdE** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,8 | 0 | 6,7 | 0 | 4,7 | 0 | 5,8 | 0 | 4,9 | 0 | 1,5 |
| 0,05 | 5,9 | 0,05 | 7,7 | 0,05 | 5,8 | 0,05 | 6,7 | 0,05 | 5,8 | 0,05 | 2,6 |
| 0,1 | 5,9 | 0,1 | 7,7 | 0,1 | 5,8 | 0,1 | 6,8 | 0,1 | 5,8 | 0,1 | 2,6 |
| 0,15 | 5,9 | 0,15 | 7,7 | 0,15 | 5,8 | 0,15 | 6,8 | 0,15 | 5,8 | 0,15 | 2,6 |
| 0,2 | 5,9 | 0,2 | 7,7 | 0,2 | 5,8 | 0,2 | 6,8 | 0,2 | 5,8 | 0,2 | 2,6 |
| 0,25 | 5,9 | 0,25 | 7,6 | 0,25 | 5,8 | 0,25 | 6,8 | 0,25 | 5,8 | 0,25 | 2,6 |
| 0,3 | 5,9 | 0,3 | 7,6 | 0,3 | 5,8 | 0,3 | 6,8 | 0,3 | 5,8 | 0,3 | 2,6 |
| 0,35 | 5,9 | 0,35 | 7,6 | 0,35 | 5,8 | 0,35 | 6,8 | 0,35 | 5,8 | 0,35 | 2,6 |
| 0,4 | 5,9 | 0,4 | 7,7 | 0,4 | 5,8 | 0,4 | 6,7 | 0,4 | 5,8 | 0,4 | 2,6 |
| 0,45 | 5,9 | 0,45 | 7,7 | 0,45 | 5,8 | 0,45 | 6,7 | 0,45 | 5,8 | 0,45 | 2,6 |
| 0,5 | 5,9 | 0,5 | 7,7 | 0,5 | 5,8 | 0,5 | 6,7 | 0,5 | 5,8 | 0,5 | 2,6 |
| 0,55 | 5,9 | 0,55 | 7,7 | 0,55 | 5,8 | 0,55 | 6,7 | 0,55 | 5,7 | 0,55 | 2,6 |
| 0,6 | 5,9 | 0,6 | 7,7 | 0,6 | 5,8 | 0,6 | 6,7 | 0,6 | 5,7 | 0,6 | 2,6 |
| 0,65 | 5,9 | 0,65 | 7,7 | 0,65 | 5,8 | 0,65 | 6,6 | 0,65 | 5,6 | 0,65 | 2,6 |
| 0,7 | 5,9 | 0,7 | 7,6 | 0,7 | 5,8 | 0,7 | 6,5 | 0,7 | 5,4 | 0,7 | 2,6 |
| 0,75 | 5,7 | 0,75 | 7,5 | 0,75 | 5,8 | 0,75 | 6,4 | 0,75 | 5,2 | 0,75 | 2,6 |
| 0,8 | 5,4 | 0,8 | 7,2 | 0,8 | 5,8 | 0,8 | 6,1 | 0,8 | 4,8 | 0,8 | 2,5 |
| 0,85 | 5,0 | 0,85 | 6,6 | 0,85 | 5,8 | 0,85 | 5,6 | 0,85 | 4,3 | 0,85 | 2,3 |
| 0,9 | 4,2 | 0,9 | 5,5 | 0,9 | 5,0 | 0,9 | 4,8 | 0,9 | 3,6 | 0,9 | 2,1 |
| 0,95 | 3,0 | 0,95 | 3,8 | 0,95 | 3,5 | 0,95 | 3,4 | 0,95 | 2,6 | 0,95 | 1,7 |
| 1 | 1,2 | 1 | 1,2 | 1 | 1,2 | 1 | 1,2 | 1 | 1,2 | 1 | 1,2 |

**HF - HFO-1234yf - HFC-245cb - HFO-1234zeZ - HCFO-1233zdE- HFO-1243zf**

| **Organics 0,2 F1234yf + 0,2 F245cb + 0,2 F1243zf + 0,2 F1234zeZ + 0,2 F1233zdE** | | **Organics 0,96 F1234yf + 0,01 F245cb + 0,01 F1243zf + 0,01 F1234zeZ + 0,01 F1233zdE** | | **Organics 0,01 F1234yf + 0,96 F245cb + 0,01 F1243zf + 0,01 F1234zeZ + 0,01 F1233zdE** | | **Organics 0,01 F1234yf + 0,01 F245cb + 0,96 F1243zf + 0,01 F1234zeZ + 0,01 F1233zdE** | | **Organics 0,01 F1234yf + 0,01 F245cb + 0,01 F1243zf + 0,96 F1234zeZ + 0,01 F1233zdE** | | **Organics 0,01 F1234yf + 0,01 F245cb + 0,01 F1243zf + 0,01 F1234zeZ + 0,96 F1233zdE** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,2 | 0 | 6,7 | 0 | 4,6 | 0 | 5,8 | 0 | 1,9 | 0 | 1,5 |
| 0,05 | 5,3 | 0,05 | 7,6 | 0,05 | 5,8 | 0,05 | 6,7 | 0,05 | 3,0 | 0,05 | 2,6 |
| 0,1 | 5,3 | 0,1 | 7,6 | 0,1 | 5,8 | 0,1 | 6,7 | 0,1 | 3,0 | 0,1 | 2,6 |
| 0,15 | 5,3 | 0,15 | 7,6 | 0,15 | 5,8 | 0,15 | 6,7 | 0,15 | 3,0 | 0,15 | 2,6 |
| 0,2 | 5,3 | 0,2 | 7,6 | 0,2 | 5,8 | 0,2 | 6,7 | 0,2 | 3,0 | 0,2 | 2,6 |
| 0,25 | 5,3 | 0,25 | 7,6 | 0,25 | 5,8 | 0,25 | 6,7 | 0,25 | 3,0 | 0,25 | 2,6 |
| 0,3 | 5,3 | 0,3 | 7,6 | 0,3 | 5,8 | 0,3 | 6,7 | 0,3 | 3,0 | 0,3 | 2,6 |
| 0,35 | 5,3 | 0,35 | 7,6 | 0,35 | 5,8 | 0,35 | 6,7 | 0,35 | 3,0 | 0,35 | 2,6 |
| 0,4 | 5,3 | 0,4 | 7,6 | 0,4 | 5,8 | 0,4 | 6,7 | 0,4 | 3,0 | 0,4 | 2,6 |
| 0,45 | 5,3 | 0,45 | 7,6 | 0,45 | 5,8 | 0,45 | 6,7 | 0,45 | 3,0 | 0,45 | 2,6 |
| 0,5 | 5,3 | 0,5 | 7,6 | 0,5 | 5,8 | 0,5 | 6,7 | 0,5 | 3,0 | 0,5 | 2,6 |
| 0,55 | 5,3 | 0,55 | 7,6 | 0,55 | 5,8 | 0,55 | 6,7 | 0,55 | 3,0 | 0,55 | 2,6 |
| 0,6 | 5,3 | 0,6 | 7,6 | 0,6 | 5,8 | 0,6 | 6,7 | 0,6 | 3,0 | 0,6 | 2,6 |
| 0,65 | 5,3 | 0,65 | 7,6 | 0,65 | 5,8 | 0,65 | 6,6 | 0,65 | 3,0 | 0,65 | 2,6 |
| 0,7 | 5,3 | 0,7 | 7,6 | 0,7 | 5,8 | 0,7 | 6,5 | 0,7 | 3,0 | 0,7 | 2,6 |
| 0,75 | 5,2 | 0,75 | 7,5 | 0,75 | 5,8 | 0,75 | 6,4 | 0,75 | 3,0 | 0,75 | 2,6 |
| 0,8 | 5,0 | 0,8 | 7,2 | 0,8 | 5,8 | 0,8 | 6,1 | 0,8 | 2,9 | 0,8 | 2,5 |
| 0,85 | 4,6 | 0,85 | 6,6 | 0,85 | 5,8 | 0,85 | 5,6 | 0,85 | 2,7 | 0,85 | 2,3 |
| 0,9 | 3,9 | 0,9 | 5,5 | 0,9 | 5,0 | 0,9 | 4,7 | 0,9 | 2,4 | 0,9 | 2,0 |
| 0,95 | 2,9 | 0,95 | 3,8 | 0,95 | 3,5 | 0,95 | 3,4 | 0,95 | 1,9 | 0,95 | 1,7 |
| 1 | 1,2 | 1 | 1,2 | 1 | 1,2 | 1 | 1,2 | 1 | 1,2 | 1 | 1,2 |

**HF - HFO-1234yf- HFO-1234zeE - HFO-1234zeZ - HCFO-1233zdE- HFO-1243zf**

| **Organics 0,2 F1234yf + 0,2 F1243zf + 0,2 F1233zdE 0,2 F1234zeE + 0,2 F1234zeZ** | | **Organics 0,96 F1234yf + 0,01 F1243zf + 0,01 F1233zdE + 0,01 F1234zeE + 0,01 F1234zeZ** | | **Organics 0,01 F1234yf + 0,96 F1243zf + 0,01 F1233zdE + 0,01 F1234zeE + 0,01 F1234zeZ** | | **Organics 0,01 F1234yf + 0,01 F1243zf + 0,96 F1233zdE + 0,01 F1234zeE + 0,01 F1234zeZ** | | **Organics 0,01 F1234yf + 0,01 F1243zf + 0,01 F1233zdE + 0,96 F1234zeE + 0,01 F1234zeZ** | | **Organics 0,01 F1234yf + 0,01 F1243zf + 0,01 F1233zdE + 0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 4,2 | 0 | 6,7 | 0 | 5,8 | 0 | 1,5 | 0 | 4,8 | 0 | 1,9 |
| 0,05 | 5,3 | 0,05 | 7,6 | 0,05 | 6,7 | 0,05 | 2,6 | 0,05 | 5,8 | 0,05 | 3,0 |
| 0,1 | 5,3 | 0,1 | 7,6 | 0,1 | 6,7 | 0,1 | 2,6 | 0,1 | 5,8 | 0,1 | 3,0 |
| 0,15 | 5,3 | 0,15 | 7,6 | 0,15 | 6,7 | 0,15 | 2,6 | 0,15 | 5,8 | 0,15 | 3,0 |
| 0,2 | 5,3 | 0,2 | 7,6 | 0,2 | 6,7 | 0,2 | 2,6 | 0,2 | 5,8 | 0,2 | 3,0 |
| 0,25 | 5,3 | 0,25 | 7,6 | 0,25 | 6,7 | 0,25 | 2,6 | 0,25 | 5,8 | 0,25 | 3,0 |
| 0,3 | 5,3 | 0,3 | 7,6 | 0,3 | 6,7 | 0,3 | 2,6 | 0,3 | 5,8 | 0,3 | 3,0 |
| 0,35 | 5,3 | 0,35 | 7,6 | 0,35 | 6,7 | 0,35 | 2,6 | 0,35 | 5,8 | 0,35 | 3,0 |
| 0,4 | 5,3 | 0,4 | 7,6 | 0,4 | 6,7 | 0,4 | 2,6 | 0,4 | 5,8 | 0,4 | 3,0 |
| 0,45 | 5,3 | 0,45 | 7,6 | 0,45 | 6,7 | 0,45 | 2,6 | 0,45 | 5,8 | 0,45 | 3,0 |
| 0,5 | 5,3 | 0,5 | 7,6 | 0,5 | 6,7 | 0,5 | 2,6 | 0,5 | 5,8 | 0,5 | 3,0 |
| 0,55 | 5,3 | 0,55 | 7,6 | 0,55 | 6,7 | 0,55 | 2,6 | 0,55 | 5,7 | 0,55 | 3,0 |
| 0,6 | 5,2 | 0,6 | 7,6 | 0,6 | 6,7 | 0,6 | 2,6 | 0,6 | 5,6 | 0,6 | 3,0 |
| 0,65 | 5,2 | 0,65 | 7,6 | 0,65 | 6,6 | 0,65 | 2,6 | 0,65 | 5,5 | 0,65 | 3,0 |
| 0,7 | 5,2 | 0,7 | 7,6 | 0,7 | 6,5 | 0,7 | 2,6 | 0,7 | 5,4 | 0,7 | 3,0 |
| 0,75 | 5,0 | 0,75 | 7,5 | 0,75 | 6,3 | 0,75 | 2,6 | 0,75 | 5,1 | 0,75 | 3,0 |
| 0,8 | 4,7 | 0,8 | 7,1 | 0,8 | 6,1 | 0,8 | 2,5 | 0,8 | 4,8 | 0,8 | 2,9 |
| 0,85 | 4,3 | 0,85 | 6,5 | 0,85 | 5,6 | 0,85 | 2,3 | 0,85 | 4,3 | 0,85 | 2,7 |
| 0,9 | 3,7 | 0,9 | 5,5 | 0,9 | 4,7 | 0,9 | 2,0 | 0,9 | 3,6 | 0,9 | 2,4 |
| 0,95 | 2,7 | 0,95 | 3,8 | 0,95 | 3,4 | 0,95 | 1,7 | 0,95 | 2,6 | 0,95 | 1,9 |
| 1 | 1,2 | 1 | 1,2 | 1 | 1,2 | 1 | 1,2 | 1 | 1,2 | 1 | 1,2 |

**HF - HFC-245cb - HFO-1234zeE - HFO-1234zeZ - HCFO-1233zdE - HFO-1243zf**

| **Organics 0,2 F245cb + 0,2 F1243zf + 0,2 F1233zdE 0,2 F1234zeE + 0,2 F1234zeZ** | | **Organics 0,96 F245cb + 0,01 F1243zf + 0,01 F1233zdE + 0,01 F1234zeE + 0,01 F1234zeZ** | | **Organics 0,01 F245cb + 0,96 F1243zf + 0,01 F1233zdE + 0,01 F1234zeE + 0,01 F1234zeZ** | | **Organics 0,01 F245cb + 0,01 F1243zf + 0,96 F1233zdE + 0,01 F1234zeE + 0,01 F1234zeZ** | | **Organics 0,01 F245cb + 0,01 F1243zf + 0,01 F1233zdE + 0,96 F1234zeE + 0,01 F1234zeZ** | | **Organics 0,01 F245cb + 0,01 F1243zf + 0,01 F1233zdE + 0,01 F1234zeE + 0,96 F1234zeZ** | |
|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE |
| | bar | | bar | | bar | | bar | | bar | | bar |
| 0 | 3,8 | 0 | 4,6 | 0 | 5,8 | 0 | 1,5 | 0 | 4,8 | 0 | 1,9 |
| 0,05 | 4,9 | 0,05 | 5,8 | 0,05 | 6,7 | 0,05 | 2,6 | 0,05 | 5,8 | 0,05 | 3,0 |
| 0,1 | 4,9 | 0,1 | 5,8 | 0,1 | 6,7 | 0,1 | 2,6 | 0,1 | 5,8 | 0,1 | 3,0 |
| 0,15 | 4,9 | 0,15 | 5,8 | 0,15 | 6,7 | 0,15 | 2,6 | 0,15 | 5,8 | 0,15 | 3,0 |
| 0,2 | 4,9 | 0,2 | 5,8 | 0,2 | 6,7 | 0,2 | 2,6 | 0,2 | 5,8 | 0,2 | 3,0 |
| 0,25 | 4,9 | 0,25 | 5,8 | 0,25 | 6,7 | 0,25 | 2,6 | 0,25 | 5,8 | 0,25 | 3,0 |
| 0,3 | 4,9 | 0,3 | 5,8 | 0,3 | 6,7 | 0,3 | 2,6 | 0,3 | 5,8 | 0,3 | 3,0 |
| 0,35 | 4,9 | 0,35 | 5,8 | 0,35 | 6,7 | 0,35 | 2,6 | 0,35 | 5,8 | 0,35 | 3,0 |
| 0,4 | 4,9 | 0,4 | 5,8 | 0,4 | 6,7 | 0,4 | 2,6 | 0,4 | 5,8 | 0,4 | 3,0 |
| 0,45 | 4,9 | 0,45 | 5,8 | 0,45 | 6,7 | 0,45 | 2,6 | 0,45 | 5,8 | 0,45 | 3,0 |
| 0,5 | 4,9 | 0,5 | 5,8 | 0,5 | 6,7 | 0,5 | 2,6 | 0,5 | 5,7 | 0,5 | 3,0 |
| 0,55 | 4,9 | 0,55 | 5,8 | 0,55 | 6,7 | 0,55 | 2,6 | 0,55 | 5,7 | 0,55 | 3,0 |
| 0,6 | 4,9 | 0,6 | 5,8 | 0,6 | 6,6 | 0,6 | 2,6 | 0,6 | 5,6 | 0,6 | 3,0 |
| 0,65 | 4,9 | 0,65 | 5,8 | 0,65 | 6,6 | 0,65 | 2,6 | 0,65 | 5,5 | 0,65 | 3,0 |
| 0,7 | 4,9 | 0,7 | 5,8 | 0,7 | 6,5 | 0,7 | 2,6 | 0,7 | 5,4 | 0,7 | 3,0 |
| 0,75 | 4,8 | 0,75 | 5,8 | 0,75 | 6,3 | 0,75 | 2,5 | 0,75 | 5,1 | 0,75 | 3,0 |
| 0,8 | 4,5 | 0,8 | 5,8 | 0,8 | 6,0 | 0,8 | 2,4 | 0,8 | 4,8 | 0,8 | 2,9 |
| 0,85 | 4,2 | 0,85 | 5,8 | 0,85 | 5,6 | 0,85 | 2,3 | 0,85 | 4,3 | 0,85 | 2,7 |
| 0,9 | 3,5 | 0,9 | 5,0 | 0,9 | 4,7 | 0,9 | 2,0 | 0,9 | 3,6 | 0,9 | 2,4 |
| 0,95 | 2,6 | 0,95 | 3,5 | 0,95 | 3,4 | 0,95 | 1,7 | 0,95 | 2,6 | 0,95 | 1,9 |
| 1 | 1,2 | 1 | 1,2 | 1 | 1,2 | 1 | 1,2 | 1 | 1,2 | 1 | 1,2 |

### Exemple 11 : Plage de température et de pression de systèmes à 6 composés

| | | |
|---|---|---|
| | Enveloppe de points d'ébullition | |

| Système à 6 composés | Température °C | Pression bar abs |
|---|---|---|
| HF-HCFO-1233xf-HFO-1234yf-HFC-245cb-HFO-1234zeE-HFO-1234zeZ | 0 à 40 | ∼1.0∼11.6 |
| HF-HCFO-1233xf-HFO-1234yf-HFC-245cb-HFO-1234zeE-HCFO-1233zdE | 0 à 40 | ∼0.9 à ∼11.6 |
| HF-HCFO-1233xf-HFO-1234yf-HFC-245cb-HFO-1234zeE-HFO-1243zf | 0 à 40 | ∼1.0 à ∼11.6 |
| HF-HCFO-1233xf-HFO-1234yf-HFC-245cb-HFO-1234zeZ-HCFO-1233zdE | 0 à 40 | ∼0.9 à ∼11.5 |
| HF-HCFO-1233xf-HFO-1234yf-HFC-245cb-HFO-1234zeZ-HFO-1243zf | 0 à 40 | ∼1.0 à ∼11.6 |
| HF-HCFO-1233xf-HFO-1234yf-HFO-1234zeE-HFO-1234zeZ-HCFO-1233zdE | 0 à 40 | ∼0.9 à ∼11.5 |
| HF-HCFO-1233xf-HFO-1234yf-HFO-1234zeE-HFO-1234zeZ-HFO-1243zf | 0 à 40 | ∼1.0 à ∼11.6 |
| HF-HCFO-1233xf-HFO-1234yf-HFO-1234zeE-HCFO-1233zdE-HFO-1243zf | 0 à 40 | ∼0.9 à ∼11.6 |
| HF-HCFO-1233xf-HFO-1234yf-HFO-1234zeZ-HCFO-1233zdE-HFO-1243zf | 0 à 40 | ∼0.9 à ∼11.6 |
| HF-HCFO-1233xf-HFC-245cb-HFO-1234zeE-HFO-1234zeZ-HCFO-1233zdE | 0 à 40 | ∼0.9 à ∼9.0 |
| HF-HCFO-1233xf-HFC-245cb-HFO-1234zeE-HFO-1234zeZ-HFO-1243zf | 0 à 40 | ∼1.0 à ∼10.3 |
| HF-HCFO-1233xf-HFC-245cb-HFO-1234zeE-HCFO-1233zdE-HFO-1243zf | 0 à 40 | ∼0.9 à ∼10.3 |
| HF-HCFO-1233xf-HFC-245cb-HFO-1234zeZ-HCFO-1233zdE-HFO-1243zf | 0 à 40 | ∼1.0 à ∼10.3 |
| HF-HCFO-1233xf-HFO-1234zeE-HFO-1234zeZ-HCFO-1233zdE-HFO-1243zf | 0 à 40 | ∼0.9 ∼10.3 |
| HF-HFO-1234yf-HFC-245cb-HFO-1234zeE-HFO-1234zeZ-HCFO-1233zdE | 0 à 40 | ∼0.9 ∼11.6 |
| HF-HFO-1234yf-HFC-245cb-HFO-1234zeE-HFO-1234zeZ-HFO-1243zf | 0 à 40 | ∼1.2 à ∼11.6 |
| HF-HFO-1234yf-HFC-245cb-HFO-1234zeE-HCFO-1233zdE-HFO-1243zf | 0 à 40 | ∼1.0 à ∼11.7 |
| HF-HFO-1234yf-HFC-245cb-HFO-1234zeZ-HCFO-1233zdE-HFO-1243zf | 0 à 40 | ∼0.9 à ∼11.6 |
| HF-HFO-1234yf-HFO-1234zeE-HFO-1234zeZ-HCFO-1233zdE-HFO-1243zf | 0 à 40 | ∼0.9 à ∼11.6 |
| HF-HFC-245cb-HFO-1234zeE-HFO-1234zeZ-HCFO-1233zdE-HFO-1243zf | 0 à 40 | ∼0.9 à ∼10.3 |

### Exemple 12 : Plage de décantation de systèmes à 6 composés

| | Plages de décantation | | |
|---|---|---|---|
| | Pourcentage massique d'HF | | |
| Système à 6 composés | Isotherme | | |
| | 0 °C | 25 °C | 40 °C |
| HF-HCFO-1233xf-HFO-1234yf-HFC-245cb-HFO-1234zeE-HFO-1234zeZ | 5-75 | 5-70 | 15-55 |
| HF-HCFO-1233xf-HFO-1234yf-HFC-245cb-HFO-1234zeE-HCFO-1233zdE | 5-75 | 5-70 | 15-55 |
| HF-HCFO-1233xf-HFO-1234yf-HFC-245cb-HFO-1234zeE-HFO-1243zf | 5-75 | 10-65 | * |
| HF-HCFO-1233xf-HFO-1234yf-HFC-245cb-HFO-1234zeZ-HCFO-1233zdE | 5-75 | 5-75 | 10-65 |
| HF-HCFO-1233xf-HFO-1234yf-HFC-245cb-HFO-1234zeZ-HFO-1243zf | 5-75 | 10-70 | 15-50 |
| HF-HCFO-1233xf-HFO-1234yf-HFO-1234zeE-HFO-1234zeZ-HCFO-1233zdE | 5-75 | 5-65 | 10-55 |
| HF-HCFO-1233xf-HFO-1234yf-HFO-1234zeE-HFO-1234zeZ-HFO-1243zf | 5-70 | 10-65 | * |
| HF-HCFO-1233xf-HFO-1234yf-HFO-1234zeE-HCFO-1233zdE-HFO-1243zf | 5-70 | 10-75 | * |
| HF-HCFO-1233xf-HFO-1234yf-HFO-1234zeZ-HCFO-1233zdE-HFO-1243zf | 5-75 | 5-70 | 10-55 |
| HF-HCFO-1233xf-HFC-245cb-HFO-1234zeE-HFO-1234zeZ-HCFO-1233zdE | 5-75 | 5-70 | 10-65 |
| HF-HCFO-1233xf-HFC-245cb-HFO-1234zeE-HFO-1234zeZ-HFO-1243zf | 5-75 | 10-70 | 15-45 |
| HF-HCFO-1233xf-HFC-245cb-HFO-1234zeE-HCFO-1233zdE-HFO-1243zf | 5-75 | 5-70 | 15-50 |
| HF-HCFO-1233xf-HFC-245cb-HFO-1234zeZ-HCFO-1233zdE-HFO-1243zf | 5-75 | 5-70 | 10-65 |
| HF-HCFO-1233xf-HFO-1234zeE-HFO-1234zeZ-HCFO-1233zdE-HFO-1243zf | 5-75 | 5-65 | 10-55 |
| HF-HFO-1234yf-HFC-245cb-HFO-1234zeE-HFO-1234zeZ-HCFO-1233zdE | 5-75 | 5-70 | 15-55 |
| HF-HFO-1234yf-HFC-245cb-HFO-1234zeE-HFO-1234zeZ-HFO-1243zf | 5-75 | 10-65 | * |
| HF-HFO-1234yf-HFC-245cb-HFO-1234zeE-HCFO-1233zdE-HFO-1243zf | 5-75 | 10-65 | * |
| HF-HFO-1234yf-HFC-245cb-HFO-1234zeZ-HCFO-1233zdE-HFO-1243zf | 5-75 | 10-70 | 15-55 |
| HF-HFO-1234yf-HFO-1234zeE-HFO-1234zeZ-HCFO-1233zdE-HFO-1243zf | 5-70 | 10-65 | * |
| HF-HFC-245cb-HFO-1234zeE-HFO-1234zeZ-HCFO-1233zdE-HFO-1243zf | 5-75 | 5-70 | 15-50 |

### Exemple 13 : Systèmes à sept composés, isotherme à 25 °C

**HF - HCFO-1233xf - HFC-245cb - HCFO-1233zdE -HFO-1234zeE - HFO-1234zeZ - HFO-1243zf**

| **Organics 0,15 F1233xf** + **0,17 F245cb** + **0,17 F1233zdE** + **0,17 F1234zeE** + **0,17 F1234zeZ** + **0,17 F1243zf** | | **Organics 0,95 F1233xf + 0,01 F245cb + 0,01 F1233zdE + 0,01 F1234zeE + 0,01 F1234zeZ + 0,01 F1243zf** | | **Organics 0,01 F1233xf + 0,95 F245cb + 0,01 F1233zdE + 0,01 F1234zeE + 0,01 F1234zeZ + 0,01 F1243zf** | | **Organics 0,01 F1233xf + 0,01 F245cb + 0,95 F1233zdE + 0,01 F1234zeE + 0,01 F1234zeZ + 0,01 F1243zf** | | **Organics 0,01 F1233xf + 0,01 F245cb + 0,01 F1233zdE + 0,95 F1234zeE + 0,01 F1234zeZ + 0,01 F1243zf** | | **Organics 0,01 F1233xf + 0,01 F245cb + 0,01 F1233zdE + 0,01 F1234zeE + 0,01 F1234zeZ + 0,95 F1243zf** | | **Organics 0,01 F1233xf + 0,01 F245cb + 0,01 F1233zdE + 0,01 F1234zeE + 0,95 F1234zeZ + 0,01 F1243zf** | |
|---|---|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | | | | | | | | | | | | | |
| | bar | | bar | | bar | | bar | | bar | | bar | | bar |
| 0 | 3,5 | 0 | 1,7 | 0 | 4,6 | 0 | 1,5 | 0 | 4,8 | 0 | 5,7 | 0 | 1,9 |
| 0,05 | 4,6 | 0,05 | 2,8 | 0,05 | 5,7 | 0,05 | 2,6 | 0,05 | 5,7 | 0,05 | 6,7 | 0,05 | 3,0 |
| 0,1 | 4,6 | 0,1 | 2,8 | 0,1 | 5,7 | 0,1 | 2,6 | 0,1 | 5,8 | 0,1 | 6,7 | 0,1 | 3,0 |
| 0,15 | 4,6 | 0,15 | 2,8 | 0,15 | 5,7 | 0,15 | 2,6 | 0,15 | 5,8 | 0,15 | 6,7 | 0,15 | 3,0 |
| 0,2 | 4,6 | 0,2 | 2,8 | 0,2 | 5,7 | 0,2 | 2,6 | 0,2 | 5,8 | 0,2 | 6,7 | 0,2 | 3,0 |
| 0,25 | 4,6 | 0,25 | 2,8 | 0,25 | 5,8 | 0,25 | 2,6 | 0,25 | 5,8 | 0,25 | 6,7 | 0,25 | 3,0 |
| 0,3 | 4,6 | 0,3 | 2,8 | 0,3 | 5,8 | 0,3 | 2,6 | 0,3 | 5,8 | 0,3 | 6,7 | 0,3 | 3,0 |
| 0,35 | 4,6 | 0,35 | 2,8 | 0,35 | 5,8 | 0,35 | 2,6 | 0,35 | 5,8 | 0,35 | 6,7 | 0,35 | 3,0 |
| 0,4 | 4,6 | 0,4 | 2,8 | 0,4 | 5,8 | 0,4 | 2,6 | 0,4 | 5,8 | 0,4 | 6,7 | 0,4 | 3,0 |
| 0,45 | 4,6 | 0,45 | 2,8 | 0,45 | 5,8 | 0,45 | 2,6 | 0,45 | 5,7 | 0,45 | 6,7 | 0,45 | 3,0 |
| 0,5 | 4,6 | 0,5 | 2,8 | 0,5 | 5,8 | 0,5 | 2,6 | 0,5 | 5,7 | 0,5 | 6,7 | 0,5 | 3,0 |
| 0,55 | 4,6 | 0,55 | 2,8 | 0,55 | 5,8 | 0,55 | 2,6 | 0,55 | 5,7 | 0,55 | 6,6 | 0,55 | 3,0 |
| 0,6 | 4,6 | 0,6 | 2,8 | 0,6 | 5,8 | 0,6 | 2,6 | 0,6 | 5,6 | 0,6 | 6,6 | 0,6 | 3,0 |
| 0,65 | 4,6 | 0,65 | 2,8 | 0,65 | 5,8 | 0,65 | 2,6 | 0,65 | 5,5 | 0,65 | 6,6 | 0,65 | 3,0 |
| 0,7 | 4,6 | 0,7 | 2,8 | 0,7 | 5,8 | 0,7 | 2,6 | 0,7 | 5,3 | 0,7 | 6,5 | 0,7 | 3,0 |
| 0,75 | 4,5 | 0,75 | 2,8 | 0,75 | 5,8 | 0,75 | 2,6 | 0,75 | 5,1 | 0,75 | 6,3 | 0,75 | 3,0 |
| 0,8 | 4,2 | 0,8 | 2,6 | 0,8 | 5,8 | 0,8 | 2,4 | 0,8 | 4,8 | 0,8 | 6,0 | 0,8 | 2,9 |
| 0,85 | 3,9 | 0,85 | 2,4 | 0,85 | 5,8 | 0,85 | 2,3 | 0,85 | 4,3 | 0,85 | 5,5 | 0,85 | 2,7 |
| 0,9 | 3,3 | 0,9 | 2,2 | 0,9 | 4,9 | 0,9 | 2,0 | 0,9 | 3,6 | 0,9 | 4,7 | 0,9 | 2,4 |
| 0,95 | 2,5 | 0,95 | 1,8 | 0,95 | 3,5 | 0,95 | 1,7 | 0,95 | 2,6 | 0,95 | 3,3 | 0,95 | 1,9 |
| 1 | 1,2 | 1 | 1,2 | 1 | 1,2 | 1 | 1,2 | 1 | 1,2 | 1 | 1,2 | 1 | 1,2 |

**HF - HCFO-1233xf - HFO-1234f - HFC-245cb - HCFO-1233zdE - HFO-1234zeE - HFO-1234zeZ**

| **Organics 0,15 F1233xf + 0,17 F1234yf + 0,17 F245cb + 0,17 F1233zdE + 0,17 F1234zeE + 0,17 F1234zeZ** | | **Organics 0,01 F1233xf + 0,95 F1234yf + 0,01 F245cb + 0,01 F1233zdE + 0,01 F1234zeE + 0,01 F1234zeZ** | | **Organics 0,01 F1233xf + 0,01 F1234yf + 0,95 F245cb + 0,01 F1233zdE + 0,01 F1234zeE + 0,01 F1234zeZ** | | **Organics 0,01 F1233xf + 0,01 F1234yf + 0,01 F245cb + 0,95 F1233zdE + 0,01 F1234zeE + 0,01 F1234zeZ** | | **Organics 0,01 F1233xf + 0,01 F1234yf + 0,01 F245cb + 0,01 F1233zdE + 0,95 F1234zeE + 0,01 F1234zeZ** | | **Organics 0,01 F1233xf + 0,01 F1234yf + 0,01 F245cb + 0,01 F1233zdE + 0,01 F1234zeE + 0,95 F1234zeZ** | | **Organics 0,95 F1233xf + 0,01 F1234yf + 0,01 F245cb + 0,01 F1233zdE + 0,01 F1234zeE + 0,01 F1234zeZ** | |
|---|---|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | | | | | | | | | | | | | |
| | bar | | bar | | bar | | bar | | bar | | bar | | bar |
| 0 | 3,6 | 0 | 6,6 | 0 | 4,6 | 0 | 1,5 | 0 | 4,8 | 0 | 1,9 | 0 | 1,7 |
| 0,05 | 4,7 | 0,05 | 7,6 | 0,05 | 5,8 | 0,05 | 2,6 | 0,05 | 5,8 | 0,05 | 3,0 | 0,05 | 2,8 |
| 0,1 | 4,7 | 0,1 | 7,6 | 0,1 | 5,8 | 0,1 | 2,6 | 0,1 | 5,8 | 0,1 | 3,0 | 0,1 | 2,8 |
| 0,15 | 4,7 | 0,15 | 7,6 | 0,15 | 5,8 | 0,15 | 2,6 | 0,15 | 5,8 | 0,15 | 3,0 | 0,15 | 2,8 |
| 0,2 | 4,7 | 0,2 | 7,6 | 0,2 | 5,8 | 0,2 | 2,6 | 0,2 | 5,8 | 0,2 | 3,0 | 0,2 | 2,8 |
| 0,25 | 4,7 | 0,25 | 7,6 | 0,25 | 5,8 | 0,25 | 2,6 | 0,25 | 5,8 | 0,25 | 3,0 | 0,25 | 2,8 |
| 0,3 | 4,7 | 0,3 | 7,6 | 0,3 | 5,8 | 0,3 | 2,6 | 0,3 | 5,8 | 0,3 | 3,0 | 0,3 | 2,8 |
| 0,35 | 4,7 | 0,35 | 7,6 | 0,35 | 5,8 | 0,35 | 2,6 | 0,35 | 5,8 | 0,35 | 3,0 | 0,35 | 2,8 |
| 0,4 | 4,7 | 0,4 | 7,6 | 0,4 | 5,8 | 0,4 | 2,6 | 0,4 | 5,8 | 0,4 | 3,0 | 0,4 | 2,8 |
| 0,45 | 4,7 | 0,45 | 7,6 | 0,45 | 5,8 | 0,45 | 2,6 | 0,45 | 5,7 | 0,45 | 3,0 | 0,45 | 2,8 |
| 0,5 | 4,7 | 0,5 | 7,6 | 0,5 | 5,8 | 0,5 | 2,6 | 0,5 | 5,7 | 0,5 | 3,0 | 0,5 | 2,8 |
| 0,55 | 4,7 | 0,55 | 7,6 | 0,55 | 5,8 | 0,55 | 2,6 | 0,55 | 5,7 | 0,55 | 3,0 | 0,55 | 2,8 |
| 0,6 | 4,7 | 0,6 | 7,6 | 0,6 | 5,8 | 0,6 | 2,6 | 0,6 | 5,6 | 0,6 | 3,0 | 0,6 | 2,8 |
| 0,65 | 4,7 | 0,65 | 7,6 | 0,65 | 5,8 | 0,65 | 2,6 | 0,65 | 5,5 | 0,65 | 3,0 | 0,65 | 2,8 |
| 0,7 | 4,7 | 0,7 | 7,6 | 0,7 | 5,8 | 0,7 | 2,6 | 0,7 | 5,3 | 0,7 | 3,0 | 0,7 | 2,8 |
| 0,75 | 4,6 | 0,75 | 7,4 | 0,75 | 5,8 | 0,75 | 2,6 | 0,75 | 5,1 | 0,75 | 3,0 | 0,75 | 2,8 |
| 0,8 | 4,4 | 0,8 | 7,1 | 0,8 | 5,8 | 0,8 | 2,5 | 0,8 | 4,8 | 0,8 | 2,9 | 0,8 | 2,6 |
| 0,85 | 4,1 | 0,85 | 6,5 | 0,85 | 5,8 | 0,85 | 2,3 | 0,85 | 4,3 | 0,85 | 2,7 | 0,85 | 2,5 |
| 0,9 | 3,5 | 0,9 | 5,5 | 0,9 | 5,0 | 0,9 | 2,0 | 0,9 | 3,6 | 0,9 | 2,4 | 0,9 | 2,2 |
| 0,95 | 2,5 | 0,95 | 3,8 | 0,95 | 3,5 | 0,95 | 1,7 | 0,95 | 2,6 | 0,95 | 1,9 | 0,95 | 1,8 |
| 1 | 1,2 | 1 | 1,2 | 1 | 1,2 | 1 | 1,2 | 1 | 1,2 | 1 | 1,2 | 1 | 1,2 |

**HF - HCFO-1233xf - HFO-1234f - HFC-245cb - HCFO-1233zdE - HFO-1234zeE - HFO-1243zf**

| **Organics 0,15 F1233xf + 0,17 F1234yf + 0,17 F245cb + 0,17 F1233zdE + 0,17 F1234zeE + 0,17 F1243zf** | | **Organics 0,01 F1233xf + 0,95 F1234yf + 0,01 F245cb + 0,01 F1233zdE + 0,01 F1234zeE + 0,01 F1243zf** | | **Organics 0,01 F1233xf + 0,01 F1234yf + 0,95 F245cb + 0,01 F1233zdE + 0,01 F1234zeE + 0,01 F1243zf** | | **Organics 0,01 F1233xf + 0,01 F1234yf + 0,01 F245cb + 0,95 F1233zdE + 0,01 F1234zeE + 0,01 F1243zf** | | **Organics 0,01 F1233xf + 0,01 F1234yf + 0,01 F245cb + 0,01 F1233zdE + 0,95 F1234zeE + 0,01 F1243zf** | | **Organics 0,01 F1233xf + 0,01 F1234yf + 0,01 F245cb + 0,01 F1233zdE + 0,01 F1234zeE + 0,95 F1243zf** | | **Organics 0,95 F1233xf + 0,01 F1234yf + 0,01 F245cb + 0,01 F1233zdE + 0,01 F1234zeE + 0,01 F1243zf** | |
|---|---|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | bar | | bar | | bar | | bar | | bar | | bar | | bar |
| 0 | 4,4 | 0 | 6,6 | 0 | 4,6 | 0 | 1,5 | 0 | 4,8 | 0 | 5,8 | 0 | 1,7 |
| 0,05 | 5,4 | 0,05 | 7,6 | 0,05 | 5,8 | 0,05 | 2,6 | 0,05 | 5,8 | 0,05 | 6,7 | 0,05 | 2,8 |
| 0,1 | 5,4 | 0,1 | 7,6 | 0,1 | 5,8 | 0,1 | 2,6 | 0,1 | 5,8 | 0,1 | 6,7 | 0,1 | 2,8 |
| 0,15 | 5,4 | 0,15 | 7,6 | 0,15 | 5,8 | 0,15 | 2,6 | 0,15 | 5,8 | 0,15 | 6,7 | 0,15 | 2,8 |
| 0,2 | 5,4 | 0,2 | 7,6 | 0,2 | 5,8 | 0,2 | 2,6 | 0,2 | 5,8 | 0,2 | 6,7 | 0,2 | 2,8 |
| 0,25 | 5,4 | 0,25 | 7,6 | 0,25 | 5,8 | 0,25 | 2,6 | 0,25 | 5,8 | 0,25 | 6,7 | 0,25 | 2,8 |
| 0,3 | 5,4 | 0,3 | 7,6 | 0,3 | 5,8 | 0,3 | 2,6 | 0,3 | 5,8 | 0,3 | 6,7 | 0,3 | 2,8 |
| 0,35 | 5,4 | 0,35 | 7,6 | 0,35 | 5,8 | 0,35 | 2,6 | 0,35 | 5,8 | 0,35 | 6,7 | 0,35 | 2,8 |
| 0,4 | 5,4 | 0,4 | 7,6 | 0,4 | 5,8 | 0,4 | 2,6 | 0,4 | 5,8 | 0,4 | 6,7 | 0,4 | 2,8 |
| 0,45 | 5,4 | 0,45 | 7,6 | 0,45 | 5,8 | 0,45 | 2,6 | 0,45 | 5,8 | 0,45 | 6,7 | 0,45 | 2,8 |
| 0,5 | 5,4 | 0,5 | 7,6 | 0,5 | 5,8 | 0,5 | 2,6 | 0,5 | 5,8 | 0,5 | 6,7 | 0,5 | 2,8 |
| 0,55 | 5,4 | 0,55 | 7,6 | 0,55 | 5,8 | 0,55 | 2,6 | 0,55 | 5,7 | 0,55 | 6,7 | 0,55 | 2,8 |
| 0,6 | 5,4 | 0,6 | 7,6 | 0,6 | 5,8 | 0,6 | 2,6 | 0,6 | 5,6 | 0,6 | 6,7 | 0,6 | 2,8 |
| 0,65 | 5,4 | 0,65 | 7,6 | 0,65 | 5,8 | 0,65 | 2,6 | 0,65 | 5,5 | 0,65 | 6,6 | 0,65 | 2,8 |
| 0,7 | 5,4 | 0,7 | 7,6 | 0,7 | 5,8 | 0,7 | 2,6 | 0,7 | 5,4 | 0,7 | 6,5 | 0,7 | 2,8 |
| 0,75 | 5,3 | 0,75 | 7,4 | 0,75 | 5,8 | 0,75 | 2,6 | 0,75 | 5,1 | 0,75 | 6,3 | 0,75 | 2,8 |
| 0,8 | 5,0 | 0,8 | 7,1 | 0,8 | 5,8 | 0,8 | 2,5 | 0,8 | 4,8 | 0,8 | 6,1 | 0,8 | 2,7 |
| 0,85 | 4,6 | 0,85 | 6,5 | 0,85 | 5,8 | 0,85 | 2,3 | 0,85 | 4,3 | 0,85 | 5,6 | 0,85 | 2,5 |
| 0,9 | 3,9 | 0,9 | 5,5 | 0,9 | 5,0 | 0,9 | 2,1 | 0,9 | 3,6 | 0,9 | 4,7 | 0,9 | 2,2 |
| 0,95 | 2,8 | 0,95 | 3,8 | 0,95 | 3,5 | 0,95 | 1,7 | 0,95 | 2,6 | 0,95 | 3,4 | 0,95 | 1,8 |
| 1 | 1,2 | 1 | 1,2 | 1 | 1,2 | 1 | 1,2 | 1 | 1,2 | 1 | 1,2 | 1 | 1,2 |

**HF - HCFO-1233xf - HFO-1234f - HFC-245cb - HCFO-1233zdE - HFO-1234zeZ - HFO-1234zeZ**

| **Organics 0,15 F1233xf + 0,17 F1234yf + 0,17 F245cb + 0,17 F1243zf + 0,17 F1233zdE + 0,17 F1234zeZ** | | **Organics 0,01 F1233xf + 0,95 F1234yf + 0,01 F245cb + 0,01 F1243zf + 0,01 F1233zdE + 0,01 F1234zeZ** | | **Organics 0,01 F1233xf + 0,01 F1234yf + 0,95 F245cb + 0,01 F1243zf + 0,01 F1233zdE + 0,01 F1234zeZ** | | **Organics 0,01 F1233xf + 0,01 F1234yf + 0,01 F245cb + 0,95 F1243zf + 0,01 F1233zdE + 0,01 F1234zeZ** | | **Organics 0,01 F1233xf + 0,01 F1234yf + 0,01 F245cb + 0,01 F1243zf + 0,95 F1233zdE + 0,01 F1234zeZ** | | **Organics 0,01 F1233xf + 0,01 F1234yf + 0,01 F245cb + 0,01 F1243zf + 0,01 F1233zdE + 0,95 F1234zeZ** | | **Organics 0,95 F1233xf + 0,01 F1234yf + 0,01 F245cb** + **0,01 F1243zf + 0,01 F1233zdE + 0,01 F1234zeZ** | |
|---|---|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | bar | | bar | | bar | | bar | | bar | | bar | | bar |
| 0 | 3,8 | 0 | 6,6 | 0 | 4,6 | 0 | 5,7 | 0 | 1,5 | 0 | 1,9 | 0 | 1,7 |
| 0,05 | 4,9 | 0,05 | 7,6 | 0,05 | 5,8 | 0,05 | 6,7 | 0,05 | 2,6 | 0,05 | 3,0 | 0,05 | 2,8 |
| 0,1 | 4,9 | 0,1 | 7,6 | 0,1 | 5,8 | 0,1 | 6,7 | 0,1 | 2,6 | 0,1 | 3,0 | 0,1 | 2,8 |
| 0,15 | 4,9 | 0,15 | 7,6 | 0,15 | 5,8 | 0,15 | 6,7 | 0,15 | 2,6 | 0,15 | 3,0 | 0,15 | 2,8 |
| 0,2 | 4,9 | 0,2 | 7,6 | 0,2 | 5,8 | 0,2 | 6,7 | 0,2 | 2,6 | 0,2 | 3,0 | 0,2 | 2,8 |
| 0,25 | 4,9 | 0,25 | 7,6 | 0,25 | 5,8 | 0,25 | 6,7 | 0,25 | 2,6 | 0,25 | 3,0 | 0,25 | 2,8 |
| 0,3 | 4,9 | 0,3 | 7,6 | 0,3 | 5,8 | 0,3 | 6,7 | 0,3 | 2,6 | 0,3 | 3,0 | 0,3 | 2,8 |
| 0,35 | 4,9 | 0,35 | 7,6 | 0,35 | 5,8 | 0,35 | 6,7 | 0,35 | 2,6 | 0,35 | 3,0 | 0,35 | 2,8 |
| 0,4 | 4,9 | 0,4 | 7,6 | 0,4 | 5,8 | 0,4 | 6,7 | 0,4 | 2,6 | 0,4 | 3,0 | 0,4 | 2,8 |
| 0,45 | 4,9 | 0,45 | 7,6 | 0,45 | 5,8 | 0,45 | 6,7 | 0,45 | 2,6 | 0,45 | 3,0 | 0,45 | 2,8 |
| 0,5 | 4,9 | 0,5 | 7,6 | 0,5 | 5,8 | 0,5 | 6,7 | 0,5 | 2,6 | 0,5 | 3,0 | 0,5 | 2,8 |
| 0,55 | 4,9 | 0,55 | 7,6 | 0,55 | 5,8 | 0,55 | 6,7 | 0,55 | 2,6 | 0,55 | 3,0 | 0,55 | 2,8 |
| 0,6 | 4,9 | 0,6 | 7,6 | 0,6 | 5,8 | 0,6 | 6,6 | 0,6 | 2,6 | 0,6 | 3,0 | 0,6 | 2,8 |
| 0,65 | 4,9 | 0,65 | 7,6 | 0,65 | 5,8 | 0,65 | 6,6 | 0,65 | 2,6 | 0,65 | 3,0 | 0,65 | 2,8 |
| 0,7 | 4,9 | 0,7 | 7,6 | 0,7 | 5,8 | 0,7 | 6,5 | 0,7 | 2,6 | 0,7 | 3,0 | 0,7 | 2,8 |
| 0,75 | 4,9 | 0,75 | 7,4 | 0,75 | 5,8 | 0,75 | 6,3 | 0,75 | 2,6 | 0,75 | 3,0 | 0,75 | 2,8 |
| 0,8 | 4,7 | 0,8 | 7,1 | 0,8 | 5,8 | 0,8 | 6,0 | 0,8 | 2,5 | 0,8 | 2,9 | 0,8 | 2,7 |
| 0,85 | 4,3 | 0,85 | 6,5 | 0,85 | 5,8 | 0,85 | 5,5 | 0,85 | 2,3 | 0,85 | 2,7 | 0,85 | 2,5 |
| 0,9 | 3,7 | 0,9 | 5,5 | 0,9 | 5,0 | 0,9 | 4,7 | 0,9 | 2,0 | 0,9 | 2,4 | 0,9 | 2,2 |
| 0,95 | 2,7 | 0,95 | 3,8 | 0,95 | 3,5 | 0,95 | 3,3 | 0,95 | 1,7 | 0,95 | 1,9 | 0,95 | 1,8 |
| 1 | 1,2 | 1 | 1,2 | 1 | 1,2 | 1 | 1,2 | 1 | 1,2 | 1 | 1,2 | 1 | 1,2 |

**HF - HCFO-1233xf - HFO-1234f - HFC-245cb -HFO-1234zeE - HFO-1234zeZ - HFO-1243zf**

| **Organics 0,15 F1233xf + 0,17 F1234yf + 0,17 F245cb + 0,17 F1243zf + 0,17 F1234zeE + 0,17 F1234zeZ** | | **Organics 0,01 F1233xf + 0,95 F1234yf + 0,01 F245cb + 0,01 F1243zf + 0,01 F1234zeE + 0,01 F1234zeZ** | | **Organics 0,01 F1233xf + 0,01 F1234yf + 0,95 F245cb + 0,01 F1243zf + 0,01 F1234zeE + 0,01 F1234zeZ** | | **Organics 0,01 F1233xf + 0,01 F1234yf + 0,01 F245cb + 0,95 F1243zf + 0,01 F1234zeE + 0,01 F1234zeZ** | | **Organics 0,01 F1233xf + 0,01 F1234yf + 0,01 F245cb + 0,01 F1243zf + 0,95 F1234zeE + 0,01 F1234zeZ** | | **Organics 0,01 F1233xf + 0,01 F1234yf + 0,01 F245cb + 0,01 F1243zf + 0,01 F1234zeE + 0,95 F1234zeZ** | | **Organics 0,95 F1233xf + 0,01 F1234yf + 0,01 F245cb + 0,01 F1243zf + 0,01 F1234zeE + 0,01 F1234zeZ** | |
|---|---|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | bar | | bar | | bar | | bar | | bar | | bar | | bar |
| 0 | 4,4 | 0 | 6,6 | 0 | 4,6 | 0 | 5,8 | 0 | 4,8 | 0 | 2,0 | 0 | 1,7 |
| 0,05 | 5,5 | 0,05 | 7,6 | 0,05 | 5,8 | 0,05 | 6,7 | 0,05 | 5,8 | 0,05 | 3,1 | 0,05 | 2,8 |
| 0,1 | 5,5 | 0,1 | 7,6 | 0,1 | 5,8 | 0,1 | 6,7 | 0,1 | 5,8 | 0,1 | 3,1 | 0,1 | 2,8 |
| 0,15 | 5,5 | 0,15 | 7,6 | 0,15 | 5,8 | 0,15 | 6,7 | 0,15 | 5,8 | 0,15 | 3,1 | 0,15 | 2,8 |
| 0,2 | 5,5 | 0,2 | 7,6 | 0,2 | 5,8 | 0,2 | 6,7 | 0,2 | 5,8 | 0,2 | 3,1 | 0,2 | 2,8 |
| 0,25 | 5,5 | 0,25 | 7,6 | 0,25 | 5,8 | 0,25 | 6,7 | 0,25 | 5,8 | 0,25 | 3,1 | 0,25 | 2,8 |
| 0,3 | 5,5 | 0,3 | 7,6 | 0,3 | 5,8 | 0,3 | 6,7 | 0,3 | 5,8 | 0,3 | 3,1 | 0,3 | 2,8 |
| 0,35 | 5,5 | 0,35 | 7,6 | 0,35 | 5,8 | 0,35 | 6,7 | 0,35 | 5,8 | 0,35 | 3,1 | 0,35 | 2,8 |
| 0,4 | 5,5 | 0,4 | 7,6 | 0,4 | 5,8 | 0,4 | 6,7 | 0,4 | 5,8 | 0,4 | 3,1 | 0,4 | 2,8 |
| 0,45 | 5,5 | 0,45 | 7,6 | 0,45 | 5,8 | 0,45 | 6,7 | 0,45 | 5,8 | 0,45 | 3,1 | 0,45 | 2,8 |
| 0,5 | 5,5 | 0,5 | 7,6 | 0,5 | 5,8 | 0,5 | 6,7 | 0,5 | 5,8 | 0,5 | 3,1 | 0,5 | 2,8 |
| 0,55 | 5,5 | 0,55 | 7,6 | 0,55 | 5,8 | 0,55 | 6,7 | 0,55 | 5,7 | 0,55 | 3,1 | 0,55 | 2,8 |
| 0,6 | 5,5 | 0,6 | 7,6 | 0,6 | 5,8 | 0,6 | 6,7 | 0,6 | 5,6 | 0,6 | 3,1 | 0,6 | 2,8 |
| 0,65 | 5,5 | 0,65 | 7,6 | 0,65 | 5,8 | 0,65 | 6,6 | 0,65 | 5,5 | 0,65 | 3,1 | 0,65 | 2,8 |
| 0,7 | 5,5 | 0,7 | 7,6 | 0,7 | 5,8 | 0,7 | 6,5 | 0,7 | 5,4 | 0,7 | 3,1 | 0,7 | 2,8 |
| 0,75 | 5,3 | 0,75 | 7,4 | 0,75 | 5,8 | 0,75 | 6,3 | 0,75 | 5,1 | 0,75 | 3,1 | 0,75 | 2,8 |
| 0,8 | 5,1 | 0,8 | 7,1 | 0,8 | 5,8 | 0,8 | 6,1 | 0,8 | 4,8 | 0,8 | 2,9 | 0,8 | 2,7 |
| 0,85 | 4,6 | 0,85 | 6,5 | 0,85 | 5,8 | 0,85 | 5,6 | 0,85 | 4,3 | 0,85 | 2,7 | 0,85 | 2,5 |
| 0,9 | 3,9 | 0,9 | 5,5 | 0,9 | 5,0 | 0,9 | 4,7 | 0,9 | 3,6 | 0,9 | 2,4 | 0,9 | 2,2 |
| 0,95 | 2,8 | 0,95 | 3,8 | 0,95 | 3,5 | 0,95 | 3,4 | 0,95 | 2,6 | 0,95 | 1,9 | 0,95 | 1,8 |
| 1 | 1,2 | 1 | 1,2 | 1 | 1,2 | 1 | 1,2 | 1 | 1,2 | 1 | 1,2 | 1 | 1,2 |

**HF - HCFO-1233xf - HFO-1234yf - HCFO-1233zdE -HFO-1234zeE - HFO-1234zeZ - HFO-1243zf**

| **Organics 0,15 F1233xf + 0,17 F1234yf + 0,17 F1233zdE + 0,17 F1243zf + 0,17 F1234zeE + 0,17 F1234zeZ** | | **Organics 0,01 F1233xf + 0,95 F1234yf + 0,01 F1233zdE + 0,01 F1243zf + 0,01 F1234zeE + 0,01 F1234zeZ** | | **Organics 0,01 F1233xf + 0,01 F1234yf + 0,95 F1233zdE + 0,01 F1243zf + 0,01 F1234zeE + 0,01 F1234zeZ** | | **Organics 0,01 F1233xf + 0,01 F1234yf + 0,01 F1233zdE + 0,95 F1243zf + 0,01 F1234zeE + 0,01 F1234zeZ** | | **Organics 0,01 F1233xf + 0,01 F1234yf + 0,01 F1233zdE + 0,01 F1243zf + 0,95 F1234zeE + 0,01 F1234zeZ** | | **Organics 0,01 F1233xf + 0,01 F1234yf + 0,01 F1233zdE + 0,01 F1243zf + 0,01 F1234zeE + 0,95 F1234zeZ** | | **Organics 0,95 F1233xf + 0,01 F1234yf + 0,01 F1233zdE + 0,01 F1243zf + 0,01 F1234zeE + 0,01 F1234zeZ** | |
|---|---|---|---|---|---|---|---|---|---|---|---|---|---|
| MASSFRAC | TOTAL | MASSFRAC | TOTAL | MASSFRACI | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | bar | | bar | | bar | | bar | | bar | | bar | | bar |
| 0 | 3,9 | 0 | 6,6 | 0 | 1,5 | 0 | 5,7 | 0 | 4,8 | 0 | 1,9 | 0 | 1,7 |
| 0,05 | 5,0 | 0,05 | 7,6 | 0,05 | 2,6 | 0,05 | 6,7 | 0,05 | 5,8 | 0,05 | 3,0 | 0,05 | 2,8 |
| 0,1 | 4,9 | 0,1 | 7,6 | 0,1 | 2,6 | 0,1 | 6,7 | 0,1 | 5,8 | 0,1 | 3,0 | 0,1 | 2,8 |
| 0,15 | 4,9 | 0,15 | 7,6 | 0,15 | 2,6 | 0,15 | 6,7 | 0,15 | 5,8 | 0,15 | 3,0 | 0,15 | 2,8 |
| 0,2 | 4,9 | 0,2 | 7,6 | 0,2 | 2,6 | 0,2 | 6,7 | 0,2 | 5,8 | 0,2 | 3,0 | 0,2 | 2,8 |
| 0,25 | 4,9 | 0,25 | 7,6 | 0,25 | 2,6 | 0,25 | 6,7 | 0,25 | 5,8 | 0,25 | 3,0 | 0,25 | 2,8 |
| 0,3 | 4,9 | 0,3 | 7,6 | 0,3 | 2,6 | 0,3 | 6,7 | 0,3 | 5,8 | 0,3 | 3,0 | 0,3 | 2,8 |
| 0,35 | 4,9 | 0,35 | 7,6 | 0,35 | 2,6 | 0,35 | 6,7 | 0,35 | 5,8 | 0,35 | 3,0 | 0,35 | 2,8 |
| 0,4 | 4,9 | 0,4 | 7,6 | 0,4 | 2,6 | 0,4 | 6,7 | 0,4 | 5,8 | 0,4 | 3,0 | 0,4 | 2,8 |
| 0,45 | 4,9 | 0,45 | 7,6 | 0,45 | 2,6 | 0,45 | 6,7 | 0,45 | 5,8 | 0,45 | 3,0 | 0,45 | 2,8 |
| 0,5 | 4,9 | 0,5 | 7,6 | 0,5 | 2,6 | 0,5 | 6,7 | 0,5 | 5,7 | 0,5 | 3,0 | 0,5 | 2,8 |
| 0,55 | 4,9 | 0,55 | 7,6 | 0,55 | 2,6 | 0,55 | 6,7 | 0,55 | 5,7 | 0,55 | 3,0 | 0,55 | 2,8 |
| 0,6 | 4,9 | 0,6 | 7,6 | 0,6 | 2,6 | 0,6 | 6,6 | 0,6 | 5,6 | 0,6 | 3,0 | 0,6 | 2,8 |
| 0,65 | 4,9 | 0,65 | 7,6 | 0,65 | 2,6 | 0,65 | 6,6 | 0,65 | 5,5 | 0,65 | 3,0 | 0,65 | 2,8 |
| 0,7 | 4,8 | 0,7 | 7,6 | 0,7 | 2,6 | 0,7 | 6,5 | 0,7 | 5,3 | 0,7 | 3,0 | 0,7 | 2,8 |
| 0,75 | 4,7 | 0,75 | 7,4 | 0,75 | 2,6 | 0,75 | 6,3 | 0,75 | 5,1 | 0,75 | 3,0 | 0,75 | 2,8 |
| 0,8 | 4,4 | 0,8 | 7,1 | 0,8 | 2,5 | 0,8 | 6,0 | 0,8 | 4,8 | 0,8 | 2,9 | 0,8 | 2,6 |
| 0,85 | 4,0 | 0,85 | 6,5 | 0,85 | 2,3 | 0,85 | 5,5 | 0,85 | 4,3 | 0,85 | 2,7 | 0,85 | 2,5 |
| 0,9 | 3,4 | 0,9 | 5,5 | 0,9 | 2,0 | 0,9 | 4,7 | 0,9 | 3,6 | 0,9 | 2,4 | 0,9 | 2,2 |
| 0,95 | 2,5 | 0,95 | 3,8 | 0,95 | 1,7 | 0,95 | 3,3 | 0,95 | 2,6 | 0,95 | 1,9 | 0,95 | 1,8 |
| 1 | 1,2 | 1 | 1,2 | 1 | 1,2 | 1 | 1,2 | 1 | 1,2 | 1 | 1,2 | 1 | 1,2 |

**HF - HFO-1234yf - HFC-245cb - HCFO-1233zdE -HFO-1234zeE - HFO-1234zeZ - HFO-1243zf**

| **Organics 0,15 F1234yf + 0,17 F245cb + 0,17 F1233zdE + 0,17 F1234zeE + 0,17 F1234zeZ + 0,17 F1243zf** | | **Organics 0,95 F1234yf + 0,01 F245cb + 0,01 F1233zdE + 0,01 F1234zeE + 0,01 F1234zeZ + 0,01 F1243zf** | | **Organics 0,01 F1234yf + 0,95 F245cb + 0,01 F1233zdE + 0,01 F1234zeE + 0,01 F1234zeZ + 0,01 F1243zf** | | **Organics 0,01 F1234yf + 0,01 F245cb + 0,95 F1233zdE + 0,01 F1234zeE + 0,01 F1234zeZ + 0,01 F1243zf** | | **Organics 0,01 F1234yf + 0,01 F245cb + 0,01 F1233zdE + 0,95 F1234zeE + 0,01 F1234zeZ + 0,01 F1243zf** | | **Organics 0,01 F1234yf + 0,01 F245cb + 0,01 F1233zdE + 0,01 F1234zeE + 0,01 F1234zeZ + 0,95 F1243zf** | | **Organics 0,01 F1234yf + 0,01 F245cb + 0,01 F1233zdE + 0,01 F1234zeE + 0,95 F1234zeZ + 0,01 F1243zf** | |
|---|---|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | bar | | bar | | bar | | bar | | bar | | bar | | bar |
| 0 | 4,3 | 0 | 6,6 | 0 | 4,6 | 0 | 1,5 | 0 | 4,8 | 0 | 5,8 | 0 | 2,0 |
| 0,05 | 5,3 | 0,05 | 7,6 | 0,05 | 5,8 | 0,05 | 2,6 | 0,05 | 5,8 | 0,05 | 6,7 | 0,05 | 3,1 |
| 0,1 | 5,3 | 0,1 | 7,6 | 0,1 | 5,8 | 0,1 | 2,6 | 0,1 | 5,8 | 0,1 | 6,7 | 0,1 | 3,1 |
| 0,15 | 5,3 | 0,15 | 7,6 | 0,15 | 5,8 | 0,15 | 2,6 | 0,15 | 5,8 | 0,15 | 6,7 | 0,15 | 3,1 |
| 0,2 | 5,3 | 0,2 | 7,6 | 0,2 | 5,8 | 0,2 | 2,6 | 0,2 | 5,8 | 0,2 | 6,7 | 0,2 | 3,1 |
| 0,25 | 5,3 | 0,25 | 7,6 | 0,25 | 5,8 | 0,25 | 2,6 | 0,25 | 5,8 | 0,25 | 6,7 | 0,25 | 3,1 |
| 0,3 | 5,3 | 0,3 | 7,6 | 0,3 | 5,8 | 0,3 | 2,6 | 0,3 | 5,8 | 0,3 | 6,7 | 0,3 | 3,1 |
| 0,35 | 5,3 | 0,35 | 7,6 | 0,35 | 5,8 | 0,35 | 2,6 | 0,35 | 5,8 | 0,35 | 6,7 | 0,35 | 3,1 |
| 0,4 | 5,3 | 0,4 | 7,6 | 0,4 | 5,8 | 0,4 | 2,6 | 0,4 | 5,8 | 0,4 | 6,7 | 0,4 | 3,1 |
| 0,45 | 5,3 | 0,45 | 7,6 | 0,45 | 5,8 | 0,45 | 2,6 | 0,45 | 5,8 | 0,45 | 6,7 | 0,45 | 3,1 |
| 0,5 | 5,3 | 0,5 | 7,6 | 0,5 | 5,8 | 0,5 | 2,6 | 0,5 | 5,8 | 0,5 | 6,7 | 0,5 | 3,1 |
| 0,55 | 5,3 | 0,55 | 7,6 | 0,55 | 5,8 | 0,55 | 2,6 | 0,55 | 5,7 | 0,55 | 6,7 | 0,55 | 3,1 |
| 0,6 | 5,3 | 0,6 | 7,6 | 0,6 | 5,8 | 0,6 | 2,6 | 0,6 | 5,6 | 0,6 | 6,7 | 0,6 | 3,1 |
| 0,65 | 5,3 | 0,65 | 7,6 | 0,65 | 5,8 | 0,65 | 2,6 | 0,65 | 5,5 | 0,65 | 6,6 | 0,65 | 3,1 |
| 0,7 | 5,3 | 0,7 | 7,6 | 0,7 | 5,8 | 0,7 | 2,6 | 0,7 | 5,4 | 0,7 | 6,5 | 0,7 | 3,1 |
| 0,75 | 5,2 | 0,75 | 7,4 | 0,75 | 5,8 | 0,75 | 2,6 | 0,75 | 5,1 | 0,75 | 6,3 | 0,75 | 3,1 |
| 0,8 | 4,9 | 0,8 | 7,1 | 0,8 | 5,8 | 0,8 | 2,5 | 0,8 | 4,8 | 0,8 | 6,1 | 0,8 | 2,9 |
| 0,85 | 4,5 | 0,85 | 6,5 | 0,85 | 5,8 | 0,85 | 2,3 | 0,85 | 4,3 | 0,85 | 5,6 | 0,85 | 2,7 |
| 0,9 | 3,9 | 0,9 | 5,5 | 0,9 | 5,0 | 0,9 | 2,1 | 0,9 | 3,6 | 0,9 | 4,7 | 0,9 | 2,4 |
| 0,95 | 2,8 | 0,95 | 3,8 | 0,95 | 3,5 | 0,95 | 1,7 | 0,95 | 2,6 | 0,95 | 3,4 | 0,95 | 1,9 |
| 1 | 1,2 | 1 | 1,2 | 1 | 1,2 | 1 | 1,2 | 1 | 1,2 | 1 | 1,2 | 1 | 1,2 |

**HF - HFO-1234zeE - HCFC-244bb- HFC-245fa - Trifluoropropyne- HFO-1225yeZ- HFO-1225zc**

| | **Organics 0,15 F1234zeE + 0,17 F244bb + 0,17 F245fa + 0,17TFP + 0,17 F1225yeZ + 0,17 F1225zc** | **Organics 0,95 F1234zeE + 0,01 F244bb + 0,01 F245fa + 0,01 TFP + 0,01 F1225yeZ + 0,01 F1225zc** | **Organics 0,01 F1234zeE + 0,95 F244bb + 0,01 F245fa + 0,01 TFP + 0,01 F1225yeZ + 0,01 F1225zc** | **Organics 0,01 F1234zeE + 0,01 F244bb + 0,95 F245fa + 0,01 TFP + 0,01 F1225yeZ + 0,01 F1225zc** | **Organics 0,01 F1234zeE + 0,01 F244bb + 0,01 F245fa + 0,95 TFP + 0,01 F1225yeZ + 0,01 F1225zc** | **Organics 0,01 F1234zeE + 0,01 F244bb + 0,01 F245fa + 0,01 TFP + 0,95 F1225yeZ + 0,01 F1225zc** | **Organics 0,01 F1234zeE + 0,01 F244bb + 0,01 F245fa + 0,01 TFP + 0,01 F1225yeZ + 0,95 F1225zc** |
|---|---|---|---|---|---|---|---|
| MASSFRAC | TOTAL | TOTAL | TOTAL | TOTAL | TOTAL | TOTAL | TOTAL |
| HF | PRESSURE | PRESSURE | PRESSURE | PRESSURE | PRESSURE | PRESSURE | PRESSURE |
| | bar | bar | bar | bar | bar | bar | bar |
| 0 | 5,3 | 4,9 | 1,0 | 1,8 | 11,3 | 5,2 | 5,3 |
| 0,05 | 6,3 | 5,8 | 2,1 | 2,9 | 12,1 | 6,2 | 6,3 |
| 0,1 | 6,3 | 5,9 | 2,1 | 2,9 | 12,0 | 6,2 | 6,3 |
| 0,15 | 6,3 | 5,9 | 2,1 | 2,9 | 11,9 | 6,2 | 6,3 |
| 0,2 | 6,3 | 5,9 | 2,1 | 2,9 | 11,8 | 6,2 | 6,3 |
| 0,25 | 6,3 | 5,9 | 2,1 | 2,9 | 11,7 | 6,2 | 6,3 |
| 0,3 | 6,3 | 5,9 | 2,1 | 2,9 | 11,6 | 6,2 | 6,3 |
| 0,35 | 6,3 | 5,9 | 2,1 | 2,9 | 11,5 | 6,2 | 6,3 |
| 0,4 | 6,2 | 5,9 | 2,1 | 2,9 | 11,5 | 6,2 | 6,3 |
| 0,45 | 6,2 | 5,8 | 2,1 | 2,9 | 11,5 | 6,2 | 6,3 |
| 0,5 | 6,2 | 5,8 | 2,1 | 2,9 | 11,5 | 6,2 | 6,2 |
| 0,55 | 6,2 | 5,8 | 2,1 | 2,9 | 11,5 | 6,2 | 6,2 |
| 0,6 | 6,1 | 5,7 | 2,1 | 2,8 | 11,6 | 6,2 | 6,1 |
| 0,65 | 6,1 | 5,6 | 2,1 | 2,8 | 11,6 | 6,2 | 6,0 |
| 0,7 | 6,0 | 5,4 | 2,0 | 2,8 | 11,7 | 6,1 | 5,9 |
| 0,75 | 5,8 | 5,2 | 2,0 | 2,8 | 11,6 | 5,9 | 5,6 |
| 0,8 | 5,5 | 4,8 | 2,0 | 2,7 | 11,3 | 5,6 | 5,2 |
| 0,85 | 5,0 | 4,3 | 2,0 | 2,5 | 10,5 | 5,1 | 4,7 |
| 0,9 | 4,2 | 3,6 | 1,9 | 2,2 | 8,9 | 4,2 | 3,8 |
| 0,95 | 3,0 | 2,6 | 1,6 | 1,8 | 6,0 | 3,0 | 2,7 |
| 1 | 1,2 | 1,2 | 1,2 | 1,2 | 1,2 | 1,2 | 1,2 |

**HF - HFO-1234zeZ - HCFC-244bb- HFC-245fa - Trifluoropropyne- HFO-1225yeZ- HFO-1225zc**

| | **Organics 0,15 F1234zeZ + 0,17 F244bb + 0,17 F245fa + 0,17TFP + 0,17 F1225yeZ + 0,17 F1225zc** | **Organics 0,95 F1234zeZ + 0,01 F244bb + 0,01 F245fa + 0,01 TFP + 0,01 F1225yeZ + 0,01 F1225zc** | **Organics 0,01 F1234zeZ + 0,95 F244bb + 0,01 F245fa + 0,01 TFP + 0,01 F1225yeZ + 0,01 F1225zc** | **Organics 0,01 F1234zeZ + 0,01 F244bb + 0,95 F245fa + 0,01 TFP + 0,01 F1225yeZ + 0,01 F1225zc** | **Organics 0,01 F1234zeZ + 0,01 F244bb + 0,01 F245fa + 0,95 TFP + 0,01 F1225yeZ + 0,01 F1225zc** | **Organics 0,01 F1234zeZ + 0,01 F244bb + 0,01 F245fa + 0,01 TFP + 0,95 F1225yeZ + 0,01 F1225zc** | **Organics 0,01 F1234zeZ + 0,01 F244bb + 0,01 F245fa + 0,01 TFP + 0,01 F1225yeZ + 0,95 F1225zc** |
|---|---|---|---|---|---|---|---|
| MASSFRAC | TOTAL | TOTAL | TOTAL | TOTAL | TOTAL | TOTAL | TOTAL |
| HF | PRESSURE | PRESSURE | PRESSURE | PRESSURE | PRESSURE | PRESSURE | PRESSURE |
| | bar | bar | bar | bar | bar | bar | bar |
| 0 | 4,8 | 2,0 | 0,9 | 1,7 | 11,3 | 5,1 | 5,3 |
| 0,05 | 5,9 | 3,1 | 2,1 | 2,8 | 12,0 | 6,2 | 6,2 |
| 0,1 | 5,9 | 3,1 | 2,1 | 2,8 | 12,0 | 6,2 | 6,3 |
| 0,15 | 5,9 | 3,1 | 2,1 | 2,8 | 11,9 | 6,2 | 6,3 |
| 0,2 | 5,8 | 3,1 | 2,1 | 2,8 | 11,8 | 6,2 | 6,3 |
| 0,25 | 5,8 | 3,1 | 2,1 | 2,8 | 11,7 | 6,2 | 6,2 |
| 0,3 | 5,8 | 3,1 | 2,1 | 2,8 | 11,6 | 6,2 | 6,2 |
| 0,35 | 5,8 | 3,1 | 2,1 | 2,8 | 11,5 | 6,2 | 6,2 |
| 0,4 | 5,8 | 3,1 | 2,1 | 2,8 | 11,4 | 6,2 | 6,2 |
| 0,45 | 5,8 | 3,1 | 2,1 | 2,8 | 11,4 | 6,2 | 6,2 |
| 0,5 | 5,8 | 3,1 | 2,0 | 2,8 | 11,5 | 6,2 | 6,2 |
| 0,55 | 5,7 | 3,1 | 2,0 | 2,8 | 11,5 | 6,2 | 6,2 |
| 0,6 | 5,7 | 3,1 | 2,0 | 2,8 | 11,5 | 6,2 | 6,1 |
| 0,65 | 5,6 | 3,1 | 2,0 | 2,8 | 11,5 | 6,2 | 6,0 |
| 0,7 | 5,6 | 3,1 | 2,0 | 2,8 | 11,5 | 6,1 | 5,8 |
| 0,75 | 5,5 | 3,1 | 2,0 | 2,8 | 11,5 | 5,9 | 5,6 |
| 0,8 | 5,2 | 2,9 | 2,0 | 2,7 | 11,3 | 5,6 | 5,2 |
| 0,85 | 4,7 | 2,7 | 2,0 | 2,5 | 10,5 | 5,0 | 4,6 |
| 0,9 | 4,0 | 2,4 | 1,9 | 2,2 | 8,9 | 4,2 | 3,8 |
| 0,95 | 2,8 | 1,9 | 1,6 | 1,8 | 6,0 | 3,0 | 2,7 |
| 1 | 1,2 | 1,2 | 1,2 | 1,2 | 1,2 | 1,2 | 1,2 |

### Exemple 14 : Plage de température et de pression de système à 7 composés

| | Enveloppe de points d'ébullition | |
|---|---|---|
| Système à 7 composés | Temp. °C | Pression bar abs |
| HF-HCFO-1233xf-HFO-1234yf-HFC-245cb-HCFO-1233zdE-HFO-1234zeE-HFO-1234zeZ | 0 à 40 | ∼0.9∼11.5 |
| HF-HCFO-1233xf-HFO-1234yf-HFC-245cb-HCFO-1233zdE-HFO-1234zeE-HFO-1243zf | 0 à 40 | ∼1.0 à ∼11.6 |
| HF-HCFO-1233xf-HFO-1234yf-HFC-245cb-HCFO-1233zdE-HFO-1234zeZ-HFO-1243zf | 0 à 40 | ∼0.9 à ∼11.5 |
| HF-HCFO-1233xf-HFO-1234yf-HFC-245cb-HFO-1234zeE-HFO-1234zeZ-HFO-1243zf | 0 à 40 | ∼1.1 à ∼11.6 |
| HF-HCFO-1233xf-HFO-1234yf-HCFO-1233zdE-HFO-1234zeE-HFO-1234zeZ-HFO-1243zf | 0 à 40 | ∼0.9 à ∼11.5 |
| HF-HCFO-1233xf-HFC-245cb-HCFO-1233zdE-HFO-1234zeE-HFO-1234zeZ-HFO-1243zf | 0 à 40 | ∼0.9 à ∼10.3 |
| HF-HFO-1234yf-HFC-245cb-HCFO-1233zdE-HFO-1234zeE-HFO-1234zeZ-HFO-1243zf | 0 à 40 | ∼1.0 à ∼11.6 |
| HF - HFO-1234zeE - HCFC-244bb- HFC-245fa - Trifluoropropyne-HFO-1225yeZ- HFO-1225zc | 0 à 40 | ∼0.7 à ∼17.5 |
| HF - HFO-1234zeZ - HCFC-244bb- HFC-245fa - Trifluoropropyne-HFO-1225yeZ- HFO-1225zc | 0 à 40 | ∼0.7 à ∼17.5 |

### Exemple 15 : Plage de décantation de système à 7 composés

| | Plages de décantation Pourcentage massique d'HF | | |
|---|---|---|---|
| Système à 7 composés | Temp 0°C | Temp 25°C | Temp 40°C |
| HF-HCFO-1233xf-HFO-1234yf-HFC-245cb-HCFO-1233zdE-HFO-1234zeE-HFO-1234zeZ | 5-75 | 5-70 | 10-60 |
| HF-HCFO-1233xf-HFO-1234yf-HFC-245cb-HCFO-1233zdE-HFO-1234zeE-HFO-1243zf | 5-75 | 10-65 | 20 |
| HF-HCFO-1233xf-HFO-1234yf-HFC-245cb-HCFO-1233zdE-HFO-1234zeZ-HFO-1243zf | 5-70 | 5-70 | 10-60 |
| HF-HCFO-1233xf-HFO-1234yf-HFC-245cb-HFO-1234zeE-HFO-1234zeZ-HFO-1243zf | 5-75 | 10-65 | * |
| HF-HCFO-1233xf-HFO-1234yf-HCFO-1233zdE-HFO-1234zeE-HFO-1234zeZ-HFO-1243zf | 5-75 | 10-65 | 15-45 |
| HF-HCFO-1233xf-HFC-245cb-HCFO-1233zdE-HFO-1234zeE-HFO-1234zeZ-HFO-1243zf | 5-75 | 5-70 | 10-55 |
| HF-HFO-1234yf-HFC-245cb-HCFO-1233zdE-HFO-1234zeE-HFO-1234zeZ-HFO-1243zf | 5-75 | 10-65 | * |
| HF - HFO-1234zeE - HCFC-244bb- HFC-245fa - Trifluoropropyne-HFO-1225yeZ- HFO-1225zc | 5-75 | 10-70 | * |
| HF - HFO-1234zeZ - HCFC-244bb- HFC-245fa - Trifluoropropyne-HFO-1225yeZ- HFO-1225zc | 5-75 | 5-70 | 10-60 |

### Exemple 16 : Systèmes à 8 composés

**HF - HCFO-1233xf - HFO-1234yf - HFC-245cb - HCFO-1233zdE - HFO-1234zeE - HFO-1234zeZ - HFO-1243zf**

| Organics 0,01 F1233xf + **0,94 F1234yf** + 0,01 F245cb +0,01 F1233zdE + 0,01 F1234zeE + 0,01 F1234zeZ + 0,01 F1243zf | | Organics 0,01 F1233xf + 0,01 F1234yf + **0,94 F245cb** +0,01 F1233zdE + 0,01 F1234zeE + 0,01 F1234zeZ + 0,01 F1243zf | | Organics 0,01 F1233xf + 0,01 F1234yf + 0,01 F245cb **+0,94 F1233zdE** + 0,01 F1234zeE + 0,01 F1234zeZ + 0,01 F1243zf | | Organics 0,01 F1233xf + 0,01 F1234yf + 0,01 F245cb +0,01 F1233zdE + **0,94 F1234zeE** + 0,01 F1234zeZ + 0,01 F1243zf | | Organics 0,01 F1233xf + 0,01 F1234yf + 0,01F 245cb +0,01 F1233zdE + 0,01 F1234zeE + **0,94 F1234zeZ** + 0,01 F1243zf | | Organics 0,01 F1233xf + 0,01 F1234yf + 0,01 F245cb +0,01 F1233zdE + 0,01 F1234zeE + 0,01 F1234zeZ **+ 0,94 F1243zf** | | Organics 0,16 F1233xf +0,14 F1234yf + 0,14 F245cb +0,14 F1233zdE + 0,14 F1234zeE + 0,14 F1234zeZ + 0,14 F1243zf | |
|---|---|---|---|---|---|---|---|---|---|---|---|---|---|
| MASSFRAC | TOTAL | MASSFRAC | TOTAL | MASSFRAC | TOTAL | MASSFRAC | TOTAL | MASSFRAC | TOTAL | MASSFRAC | TOTAL | MASSFRAC | TOTAL |
| HF | PRESSURE | HF | PRESSURE | HF | PRESSURE | HF | PRESSURE | HF | PRESSURE | HF | PRESSURE | HF | PRESSURE |
| | bar | | bar | | bar | | bar | | bar | | bar | | bar |
| 0 | 6,6 | 0 | 4,6 | 0 | 1,5 | 0 | 4,8 | 0 | 2,0 | 0 | 5,7 | 0 | 3,9 |
| 0,05 | 7,6 | 0,05 | 5,8 | 0,05 | 2,6 | 0,05 | 5,8 | 0,05 | 3,1 | 0,05 | 6,7 | 0,05 | 5,0 |
| 0,1 | 7,6 | 0,1 | 5,8 | 0,1 | 2,6 | 0,1 | 5,8 | 0,1 | 3,1 | 0,1 | 6,7 | 0,1 | 5,0 |
| 0,15 | 7,6 | 0,15 | 5,8 | 0,15 | 2,6 | 0,15 | 5,8 | 0,15 | 3,1 | 0,15 | 6,7 | 0,15 | 5,0 |
| 0,2 | 7,6 | 0,2 | 5,8 | 0,2 | 2,6 | 0,2 | 5,8 | 0,2 | 3,1 | 0,2 | 6,7 | 0,2 | 5,0 |
| 0,25 | 7,6 | 0,25 | 5,8 | 0,25 | 2,6 | 0,25 | 5,8 | 0,25 | 3,1 | 0,25 | 6,7 | 0,25 | 5,0 |
| 0,3 | 7,6 | 0,3 | 5,8 | 0,3 | 2,6 | 0,3 | 5,8 | 0,3 | 3,1 | 0,3 | 6,7 | 0,3 | 5,0 |
| 0,35 | 7,6 | 0,35 | 5,8 | 0,35 | 2,6 | 0,35 | 5,8 | 0,35 | 3,1 | 0,35 | 6,7 | 0,35 | 5,0 |
| 0,4 | 7,6 | 0,4 | 5,8 | 0,4 | 2,6 | 0,4 | 5,8 | 0,4 | 3,1 | 0,4 | 6,7 | 0,4 | 5,0 |
| 0,45 | 7,6 | 0,45 | 5,8 | 0,45 | 2,6 | 0,45 | 5,8 | 0,45 | 3,1 | 0,45 | 6,7 | 0,45 | 5,0 |
| 0,5 | 7,6 | 0,5 | 5,8 | 0,5 | 2,6 | 0,5 | 5,7 | 0,5 | 3,1 | 0,5 | 6,7 | 0,5 | 5,0 |
| 0,55 | 7,6 | 0,55 | 5,8 | 0,55 | 2,6 | 0,55 | 5,7 | 0,55 | 3,1 | 0,55 | 6,7 | 0,55 | 5,0 |
| 0,6 | 7,6 | 0,6 | 5,8 | 0,6 | 2,6 | 0,6 | 5,6 | 0,6 | 3,1 | 0,6 | 6,6 | 0,6 | 5,0 |
| 0,65 | 7,6 | 0,65 | 5,8 | 0,65 | 2,6 | 0,65 | 5,5 | 0,65 | 3,1 | 0,65 | 6,6 | 0,65 | 5,0 |
| 0,7 | 7,5 | 0,7 | 5,8 | 0,7 | 2,6 | 0,7 | 5,4 | 0,7 | 3,1 | 0,7 | 6,5 | 0,7 | 5,0 |
| 0,75 | 7,4 | 0,75 | 5,8 | 0,75 | 2,6 | 0,75 | 5,1 | 0,75 | 3,1 | 0,75 | 6,3 | 0,75 | 4,9 |
| 0,8 | 7,1 | 0,8 | 5,8 | 0,8 | 2,5 | 0,8 | 4,8 | 0,8 | 2,9 | 0,8 | 6,0 | 0,8 | 4,6 |
| 0,85 | 6,5 | 0,85 | 5,8 | 0,85 | 2,3 | 0,85 | 4,3 | 0,85 | 2,7 | 0,85 | 5,5 | 0,85 | 4,2 |
| 0,9 | 5,5 | 0,9 | 5,0 | 0,9 | 2,1 | 0,9 | 3,6 | 0,9 | 2,4 | 0,9 | 4,7 | 0,9 | 3,6 |
| 0,95 | 3,8 | 0,95 | 3,5 | 0,95 | 1,7 | 0,95 | 2,6 | 0,95 | 1,9 | 0,95 | 3,3 | 0,95 | 2,6 |
| 1 | 1,2 | 1 | 1,2 | 1 | 1,2 | 1 | 1,2 | 1 | 1,2 | 1 | 1,2 | 1 | 1,2 |

**HF-HFO-1234yf-HFC-245cb-HCFO-1233xf-HFO-1234zeE-HFO-1234zeZ-HCFO-1233zdE-HCFC-244bb**

| | **Organics 0,94 F1233xf + 0,01 F1234yf + 0,01 F245cb +0,01 F1233zdE + 0,01 F1234zeE + 0,01 F1234zeZ +0,01 F244bb** | **Organics 0,01 F1233xf + 0,94 F1234yf + 0,01 F245cb +0,01 F1233zdE + 0,01 F1234zeE + 0,01 F1234zeZ +0,01 F244bb** | **Organics 0,01 F1233xf + 0,01 F1234yf + 0,94 F245cb +0,01 F1233zdE + 0,01 F1234zeE + 0,01 F1234zeZ +0,01 F244bb** | **Organics 0,01 F1233xf + 0,01 F1234yf + 0,01 F245cb +0,94 F1233zdE + 0,01 F1234zeE + 0,01 F1234zeZ +0,01 F244bb** | **Organics 0,01 F1233xf + 0,01 F1234yf + 0,01 F245cb +0,01 F1233zdE + 0,94 F1234zeE + 0,01 F1234zeZ +0,01 F244bb** | **Organics 0,01 F1233xf + 0,01 F1234yf + 0,01 F245cb +0,01 F1233zdE + 0,01 F1234zeE + 0,94 F1234zeZ +0,01 F244bb** | **Organics 0,01 F1233xf + 0,01 F1234yf + 0,01 F245cb +0,01 F1233zdE + 0,01 F1234zeE + 0,01 F1234zeZ +0,94 F244bb** | **Organics 0,16 F1233xf + 0,14 F1234yf + 0,14 F245cb +0,14 F1233zdE + 0,14 F1234zeE + 0,14 F1234zeZ +0,14 F244bb** |
|---|---|---|---|---|---|---|---|---|
| MASSFRAC | TOTAL | TOTAL | TOTAL | TOTAL | TOTAL | TOTAL | TOTAL | TOTAL |
| HF | PRESSURE | PRESSURE | PRESSURE | PRESSURE | PRESSURE | PRESSURE | PRESSURE | PRESSURE |
| | bar | bar | bar | bar | bar | bar | bar | bar |
| 0 | 1,7 | 6,6 | 4,5 | 1,5 | 4,8 | 1,9 | 0,8 | 3,2 |
| 0,05 | 2,8 | 7,5 | 5,7 | 2,6 | 5,7 | 3,0 | 2,0 | 4,3 |
| 0,1 | 2,8 | 7,5 | 5,7 | 2,6 | 5,7 | 3,0 | 2,0 | 4,3 |
| 0,15 | 2,8 | 7,5 | 5,7 | 2,6 | 5,7 | 3,0 | 2,0 | 4,3 |
| 0,2 | 2,8 | 7,5 | 5,7 | 2,6 | 5,7 | 3,0 | 2,0 | 4,3 |
| 0,25 | 2,8 | 7,5 | 5,7 | 2,6 | 5,7 | 3,0 | 2,0 | 4,3 |
| 0,3 | 2,8 | 7,5 | 5,7 | 2,6 | 5,7 | 3,0 | 2,0 | 4,3 |
| 0,35 | 2,8 | 7,5 | 5,7 | 2,6 | 5,7 | 3,0 | 2,0 | 4,3 |
| 0,4 | 2,8 | 7,5 | 5,7 | 2,6 | 5,7 | 3,0 | 2,0 | 4,3 |
| 0,45 | 2,8 | 7,5 | 5,7 | 2,6 | 5,7 | 3,0 | 2,0 | 4,3 |
| 0,5 | 2,8 | 7,5 | 5,7 | 2,6 | 5,7 | 3,0 | 2,0 | 4,3 |
| 0,55 | 2,8 | 7,5 | 5,7 | 2,6 | 5,6 | 3,0 | 2,0 | 4,2 |
| 0,6 | 2,8 | 7,5 | 5,7 | 2,6 | 5,6 | 3,0 | 2,0 | 4,2 |
| 0,65 | 2,8 | 7,5 | 5,7 | 2,6 | 5,5 | 3,0 | 2,0 | 4,2 |
| 0,7 | 2,8 | 7,5 | 5,7 | 2,6 | 5,3 | 3,0 | 2,0 | 4,2 |
| 0,75 | 2,8 | 7,4 | 5,7 | 2,6 | 5,1 | 3,0 | 1,9 | 4,2 |
| 0,8 | 2,6 | 7,0 | 5,7 | 2,4 | 4,7 | 2,9 | 1,9 | 4,0 |
| 0,85 | 2,4 | 6,5 | 5,7 | 2,3 | 4,3 | 2,7 | 1,9 | 3,7 |
| 0,9 | 2,2 | 5,4 | 4,9 | 2,0 | 3,6 | 2,4 | 1,8 | 3,2 |
| 0,95 | 1,8 | 3,8 | 3,5 | 1,7 | 2,6 | 1,9 | 1,6 | 2,4 |
| 1 | 1,2 | 1,2 | 1,2 | 1,2 | 1,2 | 1,2 | 1,2 | 1,2 |

**HF-HFO-1234yf-HFC-245cb-HCFO-1233xf-HFO-1234zeE-HFO-1234zeZ-HCFO-1233zdE-Trifluoropropyne**

| | **Organics 0,16 F1234yf + 0,14 F245cb + 0,14 F1233xf + 0,14 F1234zeE + 0,14 F1234zeZ + 0,14 F1233zdE + 0,14 TPF** | **Organics 0,94 F1234yf + 0,01 F245cb + 0,01 F1233xf + 0,01 F1234zeE + 0,01 F1234zeZ + 0,01 F1233zdE + 0,01 TPF** | **Organics 0,01 F1234yf + 0,94 F245cb + 0,01 F1233xf + 0,01 F1234zeE + 0,01 F1234zeZ + 0,01 F1233zdE + 0,01 TPF** | **Organics 0,01 F1234yf + 0,01 F245cb + 0,94 F1233xf + 0,01 F1234zeE + 0,01 F1234zeZ + 0,01 F1233zdE + 0,01 TPF** | **Organics 0,01 F1234yf + 0,01 F245cb + 0,01 F1233xf + 0,94 F1234zeE + 0,01 F1234zeZ + 0,01 F1233zdE + 0,01 TPF** | **Organics 0,01 F1234yf + 0,01 F245cb + 0,01 F1233xf + 0,01 F1234zeE + 0,94 F1234zeZ + 0,01 F1233zdE + 0,01 TPF** | **Organics 0,01 F1234yf + 0,01 F245cb + 0,01 F1233xf + 0,01 F1234zeE + 0,01 F1234zeZ + 0,94 F1233zdE + 0,01 TPF** | **Organics 0,01 F1234yf + 0,01 F245cb + 0,01 F1233xf + 0,01 F1234zeE + 0,01 F1234zeZ + 0,01 F1233zdE + 0,94 TPF** |
|---|---|---|---|---|---|---|---|---|
| MASSFRAC | TOTAL | TOTAL | TOTAL | TOTAL | TOTAL | TOTAL | TOTAL | TOTAL |
| HF | PRESSURE | PRESSURE | PRESSURE | PRESSURE | PRESSURE | PRESSURE | PRESSURE | PRESSURE |
| | bar | bar | bar | bar | bar | bar | bar | bar |
| 0 | 5,0 | 6,7 | 4,7 | 1,8 | 4,9 | 2,0 | 1,6 | 11,2 |
| 0,05 | 6,0 | 7,6 | 5,8 | 2,9 | 5,8 | 3,1 | 2,7 | 12,0 |
| 0,1 | 6,0 | 7,6 | 5,8 | 2,9 | 5,9 | 3,1 | 2,7 | 11,9 |
| 0,15 | 6,0 | 7,6 | 5,8 | 2,9 | 5,9 | 3,1 | 2,7 | 11,8 |
| 0,2 | 6,0 | 7,6 | 5,8 | 2,9 | 5,9 | 3,1 | 2,7 | 11,7 |
| 0,25 | 6,0 | 7,6 | 5,8 | 2,9 | 5,8 | 3,1 | 2,7 | 11,6 |
| 0,3 | 6,0 | 7,6 | 5,8 | 2,9 | 5,8 | 3,1 | 2,7 | 11,5 |
| 0,35 | 6,0 | 7,6 | 5,8 | 2,9 | 5,8 | 3,1 | 2,7 | 11,5 |
| 0,4 | 6,0 | 7,6 | 5,8 | 2,9 | 5,8 | 3,1 | 2,7 | 11,4 |
| 0,45 | 6,0 | 7,6 | 5,8 | 2,9 | 5,8 | 3,1 | 2,7 | 11,4 |
| 0,5 | 6,0 | 7,6 | 5,8 | 2,9 | 5,8 | 3,1 | 2,7 | 11,4 |
| 0,55 | 6,0 | 7,6 | 5,8 | 2,9 | 5,8 | 3,1 | 2,7 | 11,5 |
| 0,6 | 6,0 | 7,6 | 5,8 | 2,9 | 5,7 | 3,1 | 2,7 | 11,5 |
| 0,65 | 6,0 | 7,6 | 5,8 | 2,9 | 5,6 | 3,1 | 2,7 | 11,5 |
| 0,7 | 6,0 | 7,6 | 5,8 | 2,9 | 5,4 | 3,1 | 2,7 | 11,5 |
| 0,75 | 5,9 | 7,5 | 5,8 | 2,9 | 5,2 | 3,1 | 2,7 | 11,5 |
| 0,8 | 5,6 | 7,1 | 5,8 | 2,8 | 4,9 | 3,0 | 2,6 | 11,2 |
| 0,85 | 5,1 | 6,6 | 5,8 | 2,6 | 4,4 | 2,8 | 2,4 | 10,4 |
| 0,9 | 4,3 | 5,5 | 5,0 | 2,2 | 3,6 | 2,4 | 2,1 | 8,8 |
| 0,95 | 3,1 | 3,8 | 3,5 | 1,8 | 2,6 | 1,9 | 1,7 | 5,9 |
| 1 | 1,2 | 1,2 | 1,2 | 1,2 | 1,2 | 1,2 | 1,2 | 1,2 |

**HF-HFO-1234yf-HFC-245cb-HCFO-1233xf-HFO-1234zeE-HFO-1234zeZ-HCFO-1233zdE-HFC-245fa**

| | **Organics 0,16 F1234yf + 0,14 F245cb + 0,14 F1233xf + 0,14 F1234zeE + 0,14 F1234zeZ + 0,14 F1233zdE + 0,14 F245fa** | **Organics 0,94 F1234yf + 0,01 F245cb + 0,01 F1233xf + 0,01 F1234zeE + 0,01 F1234zeZ + 0,01 F1233zdE + 0,01 F245fa** | **Organics 0,01 F1234yf + 0,94 F245cb + 0,01 F1233xf + 0,01 F1234zeE + 0,01 F1234zeZ + 0,01 F1233zdE + 0,01 F245fa** | **Organics 0,01 F1234yf + 0,01 F245cb + 0,94 F1233xf + 0,01 F1234zeE + 0,01 F1234zeZ + 0,01 F1233zdE + 0,01 F245fa** | **Organics 0,01 F1234yf + 0,01 F245cb + 0,01 F1233xf + 0,94 F1234zeE + 0,01 F1234zeZ + 0,01 F1233zdE + 0,01 F245fa** | **Organics 0,01 F1234yf + 0,01 F245cb + 0,01 F1233xf + 0,01 F1234zeE + 0,94 F1234zeZ + 0,01 F1233zdE + 0,01 F245fa** | **Organics 0,01 F1234yf + 0,01 F245cb + 0,01 F1233xf + 0,01 F1234zeE + 0,01 F1234zeZ + 0,94 F1233zdE + 0,01 F245fa** | **Organics 0,01 F1234yf + 0,01 F245cb + 0,01 F1233xf + 0,01 F1234zeE + 0,01 F1234zeZ + 0,01 F1233zdE + 0,94 F245fa** |
|---|---|---|---|---|---|---|---|---|
| MASSFRAC | TOTAL | TOTAL | TOTAL | TOTAL | TOTAL | TOTAL | TOTAL | TOTAL |
| HF | PRESSURE | PRESSURE | PRESSURE | PRESSURE | PRESSURE | PRESSURE | PRESSURE | PRESSURE |
| | bar | bar | bar | bar | bar | bar | bar | bar |
| 0 | 3,4 | 6,6 | 4,5 | 1,7 | 4,8 | 1,9 | 1,5 | 1,6 |
| 0,05 | 4,5 | 7,5 | 5,7 | 2,8 | 5,7 | 3,0 | 2,6 | 2,8 |
| 0,1 | 4,5 | 7,5 | 5,7 | 2,8 | 5,7 | 3,0 | 2,6 | 2,8 |
| 0,15 | 4,5 | 7,5 | 5,7 | 2,8 | 5,7 | 3,0 | 2,6 | 2,8 |
| 0,2 | 4,5 | 7,5 | 5,7 | 2,8 | 5,7 | 3,0 | 2,6 | 2,8 |
| 0,25 | 4,5 | 7,5 | 5,7 | 2,8 | 5,7 | 3,0 | 2,6 | 2,8 |
| 0,3 | 4,5 | 7,5 | 5,7 | 2,8 | 5,7 | 3,0 | 2,6 | 2,8 |
| 0,35 | 4,5 | 7,5 | 5,7 | 2,8 | 5,7 | 3,0 | 2,6 | 2,8 |
| 0,4 | 4,5 | 7,5 | 5,7 | 2,8 | 5,7 | 3,0 | 2,6 | 2,8 |
| 0,45 | 4,5 | 7,5 | 5,7 | 2,8 | 5,7 | 3,0 | 2,6 | 2,8 |
| 0,5 | 4,5 | 7,5 | 5,7 | 2,8 | 5,7 | 3,0 | 2,6 | 2,8 |
| 0,55 | 4,5 | 7,5 | 5,7 | 2,8 | 5,6 | 3,0 | 2,6 | 2,8 |
| 0,6 | 4,5 | 7,5 | 5,7 | 2,8 | 5,6 | 3,0 | 2,6 | 2,8 |
| 0,65 | 4,5 | 7,5 | 5,7 | 2,8 | 5,5 | 3,0 | 2,6 | 2,8 |
| 0,7 | 4,5 | 7,5 | 5,7 | 2,8 | 5,3 | 3,0 | 2,6 | 2,8 |
| 0,75 | 4,4 | 7,4 | 5,7 | 2,8 | 5,1 | 3,0 | 2,6 | 2,8 |
| 0,8 | 4,2 | 7,1 | 5,8 | 2,6 | 4,7 | 2,9 | 2,5 | 2,7 |
| 0,85 | 3,9 | 6,5 | 5,7 | 2,5 | 4,3 | 2,7 | 2,3 | 2,5 |
| 0,9 | 3,3 | 5,4 | 4,9 | 2,2 | 3,6 | 2,4 | 2,0 | 2,2 |
| 0,95 | 2,4 | 3,8 | 3,5 | 1,8 | 2,6 | 1,9 | 1,7 | 1,8 |
| 1 | 1,2 | 1,2 | 1,2 | 1,2 | 1,2 | 1,2 | 1,2 | 1,2 |

**HF-HFO-1234yf-HFC-245cb-HCFO-1233xf-HFO-1234zeE-HFO-1234zeZ-HCFO-1233zdE-HFO-1225yeZ**

| | **Organics 0,16 F1234yf + 0,14 F245cb + 0,14 F1233xf + 0,14 F1234zeE + 0,14 F1234zeZ + 0,14 F1233zdE + 0,14 F1225yeZ** | **Organics 0,94 F1234yf + 0,01 F245cb + 0,01 F1233xf + 0,01 F1234zeE + 0,01 F1234zeZ + 0,01 F1233zdE + 0,01 F1225yeZ** | **Organics 0,01 F1234yf + 0,94 F245cb + 0,01 F1233xf + 0,01 F1234zeE + 0,01 F1234zeZ + 0,01 F1233zdE + 0,01 F1225yeZ** | **Organics 0,01 F1234yf + 0,01 F245cb + 0,94 F1233xf + 0,01 F1234zeE + 0,01 F1234zeZ + 0,01 F1233zdE + 0,01 F1225yeZ** | **Organics 0,01 F1234yf + 0,01 F245cb + 0,01 F1233xf + 0,94 F1234zeE + 0,01 F1234zeZ + 0,01 F1233zdE + 0,01 F1225yeZ** | **Organics 0,01 F1234yf + 0,01 F245cb + 0,01 F1233xf + 0,01 F1234zeE + 0,94 F1234zeZ + 0,01 F1233zdE + 0,01 F1225yeZ** | **Organics 0,01 F1234yf + 0,01 F245cb + 0,01 F1233xf + 0,01 F1234zeE + 0,01 F1234zeZ + 0,94 F1233zdE + 0,01 F1225yeZ** | **Organics 0,01 F1234yf + 0,01 F245cb + 0,01 F1233xf + 0,01 F1234zeE + 0,01 F1234zeZ + 0,01 F1233zdE + 0,94 F1225yeZ** |
|---|---|---|---|---|---|---|---|---|
| MASSFRAC | TOTAL | TOTAL | TOTAL | TOTAL | TOTAL | TOTAL | TOTAL | TOTAL |
| HF | PRESSURE | PRESSURE | PRESSURE | PRESSURE | PRESSURE | PRESSURE | PRESSURE | PRESSURE |
| | bar | bar | bar | bar | bar | bar | bar | bar |
| 0 | 3,8 | 6,6 | 4,6 | 1,7 | 4,8 | 1,9 | 1,5 | 5,1 |
| 0,05 | 4,9 | 7,6 | 5,8 | 2,8 | 5,8 | 3,0 | 2,6 | 6,1 |
| 0,1 | 4,9 | 7,6 | 5,8 | 2,8 | 5,8 | 3,0 | 2,6 | 6,1 |
| 0,15 | 4,9 | 7,6 | 5,8 | 2,8 | 5,8 | 3,0 | 2,6 | 6,1 |
| 0,2 | 4,9 | 7,5 | 5,8 | 2,8 | 5,8 | 3,0 | 2,6 | 6,1 |
| 0,25 | 4,9 | 7,6 | 5,8 | 2,8 | 5,8 | 3,0 | 2,6 | 6,1 |
| 0,3 | 4,9 | 7,6 | 5,8 | 2,8 | 5,8 | 3,0 | 2,6 | 6,1 |
| 0,35 | 4,9 | 7,6 | 5,8 | 2,8 | 5,8 | 3,0 | 2,6 | 6,1 |
| 0,4 | 4,9 | 7,6 | 5,8 | 2,8 | 5,8 | 3,0 | 2,6 | 6,1 |
| 0,45 | 4,9 | 7,6 | 5,8 | 2,8 | 5,8 | 3,0 | 2,6 | 6,1 |
| 0,5 | 4,9 | 7,6 | 5,8 | 2,8 | 5,7 | 3,0 | 2,6 | 6,1 |
| 0,55 | 4,9 | 7,6 | 5,8 | 2,8 | 5,7 | 3,0 | 2,6 | 6,1 |
| 0,6 | 4,9 | 7,6 | 5,8 | 2,8 | 5,6 | 3,0 | 2,6 | 6,1 |
| 0,65 | 4,9 | 7,6 | 5,8 | 2,8 | 5,5 | 3,0 | 2,6 | 6,1 |
| 0,7 | 5,0 | 7,5 | 5,8 | 2,8 | 5,3 | 3,0 | 2,6 | 6,0 |
| 0,75 | 4,8 | 7,4 | 5,8 | 2,8 | 5,1 | 3,0 | 2,6 | 5,8 |
| 0,8 | 4,6 | 7,1 | 5,8 | 2,7 | 4,8 | 2,9 | 2,5 | 5,5 |
| 0,85 | 4,2 | 6,5 | 5,8 | 2,5 | 4,3 | 2,7 | 2,3 | 5,0 |
| 0,9 | 3,6 | 5,5 | 4,9 | 2,2 | 3,6 | 2,4 | 2,1 | 4,2 |
| 0,95 | 2,6 | 3,8 | 3,5 | 1,8 | 2,6 | 1,9 | 1,7 | 2,9 |
| 1 | 1,2 | 1,2 | 1,2 | 1,2 | 1,2 | 1,2 | 1,2 | 1,2 |

**HF-HFO-1234yf-HFC-245cb-HCFO-1233xf-HFO-1234zeE-HFO-1234zeZ-HCFC-1233zdE-HFO-1225zc**

| | **Organics 0,16 F1234yf + 0,14 F245cb + 0,14 F1233xf + 0,14 F1234zeE + 0,14 F1234zeZ + 0,14 F1233zdE + 0,14 F1225zc** | **Organics 0,94 F1234yf + 0,01 F245cb + 0,01 F1233xf + 0,01 F1234zeE + 0,01 F1234zeZ + 0,01 F1233zdE + 0,01 F1225zc** | **Organics 0,01 F1234yf + 0,94 F245cb + 0,01 F1233xf + 0,01 F1234zeE + 0,01 F1234zeZ + 0,01 F1233zdE + 0,01 F1225zc** | **Organics 0,01 F1234yf + 0,01 F245cb + 0,94 F1233xf + 0,01 F1234zeE + 0,01 F1234zeZ + 0,01 F1233zdE + 0,01 F1225zc** | **Organics 0,01 F1234yf + 0,01 F245cb + 0,01 F1233xf + 0,94 F1234zeE + 0,01 F1234zeZ + 0,01 F1233zdE + 0,01 F1225zc** | **Organics 0,01 F1234yf + 0,01 F245cb + 0,01 F1233xf + 0,01 F1234zeE + 0,94 F1234zeZ + 0,01 F1233zdE + 0,01 F1225zc** | **Organics 0,01 F1234yf + 0,01 F245cb + 0,01 F1233xf + 0,01 F1234zeE + 0,01 F1234zeZ + 0,94 F1233zdE + 0,01 F1225zc** | **Organics 0,01 F1234yf + 0,01 F245cb + 0,01 F1233xf + 0,01 F1234zeE + 0,01 F1234zeZ + 0,01 F1233zdE + 0,94 F1225zc** |
|---|---|---|---|---|---|---|---|---|
| MASSFRAC | TOTAL | TOTAL | TOTAL | TOTAL | TOTAL | TOTAL | TOTAL | TOTAL |
| HF | PRESSURE | PRESSURE | PRESSURE | PRESSURE | PRESSURE | PRESSURE | PRESSURE | PRESSURE |
| | bar | bar | bar | bar | bar | bar | bar | bar |
| 0 | 3,9 | 6,6 | 4,6 | 1,7 | 4,8 | 1,9 | 1,5 | 5,2 |
| 0,05 | 4,9 | 7,6 | 5,8 | 2,8 | 5,8 | 3,0 | 2,6 | 6,2 |
| 0,1 | 4,9 | 7,6 | 5,8 | 2,8 | 5,8 | 3,0 | 2,6 | 6,2 |
| 0,15 | 4,9 | 7,6 | 5,8 | 2,8 | 5,8 | 3,0 | 2,6 | 6,2 |
| 0,2 | 4,9 | 7,6 | 5,8 | 2,8 | 5,8 | 3,0 | 2,6 | 6,2 |
| 0,25 | 4,9 | 7,6 | 5,8 | 2,8 | 5,8 | 3,0 | 2,6 | 6,2 |
| 0,3 | 4,9 | 7,6 | 5,8 | 2,8 | 5,8 | 3,0 | 2,6 | 6,2 |
| 0,35 | 4,9 | 7,6 | 5,8 | 2,8 | 5,8 | 3,0 | 2,6 | 6,2 |
| 0,4 | 4,9 | 7,6 | 5,8 | 2,8 | 5,8 | 3,0 | 2,6 | 6,2 |
| 0,45 | 4,9 | 7,6 | 5,8 | 2,8 | 5,8 | 3,0 | 2,6 | 6,2 |
| 0,5 | 4,9 | 7,6 | 5,8 | 2,8 | 5,7 | 3,0 | 2,6 | 6,1 |
| 0,55 | 4,9 | 7,6 | 5,8 | 2,8 | 5,7 | 3,0 | 2,6 | 6,1 |
| 0,6 | 4,9 | 7,6 | 5,8 | 2,8 | 5,6 | 3,0 | 2,6 | 6,0 |
| 0,65 | 4,9 | 7,6 | 5,8 | 2,8 | 5,5 | 3,0 | 2,6 | 5,9 |
| 0,7 | 4,9 | 7,5 | 5,8 | 2,8 | 5,3 | 3,0 | 2,6 | 5,8 |
| 0,75 | 4,8 | 7,4 | 5,8 | 2,8 | 5,1 | 3,0 | 2,6 | 5,5 |
| 0,8 | 4,6 | 7,1 | 5,8 | 2,7 | 4,8 | 2,9 | 2,5 | 5,1 |
| 0,85 | 4,2 | 6,5 | 5,8 | 2,5 | 4,3 | 2,7 | 2,3 | 4,6 |
| 0,9 | 3,5 | 5,5 | 4,9 | 2,2 | 3,6 | 2,4 | 2,1 | 3,8 |
| 0,95 | 2,6 | 3,8 | 3,5 | 1,8 | 2,6 | 1,9 | 1,7 | 2,7 |
| 1 | 1,2 | 1,2 | 1,2 | 1,2 | 1,2 | 1,2 | 1,2 | 1,2 |

### Exemple 17 : Plage de température et de pression de système à 8 composés

| | Enveloppe de points d'ébullition | |
|---|---|---|
| Système à 8 composés | Température °C | Pression bar abs |
| HF-HCFO-1233xf-HFO-1234yf-HFC-245cb-HCFO-1233zdE-HFO-1234zeE-HFO-1234zeZ-HFO-1243zf | 0 à 40 | ∼1.0 ∼11.5 |
| HF-HFO-1234yf-HFC-245cb-HCFO-1233xf-HFO-1234zeE-HFO-1234zeZ-HCFO-1233zdE-HCFC-244bb | 0 à 40 | ∼0.7 à ∼11.5 |
| HF-HFO-1234yf-HFC-245cb-HCFO-1233xf-HFO-1234zeE-HFO-1234zeZ-HCFO-1233zdE-Trifluoropropyne | 0 à 40 | ∼1.0 à ∼17.4 |
| HF-HFO-1234yf-HFC-245cb-HCFO-1233xf-HFO-1234zeE-HFO-1234zeZ-HCFO-1233zdE-HFC-245fa | 0 à 40 | ∼0.9 à ∼11.5 |
| HF-HFO-1234yf-HFC-245cb-HCFO-1233xf-HFO-1234zeE-HFO-1234zeZ-HCFO-1233zdE-HFO-1225yeZ | 0 à 40 | ∼1.0 à ∼11.5 |
| HF-HFO-1234yf-HFC-245cb-HCFO-1233xf-HFO-1234zeE-HFO-1234zeZ-HCFC-1233zdE-HFO-1225zc | 0 à 40 | ∼1.0 à ∼11.5 |

### Exemple 18 : Plages de décantation de système à 8 composés

| | **Plages de décantation Pourcentage massique d'HF** | | |
|---|---|---|---|
| **Système à 8 composés** | **Température** | | |
| | **0 °C** | **25 °C** | **40 °C** |
| **HF-HCFO-1233xf-HFO-1234yf-HFC-245cb-HCFO-1233zdE-HFO-1234zeE-HFO-1234zeZ-HFO-1243zf** | **5-75%** | **5-70%** | **15-50%** |
| **HF-HFO-1234yf-HFC-245cb-HCFO-1233xf-HFO-1234zeE-HFO-1234zeZ-HCFO-1233zdE-HCFC-244bb** | **5-80** | **5-75** | **5-70** |
| **HF-HFO-1234yf-HFC-245cb-HCFO-1233xf-HFO-1234zeE-HFO-1234zeZ-HCFO-1233zdE-Trifluoropropyne** | **5-75** | **10-65** | ***** |
| **HF-HFO-1234yf-HFC-245cb-HCFO-1233xf-HFO-1234zeE-HFO-1234zeZ-HCFO-1233zdE-HFC-245fa** | **5-75** | **5-70** | **10-60** |
| **HF-HFO-1234yf-HFC-245cb-HCFO-1233xf-HFO-1234zeE-HFO-1234zeZ-HCFO-1233zdE-HFO-1225yeZ** | **5-75** | **5-70** | **15-55** |
| **HF-HFO-1234yf-HFC-245cb-HCFO-1233xf-HFO-1234zeE-HFO-1234zeZ-HCFC-1233zdE-HFO-1225zc** | **5-75** | **5-65** | **15-50** |

### Exemple 19 : Systèmes à 13 composés

**HF - HFO-1234yf - HFC-245cb - HCFO-1233xf - HCFO-1233zdE -HFO-1234zeE - HFO-1234zeZ - HFC-1243zf - HCFC-244bb - TFP - HFC-245fa - HFO-1225yeZ - HFO-1225zc**

| | **Organics 0,087 F1234yf +0,083 F245cb + 0,083 F1233xf + 0,083 F1233zdE + 0,083 F1234zeE + 0,083 F1234zeZ + 0,083 F1243zf + 0,083 F244bb + 0,083 F245fa + 0,083 TFP + 0,083 F1225yeZ + 0,83 F1225zc** | **Organics 0,89 F1234yf +0,01 F245cb + 0,01 F1233xf + 0,01 F1233zdE + 0,01 F1234zeE + 0,01 F1234zeZ + 0,01 F1243zf + 0,01 F244bb + 0,01 F245fa + 0,01 TFP + 0,01 F1225yeZ + 0,1 F1225zc** | **Organics 0,01 F1234yf +0,01 F245cb + 0,89 F1233xf + 0,01 F1233zdE + 0,01 F1234zeE + 0,01 F1234zeZ + 0,01 F1243zf + 0,01 F244bb + 0,01 F245fa + 0,01 TFP + 0,01 F1225yeZ + 0,1 F1225zc** | **Organics 0,01 F1234yf +0,01 F245cb + 0,01 F1233xf + 0,01 F1233zdE + 0,89 F1234zeE + 0,01 F1234zeZ + 0,01 F1243zf + 0,01 F244bb + 0,01 F245fa + 0,01 TFP + 0,01 F1225yeZ + 0,1 F1225zc** | **Organics 0,01 F1234yf +0,01 F245cb + 0,01 F1233xf + 0,89 F1233zdE + 0,01 F1234zeE + 0,01 F1234zeZ + 0,01 F1243zf + 0,01 F244bb + 0,01 F245fa + 0,01 TFP + 0,01 F1225yeZ + 0,1 F1225zc** |
|---|---|---|---|---|---|
| MASSFRAC | TOTAL | TOTAL | TOTAL | TOTAL | TOTAL |
| HF | PRESSURE | PRESSURE | PRESSURE | PRESSURE | PRESSURE |
| | bar | bar | bar | bar | bar |
| 0 | 4,5 | 6,5 | 1,9 | 4,8 | 1,7 |
| 0,05 | 5,6 | 7,5 | 3,0 | 5,8 | 2,9 |
| 0,1 | 5,6 | 7,5 | 3,0 | 5,8 | 2,9 |
| 0,15 | 5,6 | 7,5 | 3,0 | 5,8 | 2,8 |
| 0,2 | 5,6 | 7,5 | 3,0 | 5,8 | 2,8 |
| 0,25 | 5,6 | 7,5 | 3,0 | 5,8 | 2,8 |
| 0,3 | 5,6 | 7,5 | 3,0 | 5,8 | 2,8 |
| 0,35 | 5,6 | 7,5 | 3,0 | 5,8 | 2,8 |
| 0,4 | 5,6 | 7,5 | 3,0 | 5,8 | 2,8 |
| 0,45 | 5,5 | 7,5 | 3,0 | 5,8 | 2,8 |
| 0,5 | 5,5 | 7,5 | 3,0 | 5,8 | 2,8 |
| 0,55 | 5,5 | 7,5 | 3,0 | 5,7 | 2,8 |
| 0,6 | 5,5 | 7,5 | 3,0 | 5,6 | 2,8 |
| 0,65 | 5,5 | 7,5 | 3,0 | 5,5 | 2,8 |
| 0,7 | 5,4 | 7,5 | 3,0 | 5,4 | 2,8 |
| 0,75 | 5,3 | 7,3 | 3,0 | 5,2 | 2,8 |
| 0,8 | 5,1 | 7,0 | 2,8 | 4,8 | 2,7 |
| 0,85 | 4,6 | 6,4 | 2,6 | 4,3 | 2,5 |
| 0,9 | 3,9 | 5,4 | 2,3 | 3,6 | 2,2 |
| 0,95 | 2,8 | 3,7 | 1,8 | 2,6 | 1,8 |
| 1 | 1,2 | 1,2 | 1,2 | 1,2 | 1,2 |

### Exemple 20 : Plage de température et de pression de système à 13 composés

| | Enveloppe de points d'ébullition | |
|---|---|---|
| **Système à 13 composés** | Température °C | Pression bar abs |
| HF - HFO-1234yf - HFC-245cb - HCFO-1233xf - HCFO-1233zdE - HFO-1234zeE - HFO-1234zeZ - HFC-1243zf - HCFC-244bb - TFP - HFC-245fa - HFO-1225yeZ - HFO-1225zc | 0 à 40 | ∼0,7 ∼18.0 |

### Exemple 21 : Plages de décantation de système à 13 composés

| | **Plages de décantation Pourcentage massique d'HF** | | |
|---|---|---|---|
| **Système à 13 composés** | **Température** | | |
| | **0°C** | **25 °C** | **40 °C** |
| **HF - HFO-1234yf - HFC-245cb - HCFO-1233xf - HCFO-1233zdE - HFO-1234zeE - HFO-1234zeZ - HFC-1243zf - HCFC-244bb - TFP - HFC-245fa - HFO-1225yeZ - HFO-1225zc** | **5-75%** | **10-70%** | **15-60%** |

## Revendications

1. Composition azéotropique ou quasi-azéotropique comprenant du fluorure d'hydrogène, du Z-1,3,3,3-tetrafluoropropene, et un ou plusieurs composé(s) (hydro)halogénocarboné(s) choisi(s) parmi le 1,1,1,2,2-pentafluoropropane, le 2,3,3,3-tetrafluoropropene, le 3,3,3-trifluoropropene, le 3,3,3-trifluoro-2-chloropropene, le E-3,3,3-trifluoro-1-chloropropene, le trifluoropropyne, le 1,1,1,3,3-pentafluoropropène, le 1,1,1,2,3-pentafluoropropène et le 2-chloro,1,1,1,2-tetrafluoropropane ; **caractérisé en ce que** ladite composition comprend de 1 à 95 % en poids de fluorure d'hydrogène et de 99 à 5 % en poids de la somme des composés organiques, et le point d'ébullition de ladite composition est compris entre -20 °C et 80 °C, à une pression comprise entre 0,1 et 44 bars absolue.

2. Composition selon la revendication 1 comprenant du fluorure d'hydrogène, du Z-1,3,3,3-tetrafluoropropene et au moins un ou plusieurs composé(s) organique(s) choisi parmi le 3,3,3-trifluoro-2-chloropropene, le E-3,3,3-trifluoro-1-chloropropene, le 3,3,3-trifluoropropene, le 1,1,1,2,2-pentafluoropropane et le 2,3,3,3-tetrafluoropropene.

3. Composition selon l'une quelconque des revendications précédentes dans laquelle la composition est hétéro-azéotropique ou quasi-hétéro-azéotropique.

4. Composition selon l'une quelconque des revendications précédentes comprenant du fluorure d'hydrogène, du Z-1,3,3,3-tetrafluoropropene, du 3,3,3-trifluoro-2-chloropropene et éventuellement un ou plusieurs composé(s) choisi(s) parmi le E-3,3,3-trifluoro-1-chloropropene, le 3,3,3-trifluoropropene, le 1,1,1,2,2-pentafluoropropane et le 2,3,3,3-tetrafluoropropene.

5. Composition selon l'une quelconque des revendications précédentes comprenant du fluorure d'hydrogène, du Z-1,3,3,3-tetrafluoropropene, du E-3,3,3-trifluoro-1-chloropropene et éventuellement un ou plusieurs composé(s) choisi(s) parmi le 3,3,3-trifluoropropene, le 1,1,1,2,2-pentafluoropropane et le 2,3,3,3-tetrafluoropropene.

6. Composition selon l'une quelconque des revendications précédentes comprenant du fluorure d'hydrogène, du Z-1,3,3,3-tetrafluoropropene, du 3,3,3-trifluoropropene et éventuellement un ou plusieurs composé(s) choisi(s) parmi le 1,1,1,2,2-pentafluoropropane et le 2,3,3,3-tetrafluoropropene.

7. Composition selon l'une quelconque des revendications précédentes comprenant du fluorure d'hydrogène, du Z-1,3,3,3-tetrafluoropropene, du 1,1,1,2,2-pentafluoropropane et éventuellement le 2,3,3,3-tetrafluoropropene.

8. Composition selon l'une quelconque des revendications précédentes comprenant du fluorure d'hydrogène, du Z-1,3,3,3-tetrafluoropropene, et du 2,3,3,3-tetrafluoropropene.

9. Composition selon l'une quelconque des revendications précédentes comprenant de 5 à 85 % en poids de fluorure d'hydrogène et de 95 à 15 % en poids de la somme des composés organiques.

10. Composition selon l'une quelconque des revendications précédentes dans laquelle le point d'ébullition de la dite composition est compris entre 0 °C et 40 °C et préférentiellement à une pression comprise entre 0,7 et 18 bars absolue, avantageusement entre 0,9 et 12,5 bars absolue.

## Patentansprüche

1. Azeotrope oder quasiazeotrope Zusammensetzung, umfassend Fluorwasserstoff, Z-1,3,3,3-Tetrafluorpropen und eine oder mehrere (Hydro)halogenkohlenstoffverbindungen, die aus 1,1,1,2,2-Pentafluorpropan, 2,3,3,3-Tetrafluorpropen, 3,3,3-Trifluorpropen, 3,3,3-Trifluor-2-chlorpropen, E-3,3,3-Trifluor-1-chlorpropen, Trifluorpropin, 1,1,1,3,3-Pentafluorpropen, 1,1,1,2,3-Pentafluorpropen und 2-Chlor-1,1,1,2-tetrafluorpropan ausgewählt sind, **dadurch gekennzeichnet, dass** die Zusammensetzung 1 bis 95 Gew.-% Fluorwasserstoff und 99 bis 5 Gew.-% der Summe der organischen Verbindungen umfasst und der Siedepunkt der Zusammensetzung bei einem Druck zwischen 0,1 und 44 bar absolut zwischen -20 °C und 80 °C liegt.

2. Zusammensetzung nach Anspruch 1, umfassend Fluorwasserstoff, Z-1,3,3,3-Tetrafluorpropen und mindestens eine oder mehrere organische Verbindungen, die aus 3,3,3-Trifluor-2-chlorpropen, E-3,3,3-Trifluor-1-chlorpropen, 3,3,3-Trifluorpropen, 1,1,1,2,2-Pentafluorpropan und 2,3,3,3-Tetrafluorpropen ausgewählt sind.

3. Zusammensetzung nach einem der vorhergehenden Ansprüche, wobei die Zusammensetzung heteroazeotrop oder quasiheteroazeotrop ist.

4. Zusammensetzung nach einem der vorhergehenden Ansprüche, umfassend Fluorwasserstoff, Z-1,3,3,3-Tetrafluorpropen, 3,3,3-Trifluor-2-chlorpropen und gegebenenfalls eine oder mehrere Verbindungen, die aus E-3,3,3-Trifluor-1-chlorpropen, 3,3,3-Trifluorpropen, 1,1,1,2,2-Pentafluorpropan und 2,3,3,3-Tetrafluorpropen ausgewählt sind.

5. Zusammensetzung nach einem der vorhergehenden Ansprüche, umfassend Fluorwasserstoff, Z-1,3,3,3-Tetrafluorpropen, E-3,3,3-Trifluor-1-chlorpropen und gegebenenfalls eine oder mehrere Verbindungen, die aus 3,3,3-Trifluorpropen, 1,1,1,2,2-Pentafluorpropan und 2,3,3,3-Tetrafluorpropen ausgewählt sind.

6. Zusammensetzung nach einem der vorhergehenden Ansprüche, umfassend Fluorwasserstoff, Z-1,3,3,3-Tetrafluorpropen, 3,3,3-Trifluorpropen und gegebenenfalls eine oder mehrere Verbindungen, die aus 1,1,1,2,2-Pentafluorpropan und 2,3,3,3-Tetrafluorpropen ausgewählt sind.

7. Zusammensetzung nach einem der vorhergehenden Ansprüche, umfassend Fluorwasserstoff, Z-1,3,3,3-Tetrafluorpropen, 1,1,1,2,2-Pentafluorpropan und gegebenenfalls 2,3,3,3-Tetrafluorpropen.

8. Zusammensetzung nach einem der vorhergehenden Ansprüche, umfassend Fluorwasserstoff, Z-1,3,3,3-Tetrafluorpropen und 2,3,3,3-Tetrafluorpropen.

9. Zusammensetzung nach einem der vorhergehenden Ansprüche, umfassend 5 bis 85 Gew.-% Fluorwasserstoff und 95 bis 15 Gew.-% der Summe der organischen Verbindungen.

10. Zusammensetzung nach einem der vorhergehenden Ansprüche, wobei der Siedepunkt der Zusammensetzung zwischen 0 °C und 40 °C und vorzugsweise bei einem Druck zwischen 0,7 und 18 bar absolut, vorteilhafterweise zwischen 0,9 und 12,5 bar absolut, liegt.

## Claims

1. Azeotropic or quasi-azeotropic composition comprising hydrogen fluoride, Z-1,3,3,3-tetrafluoropropene and one or more (hydro)halocarbon compounds chosen from 1,1,1,2,2-pentafluoropropane, 2,3,3,3-tetrafluoropropene, 3,3,3-trifluoropropene, 3,3,3-trifluoro-2-chloropropene, E-3,3,3-trifluoro-1-chloropropene, trifluoropropyne, 1,1,1,3,3-pentafluoropropene, 1,1,1,2,3-pentafluoropropene and 2-chloro-1,1,1,2-tetrafluoropropane; **characterized in that** said composition comprises from 1% to 95% by weight of hydrogen fluoride and from 99% to 5% by weight of the sum of the organic compounds, and the boiling point of said composition is between -20°C and 80°C, at a pressure of between 0.1 and 44 bar absolute.

2. Composition according to Claim 1, comprising hydrogen fluoride, Z-1,3,3,3-tetrafluoropropene and at least one or more organic compounds chosen from 3,3,3-trifluoro-2-chloropropene, E-3,3,3-trifluoro-1-chloropropene, 3,3,3-trifluoropropene, 1,1,1,2,2-pentafluoropropane and 2,3,3,3-tetrafluoropropene.

3. Composition according to either one of the preceding claims, wherein the composition is heteroazeotropic or quasi-heteroazeotropic.

4. Composition according to any one of the preceding claims, comprising hydrogen fluoride, Z-1,3,3,3-tetrafluoropropene, 3,3,3-trifluoro-2-chloropropene and optionally one or more compounds chosen from E-3,3,3-trifluoro-1-chloropropene, 3,3,3-trifluoropropene, 1,1,1,2,2-pentafluoropropane and 2,3,3,3-tetrafluoropropene.

5. Composition according to any one of the preceding claims, comprising hydrogen fluoride, Z-1,3,3,3-tetrafluoropropene, E-3,3,3-trifluoro-1-chloropropene and optionally one or more compounds chosen from 3,3,3-trifluoropropene, 1,1,1,2,2-pentafluoropropane and 2,3,3,3-tetrafluoropropene.

6. Composition according to any one of the preceding claims, comprising hydrogen fluoride, Z-1,3,3,3-tetrafluoropropene, 3,3,3-trifluoropropene and optionally one or more compounds chosen from 1,1,1,2,2-pentafluoropropane and 2,3,3,3-tetrafluoropropene.

7. Composition according to any one of the preceding claims, comprising hydrogen fluoride, Z-1,3,3,3-tetrafluoropropene, 1,1,1,2,2-pentafluoropropane and optionally 2,3,3,3-tetrafluoropropene.

8. Composition according to any one of the preceding claims, comprising hydrogen fluoride, Z-1,3,3,3-tetrafluoropropene and 2,3,3,3-tetrafluoropropene.

9. Composition according to any one of the preceding claims, comprising from 5% to 85% by weight of hydrogen fluoride and from 95% to 15% by weight of the sum of the organic compounds.

10. Composition according to any one of the preceding claims, in which the boiling point of said composition is between 0°C and 40°C and preferably at a pressure of between 0.7 and 18 bar absolute, advantageously between 0.9 and 12.5 bar absolute.
